# EUROPEAN PATENT APPLICATION

(11) **EP 2 548 947 A1**
(43) Date of publication of application: **23.01.2013**
(21) Application number: 11756255.3
(22) Date of filing: 14.03.2011
(51) Int. Cl.: C12N 1/19, C07K 14/78, C12N 15/09, C12R 1/645

(54) **TRANSFORMANT WHICH PRODUCES GLYCINE REPEAT PROTEIN**

(30) Priority: 15.03.2010 JP 2010057484
(71) Applicant: Sumitomo Chemical Company, Limited, Tokyo 104-8260 (JP); Dainippon Sumitomo Pharma Co., Ltd., Osaka 541-8524 (JP); Koken Co., Ltd., Tokyo 112-0004 (JP)
(72) Inventor: NISHIO, Shoichi, Ikeda-shi Osaka 563-0029 (JP)
(74) Representative: Vossius & Partner
(86) International application number: PCT/JP2011/055960
(87) International publication number: WO 2011/115070

(57) **Abstract**

Disclosed is a transformant in which polynucleotides comprising a nucleotide sequence encoding the amino acid sequence of all of the following proteins (1) to (3) are transfected into a microbial cell:
(1) a protein that is capable of binding to FK506 and has a molecular weight of 15,000 or more and 60,000 or less;
(2) a prolyl hydroxylase; and
(3) a glycine repeat sequence protein having the following characteristics (A) and (B):
<characteristic (A)> the polypeptide chain of the glycine repeat sequence protein having a continuous, Gly-Xaa-Yaa repeating sequence, and
<characteristic (B)> an imino acid being contained in the continuous, Gly-Xaa-Yaa repeating sequence of the polypeptide chain of the glycine repeat sequence protein.

## Description

### Technical Field

The present invention relates to a transformant that produces a glycine repeat sequence protein and to a process for obtaining a glycine repeat sequence protein.

### Background Art

A group of proteins having a gly12cine repeating sequence are present within a living body and are known to play important roles in maintaining the structures of tissues, intercellular adhesion, wound healing etc., in a living organism. The group of these proteins is characterized in that each protein has a high content of imino acid (e.g. proline or hydroxyproline) and possesses a unique triple-helical structure, and is collectively referred to as collagen.
Collagens are contained in various types of body tissues, typically including skin, tendon, cartilage, ligament, blood vessel and bone. Due to high tensile strength of collagens, body tissues containing collagens have a high tolerance for mechanical stress.
Natural or artificial glycine repeat sequence proteins as represented by collagens, which have excellent physical properties as well as a variety of bioactivities, are available as pharmaceuticals, industrial products, cosmetics, or foods, and so, it can be regarded as a commercially valuable versatile material.
Glycine repeat sequence proteins which are commercially produced are mainly natural form collagen, which are produced by purification from animal skins, tendons, bones and others.

Collagens are found in substantially all of the multicellular animals and occupy an approximately 30% of the protein mass present in the body. It is known that higher animals may have 20 or more different types of collagen. Collagens which are present in high amounts in a living organism include Type I collagen, Type II collagen and Type III collagen. Type I collagen is a major fibrillar collagen present in bone, tendon and skin, which is a heterotrimeric molecule comprising two α1(I) chains and one α2(I) chain. Type II collagen is a collagen present in cartilage and vitreous body, which is a homotrimeric molecule comprising three identical α1(II) chains. Type III collagen is a collagen present in skin and blood vessel, which is a homotrimeric molecule comprising three identical α1(III) chains. Type I, Type II and Type III collagens can be purified from body tissues, for example, by the procedures described in Non-Patent Documents 1, 4 and 5.

As techniques for producing glycine repeat sequence proteins with improved physical properties and bioactivities, there have been developed techniques for producing proteins which have a non-naturally occurring, artificial glycine-repeating sequence, as described below. Non-Patent Document 1 discloses a process for producing a glycine repeat sequence protein, in which chemically synthesized peptides are subjected to formation of a triple-helical structure, followed by chemical polymerization. Patent Document 2 discloses a technique for producing a polymer which forms a triple helix by employing self-assembling of chemically synthesized peptides. However, these methods in which chemical synthesis is employed use expensive raw materials and require multistep procedures, and thus there is a need for a simple production process using inexpensive raw materials.
As techniques for producing glycine repeat sequence proteins using inexpensive raw materials, there have been developed production processes employing recombinant cells. Non-Patent Document 6 discloses Type I collagen with hydroxylated proline residues, produced by coexpressing a precursor of Type I collagen and a prolyl hydroxylase in a yeast cell. On the other hand, Non-Patent Document 1 discloses Type I or Type III collagen with hydroxylated proline residues is prepared by coexpressing a precursor of a Type I or Type III collagen and a prolyl hydroxylase in a yeast cell. Patent Document 3 discloses a process for producing a non-naturally occurring, artificial glycine repeat sequence protein using a gene recombinant cell. However, there is a need for a more efficient process for producing a highly functional, glycine repeat sequence protein.

### Prior Art Documents

### Patent Documents

Patent Document 1: WO 97/38710 A
Patent Document 2: JP 2005-263784 A
Patent Document 3: USP 5710252

### Non-Patent Documents

Non-Patent Document 1: Masao Tanihara supervised, "Production and Development of Applications of Collagen," ISBN: 978-4-7813-0071-3, CMC Publishing Co., Ltd.;
Non-Patent Document 2: Volume Editors: Brinckmann, J., Notbohm, H., Muller, P.K., Collagen primer in Structure, Processing and Assembly, Topics in Current Chemistry Vol. 247, 2005;
Non-Patent Document 3: Gelse, K., Poschl, E., Aigner, T., Collagens - structure, function and biosynthesis, Advanced Drug Delivery Reviews, 55:1531-1546 (2003);
Non-Patent Document 4: Miller et al., Methods In Enzymology, 82: 33-64 (1982), Academic Press;
Non-Patent Document 5: Byers et al., Biochemistry, 13:5243-5248 (1974);
Non-Patent Document 6: P. David Toman et al., J. Biol. Chem., Vol. 275, No. 30, July 28, p23303-23309 (2000).

### Disclosure of the Invention

### Problems to be Solved by the Invention

There has been a need to find a transformant producing a glycine repeat sequence protein which is usable as a high performance versatile material that is more commercially valuable for pharmaceuticals, industrial products, cosmetics, foods etc., in a state where its inherent physical properties are not impaired (e.g. in a state where changes in the physical properties related to the hydrophilicity of glycine repeat sequence proteins are reduced so as to exhibit more lipophilic properties that are similar to those inherently possessed by glycine repeat sequence proteins), and there has been a need to develop a process for producing a glycine repeat sequence protein using the obtained new transformant.

### Means for Solving the Problems

The present invention provides:
1. A transformant in which all of the following polynucleotides (1), (2) and (3) are transfected into a microbial cell:
   (1) a polynucleotide comprising a nucleotide sequence encoding the amino acid sequence of an FK506 binding protein that is capable of binding to FK506 and has a molecular weight of 15,000 or more and 60,000 or less;
   (2) a polynucleotide comprising a nucleotide sequence encoding the amino acid sequence of a prolyl hydroxylase; and
   (3) a polynucleotide comprising a nucleotide sequence encoding the amino acid sequence of a glycine repeat sequence protein having the following characteristics (A) and (B):
      <characteristic (A)> the polypeptide chain of the glycine repeat sequence protein having a continuous, Gly-Xaa-Yaa repeating sequence, and
      <characteristic (B)> an imino acid (e.g. proline or hydroxyproline) being contained in the continuous, Gly-Xaa-Yaa repeating sequence of the polypeptide chain of the glycine repeat sequence protein,
      wherein Xaa and Yaa each represent any amino acid;
2. The transformant according to the above 1, which produces all of the proteins encoded by the polynucleotides (1), (2) and (3) within the cell;
3. The transformant according to the above 1 or 2, wherein the FK506 binding protein is FKBP23 or FKBP19;
4. The transformant according to the above 1 or 2, wherein the FK506 binding protein is FKBP23;
5. The transformant according to any one of the above 1 to 4, wherein the polynucleotide comprising a nucleotide sequence encoding the amino acid sequence of the FK506 binding protein is linked to downstream of a yeast derived promoter;
6. The transformant according to any one of the above 1 to 5, which further produces a lysyl hydroxylase within the cell;
7. The transformant according to the above 6, wherein the lysyl hydroxylase is at least one lysyl hydroxylase selected from lysyl hydroxylase 1 and lysyl hydroxylase 3;
8. The transformant according to any one of the above 1 to 5, which further produces Hsp47 within the cell;
9. The transformant according to any one of the above 1 to 8, wherein the prolyl hydroxylase is prolyl 4-hydroxylase;
10. The transformant according to any one of the above 1 to 8, wherein the prolyl hydroxylase is an enzyme comprising a prolyl 4-hydroxylase α subunit and a prolyl 4-hydroxylase β subunit;
11. The transformant according to the above 10, wherein a yeast α-factor prepro sequence is fused at the amino terminus of the prolyl 4-hydroxylase β subunit;
12. The transformant according to the above 10 or 11, wherein the prolyl 4-hydroxylase α subunit is an α1 subunit, an α2 subunit, or an α3 subunit;
13. The transformant according to any one of the above 1 to 12, wherein at least one polynucleotide comprising a nucleotide sequence encoding the amino acid sequence of the prolyl hydroxylase protein is linked to downstream of a yeast derived promoter;
14. The transformant according to any one of the above 1 to 13, wherein the glycine repeat sequence protein having the characteristics (A) and (B) is at least one collagen selected from among collagens of Type I to Type XXIX;
15. The transformant according to any one of the above 1 to 13, wherein the glycine repeat sequence protein having the characteristics (A) and (B) is at least one collagen selected from among collagen Type I, collagen Type II and collagen Type III;
16. The transformant according to any one of the above 1 to 15, wherein the microbial cell is an eukaryote cell;
17. The transformant according to the above 16, wherein the eukaryote cell is a yeast cell;
18. The transformant according to the above 17, wherein the yeast cell is a methanol-utilizing yeast cell;
19. The transformant according to the above 18, wherein the methanol-utilizing yeast cell is Komagataella pastoris;
20. A glycine repeat sequence protein having the characteristics (A) and (B) which is obtainable by being produced by the transformant according to any one of the above 1 to 19;
21. A process for producing a glycine repeat sequence protein, comprising:
   a first step of transfecting all of the following polynucleotides (1), (2) and (3) into a microbial cell:
      (1) a polynucleotide comprising a nucleotide sequence encoding the amino acid sequence of an FK506 binding protein that is capable of binding to FK506 and has a molecular weight of 15,000 or more and 60,000 or less,
      (2) a polynucleotide comprising a nucleotide sequence encoding the amino acid sequence of a prolyl hydroxylase, and
      (3) a polynucleotide comprising a nucleotide sequence encoding the amino acid sequence of a glycine repeat sequence protein having the following characteristics (A) and (B):
         <characteristic (A)> the polypeptide chain of the glycine repeat sequence protein having a continuous, Gly-Xaa-Yaa repeating sequence, and
         <characteristic (B)> an imino acid (e.g. proline or hydroxyproline) being contained in the continuous, Gly-Xaa-Yaa repeating sequence of the polypeptide chain of the glycine repeat sequence protein,
      wherein Xaa and Yaa each represent any amino acid;
   a second step of culturing the transformant resulting from the first step, thereby producing the glycine repeat sequence protein; and
   a third step of collecting the glycine repeat sequence protein produced in the second step; and 22. A glycine repeat sequence protein produced by the process according to the above 21.

### Effects of the Invention

According to the present invention, there can be provided a transformant producing a glycine repeat sequence protein which is usable as a high performance versatile material that is more commercially valuable for pharmaceuticals, industrial products, cosmetics, foods etc., in a state where its inherent physical properties are not impaired (e.g. in a state where changes in the physical properties related to the hydrophilicity of glycine repeat sequence proteins are reduced so as to exhibit more lipophilic properties that are similar to those inherently possessed by glycine repeat sequence proteins), and a process for producing a glycine repeat sequence protein using the transformant.

### Brief Description of the Drawings

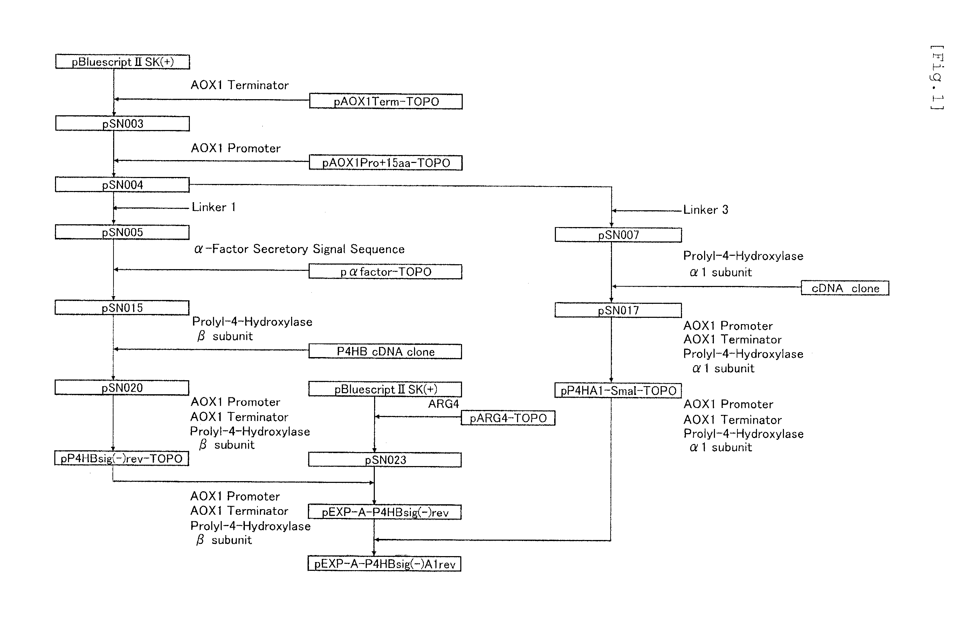

Fig. 1 is a flow chart illustrating a process of constructing a plasmid for transfection of an expression cassette for a prolyl 4-hydroxylase α1 subunit and an expression cassette for a prolyl 4-hydroxylase β subunit.

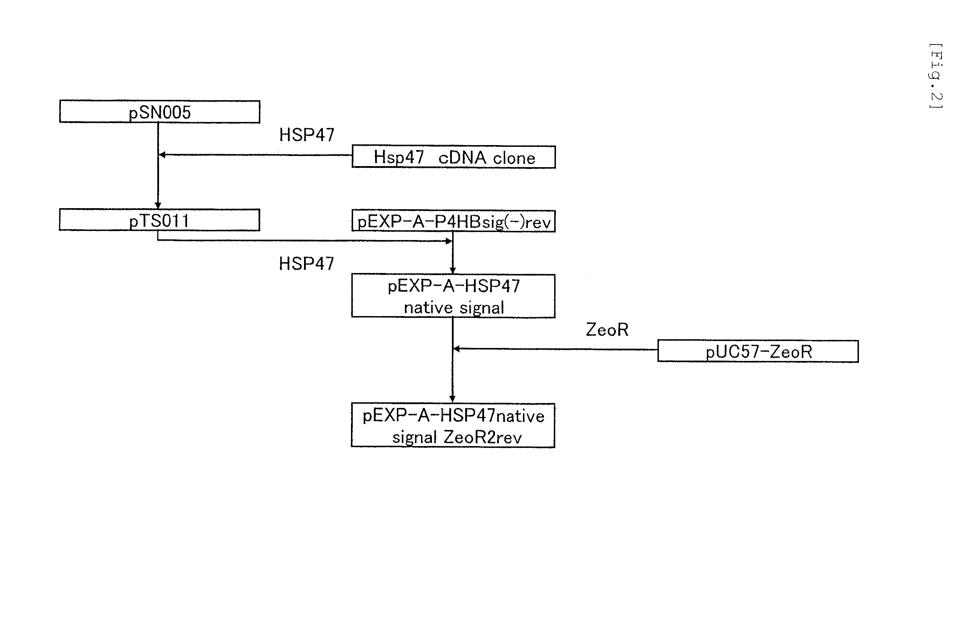

Fig. 2 is a flow chart illustrating a process of constructing a plasmid for transfection of an expression cassette for an FK506 binding protein (FKBP).

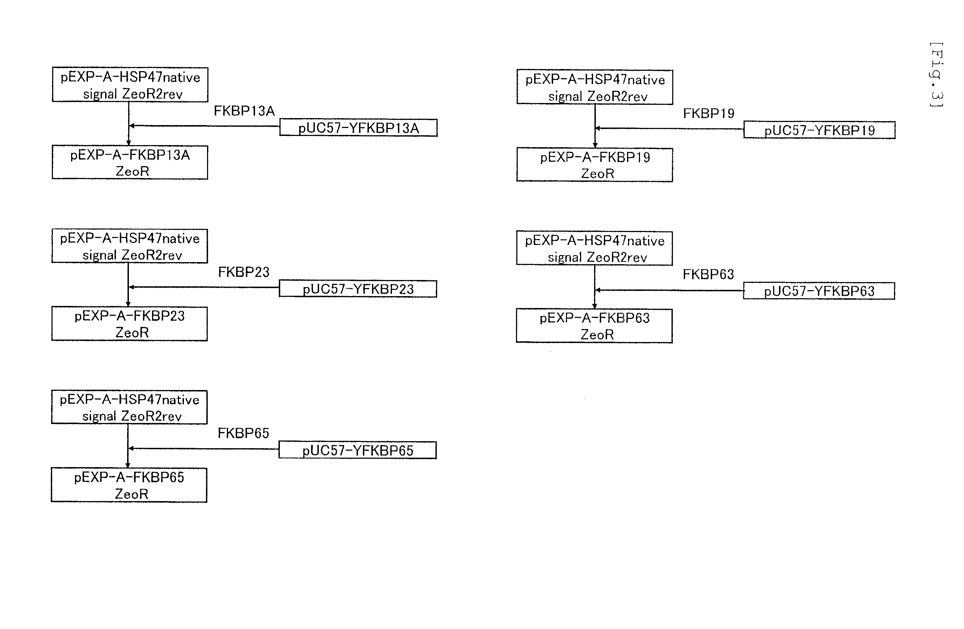

Fig. 3 is a flow chart illustrating a process of constructing a plasmid for transfection of an expression cassette for an FK506 binding protein (FKBP).

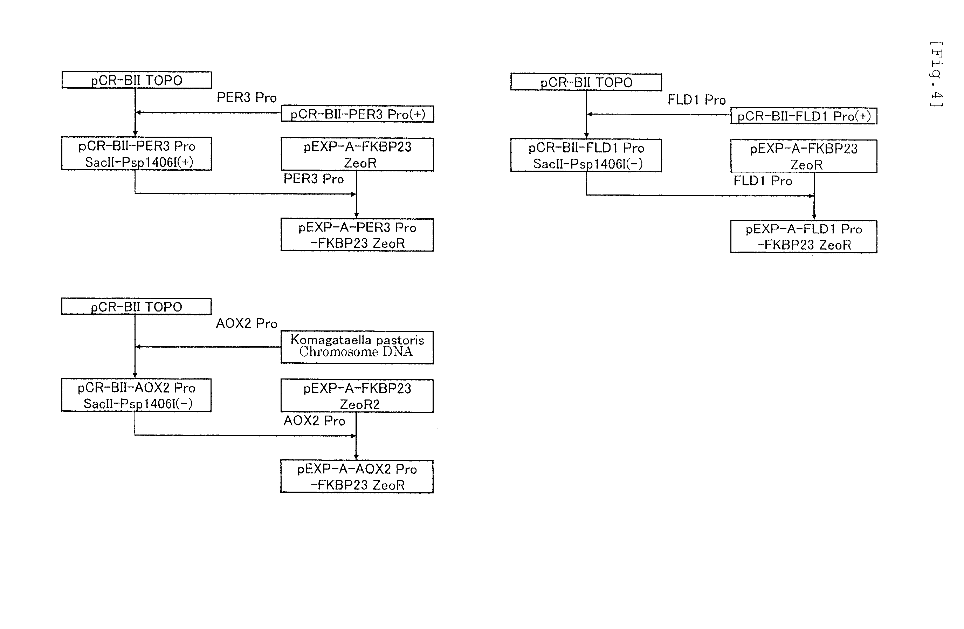

Fig. 4 is a flow chart illustrating a process of constructing a plasmid for transfection of an expression cassette for an FK506 binding protein (FKBP).

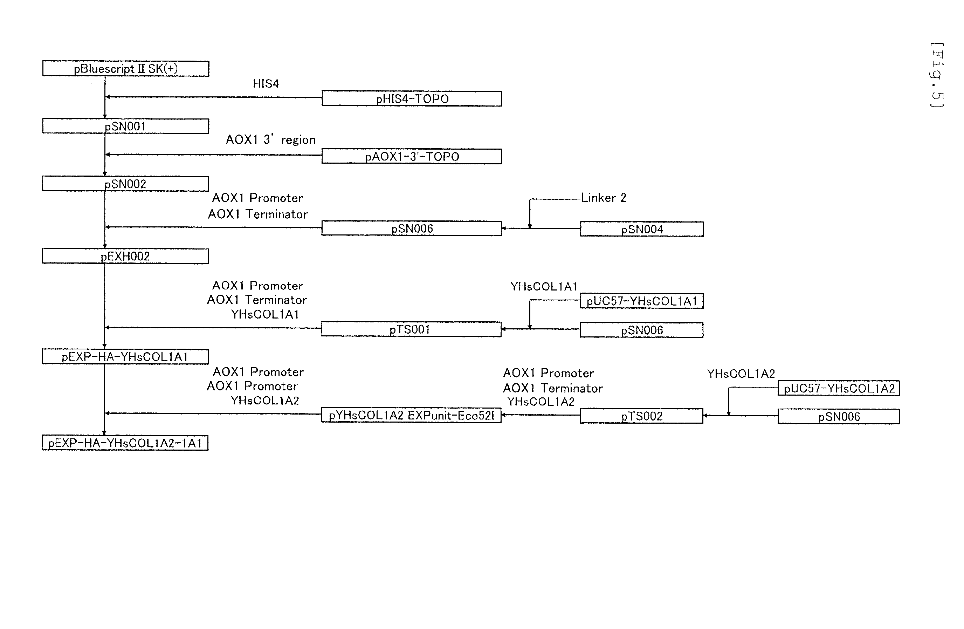

Fig. 5 is a flow chart illustrating a process of constructing a plasmid for transfection of an expression cassette for human collagen Type I α1 and an expression cassette for human collagen Type I α2.

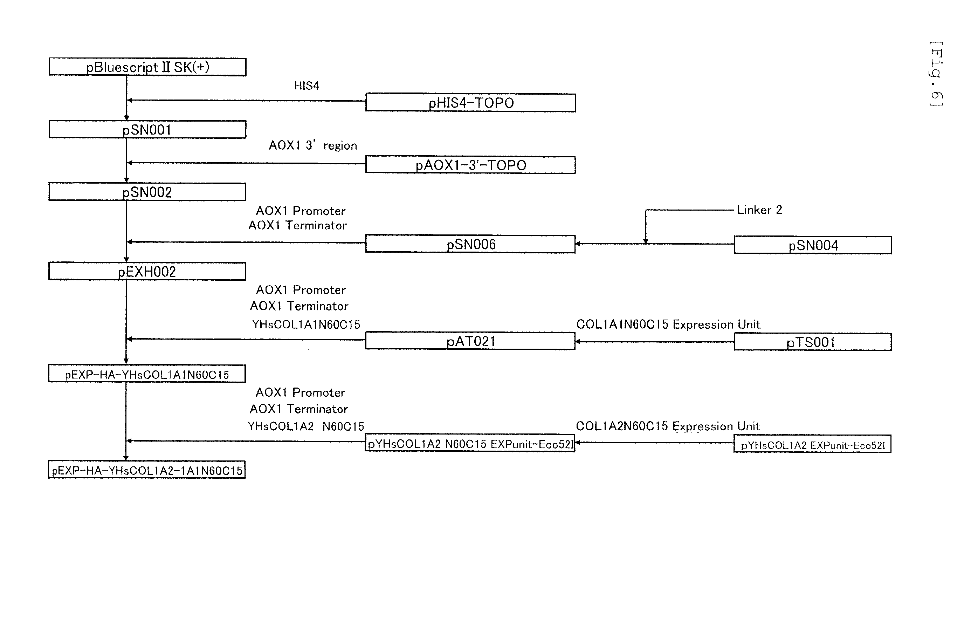

Fig. 6 is a flow chart illustrating a process of constructing a plasmid for transfection of an expression cassette for a fusion polypeptide consisting of the N-terminal non-helix region, 60 N-terminal residues of the helix region, 15 C-terminal residues of the helix region and the C-terminal non-helix region of human collagen Type I α1 and an expression cassette for a fusion polypeptide consisting of the N-terminal non-helix region, 60 N-terminal residues of the helix region, 15 C-terminal residues of the helix region and the C-terminal non-helix region of human collagen Type I α2.

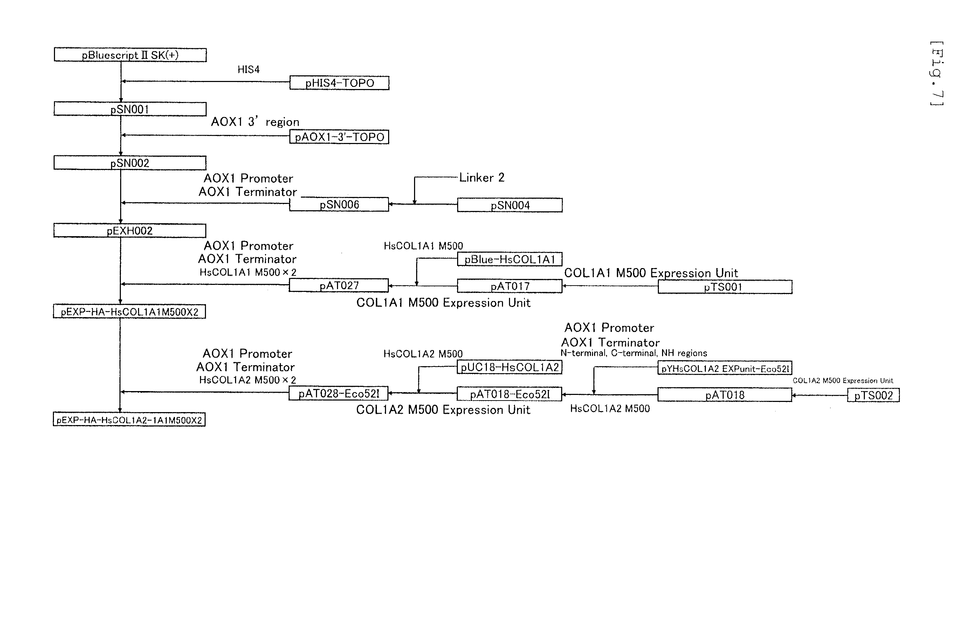

Fig. 7 is a flow chart illustrating a process of constructing a plasmid for transfection of an expression cassette for a fusion polypeptide consisting of the N-terminal non-helix region, 27 N-terminal residues of the helix region, a dimer made of 522 central residues of the helix region, 15 C-terminal residues of the helix region and the C-terminal non-helix region of human collagen Type I α1 and an expression cassette for a fusion polypeptide consisting of the N-terminal non-helix region, 27 N-terminal residues of the helix region, a dimer made of 522 central residues of the helix region, 15 C-terminal residues of the helix region and the C-terminal non-helix region of human collagen Type I α2.

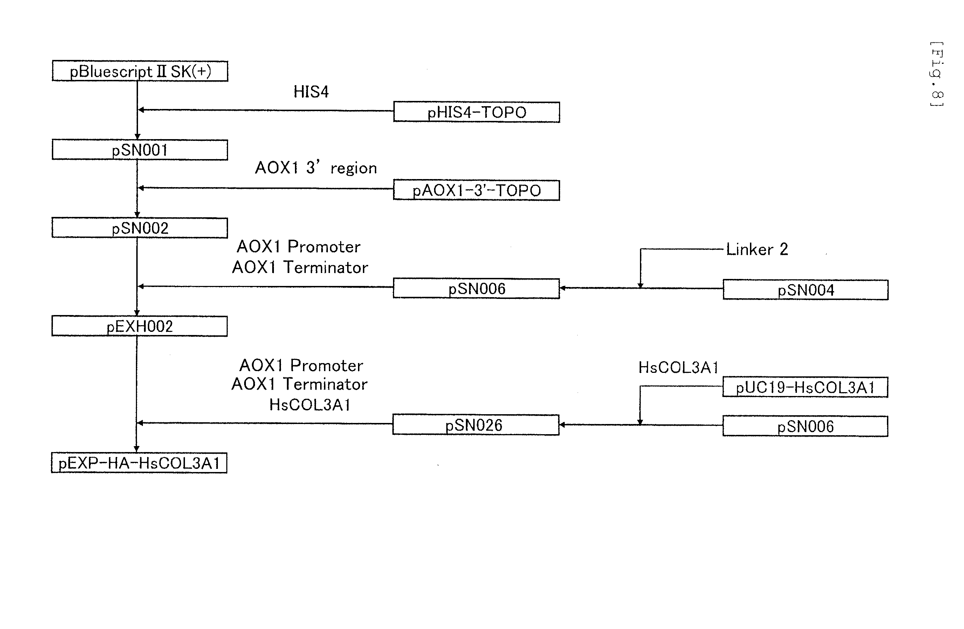

Fig. 8 is a flow chart illustrating a process of constructing a plasmid for introducing an expression cassette for human collagen Type III.

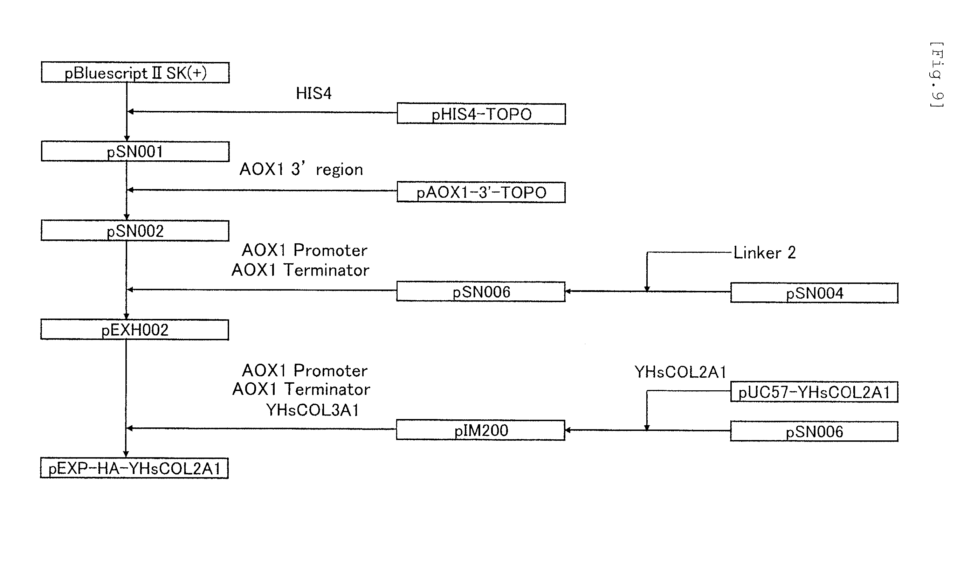

Fig. 9 is a flow chart illustrating a process of constructing a plasmid for transfection of an expression cassette for human collagen Type II.

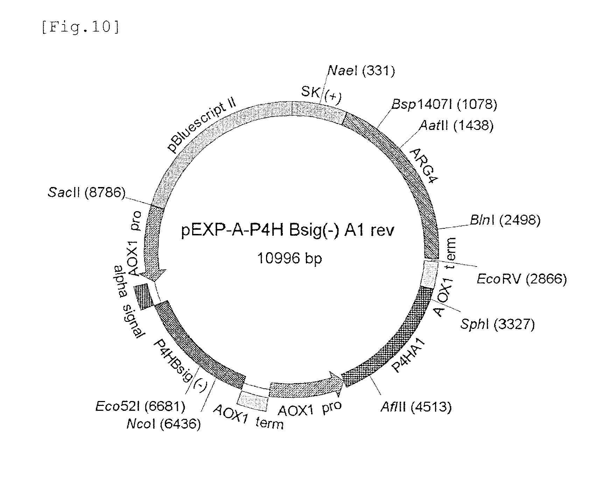

Fig. 10 is a schematic diagram showing a plasmid for transfection of an expression cassette for a prolyl 4-hydroxylase α1 subunit and an expression cassette for a prolyl 4-hydroxylase β subunit.

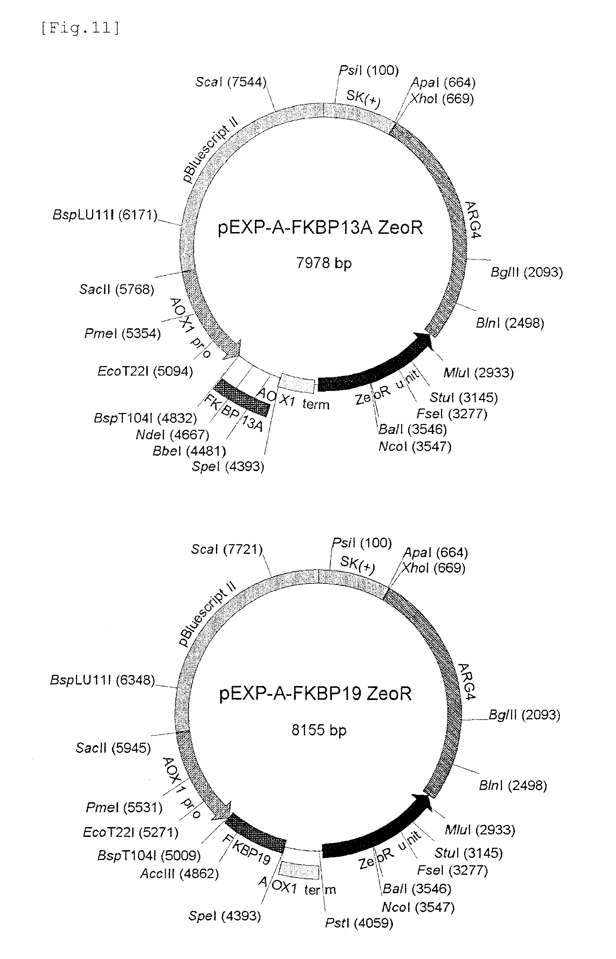

Fig. 11 is a schematic diagram showing a plasmid for transfection of an expression cassette for an FKBP.

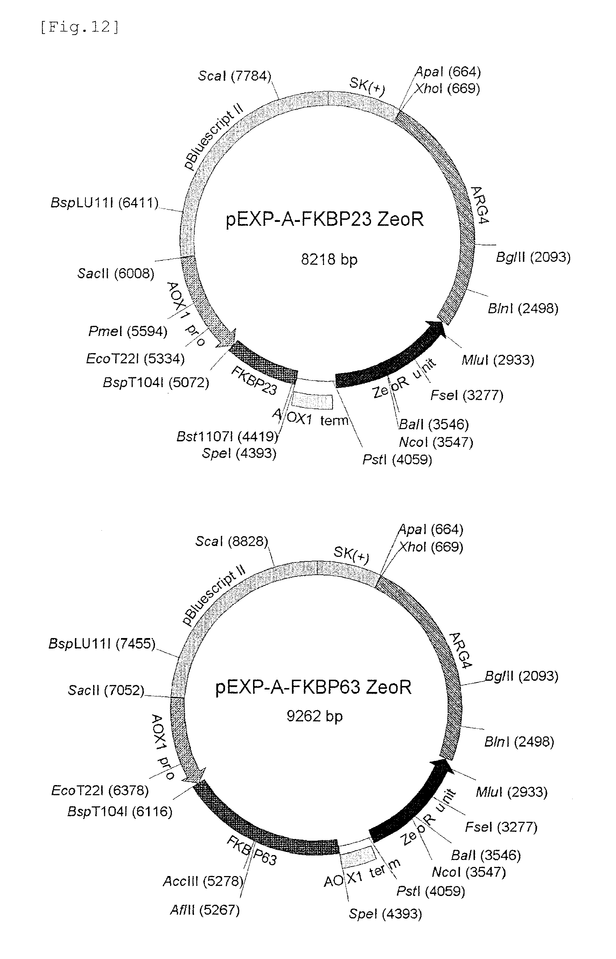

Fig. 12 is a schematic diagram showing a plasmid for transfection of an expression cassette for an FKBP.

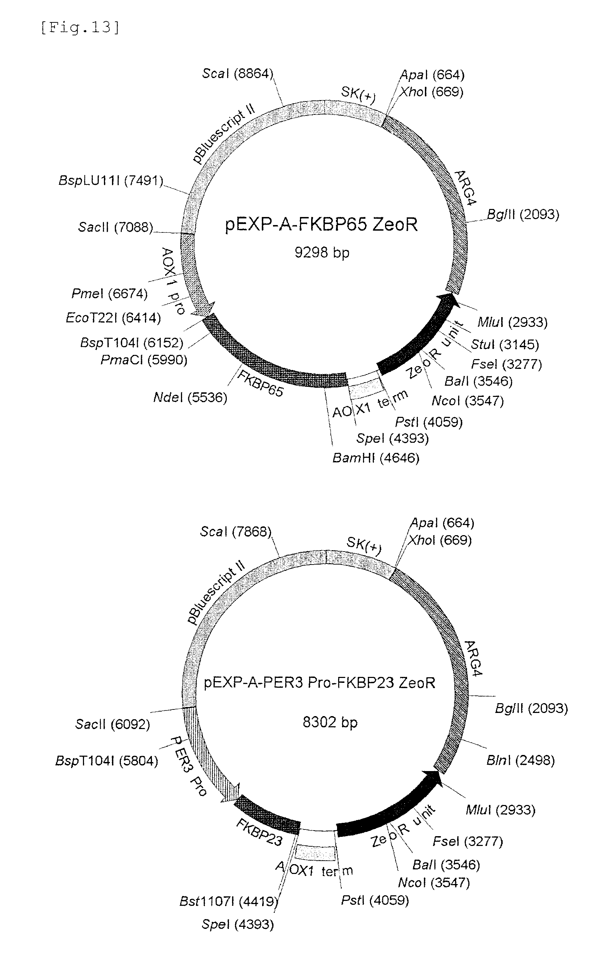

Fig. 13 is a schematic diagram showing a plasmid for transfection of an expression cassette for an FKBP.

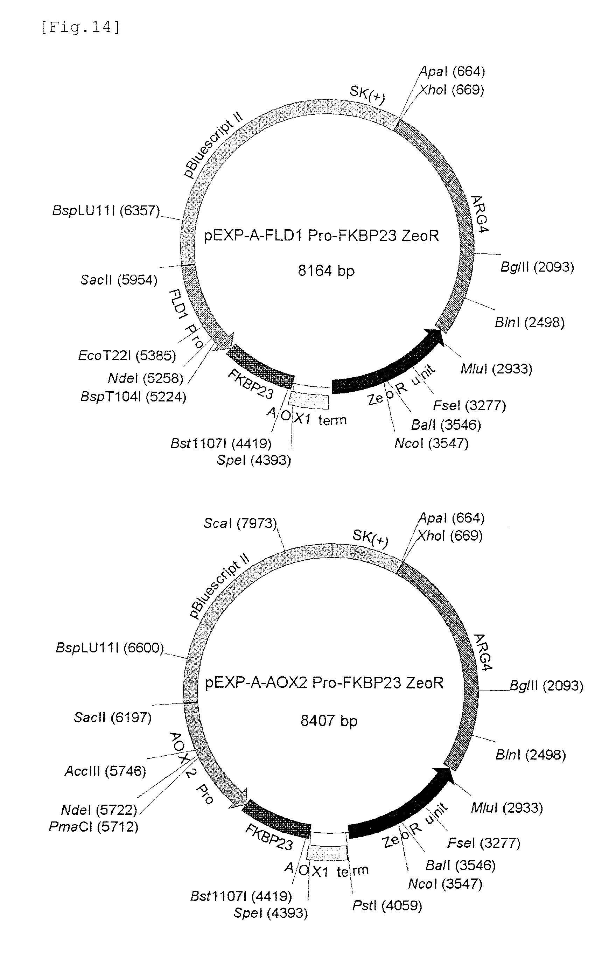

Fig. 14 is a schematic diagram showing a plasmid for transfection of an expression cassette for an FKBP.

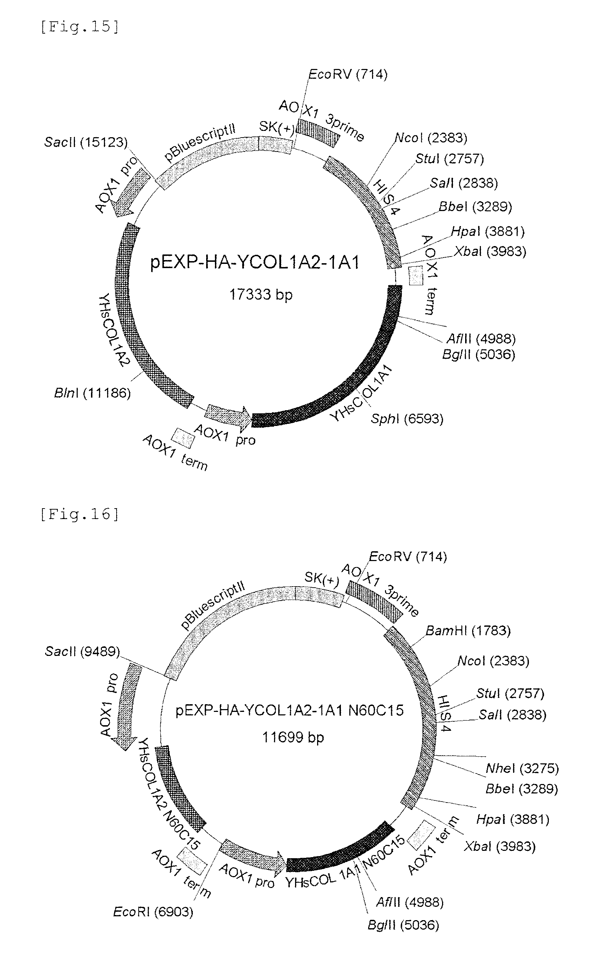

Fig. 15 is a schematic diagram showing a plasmid for transfection of an expression cassette for human collagen Type I.

Fig. 16 is a schematic diagram showing a plasmid for transfection of an expression cassette for a fusion polypeptide consisting of the N-terminal non-helix region, 60 N-terminal residues of the helix region, 15 C-terminal residues of the helix region and the C-terminal non-helix region of human collagen Type I α1 and an expression cassette for a fusion polypeptide consisting of the N-terminal non-helix region, 60 N-terminal residues of the helix region, 15 C-terminal residues of the helix region and the C-terminal non-helix region of human collagen Type I α2.

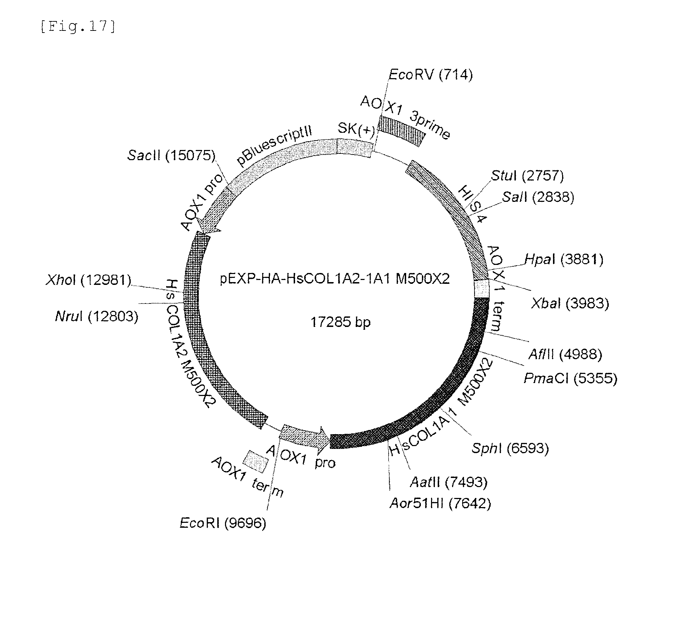

Fig. 17 is a schematic diagram showing a plasmid for transfection of an expression cassette for a fusion polypeptide consisting of the N-terminal non-helix region, 27 N-terminal residues of the helix region, a dimer made of 522 central residues of the helix region, 15 C-terminal residues of the helix region and the C-terminal non-helix region of human collagen Type I α1 and an expression cassette for a fusion polypeptide consisting of the N-terminal non-helix region, 27 N-terminal residues of the helix region, a dimer made of 522 central residues of the helix region, 15 C-terminal residues of the helix region and the C-terminal non-helix region of human collagen Type I α2.

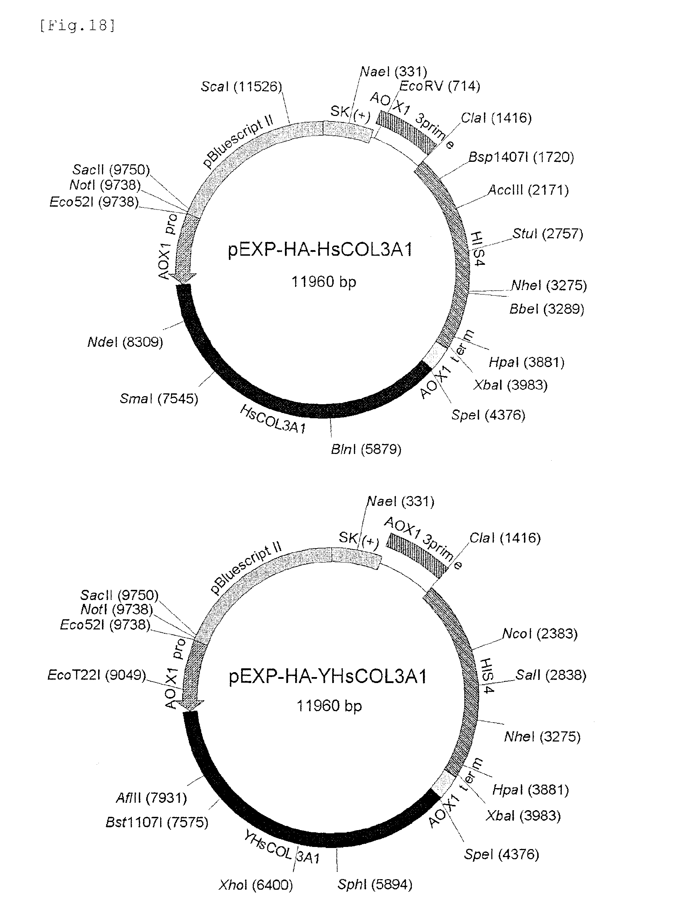

Fig. 18 is a schematic diagram showing a plasmid for transfection of an expression cassette for human collagen Type III.

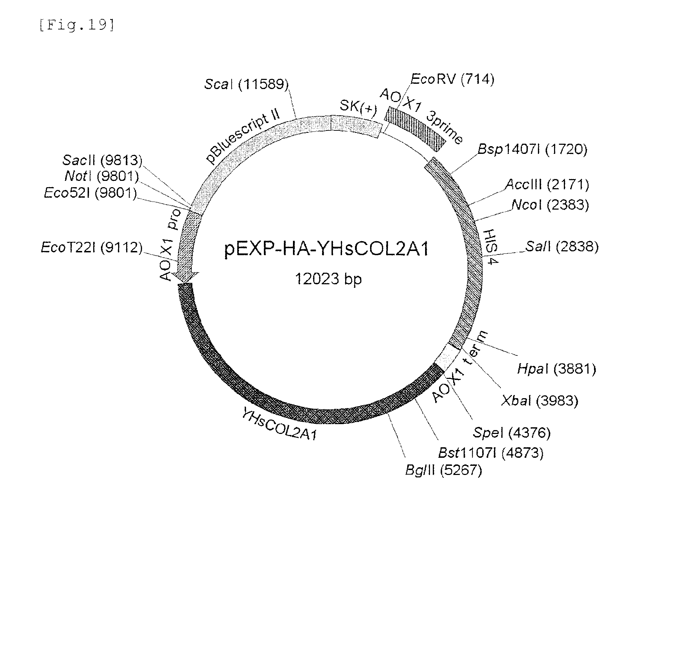

Fig. 19 is a schematic diagram showing a plasmid for transfection of an expression cassette for human collagen Type II.

### Mode for Carrying Out the Invention

It is considered that the invention described herein is not limited to specific methodologies, protocols, and reagents described, but may be modified. It is also considered that the terms used herein are used merely for description of specific embodiments, without intention to limit the scope of the present invention.
Unless otherwise specified, all technical and chemical terms used herein have the same meanings as commonly understood by those with an ordinary skill in the art to which the present invention belongs. Although any methods and materials similar or equivalent to those described herein may be used in the exploitation or verification of the invention, preferred methods, apparatuses and materials are described below.

A transformant of the present invention is characterized in that the transformant obtained by transfecting all of the following polynucleotides (1), (2) and (3) into a microbial cell:
(1) a polynucleotide comprising a nucleotide sequence encoding the amino acid sequence of an FK506 binding protein that is capable of binding to FK506 and has a molecular weight of 15,000 or more and 60,000 or less;
(2) a polynucleotide comprising a nucleotide sequence encoding the amino acid sequence of a prolyl hydroxylase; and
(3) a polynucleotide comprising a nucleotide sequence encoding the amino acid sequence of a glycine repeat sequence protein having the following characteristics (A) and (B):
   <characteristic (A)> the polypeptide chain of the glycine repeat sequence protein having a continuous, Gly-Xaa-Yaa repeating sequence, and
   <characteristic (B)> an imino acid being contained in the continuous, Gly-Xaa-Yaa repeating sequence of the polypeptide chain of the glycine repeat sequence protein,
      wherein Xaa and Yaa each represent any amino acid.
   Accordingly, the transformant of the present invention is a microbial cell which has been transformed with the above-mentioned polynucleotides (1), (2) and (3).

An FK506 binding protein (FKBP) refers collectively to proteins comprising the amino acid sequence of an FKBP domain which is found in proteins forming a complex with an immunosuppressant FK506. A polynucleotide comprising a nucleotide sequence encoding the amino acid sequence of an FK506 binding protein which is used for the production of the transformant of the present invention can be a polynucleotide comprising a nucleotide sequence encoding the amino acid sequence of an FK506 binding protein which has a molecular weight of 15,000 or more and 60,000 or less. Further, the FK506 binding protein can be preferably FKBP23 or FKBP19, and more preferably FKBP23. Species from which the FK506 binding protein is derived are not limited in particular, and the FK506 binding protein can be an FK506 binding protein derived from an animal, preferably a homeotherm, more preferably a mammal, further preferably a human.

Examples of the FK506 binding protein can include human FKBP23 comprising the amino acid sequence indicated by SEQ ID NO:74, human FKBP19 comprising the amino acid sequence indicated by SEQ ID NO:75, and an FK506 binding protein having an amino acid deletion, substitution or addition in these amino acid sequences. In addition, the FK506 binding protein may be an FK506 binding protein having an amino acid sequence which has homology with these amino acid sequences, for example, a protein comprising an amino acid sequence derived from an orthologue gene from a different species. Examples of an amino acid sequence derived from an orthologue gene of human FKBP23 can include the amino acid sequence of dog FKBP23 (NCBI accession: XP_545546), chimpanzee FKBP23 (NCBI accession: XP_515942), bovine FKBP23 (NCBI accession: XP_871858), rat FKBP23 (NCBI accession: XP_215758), mouse FKBP23 (NCBI accession: NP_034352), chicken FKBP23 (NCBI accession: XP_421981) and the like. Examples of an amino acid sequence derived from an orthologue gene of human FKBP19 can include the amino acid sequence of dog FKBP19 (NCBI accession: XP_851205), chimpanzee FKBP19 (NCBI accession: XP_001159891), bovine FKBP19 (NCBI accession: NP_001039397), rat FKBP19 (NCBI accession: NP_001013123), mouse FKBP19 (NCBI accession: NP_077131) and the like. Further, the FK506 binding protein may be an FK506 binding protein which has an amino acid deletion, substitution or addition in an amino acid sequence derived from these orthologue genes.

In addition, examples of a polynucleotide comprising a nucleotide sequence encoding the amino acid sequence of the FK506 binding protein can include a polynucleotide comprising the nucleotide sequence indicated by SEQ ID NO:76, a polynucleotide comprising the nucleotide sequence indicated by SEQ ID NO:77, a polynucleotide comprising the nucleotide sequence indicated by SEQ ID NO:78 and a polynucleotide comprising the nucleotide sequence indicated by SEQ ID NO:79, and more preferably, a polynucleotide comprising a nucleotide sequence encoding the amino acid sequence of the FK506 binding protein can be a polynucleotide comprising the nucleotide sequence indicated by SEQ ID NO:76 or a polynucleotide comprising the nucleotide sequence indicated by SEQ ID NO:78. In addition, included can be a polynucleotide having a deletion, substitution, or addition of one or more bases in the nucleotide sequences of these polynucleotides; and a polynucleotide comprising a partial nucleotide sequence of these polynucleotides. Further, included may be a polynucleotide comprising a nucleotide sequence having homology with the nucleotide sequences of these polynucleotides, for example, a polynucleotide comprising a nucleotide sequence derived from the coding sequence (CDS) of an orthologue gene from a different species. Examples of the CDS of an orthologue gene of human FKBP23 can include dog FKBP23 CDS (NCBI accession: XM_545546), chimpanzee FKBP23 CDS (NCBI accession: XM_515942), bovine FKBP23 CDS (NCBI accession: XM_866765), rat FKBP23 CDS (NCBI accession: XM_215758), mouse FKBP23 CDS (NCBI accession: NM_010222), chicken FKBP23 CDS (NCBI accession: XM_421981) and the like. Examples of the CDS of an orthologue gene of human FKBP19 can include dog FKBP19 CDS (NCBI accession: XM_846112), chimpanzee FKBP19 CDS (NCBI accession: XM_001159891), bovine FKBP19 CDS (NCBI accession: NM_001045932), rat FKBP19 CDS (NCBI accession: NM_001013105), mouse FKBP19 CDS (NCBI accession: NM_024169) and the like. Further, included may be a polynucleotide having a deletion, substitution or addition of one or more bases in the nucleotide sequence of a polynucleotide comprising a nucleotide sequence derived from these CDSs; and a polynucleotide comprising a partial nucleotide sequence of a nucleotide sequence derived from these CDSs.

Examples of a polynucleotide comprising a nucleotide sequence encoding a prolyl hydroxylase which is used for the production of the transformant of the present inventions can include a polynucleotide comprising a nucleotide sequence encoding the amino acid sequence of prolyl 4-hydroxylase. Prolyl 4-hydroxylase is an enzyme which catalyzes the reaction of hydroxylation of the 4-position of the proline residues located at Yaa sites in the Gly-Xaa-Yaa repeating sequence, converting the proline to 4-hydroxyproline. A glycine repeat sequence protein forms a unique triple-helical structure. The hydroxylation of the proline residues located at Yaa sites results in an improved stability of the triple-helical structure of the glycine repeat sequence protein. The origin of the prolyl 4-hydroxylase is not limited in particular, and the prolyl 4-hydroxylase can be a prolyl 4-hydroxylase derived from an animal, preferably a higher animal, more preferably a human.
The prolyl 4-hydroxylase can be a prolyl hydroxylase comprising an α subunit and a β subunit, wherein the α subunit can be, for example, an α1 subunit, an α2 subunit, or an α3 subunit, preferably an α1 subunit.

Examples of the prolyl 4-hydroxylase α subunit can include a human prolyl 4-hydroxylase α1 subunit (P4Hα1) comprising the amino acid sequence indicated by SEQ ID NO:80, a human prolyl 4-hydroxylase α2 subunit (P4Hα2) comprising the amino acid sequence indicated by SEQ ID NO:81 and a human prolyl 4-hydroxylase α3 subunit (P4Hα3) comprising the amino acid sequence indicated by SEQ ID NO:82. Examples of the prolyl 4-hydroxylase β subunit (P4Hβ) can include human PH4β comprising the amino acid sequence indicated by SEQ ID NO:83. Further, included may be a protein having an amino acid deletion, substitution or addition in these amino acid sequences and a protein having an amino acid sequence which has homology with these amino acid sequences, for example, a protein comprising an amino acid sequence derived from an orthologue gene from a different species. Examples of an amino acid sequence derived from an orthologue gene of human P4Hα1 can include the amino acid sequence of dog P4Hα1 (NCBI accession: XP_862257), chimpanzee P4Hα1 (NCBI accession: XP_001141043), bovine P4Hα1 (NCBI accession: NP_001069238), rat P4Hα1 (NCBI accession: NP_742059), mouse P4Hα1 (NCBI accession: NP_035160) and chicken P4Hα1 (NCBI accession: XP_421583). Examples of an amino acid sequence derived from an orthologue gene of human P4Hα2 can include the amino acid sequence of dog P4Hα2 (NCBI accession: XP_860637), chimpanzee P4Hα2 (NCBI accession: XP_001162222), bovine P4Hα2 (NCBI accession: NP_001029465), rat P4Hα2 (NCBI accession: XP_340799), mouse P4Hα2 (NCBI accession: NP_035161) and chicken P4Hα2 (NCBI accession: NP_001006155). Examples of an amino acid sequence derived from an orthologue gene of human P4Hα3 can include the amino acid sequence of dog P4Hα3 (NCBI accession: XP_851718), chimpanzee P4Hα3 (NCBI accession: XP_001174896), bovine P4Hα3 (NCBI accession: NP_001001598), rat P4Hα3 (NCBI accession: NP_942070), mouse P4Hα3 (NCBI accession: NP_796135) and chicken P4Hα3 (NCBI accession: XP_417248). Examples of an amino acid sequence derived from an orthologue gene of human P4Hβcan include the amino acid sequence of dog P4Hβ (NCBI accession: XP_540488), chimpanzee P4Hβ (NCBI accession: XP_001164396), bovine P4Hβ (NCBI accession: NP_776560), rat P4Hβ (NCBI accession: NP_037130), mouse P4Hβ (NCBI accession: NP_035162) and chicken P4Hβ (NCBI accession: XP_420095). Further, included may be a prolyl 4-hydroxylase which has an amino acid deletion, substitution or addition in an amino acid sequence derived from these orthologue genes.

In addition, examples of a polynucleotide comprising a nucleotide sequence encoding the amino acid sequence of the prolyl 4-hydroxylase as described above can include the polynucleotide indicated by SEQ ID NO:84, which comprises a nucleotide sequence encoding the amino acid sequence of human P4Hα1; the polynucleotide indicated by SEQ ID NO:85, which comprises a nucleotide sequence encoding the amino acid sequence of human P4Hα2; the polynucleotide indicated by SEQ ID NO:86, which comprises a nucleotide sequence encoding the amino acid sequence of human P4Hα3; and the polynucleotide indicated by SEQ ID NO:87, which comprises a nucleotide sequence encoding the amino acid sequence of human P4Hβ. More preferably, a polynucleotide comprising a nucleotide sequence encoding the amino acid sequence of the prolyl 4-hydroxylase as described above can be the polynucleotide indicated by SEQ ID NO:84 or the polynucleotide indicated by SEQ ID NO:87. In addition, included can be a polynucleotide having a deletion, substitution or addition of one or more bases in the nucleotide sequences of these polynucleotides; and a polynucleotide comprising a partial nucleotide sequence of these polynucleotides. Further, included may be a polynucleotide comprising a nucleotide sequence having homology with the nucleotide sequences of these polynucleotides, for example, a polynucleotide comprising a nucleotide sequence derived from the coding sequence (CDS) of an orthologue gene from a different species. Examples of the CDS of an orthologue gene of human P4Hα1 can include dog P4HA1 CDS (NCBI accession: XM_857164), chimpanzee P4HA1 CDS (NCBI accession: XM_001141043), bovine P4HA1 CDS (NCBI accession: NM_001075770), rat P4HA1 CDS (NCBI accession: NM_172062), mouse P4HA1 CDS (NCBI accession: NM_011030), chicken P4HA1 CDS (NCBI accession: XM_421583) and the like. Examples of the CDS of an orthologue gene of human P4Hα2 can include dog P4HA2 CDS (NCBI accession: XM_855544), chimpanzee P4HA2 CDS (NCBI accession: XM_001162222), bovine P4HA2 CDS (NCBI accession: NM_001034293), rat P4HA2 CDS (NCBI accession: XM_340798), mouse P4HA2 CDS (NCBI accession: NM_011031), chicken P4HA2 CDS (NCBI accession: NM_001006155) and the like. Examples of the CDS of an orthologue gene of human P4Hα3 can include dog P4HA3 CDS (NCBI accession: XM_846625), chimpanzee P4HA3 CDS (NCBI accession: XM_001174896), bovine P4HA3 CDS (NCBI accession: NM_001001598), rat P4HA3 CDS (NCBI accession: NM_198775), mouse P4HA3 CDS (NCBI accession: NM_177161), chicken P4HA3 CDS (NCBI accession: XM_417248) and the like. Examples of the CDS of an orthologue gene of human P4Hβcan include dog P4HB CDS (NCBI accession: XM_540488), chimpanzee P4HB CDS (NCBI accession: XM_001164396), bovine P4HB CDS (NCBI accession: NM_174135), rat P4HB CDS (NCBI accession: NM_012998), mouse P4HB CDS (NCBI accession: NM_011032), chicken P4HB CDS (NCBI accession: XM_420095) and the like. Further, included may be a polynucleotide having a deletion, substitution or addition of one or more bases in the nucleotide sequence of a polynucleotide comprising a nucleotide sequence derived from these CDSs; and a polynucleotide comprising a partial nucleotide sequence of a nucleotide sequence derived from these CDSs.

A protein comprising a glycine-repeating sequence according to the present invention is not limited in particular, as long as the protein is a glycine repeat sequence protein having the characteristics (A) and (B) described below. Such protein according to the present invention can be, for example, at least one collagen selected from among collagens of Type I to Type XXIX, preferably a Fibril-forming collagen (Type I collagen, Type II collagen, Type III collagen, Type V collagen, Type XI collagen, or Type XXIV collagen, Type XXVII collagen), more preferably Type I collagen, Type II collagen, or Type III collagen. Also, included can be a glycine repeat sequence protein which consists of a partial region of these collagens. The origin of the collagens is not limited in particular, and the collagens can be a collagen derived from an animal, preferably a higher animal, further preferably a human.
<Characteristic (A)>
   The polypeptide chain of the glycine repeat sequence protein having a continuous, Gly-Xaa-Yaa repeating sequence.
<Characteristic (B)>
   An imino acid (for example, proline or hydroxyproline) being contained in the continuous, Gly-Xaa-Yaa repeating sequence of the polypeptide chain of the glycine repeat sequence protein,
   wherein Xaa and Yaa each represent any amino acid.
In characteristic (A), the number of repeats of the repeating sequence Gly-Xaa-Yaa can be 25 or more and 362 or less, for example. In characteristic (B), the content of the imino acid which is contained in the Gly-Xaa-Yaa repeating sequence can be 19.8% or more and 38.7% or less, for example.

Examples of the Type I collagen can include a human Type I α1 collagen precursor (Hs Type I α1) comprising the amino acid sequence indicated by SEQ ID NO:88 and a human Type I α2 collagen precursor (Hs Type I α2) comprising the amino acid sequence indicated by SEQ ID NO:89. Examples of the Type II collagen can include a human Type II α1 collagen precursor (Hs Type II α1) comprising the amino acid sequence indicated by SEQ ID NO:90. Examples of the Type III collagen can include a human Type III α1 collagen precursor (Hs Type III α1) comprising the amino acid sequence indicated by SEQ ID NO:91. Further, included can be a protein having an amino acid deletion, substitution or addition in these amino acid sequences. In addition, included may be a protein having an amino acid sequence which has homology with these amino acid sequences, for example, a protein comprising an amino acid sequence derived from an orthologue from a different species. Examples of an amino acid sequence derived from an orthologue gene of human Type I α1 can include the amino acid sequence of dog Type I α1 (NCBI accession: NP_001003090), chimpanzee Type I α1 (NCBI accession: XP_001169320), bovine Type I α1 (NCBI accession: NP_001029211), rat Type I α1 (NCBI accession: XP_213440) and mouse Type I α1 (NCBI accession: NP_031768). Examples of an amino acid sequence derived from an orthologue gene of human Type I α2 can include the amino acid sequence of dog Type I α2 (NCBI accession: NP_001003187), chimpanzee Type I α2 (NCBI accession: XP_519207), bovine Type I α2 (NCBI accession: NP_776945), rat Type I α2 (NCBI accession: NP_445808) and mouse Type I α2 (NCBI accession: NP_031769). Examples of an amino acid sequence derived from an orthologue gene of human Type II α1 can include the amino acid sequence of dog Type II α1 (NCBI accession: NP_001006952), chimpanzee Type II α1 (NCBI accession: XP_509026), rat Type II α1 (NCBI accession: NP_037061), mouse Type II α1 (NCBI accession: NP_112440) and chicken Type II α1 (NCBI accession: NP_989757). Examples of an amino acid sequence derived from an orthologue gene of human Type III α1 can include the amino acid sequence of dog Type III α1 (NCBI accession: XP_851009), chimpanzee Type III α1 (NCBI accession: XP_001163665), bovine Type III α1 (NCBI accession: NP_001070299), rat Type III α1 (NCBI accession: NP_114474), mouse Type III α1 (NCBI accession: NP_034060) and chicken Type III α1 (NCBI accession: XP_421847). Further, included may be a protein which has an amino acid deletion, substitution or addition in an amino acid sequence derived from these orthologue genes.
In the case where the glycine repeat sequence protein has been expressed within a yeast and the produced prolyl hydroxylase is present within the yeast, a glycine repeat sequence protein may be produced in which proline residues of the glycine repeat sequence protein are hydroxylated by the prolyl hydroxylase. The degree of hydroxylation of the proline residues of a glycine repeat sequence protein may be determined in known methods for amino acid composition analysis. In addition, the ability for fibril formation of a glycine repeat sequence protein may be determined, for example, by the following procedures. In particular, for example, a solution of a purified glycine repeat sequence protein is readjusted to a salt concentration of 1x D-PBS(-) and a pH of from 7.3 to 7.4 and then kept at a temperature of 37°C, thereby leading to reorientation of the glycine repeat sequence protein molecule and then clouding of the solution. Such clouding may be regarded as an indication of the ability for fibril formation of the glycine repeat sequence protein. Accordingly, by means of this property, the ability for fibril formation of a glycine repeat sequence protein may be determined by keeping a solution of a glycine repeat sequence protein, whose concentration is 0.05%, at a temperature of 37°C, at a salt concentration of 1x D-PBS(-), and at a pH of from 7.3 to 7.4, and measuring the absorbance of the solution over time during the incubation period.

Examples of a glycine repeat sequence protein consisting of a partial region of the above-mentioned collagens can include a precursor of a fusion polypeptide (Hs Type I α1 N60C15) comprising the amino acid sequence indicated by SEQ ID NO:92, which consists of the N-terminal non-helix region, 60 N-terminal residues of the helix region, 15 C-terminal residues of the helix region and the C-terminal non-helix region of human collagen Type I α1; a precursor of a fusion polypeptide (Hs Type I α2 N60-C15) comprising the amino acid sequence indicated by SEQ ID NO:93, which consists of the N-terminal non-helix region, 60 N-terminal residues of the helix region, 15 C-terminal residues of the helix region and the C-terminal non-helix region of human collagen Type I α2; a precursor of a fusion polypeptide (Hs Type I α1 N27-M522-C15) comprising the amino acid sequence indicated by SEQ ID NO:94, which consists of the N-terminal non-helix region, 27 N-terminal residues of the helix region, 522 central residues of the helix region, 15 C-terminal residues of the helix region and the C-terminal non-helix region of human collagen Type I α1; a precursor of a fusion polypeptide (Hs Type I α2 N27-M522-C15) comprising the amino acid sequence indicated by SEQ ID NO:95, which consists of the N-terminal non-helix region, 27 N-terminal residues of the helix region, 522 central residues of the helix region, 15 C-terminal residues of the helix region and the C-terminal non-helix region of human collagen Type I α2; a precursor of a fusion polypeptide (Hs Type I α1 N27-M522X2-C15) comprising the amino acid sequence indicated by SEQ ID NO:96, which consists of the N-terminal non-helix region, 27 N-terminal residues of the helix region, a dimer made of 522 central residues of the helix region, 15 C-terminal residues of the helix region and the C-terminal non-helix region of human collagen Type I α1; and a precursor of a fusion polypeptide (Hs Type I α2 N27-M522x2-C15) comprising the amino acid sequence indicated by SEQ ID NO:97, which consists of the N-terminal non-helix region, 27 N-terminal residues of the helix region, a dimer made of 522 central residues of the helix region, 15 C-terminal residues of the helix region and the C-terminal non-helix region of human collagen Type I α2.

Examples of a polynucleotide comprising a nucleotide sequence encoding a glycine repeat sequence protein which is used for the production of the transformant of the present invention can include a polynucleotide comprising a nucleotide sequence encoding the amino acid sequence of the above-mentioned human Type I α1 collagen precursor, wherein the nucleotide sequence is indicated by SEQ ID NO:98; a polynucleotide comprising a nucleotide sequence encoding the amino acid sequence of the above-mentioned human Type I α1 collagen precursor, wherein the nucleotide sequence is indicated by SEQ ID NO:99; a polynucleotide comprising a nucleotide sequence encoding the amino acid sequence of the above-mentioned human Type I α2 collagen precursor, wherein the nucleotide sequence is indicated by SEQ ID NO:100; a polynucleotide comprising a nucleotide sequence encoding the amino acid sequence of the above-mentioned human Type I α2 collagen precursor, wherein the nucleotide sequence is indicated by SEQ ID NO:101; a polynucleotide comprising a nucleotide sequence encoding the amino acid sequence of the above-mentioned human Type II α1 collagen precursor, wherein the nucleotide sequence is indicated by SEQ ID NO:102; a polynucleotide comprising a nucleotide sequence encoding the amino acid sequence of the above-mentioned human Type II α1 collagen precursor, wherein the nucleotide sequence is indicated by SEQ ID NO:103; a polynucleotide comprising a nucleotide sequence encoding the amino acid sequence of the above-mentioned human Type III α1 collagen precursor, wherein the nucleotide sequence is indicated by SEQ ID NO:104; and a polynucleotide comprising a nucleotide sequence encoding the amino acid sequence of the above-mentioned human Type III α1 collagen precursor, wherein the nucleotide sequence is indicated by SEQ ID NO:105; and more preferably, a polynucleotide comprising a nucleotide sequence encoding a glycine repeat sequence protein which is used for the production of the transformant of the present invention can be a polynucleotide comprising a nucleotide sequence encoding the amino acid sequence of the above-mentioned human Type I α1 collagen precursor, wherein the nucleotide sequence is indicated by SEQ ID NO:98; a polynucleotide comprising a nucleotide sequence encoding the amino acid sequence of the above-mentioned human Type I α2 collagen precursor, wherein the nucleotide sequence is indicated by SEQ ID NO:100; a polynucleotide comprising a nucleotide sequence encoding the amino acid sequence of the above-mentioned human Type II α1 collagen precursor, wherein the nucleotide sequence is indicated by SEQ ID NO:102; or a polynucleotide comprising a nucleotide sequence encoding the amino acid sequence of the above-mentioned human Type III α1 collagen precursor, wherein the nucleotide sequence is indicated by SEQ ID NO:104. In addition, included can be a polynucleotide having a deletion, substitution or addition of one or more bases in the nucleotide sequences of these polynucleotides; and a polynucleotide comprising a partial nucleotide sequence of these polynucleotides. Further, included may be a polynucleotide comprising a nucleotide sequence having homology with the nucleotide sequences of these polynucleotides, for example, a polynucleotide comprising a nucleotide sequence derived from the coding sequence (CDS) of an orthologue gene from a different species.

Examples of the CDS of an orthologue gene of human Type I α1 can include dog Type I α1 CDS (NCBI accession: NM_001003090), chimpanzee Type I α1 CDS (NCBI accession: XM_001169320), bovine Type I α1 CDS (NCBI accession: NM_001034039), rat Type I α1 CDS (NCBI accession: XM_213440), mouse Type I α1 CDS (NCBI accession: NM_007742) and the like. Examples of the CDS of an orthologue gene of human Type I α2 can include dog Type I α2 CDS (NCBI accession: NM_001003187), chimpanzee Type I α2 CDS (NCBI accession: XM_519207), bovine Type I α2 CDS (NCBI accession: NM_174520), rat Type I α2 CDS (NCBI accession: NM_053356), mouse Type I α2 CDS (NCBI accession: NM_007743) and the like. Examples of the CDS of an orthologue gene of human Type II α1 can include dog Type II α1 CDS (NCBI accession: NM_001006951), chimpanzee Type II α1 CDS (NCBI accession: XM_509026), rat Type II α1 CDS (NCBI accession: NM_012929), mouse Type II α1 CDS (NCBI accession: NM_031163), chicken Type II α1 CDS (NCBI accession: NM_204426) and the like. Examples of the CDS of an orthologue gene of human Type III α1 can include dog Type III α1 CDS (NCBI accession: XM_845916), chimpanzee Type III α1 CDS (NCBI accession: XM_001163665), bovine Type III α1 CDS (NCBI accession: NM_001076831), rat Type III α1 CDS (NCBI accession: NM_032085), mouse Type III α1 CDS (NCBI accession: NP_009930), chicken Type III α1 CDS (NCBI accession: XM_421847) and the like. Further, included may be a polynucleotide having a deletion, substitution or addition of one ore more bases in the nucleotide sequence of a polynucleotide comprising a nucleotide sequence derived from these CDSs; and a polynucleotide comprising a partial nucleotide sequence of a nucleotide sequence derived from these CDSs.

Examples of a polynucleotide comprising a nucleotide sequence encoding a glycine repeat sequence protein consisting of a partial region of the above-mentioned collagens can include a polynucleotide comprising a nucleotide sequence encoding the amino acid sequence of the Hs Type I α1 N60C15 precursor which is indicated by SEQ ID NO:106, a polynucleotide comprising a nucleotide sequence encoding the amino acid sequence of the Hs Type I α2 N60-C15 precursor which is indicated by SEQ ID NO:107, a polynucleotide comprising a nucleotide sequence encoding the amino acid sequence of the Hs Type I α1 N27-M522-C15 precursor which is indicated by SEQ ID NO:108, a polynucleotide comprising a nucleotide sequence encoding the amino acid sequence of the Hs Type I α2 N27-M522-C15 precursor which is indicated by SEQ ID NO:109, a polynucleotide comprising a nucleotide sequence encoding the amino acid sequence of the Hs Type I α1 N27-M522X2-C15 precursor which is indicated by SEQ ID NO:110, and a polynucleotide comprising a nucleotide sequence encoding the amino acid sequence of the Hs Type I α2 N27-M522x2-C15 precursor which is indicated by SEQ ID NO:111.

The "transformant of the present invention" as described above may be further a transformant which is obtained by transfecting an additional polynucleotide comprising a nucleotide sequence encoding the amino acid sequence of a "lysyl hydroxylase," that is, a transformant which produces all of the following proteins (1) to (4):
(1) an FK506 binding protein that is capable of binding to FK506 and has a molecular weight of 15,000 or more and 60,000 or less;
(2) a prolyl hydroxylase;
(3) a glycine repeat sequence protein having the following characteristics (A) and (B):
   <characteristic (A)> the polypeptide chain of the glycine repeat sequence protein having a continuous, Gly-Xaa-Yaa repeating sequence, and
   <characteristic (B)> an imino acid (e.g. proline or hydroxyproline) being contained in the continuous, Gly-Xaa-Yaa repeating sequence in the polypeptide chain of the glycine repeat sequence protein
   wherein Xaa and Yaa each represent any amino acid; and
(4) a lysyl hydroxylase.
Such transformant is capable of efficiently producing a glycine repeat sequence protein with hydoxylated lysine residues in the amino acid sequence. Such transformant can produce a glycine repeat sequence protein with an enhanced ability for fibril formation and an increased stability. For example, a transformant which produces all of the above-mentioned proteins (1) to (4) allows the production of a glycine repeat sequence protein in which 20% or more, preferably 40% or more, of the total lysine residues located at Yaa's in the Gly-Xaa-Yaa repeating sequence have been hydroxylated.
A glycine repeat sequence protein with hydroxylated lysine residues is one that exhibits an enhanced ability for fibril formation and has a more stable triple-helical structure, and thus can be one that is usable as a high performance versatile material which is more commercially valuable for pharmaceuticals, industrial products, cosmetics, foods etc.

In the present invention, a "lysyl hydroxylase" can include lysyl hydroxylase 1, lysyl hydroxylase 3 and the like.
The origin of a "lysyl hydroxylase" is not limited in particular, and a "lysyl hydroxylase" is preferably derived, for example, from a higher animal, further preferably from a human.
Lysyl hydroxylase 1 and lysyl hydroxylase 3 are homodimer-forming enzymes and hydroxylate the δ position of the lysines located at Yaa's in the Gly-Xaa-Yaa repeating sequence found in the helix structure of a glycine repeat sequence protein.
Examples of the lysyl hydroxylase can include human lysyl hydroxylase (LH1) comprising the amino acid sequence indicated by SEQ ID NO:116 and human lysyl hydroxylase 3 (LH3) comprising the amino acid sequence indicated by SEQ ID NO:118. Further, included can be a protein having an amino acid deletion, substitution or addition in these amino acid sequences. In addition, included may be a protein homologous to these amino acid sequences, for example, a protein comprising an amino acid sequence derived from an orthologue gene from a different species. Examples of an amino acid sequence derived from an orthologue gene of human LH1 can include the amino acid sequence of dog LH1 (NCBI accession: XP_865470), chimpanzee LH1 (NCBI accession: XP_001142937), bovine LH1 (NCBI accession: NP_776573), rat LH1 (NCBI accession: NP_446279), mouse LH1 (NCBI accession: NP_035252) and chicken LH1 (NCBI accession: NP_001005618). Examples of an amino acid sequence derived from an orthologue gene of human LH3 can include the amino acid sequence of dog LH3 (NCBI accession: XP_858413), chimpanzee LH3 (NCBI accession: XP_001153979), bovine LH3 (NCBI accession: XP_887254), rat LH3 (NCBI accession: NP_835202), mouse LH3 (NCBI accession: NP_036092) and the like. Further, included may be a lysyl hydroxylase having an amino acid deletion, substitution or addition in an amino acid sequence derived from these orthologue genes.

Examples of a polynucleotide comprising a nucleotide sequence encoding the amino acid sequence of a "lysyl hydroxylase" which can be used for the production of the transformant of the present invention can include a polynucleotide comprising a nucleotide sequence encoding lysyl hydroxylase 1 (LH1) and a polynucleotide comprising a nucleotide sequence encoding lysyl hydroxylase 3 (LH3). Examples of a polynucleotide comprising a nucleotide sequence encoding the above-mentioned lysyl hydroxylase 1 can include a polynucleotide comprising a nucleotide sequence encoding the amino acid sequence of human LH1 which is indicated by SEQ ID NO:117 and a polynucleotide comprising a nucleotide sequence encoding the amino acid sequence of human LH3 which is indicated by SEQ ID NO:119. In addition, included can be a polynucleotide having a deletion, substitution or addition of one or more bases in the nucleotide sequences of these polynucleotides; and a polynucleotide comprising a partial nucleotide sequence of these polynucleotides. Further, included may be a polynucleotide comprising a nucleotide sequence homologous to the nucleotide sequences of these polynucleotides, for example, a polynucleotide comprising a nucleotide sequence derived from the coding sequence (CDS) of an orthologue gene from a different species. Examples of the CDS of an orthologue gene of human LH1 can include dog PLOD1 CDS (NCBI accession: XM_860377), chimpanzee PLOD1 CDS (NCBI accession: XM_001142937), bovine PLOD1 CDS (NCBI accession: NM_174148), rat PLOD1 CDS (NCBI accession: NM_053827), mouse PLOD1 CDS (NCBI accession: NM_011122), chicken PLOD1 CDS (NCBI accession: NM_001005618) and the like. Examples of the CDS of an orthologue gene of human LH3 can include dog PLOD3 CDS (NCBI accession: XM_853320), chimpanzee PLOD3 CDS (NCBI accession: XM_001153979), bovine PLOD3 CDS (NCBI accession: XM_882161), rat PLOD3 CDS (NCBI accession: NM_178101), mouse PLOD3 CDS (NCBI accession: NM_011962) and the like. Further, included may be a polynucleotide having a deletion, substitution or addition of one or more bases in the nucleotide sequence of a polynucleotide comprising a nucleotide sequence derived from the CDSs of these orthologue genes; and a polynucleotide comprising a partial nucleotide sequence of a nucleotide sequence derived from the CDSs of these orthologue genes.

Here, the above-mentioned polynucleotide comprising a nucleotide sequence encoding the amino acid sequence of a lysyl hydroxylase is preferably linked, for example, to downstream of a promoter derived from a yeast, for example, the promoter of the alcohol oxidase 1 gene or the like.

The "transformant of the present invention" as described above may be further a transformant which is obtained by additionally transfeting a polynucleotide comprising a nucleotide sequence encoding the amino acid sequence of "Hsp47", that is, a transformant which produces all of the following proteins (1) to (3) and (5):
(1) an FK506 binding protein that is capable of binding to FK506 and has a molecular weight of 15,000 or more and 60,000 or less:
(2) a prolyl hydroxylase;
(3) a glycine repeat sequence protein having the following characteristics (A) and (B):
   <characteristic (A)> the polypeptide chain of the glycine repeat sequence protein having a continuous, Gly-Xaa-Yaa repeating sequence, and
   <characteristic (B)> an imino acid (e.g. proline or hydroxyproline) being contained in the continuous, Gly-Xaa-Yaa repeating sequence of the polypeptide chain of the glycine repeat sequence protein
   wherein Xaa and Yaa each represent any amino acid; and (5) Hsp47.
Such transformant is capable of efficiently producing a protein which has been modified with neutral sugars and has a smaller content of the neutral sugars. Such yeast reduces a change of the physical properties relating to the hydrophilicity of glycine repeat sequence proteins which result from neutral sugars, thereby making it possible to produce a glycine repeat sequence protein having more lipophilic properties that are similar to those inherently possessed by glycine repeat sequence proteins. Such glycine repeat sequence protein is a protein having a more stable triple-helical structure, and thus can be a glycine repeat sequence protein which is usable as a high performance versatile material that is more commercially valuable for pharmaceuticals, industrial products, cosmetics, foods etc.

"Hsp47" is a stress response protein localized in the endoplasmic reticulum lumen, which is generally referred to as Heat shock protein 47.
In the present invention, "Hsp47" is not particularly limited by the origin, and for example, is preferably derived from a higher animal, further preferably from a human. Examples can include human Hsp47, which has the amino acid sequence indicated by SEQ ID NO:120. In addition, included can be a protein having a deletion, substitution or addition of an amino acid sequence in the amino acid sequences of these Hsp47s. Also, included may be a protein having homology with these amino acid sequences, for example, a protein comprising an amino acid sequence derived from an orthologue from a different species. Examples of an amino acid sequence derived from an orthologue gene of human Hsp47 can include the amino acid sequence of dog Hsp47 (NCBI accession: XP_542305), chimpanzee Hsp47 (NCBI accession: XP_001174979), bovine Hsp47 (NCBI accession: NP_001039528), rat Hsp47 (NCBI accession: NP_058869), mouse Hsp47 (NCBI accession: NP_033955), chicken Hsp47 (NCBI accession: NP_990622) and the like. Further, included may be a protein having a deletion, substitution or addition of an amino acid sequence in an amino acid sequence derived from these orthologue genes.

Examples of a polynucleotide comprising a nucleotide sequence encoding the amino acid sequence of "Hsp47" which can be used for the production of the transformant of the present invention can include a polynucleotide comprising the nucleotide sequence indicated by SEQ ID NO:121 and others. In addition, included may be a polynucleotide having an artificial deletion, substitution or addition of one or more bases in the nucleotide sequence of this polynucleotide; and also a polynucleotide comprising a nucleotide sequence encoding an amino acid sequence which has a deletion, substitution or addition in the amino acid sequence of Hsp47. In addition, included may be a polynucleotide comprising a nucleotide sequence having homology with these polynucleotides, for example, a polynucleotide comprising a nucleotide sequence derived from the coding sequence (CDS) of an orthologue gene from a different species. Examples of the CDS of an orthologue gene of human Hsp47 can include dog Hsp47 CDS (NCBI accession: XM_542305), chimpanzee Hsp47 CDS (NCBI accession: XM_001174979), bovine Hsp47 CDS (NCBI accession: NM_001046063), rat Hsp47 CDS (NCBI accession: NM_017173), mouse Hsp47 CDS (NCBI accession: NM_009825), chicken Hsp47 CDS (NCBI accession: NM_205291) and the like. Further, included may be a polynucleotide having a deletion, substitution or addition of one or more bases in the nucleotide sequence of a polynucleotide comprising a nucleotide sequence derived from the CDSs of these orthologue genes; and a polynucleotide comprising a partial nucleotide sequence of a nucleotide sequence derived from the CDSs of these orthologue genes.

Here, the above-mentioned polynucleotide comprising a nucleotide sequence encoding the amino acid sequence of Hsp47 is preferably linked, for example, to downstream of a promoter derived from an yeast, for example, the promoter of the alcohol oxidase 1 gene or the like.

The transformant of the present invention may be produced by transfecting all of the below-mentioned polynucleotides (1), (2) and (3) into a host cell. These polynucleotides may be produced by cDNA cloning, assembly PCR using chemically synthesized oligonucleotides, or genetic engineering procedures. Clones, such as commercially available cDNAs, may be also obtained and used.
(1) a polynucleotide comprising a nucleotide sequence encoding the amino acid sequence of an FK506 binding protein that is capable of binding to FK506 and has a molecular weight of 15,000 or more and 60,000 or less;
(2) a polynucleotide comprising a nucleotide sequence encoding a prolyl hydroxylase; and
(3) a polynucleotide comprising a nucleotide sequence encoding the amino acid sequence of a glycine repeat sequence protein having the following characteristics (A) and (B):
   <characteristic (A)> the polypeptide chain of the glycine repeat sequence protein having a continuous, Gly-Xaa repeating sequence; and
   <characteristic (B)> an imino acid (e.g. proline or hydroxyproline) being contained in the continuous, Gly-Xaa-Yaa repeating sequence of the polypeptide chain of the glycine repeat sequence protein,
wherein Xaa and Yaa each represent any amino acid.

A transformant in which the above-mentioned polynucleotides are efficiently expressed can be produced by linking each of the polynucleotides, for example, to a promoter, a terminator, a polynucleotide encoding a signal sequence etc. which allows efficient expression of the polynucleotide within a cell, thereby constructing an expression cassette; and transfecting each of the constructed expression cassettes for the polynucleotides into a host cell.
These expression cassettes can be constructed by linking the polynucleotide to downstream of a promoter and to upstream of a terminator and optionally replacing the polynucleotide encoding signal sequence at the amino-terminal, by means of standard genetic engineering procedures.
Host-vector systems which are used for constructing the expression cassettes can include, for example, but not especially limited to, the host-vector system using bacteria such as Escherichia, Bacillus or Pseudomonas as the host, and a bacteriophage, a plasmid or a cosmid as the vector; the host-vector system using the yeast such as Komagataella, Saccharomyces, Hansenula, Candida or Ogataea as the host, and an episomal plasmid, a ARS-CEN plasmid or a plasmid integrated into the chromosome as the vector; or the like. Preferably, Escherichia can be used as a host and plasmid can be used as a vector.

The promoter is not limited in particular, as long as it functions within a host cell which is used for producing the transformant of the present invention. Inducible promoters, of which the transcriptional activity may be induced by specific nutrients or substrates, may be preferably used.
Examples of the promoter can include the promoters of the following genes: the galactose metabolizing enzyme genes (GAL1, GAL10), the inhibitory acid phosphatase gene (PHO5), the glyceraldehyde-3-phosphate dehydrogenase gene (TD), the phosphoglycerate kinase gene (PGK), the alcohol oxidase genes (ADH1, AOX1, AOX2, MOX, AOD1), the formate dehydrogenase genes (FDH1, FMD1), the dihydroxyacetone synthase gene (DAS), the peroxisome membrane protein biogenesis gene (PER3), and the formaldehyde dehydrogenase gene (FLD1). As the terminator, any terminator may be effectively used as long as it functions within a host cell which is used for producing the transformant of the present invention, and examples of the terminator can include the terminators of the alcohol oxidase gene (AOX1) and formaldehyde dehydrogenase (FLD1). As the signal sequence, examples can include the prepro sequence of yeast α-factor, the signal sequence of yeast invertase, the signal sequences of yeast acid phosphatase, and others.

The transformant of the present invention can be produced by transforming a host cell using a plasmid comprising the above-mentioned polynucleotides. The use of a plasmid comprising the above-mentioned polynucleotides which have been formed as their respective expression cassettes will allow one to produce a transformant which efficiently expresses the polynucleotides.
Examples of host-vector systems which are used for producing the transformant of the present invention can include a host-vector system where bacteria (e.g. Escherichia, Bacillus, Pseudomonas) is used as a host and a bacteriophage, a plasmid or a cosmid is used as a vector; a host-vector system where a yeast (e.g. Saccharomyces) is used as a host and an episomal plasmid or an ARS-CEN plasmid is used as a vector; a host-vector system where a yeast (e.g. Komagataella, Saccharomyces, Hansenula, Pichia, Candida, Ogataea) is used as a host and a chromosome integration plasmid is used as a vector; a host-vector system where filamentous fungi (e.g. Aspergillus, Trichoderma) is used as a host and a chromosome integration plasmid is used as a vector. Preferably, the host can be a eukaryotic microorganism (yeast, filamentous fungus).

As a more preferable host which is used for producing the transformant of the present invention, a yeast can be employed, for example, Saccharomyces cerevisiae, Komagataella pastoris, Hansenula polymorpha, Pichia methanolica, Candida Boidini, Ogataea minuta. In cases where Saccharomyces cerevisiae is used as a host, the vector can be a chromosome integration plasmid (YIp type), a plasmid comprising the replication initiation region of the 2-µm DNA yeast endogenous plasmid (YEp type) and a plasmid comprising a self-replication region derived from the chromosome (YCp type). In these cases, a plasmid serving as a shuttle vector, where the replication origin which is replicable in Escherichia coli is inserted into the plasmid, can be used to easily construct the respective plasmid into which the above-mentioned polynucleotides have been introduced. In cases where Komagataella pastoris, Hansenula polymorpha, Pichia methanolica, Candida Boidini or Ogataea minuta are used as a host, the vector can be a chromosome integration plasmid. In these cases, a plasmid serving as a shuttle vector, where the replication origin which is replicable in Escherichia coli cells is inserted into the plasmid can be used to easily construct the respective plasmids into which the above-mentioned polynucleotides have been introduced. Further preferably, Komagataella pastoris, Hansenula polymorpha, Pichia methanolica, Candida Boidini or Ogataea minuta, which are methanol-utilizing yeasts, can be used as a host, and a chromosome integration plasmid can be used as a vector. Particularly, Komagataella pastoris may be suitably used as a host, and plasmids which are commercially available, such as pAO815 and pPIC9K, as well as plasmids produced by incorporating vector elements: a homologous recombination region, a maker, a promoter, a terminator etc. may be suitably employed as a vector.

A chromosome integration plasmid has, on the plasmid, a homologous recombination region which comprises a nucleotide sequence homologous to a nucleotide sequence of the host chromosome, whereby the plasmid is integrated in this region into the host chromosome and thus stably retained in the host cell. Examples of a homologous recombination region include, but not limited to, amino acid synthetic pathway gene, nucleic acid synthetic pathway gene, ribosomal DNAs, Ty (Transposon of Yeast) elements and the like. Markers can include genes inducing transformation by introducing them into a host cell, for example, amino acid synthetic pathway gene, nucleic acid synthetic pathway gene, antibiotic-resistance genes and the like. The amino acid or the nucleic acid synthetic pathway gene can include, but not limited to, for example, LEU2, HIS4, ARG4, TRP1, URA3, ADE2 and the like. Further, the antibiotic-resistant gene can include, but not limited to, for example, genes which induce tolerance to antibiotics such as Zeocyn, Blasticidin S, Geneticin, G418, Chloramphenicol and bleomycin.
The amino acid or the nucleic acid synthetic pathway gene can complement a host deficient in a gene for amino acid synthesis, nucleic acid synthesis or the like so that the gene may be also used as a maker for selecting transformants. In addition, markers may be used to serve as a guide for selecting a transformant in which the chromosome integration plasmid has been incorporated into the host chromosome.

Method for introducing into a host cell a plasmid into which the above-mentioned polynucleotide has been incorporated are not limited in particular, and can include, for example, procedures using calcium chloride, spheroplasts, protoplasts, electroporation, and others, when bacteria (e.g. Escherichia, Bacillus, Pseudomonas) are used as a host. In cases where yeasts (e.g. Komagataella, Saccharomyces, Hansenula, Candida, Ogataea) are used as a host, lithium processes, spheroplast procedures, electroporation techniques, and others can be employed. In cases where filamentous fungi (e.g. Aspergillus, Trichoderma) are used as a host, protoplast-PEG procedures, protoplast-electroporation techniques, and others can be employed.

The transformant of the present invention is characterized in that all of the following proteins (1), (2) and (3) are produced within the cell:
(1) an FK506 binding protein that is capable of binding to FK506 and has a molecular weight of 15,000 or more and 60,000 or less;
(2) a prolyl hydroxylase; and
(3) a glycine repeat sequence protein having the following characteristics (A) and (B):
   <characteristic (A)> the polypeptide chain of the glycine repeat sequence protein having a continuous, Gly-Xaa-Yaa repeating sequence, and
   <characteristic (B)> an imino acid (e.g. proline or hydroxyproline) being contained in the continuous, Gly-Xaa-Yaa repeating sequence of the polypeptide chain of the glycine repeat sequence protein,
wherein Xaa and Yaa each represent any amino acid.

All of the above-mentioned proteins (1) to (3) can be produced by culturing the transformant of the present invention in accordance with standard microbiological techniques. As a medium used for culturing the transformant of the present invention, ones known in the art can be used, and the culturing can be carried out by methods in accordance with standard microbiological techniques.
The medium is not limited in particular, and either of a synthetic medium or a natural medium can be used. For example, the synthetic medium can include mediums containing not only a carbon source (such as a variety of sugars, glycerol, methanol) and a nitrogen source (such as urea, aqueous ammonia, ammonium salts, or nitrate salts), but also mineral salts (such as mineral salts of Mg, Ca, Fe, Na, K, Mn, Co, Mo, B, Zn, I, Cu, or the like), micronutrients (such as a range of vitamins, a range of amino acids or nucleotides), and others. The natural medium can include mediums which contain as the medium components, for example, yeast extract, peptone, casein, and the like, in addition to the components of the synthetic mediums. Further, in cases where an inducible promoter is employed in an above-mentioned expression cassette used for producing the transformant of the present invention, a nutrient, substrate or the like which activates the inducible promoter, for example, galactose, lactose, sucrose, methanol, IPTG and the like may be added to the medium to activate the promoter.

When the transformant of the present invention is a methanol-utilizing yeast, all of the above-mentioned proteins (1), (2) and (3) can be efficiently expressed by using a methanol containing medium. Examples of the medium can include MM medium (1.34% Yeast Nitrogen Base, 4 x 10⁻⁵% biotin, 0.5% methanol), BMM medium (100 mM potassium phosphate buffer, pH 6.0, 1.34% Yeast Nitrogen Base, 4 x 10⁻⁵% biotin, 0.5% methanol), Basal Salt medium (Pichia Protocol, ISBN:0-89603-421-6, Humana Press), FM22 medium (Pichia Protocol, ISBN:0-89603-421-6, Humana Press) and others. The pH of a medium is neutral or weak basic, or weak acidic. Particularly, a medium adjusted to a pH of from 3 to 8, more preferably a pH of from 4 to 7, may be used.

The culturing is preferably carried out in the form of liquid culture and the culturing temperature is preferably between 15°C and 40°C. The culturing period is preferably between 1 and 1,000 hours. The culturing is preferably carried out under conditions with shaking or stirring and can be performed under aeration with air, oxygen. In addition, the culturing is preferably carried out in batch culture, semibatch culture or continuous culture. During the culturing period, feeding of a concentrated medium component or components can be carried out, if needed.
Further, pre-culture can be performed prior to the above-mentioned culture (main culture), if needed. Examples of mediums for pre-culture can include, but not limited to, YNB medium (0.67% Yeast Nitrogen Base, 4 x 10⁻⁵% biotin, 2% glucose), YPD medium (1% yeast extract, 2% peptone, 2% glucose), BMGY medium (1% yeast extract, 2% peptone, 100 mM potassium phosphate buffer, pH 6.0, 1.34% Yeast Nitrogen Base, 4 x 10⁻⁵% biotin, 1% methanol) and others. Culturing conditions for pre-culture are not limited in particular. The culturing period is preferably between 10 and 100 hours, and the culturing temperature is preferably between 15°C and 40°C. Pre-culture is preferably carried out under conditions with shaking or stirring and can be performed under aeration with air, oxygen. Culturing for pre-culture is preferably carried out in batch culture.

The expression of all of the above-mentioned proteins (1), (2) and (3) can be detected by standard biochemical and protein-engineering procedures, including, for example, methods using immunoassays. Specifically, an enzyme-linked immunosorbent assay (ELISA), a radioimmunoassay (RIA), and an immunobead-trapping assay, a western blot analysisetc. may be used. Preferably, a western blot analysis may be used.

The present invention comprises a glycine repeat sequence protein (referred to as a protein of the present invention) which is characterized in that the protein is obtainable by being produced by the transformant of the present invention. Using the transformant of the present invention allows one to obtain a glycine repeat sequence protein with a small content of neutral sugars. In addition, the protein of the present invention is a high performance versatile material (for example, in which changes in the physical properties related to the hydrophilicity of glycine repeat sequence proteins which result from neutral sugars are reduced so as to exhibit more lipophilic properties that are similar to those inherently possessed by glycine repeat sequence proteins) that is more commercially valuable for pharmaceuticals, industrial products, cosmetics and foods etc.

Quantitative determination of neutral sugars contained in a glycine repeat sequence protein may be carried out by standard biochemical procedures. In particular, neutral sugars may be quantitatively determined by phenol-sulfuric acid method, monosaccharide composition analysis using liquid chromatography or the like.

In addition, the ability for fibril formation of a glycine repeat sequence protein may be determined, for example, by the following procedures. For example, a solution of a purified glycine repeat sequence protein is readjusted to a salt concentration of 1x D-PBS(-) and a pH of from 7.3 to 7.4 and then kept at temperature of 37°C, thereby leading to reorientation of the glycine repeat sequence protein molecule and then clouding of the solution. Such clouding may be regarded as an indication of the ability for fibril formation of the glycine repea sequence t protein. Accordingly, by means of this property, the ability for fibril formation of a glycine repeat sequence protein may be determined by keeping a solution containing 0.05% of glycine repeat sequence protein at a temperature of 37°C, at a salt concentration of 1x D-PBS(-) and at a pH of from 7.3 to 7.4; and measuring the absorbance of the solution over time during the incubation period.

The present invention comprises a process for obtaining a glycine repeat sequence protein (referred to as an process obtained by the present invention), characterized by comprising the following steps:
a first step of transfecting all of the following polynucleotides (1), (2) and (3) into a microbial cell:
   (1) a polynucleotide comprising a nucleotide sequence encoding the amino acid sequence of an FK506 binding protein that is capable of binding to FK506 and has a molecular weight of 15,000 or more and 60,000 or less,
   (2) a polynucleotide comprising a nucleotide sequence encoding the amino acid sequence of a prolyl hydroxylase, and
   (3) a polynucleotide comprising a nucleotide sequence encoding the amino acid sequence of a glycine repeat sequence protein having the following characteristics (A) and (B):
      <characteristic (A)> the polypeptide chain of the glycine repeat sequence protein having a continuous, Gly-Xaa-Yaa repeating sequence, and
      <characteristic (B)> an imino acid (e.g. proline or hydroxyproline) being contained in the continuous, Gly-Xaa-Yaa repeating sequence of the polypeptide chain of the glycine repeat sequence protein,
   wherein Xaa and Yaa each represent any amino acid;
a second step of culturing the transformant resulting from the first step, thereby producing the glycine repeat sequence protein; and
a third step of collecting the glycine repeat sequence protein produced in the second step.
The first step can be performed by the procedures described for the transformant of the present invention as described above. The second step can be performed in similar ways to those in which the transformant of the present invention is used to produce the proteins encoded by the above-mentioned polynucleotides (1), (2) and (3).
The third step can be performed in accordance with standard biochemical procedures. In particular, the third step can be performed by purifying the glycine repeat sequence protein produced in the second step from the microbial cells and the culture supernatant.

Methods for carrying out the above-mentioned purification are not limited in particular. Purification can be effected, for example, by combining a disrupting and solubilizing step and a fractionating and refining step. Specifically, the disrupting and solubilizing step can include methods by which disruption is physically effected with ultrasound, glass beads or others; methods by which solubilization is effected using enzymes, acids, alkalis, surfactants or others; solvent extraction methods using organic solvents, buffers or others. The fractionating and refining step can include precipitation methods using salting out, solvent precipitation, isoelectric precipitation or others; column chromatographies such as ion-exchange chromatography, gel filtration chromatography, hydrophobic interaction chromatography, affinity chromatography and others, ultrafiltration, lyophilization, crystallization, dialysis and other methods. More specifically, the purification may be effected, for example, using procedures comprising the following steps (a) to (e) of:
(a) disrupting the microbial cells with glass beads;
(b) adding a protease or proteases to the resulting disrupted solution and degrading contaminated proteins;
(c) collecting the supernatant by centrifuging the disrupted solution after the degradation;
(d) salting-out and refining the protein from the collected supernatant under various pH conditions; and
(e) dissolving the precipitate collected by the salting-out, followed by desalting and removing particles.

The glycine repeat sequence protein obtained by the the process obtained by the present invention is a versatile material (for example, in which changes in the physical properties related to the hydrophilicity of glycine repeat sequence proteins which result from neutral sugars are reduced so as to exhibit more lipophilic properties that are similar to those inherently possessed by glycine repeat sequence proteins) that is more commercially valuable for pharmaceuticals, industrial products, cosmetics and foods etc. This glycine repeat sequence protein would be also preferred due to its potentiality of inducing low immunological antigenicity.

### EXAMPLES

The present invention will be described in more detail below by way of Examples, and the present invention is not limited thereto.
In methods for cloning genes and constructing plasmids, methods described in "Molecular Cloning: A Laboratory Manual 2nd edition," Cold Spring Harbor Laboratory Press (1989), ISBN 0-87969-309-6; "Current Protocols in Molecular Biology," John Wiley & Sons, Inc. (1987), ISBN 0-471-50338-X; and the like can be cited as reference. The cloning procedures and others will be described below in detail.

### Example 1 (Cloning)

(1-1) Cloning of a DNA encoding histidinol dehydrogenase (Construction of pHIS4-TOPO)

The oligonucleotides 1 and 2 mentioned below were synthesized. Komagataella pastoris NRRL Y-11430 (ATCC 76273) which was commercially available from the American Type Culture Collection (ATCC) was purchased. A DNA fragment encoding histidinol dehydrogenase (HIS4) was amplified by PCR using the oligonucleotides 1 and 2 as primers and a genomic DNA of ATCC 76273 as a template. The genomic DNA was prepared using Genomic-tip 100/G (QIAGEN) and Genomic DNA Buffer Set (QIAGEN).
The oligonucleotides 1 and 2 used were:
(a) oligonucleotide 1: GATCTCCTGATGACTGACTCACTGATAATA (SEQ ID NO:1), and
(b) oligonucleotide 2: TAATTAAATAAGTCCCAGTTTCTCCATACG (SEQ ID NO:2).
The composition of the reaction solution is given as follows:
(a) genomic DNA (15 ng/µl), 1 µl;
(b) primers (10 pmol/µl), 1.5 µl each;
(c) 10x AccuPrime Pfx reaction mix (Invitrogen), 5 µl;
(d) AccuPrime Pfx DNA polymerase (2.5 U/µl, Invitrogen), 0.5 µl;
(e) sterile distilled water 40.5 µl.
The PCR was conducted under conditions where the reaction solution was heated at 95°C for 2 minutes and then subjected to 35 cycles of denaturation at 95°C for 15 seconds, annealing at 60°C for 30 seconds, and extension at 68°C for 2.5 minutes, followed by additionally keeping the reaction solution at 68°C for 5 minutes.
An about 2.6-kb DNA fragment resulted from the PCR was isolated by agarose gel electrophoresis, followed by extraction and purification from the gel using MinElute Gel Extraction Kit (QIAGEN). The about 2.6-kb DNA fragment was ligated into the "PCR Product insertion site" of a pCR-BluntII-TOPO plasmid (Invitrogen), and the resulting ligation solution was used for transformation of E. coli (One Shot TOP10F' Chemically Competent E. coli, Invitrogen). For cloning into the plasmid, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the DNA fragment encoding HIS4 had been inserted (which may be referred to hereinafter as pHIS4-TOPO) was isolated from the cultured cells to give pHIS4-TOPO.

### (1-2) Cloning of a DNA encoding arginosuccinate lyase (Construction of pARG4-TOPO)

The oligonucleotides 3 and 4 mentioned below were synthesized. A DNA fragment encoding arginosuccinate lyase (ARG4) was amplified by PCR using the oligonucleotides 3 and 4 as primers and a genomic DNA of ATCC 76273 (see, Example 1(1-1)) as a template. The genomic DNA was prepared using Genomic-tip 100/G (QIAGEN) and Genomic DNA Buffer Set (QIAGEN).
The oligonucleotides 3 and 4 used were:
(a) oligonucleotide 3: ACGAAAATATGGTACCTGCCCTCAC (SEQ ID NO:3), and
(b) oligonucleotide 4: GTTCTATCTACCCGAGGAAACCGATACATA (SEQ ID NO:4).
The composition of the reaction solutions is given as follows:
(a) genomic DNA (15 ng/µl), 1 µl;
(b) primers (10 pmol/µl), 1.5 µl each;
(c) 10x AccuPrime Pfx reaction mix (Invitrogen), 5 µl;
(d) AccuPrime Pfx DNA polymerase (2.5 U/µl, Invitrogen), 0.5 µl;
(e) sterile distilled water 40.5 µl.
The PCR was conducted under conditions where the reaction solution was heated at 95°C for 2 minutes and then subjected to 35 cycles of denaturation at 95°C for 15 seconds, annealing at 65°C for 30 seconds, and extension at 68°C for 2.5 minutes, followed by additionally keeping the reaction solution at 68°C for 5 minutes.
An about 2.2-kb DNA fragment resulted from the PCR was isolated by agarose gel electrophoresis, followed by extraction and purification from the gel using MinElute Gel Extraction Kit (QIAGEN). The about 2.2-kb DNA fragment was ligated into the "PCR Product insertion site" of a pCR-BluntII-TOPO plasmid (Invitrogen), and the resulting ligation solution was used for transformation of E. coli (One Shot TOP10F' Chemically Competent E. coli, Invitrogen). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the DNA fragment encoding ARG4 had been inserted (which may be referred to hereinafter as pARG4-TOPO) was isolated from the cultured cells to give pARG4-TOPO.

### (1-3) Cloning of the alcohol dehydrogenase 1 promoter (Construction of pAOX1Pro+15aa-TOPO)

The oligonucleotides 5 and 6 mentioned below were synthesized. The promoter of alcohol dehydrogenase 1 (AOX1) was amplified by PCR using the oligonucleotides 5 and 6 as primers and a genomic DNA of ATCC 76273 (see, Example 1(1-1)) as a template. The genomic DNA was prepared using Genomic-tip 100/G (QIAGEN) and Genomic DNA Buffer Set (QIAGEN).
The oligonucleotides 5 and 6 used were:
(a) oligonucleotide 5: AGATCTAACATCCAAAGACGAAAGGTT (SEQ ID NO:5), and
(b) oligonucleotide 6: ATCCACCACCTAGAACTAGGATATCAAAC (SEQ ID NO:6).
The composition of the reaction solution is given as follows:
(a) genomic DNA (15 ng/µl), 1 µl;
(b) primers (10 pmol/µl), 1.5 µl each;
(c) 10x AccuPrime Pfx reaction mix (Invitrogen), 5 µl;
(d) AccuPrime Pfx DNA polymerase (2.5 U/µl, Invitrogen), 0.5 µl;
(e) sterile distilled water 40.5 µl.
The PCR was conducted under conditions where the reaction solution was heated at 95°C for 2 minutes and then subjected to 35 cycles of denaturation at 95°C for 15 seconds, annealing at 62°C for 30 seconds, and extension at 68°C for 1 minute, followed by additionally keeping the reaction solution at 68°C for 5 minutes.
An about 1.0-kb DNA fragment resulted from the PCR was isolated by agarose gel electrophoresis, followed by extraction and purification from the gel using MinElute Gel Extraction Kit (QIAGEN). The about 1.0-kb DNA fragment was ligated into the "PCR Product insertion site" of a pCR-BluntII-TOPO plasmid (Invitrogen), and the resulting ligation solution was used for transformation of E. coli (One Shot TOP10F' Chemically Competent E. coli, Invitrogen). For the ligation reaction, a Zero-Blunt TOPO PCR cloning kit (Invitrogen) was used.
On LB agar medium containing 50 µg/ml of kanamycin, the E. coli cells which had been subjected transformation were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the AOX1 promoter had been inserted (which may be referred to hereinafter as pAOX1Pro+15aa-TOPO) was isolated from the cultured cells to give pARG4pAOX1Pro+15aa-TOPO.

### (1-4) Cloning of the peroxisome matrix protein promoter (Construction of pCR-BII-PER3 Pro SacII-Psp1406I(+))

The oligonucleotides 7 and 8 mentioned below were synthesized. The promoter of peroxisome matrix protein (PER3) was amplified by PCR using the oligonucleotides 7 and 8 as primers and a genomic DNA of ATCC 76273 (see, Example 1(1-1)) as a template. The genomic DNA was prepared using Genomic-tip 100/G (QIAGEN) and Genomic DNA Buffer Set (QIAGEN).
The oligonucleotides 7 and 8 used were:
(a) oligonucleotide 7: CTAAGGGTCTCACTGGTGTTTCAGC (SEQ ID NO:7), and
(b) oligonucleotide 8: AGTTCCTTGCAACTGTAGTGGTCG (SEQ ID NO:8).
The composition of the reaction solutions is given as follows:
(a) genomic DNA (15 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl (1 U);
(g) sterile distilled water 33 µl.
The PCR was conducted under conditions where the reaction solution was heated at 94°C for 2 minutes and then subjected to 30 cycles of denaturation at 94°C for 15 seconds, and each of annealing and extension at 68°C for 70 seconds, followed by additionally keeping the reaction solution at 68°C for 70 seconds.
An about 1.1-kb DNA fragment resulted from the PCR was isolated by agarose gel electrophoresis, followed by extraction and purification from the gel using MinElute Gel Extraction Kit (QIAGEN). The about 1.1-kb DNA fragment was ligated into the "PCR Product insertion site" of a pCR-BluntII-TOPO plasmid (Invitrogen), and the resulting ligation solution was used for transformation of E. coli (Competent high DH5α, Toyobo Co., Ltd.). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the PER3 promoter had been inserted (which may be referred to hereinafter as pCR-BII-PER3 Pro(+)) was isolated from the cultured cells to give pCR-BII-PER3 Pro(+).
The oligonucleotides 9 and 10 mentioned below were synthesized. A DNA fragment which had a restriction enzyme recognition sequence added at each end of the PER3 promoter was amplified by PCR using the oligonucleotides 9 and 10 as primers and the pCR-BII-PER3 Pro(+) plasmid as a template.
The oligonucleotides 9 and 10 used were:
(a) oligonucleotide 9: AACCGCGGCTCGTCACTATCGTCGTTG (SEQ ID NO:9), and
(b) oligonucleotide 10: CGAACGTTACCTGAAGATAGGTAAAAAAAAATTGC (SEQ ID NO:10).
The composition of the reaction solutions is given as follows:
(a) plasmid solution (1 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase(1 U/µl, Toyobo Co., Ltd.), 1 µl (1 U);
(g) sterile distilled water 33 µl.
The PCR was conducted under conditions where the reaction solution was heated at 94°C for 2 minutes and then subjected to 5 cycles of denaturation at 94°C for 15 seconds, annealing at 60°C for 30 seconds, and extension at 68°C for 1 minute and then 20 cycles of denaturation at 94°C for 15 seconds, and each of annealing and extension at 68°C for 1 minute, followed by additionally keeping the reaction solution at 68°C for 1 minutes.
An about 1.0-kb DNA fragment resulted from the PCR was isolated by agarose gel electrophoresis, followed by extraction and purification from the gel using MinElute Gel Extraction Kit (QIAGEN). The about 1.0-kb DNA fragment was ligated into the "PCR Product insertion site" of a pCR-BluntII-TOPO plasmid (Invitrogen), and the resulting ligation solution was used for transformation of E. coli (Competent high DH5α, Toyobo Co., Ltd.). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the PER3 promoter had been inserted (which may be referred to hereinafter as pCR-BII-PER3 Pro SacII-Psp1406I(+)) was isolated from the cultured cells to give pCR-BII-PER3 Pro SacII-Psp1406I(+).

### (1-5) Cloning of the alcohol dehydrogenase 2 promoter (Construction of pCR-BII-AOX2 Pro SacII-Psp1406I(-))

The oligonucleotides 11 and 12 mentioned below were synthesized. A DNA fragment which had a restriction enzyme recognition sequence added at each end of the alcohol dehydrogenase 2 (AOX2) promoter was amplified by PCR using the oligonucleotides 11 and 12 as primers and a genomic DNA of ATCC 76273 (see, Example 1(1-1)) as a template. The genomic DNA was prepared using Genomic-tip 100/G (QIAGEN) and Genomic DNA Buffer Set (QIAGEN).
The oligonucleotides 11 and 12 used were:
(a) oligonucleotide 11: AACCGCGGCTAGTAGAACTTTGACATCTGCTA (SEQ ID NO:11), and
(b) oligonucleotide 12: CGAACGTTTTGATTTGTTTGTGGGGATTTAG (SEQ ID NO:12).
The composition of the reaction solutions is given as follows:
(a) genomic DNA (15 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl (1 U);
(g) sterile distilled water 33 µl.
The PCR was conducted under conditions where the reaction solution was heated at 94°C for 2 minutes and then subjected to 5 cycles of denaturation at 94°C for 15 seconds, annealing at 60°C for 30 seconds, and extension at 68°C for 70 seconds and then 30 cycles of denaturation at 94°C for 15 seconds, and each of annealing and extension at 68°C for 70 seconds, followed by additionally keeping the reaction solution at 68°C for 70 seconds.
An about 1.1-kb DNA fragment resulted from the PCR was isolated by agarose gel electrophoresis, followed by extraction and purification from the gel using MinElute Gel Extraction Kit (QIAGEN). The about 1.1-kb DNA fragment was ligated into the "PCR Product insertion site" of a pCR-BluntII-TOPO plasmid (Invitrogen), and the resulting ligation solution was used for transformation of E. coli (Competent high DH5α, Toyobo Co., Ltd.). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the AOX2 promoter had been inserted (which may be referred to hereinafter as pCR-BII-AOX2 Pro SacII-Psp1406I(-)) was isolated from the cultured cells to give pCR-BII-AOX2 Pro SacII-Psp1406I(-).

### (1-6) Cloning of the formaldehyde dehydrogenase promoter (Construction of pCR-BII-FLD1 Pro SacII-Psp1406I(-))

The oligonucleotides 13 and 14 mentioned below were synthesized. The promoter of formaldehyde dehydrogenase (FLD1) was amplified by PCR using the oligonucleotides 13 and 14 as primers and a genomic DNA of ATCC 76273 (see, Example 1(1-1)) as a template. The genomic DNA was prepared using Genomic-tip 100/G (QIAGEN) and Genomic DNA Buffer Set (QIAGEN).
The oligonucleotides 13 and 14 used were:
(a) oligonucleotide 13: GCAGTGTTGGCTAACGTCTATTCG (SEQ ID NO:13), and
(b) oligonucleotide 14: ACTTCATGGGCTCTTGGAGGAAG (SEQ ID NO:14).
The composition of the reaction solutions is given as follows:
(a) genomic DNA (15 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl (1 U);
(g) sterile distilled water 33 µl.
The PCR was conducted under conditions where the reaction solution was heated at 94°C for 2 minutes and then subjected to 30 cycles of denaturation at 94°C for 15 seconds, and each of annealing and extension at 68°C for 70 seconds, followed by additionally keeping the reaction solution at 68°C for 70 seconds.
An about 1.3-kb DNA fragment resulted from the PCR was isolated by agarose gel electrophoresis, followed by extraction and purification from the gel using MinElute Gel Extraction Kit (QIAGEN). The about 1.3-kb DNA fragment was ligated into the "PCR Product insertion site" of a pCR-BluntII-TOPO plasmid (Invitrogen), and the resulting ligation solution was used for transformation of E. coli (Competent high DH5α, Toyobo Co., Ltd.). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the FLD1 promoter had been inserted (which may be referred to hereinafter as pCR-BII-FLD1 Pro(+)) was isolated from the cultured cells to give pCR-BII-FLD1 Pro(+).
The oligonucleotides 15 and 16 mentioned below were synthesized. A DNA fragment which had a restriction enzyme recognition sequence added at each end of the FLD1 promoter was amplified by PCR using the oligonucleotides 15 and 16 as primers and the pCR-BII-FLD1 Pro(+) plasmid as a template.
The oligonucleotides 15 and 16 used were:
(a) oligonucleotide 15: AACCGCGGCCTGAATACCGTAACATAGTGAC (SEQ ID NO:15), and
(b) oligonucleotide 16: CGAACGTTTCAAGAATTGTATGAACAAGCAAAG (SEQ ID NO:16).
The composition of the reaction solutions is given as follows:
(a) plasmid solution (1 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl (1 U);
(g) sterile distilled water 33 µl.
The PCR was conducted under conditions where the reaction solution was heated at 94°C for 2 minutes and then subjected to 5 cycles of denaturation at 94°C for 15 seconds, annealing at 60°C for 30 seconds, and extension at 68°C for 1 minute and then 20 cycles of denaturation at 94°C for 15 seconds, and each of annealing and extension at 68°C for 1 minute, followed by additionally keeping the reaction solution at 68°C for 1 minutes.
An about 0.9-kb DNA fragment resulted from the PCR was isolated by agarose gel electrophoresis, followed by extraction and purification from the gel using MinElute Gel Extraction Kit (QIAGEN). The about 0.9-kb DNA fragment was ligated into the "PCR Product insertion site" of a pCR-BluntII-TOPO plasmid (Invitrogen), and the resulting ligation solution was used for transformation of E. coli (Competent high DH5α, Toyobo Co., Ltd.). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the FLD1 promoter had been inserted (which may be referred to hereinafter as pCR-BII-FLD1 Pro SacII-Psp1406I(-)) was isolated from the cultured cells to give pCR-BII-FLD1 Pro SacII-Psp1406I(-).

### (1-7) Cloning of the alcohol dehydrogenase 1 terminator (Construction of pAOX1 term-TOPO)

The oligonucleotides 17 and 18 mentioned below were synthesized. The terminator of alcohol dehydrogenase 1 (AOX1) was amplified by PCR using the oligonucleotides 17 and 18 as primers and a genomic DNA of ATCC 76273 (see, Example 1(1-1)) as a template. The genomic DNA was prepared using Genomic-tip 100/G (QIAGEN) and Genomic DNA Buffer Set (QIAGEN).
The oligonucleotides 17 and 18 used were:
(a) oligonucleotide 17: CCTTAGACATGACTGTTCCTCAGTTC (SEQ ID NO:17), and
(b) oligonucleotide 18: GCACAAACGAACGTCTCACTTAAT (SEQ ID NO:18).
The composition of the reaction solutions is given as follows:
(a) genomic DNA (15 ng/µl), 1 µl;
(b) primers (10 pmol/µl), 1.5 µl each;
(c) 10x AccuPrime Pfx reaction mix (Invitrogen), 5 µl;
(d) AccuPrime Pfx DNA polymerase (2.5 U/µl, Invitrogen), 0.5 µl;
(e) sterile distilled water 40.5 µl.
The PCR was conducted under conditions where the reaction solution was heated at 95°C for 2 minutes and then subjected to 35 cycles of denaturation at 95°C for 15 seconds, annealing at 60°C for 30 seconds, and extension at 68°C for 30 seconds, followed by additionally keeping the reaction solution at 68°C for 5 minutes.
An about 0.3-kb DNA fragment resulted from the PCR was isolated by agarose gel electrophoresis, followed by extraction and purification from the gel using MinElute Gel Extraction Kit (QIAGEN). The about 0.3-kb DNA fragment was ligated into the "PCR Product insertion site" of a pCR-BluntII-TOPO plasmid (Invitrogen), and the resulting ligation solution was used for transformation of E. coli (One Shot TOP10F' Chemically Competent E. coli, Invitrogen). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the AOX1 terminator had been inserted (which may be referred to hereinafter as pAOX1 term-TOPO) was isolated from the cultured cells to give pAOX1 term-TOPO.

### (1-8) Cloning of a 3'-downstream non-coding region of the alcohol dehydrogenase 1 gene (Construction of pAOX1-3'-TOPO)

The oligonucleotides 19 and 20 mentioned below were synthesized. A 3'-downstream non-coding region of the alcohol dehydrogenase 1 (AOX1) gene was amplified by PCR using the oligonucleotides 19 and 20 as primers and a genomic DNA of ATCC 76273 (see, Example 1(1-1)) as a template. The genomic DNA was prepared using Genomic-tip 100/G (QIAGEN) and Genomic DNA Buffer Set (QIAGEN).
The oligonucleotides 19 and 20 used were:
(a) oligonucleotide 19: TCGAGTATCTATGATTGGAAGTATGGGAAT (SEQ ID NO:19), and
(b) oligonucleotide 20: GATCTTGAGATAAATTTCACGTTTAAAATC (SEQ ID NO:20).
The composition of the reaction solutions is given as follows:
(a) genomic DNA (15 ng/µl), 1 µl;
(b) primers (10 pmol/µl), 1.5 µl each;
(c) 10x AccuPrime Pfx reaction mix (Invitrogen), 5 µl;
(d) AccuPrime Pfx DNA polymerase (2.5 U/µl, Invitrogen), 0.5 µl;
(e) sterile distilled water 40.5 µl.
The PCR was conducted under conditions where the reaction solution was heated at 94°C for 2 minutes and then subjected to 35 cycles of denaturation at 95°C for 15 seconds, annealing at 58°C for 30 seconds, and extension at 68°C for 50 seconds, followed by additionally keeping the reaction solution at 68°C for 5 minutes.
An about 0.8-kb DNA fragment resulted from the PCR was isolated by agarose gel electrophoresis, followed by extraction and purification from the gel using MinElute Gel Extraction Kit (QIAGEN). The about 0.8-kb DNA fragment was ligated into the "PCR Product insertion site" of a pCR-BluntII-TOPO plasmid (Invitrogen), and the resulting ligation solution was used for transformation of E. coli (One Shot TOP10F' Chemically Competent E. coli, Invitrogen). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the 3'-downstream non-coding region of AOX1 had been inserted (which may be referred to hereinafter as pAOX1-3'-TOPO) was isolated from the cultured cells to give pAOX1-3'-TOPO.

### (1-9) Cloning of a DNA encoding α factor (Construction of pαfactor-TOPO)

The oligonucleotides 21 and 22 mentioned below were synthesized. Saccharomyces cerevisiae S288C (NBRC 1136) which was commercially available from the National Institute of Technology and Evaluation (NITE) was purchased. A DNA fragment encoding a factor was amplified by PCR using the oligonucleotides 21 and 22 as primers and a genomic DNA of NBRC 1136 as a template. The genomic DNA was prepared using Genomic-tip 100/G (QIAGEN) and Genomic DNA Buffer Set (QIAGEN).
The oligonucleotides 21 and 22 used were:
(a) oligonucleotide 21: TCAAACAAGAAGATTACAAACTATCAATTTCA (SEQ ID NO:21), and
(b) oligonucleotide 22: GTACGAGCTAAAAGTACAGTGGGAACAAA (SEQ ID NO:22).
The composition of the reaction solutions is given as follows:
(a) genomic DNA (15 ng/µl), 1 µl;
(b) dNTPs (mix, 2 mM each), 5 µl;
(c) MgS0₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl (1 U);
(g) sterile distilled water 33 µl.
The PCR was conducted under conditions where the reaction solution was heated at 94°C for 3 minutes and then subjected to 10 cycles of denaturation at 94°C for 15 seconds, annealing at 60°C for 30 seconds, and extension at 68°C for 1 minute and then 25 cycles of denaturation at 94°C for 15 seconds, and each of annealing and extension at 68°C for 1 minute, followed by additionally keeping the reaction solution at 68°C for 5 minutes.
An about 0.6-kb DNA fragment resulted from the PCR was isolated by agarose gel electrophoresis, followed by extraction and purification from the gel using MinElute Gel Extraction Kit (QIAGEN). The about 0.6-kb DNA fragment was ligated into the "PCR Product insertion site" of a pCR-BluntII-TOPO plasmid (Invitrogen), and the resulting ligation solution was used for transformation of E. coli (One Shot TOP10F' Chemically Competent E. coli, Invitrogen). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the DNA fragment encoding α factor had been inserted (which may be referred to hereinafter as pa.factor-TOPO) was isolated from the cultured cells to give pαfactor-TOPO.

### (1-10) Cloning of a DNA encoding human collagen Type I α1 (Construction of pBlue-HsCOL1A1)

The oligonucleotides 23 and 24 mentioned below were synthesized. To these oligonucleotides, a phosphate group was added at their 5' end using T4 polynucleotide kinase (Toyobo Co., Ltd.). Then, a DNA fragment encoding human collagen Type I α1 was amplified by PCR using the phosphorylated oligonucleotides 23 and 24 as primers and Human brain cDNAs (Toyobo Co., Ltd.) as a template.
The oligonucleotides 23 and 24 used were:
(a) oligonucleotide 23: CAGCCACAAAGAGTCTACATGTCTAGG (SEQ ID NO:23), and
(b) oligonucleotide 24: AGGTTGGGATGGAGGGAGTT (SEQ ID NO:24).
The composition of the reaction solutions is given as follows:
(a) cDNA solution, 5 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD -plus- (Toyobo Co., Ltd.), 5 µl;
(f) KOD-plus- DNA polymerase (1 U/µl, Toyobo Co., Ltd.), 1 µl;
(g) sterile distilled water 29 µl.
The PCR was conducted under conditions where the reaction solution was heated at 94°C for 2 minutes and then subjected to 35 reaction cycles of denaturation at 98°C for 20 seconds, annealing at 58°C for 10 seconds, and extension at 74°C for 5 minutes.
An about 4.5-kb DNA fragment resulted from the PCR was purified using MagExtractor PCR&Gel cleanup (Toyobo Co., Ltd.).
A pBluescriptII KS(+) plasmid (Stratagene) was digested with a restriction enzyme EcoRV, dephosphorylated with an alkaline phosphatase, and then purified using MagExtractor PCR&Gel cleanup (Toyobo Co., Ltd.).
The about 4.5-kb DNA fragment and the dephosphorylated plasmid were ligated, and the resulting ligation solution was used for transformation of E. coli (Competent high DH5α, Toyobo Co., Ltd.). For the ligation reaction, a Ligation high (Toyobo Co., Ltd.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using MagExtractor plasmid (Toyobo Co., Ltd.), the plasmid into which the DNA fragment encoding human collagen Type I α1 had been inserted (which may be referred to hereinafter as pBlue-HsCOL1A1) was isolated from the cultured cells to give pBlue-HsCOL1A1.

### (1-11) Cloning of a DNA encoding human collagen Type I α2 (Construction of pUC18-HsCOL1A2)

Total RNA derived from Human Neonatal Dermal Fibroblasts was obtained from Cell Applications, Inc. CDNAs were synthesized using ReverTra Plus (Toyobo Co., Ltd.). The oligonucleotides 25 and 26 mentioned below were synthesized. A DNA fragment encoding human collagen Type I α2 was amplified by PCR using the oligonucleotides 25 and 26 as primers and the synthesized cDNAs as a template.
The oligonucleotides 25 and 26 used were:
(a) oligonucleotide 25: GCCAAGCTTGCATGCTCAGCTTTGTGGATACGCGGAC (SEQ ID NO:25), and
(b) oligonucleotide 26:
   CGGTACCCGGGGATCCTTATTTGAAACAGACTGGGCCAATGTCC (SEQ ID NO:26).
The composition of the reaction solutions is given as follows:
(a) cDNA solution, 5 µl;
(b) dNTPs (mix, 2 mM each), 5 µl;
(c) MgSO₄(25 mM), 2 µl;
(d) primers (10 pmol/µl), 1.5 µl each;
(e) 10x PCR buffer for KOD -plus- (Toyobo Co., Ltd.), 5 µl;
(f) KOD-plus- DNA polymerase (1 U/µl, Toyobo Co., Ltd.), 1 µl;
(g) sterile distilled water 29 µl.
The PCR was conducted under conditions where the reaction solution was heated at 94°C for 2 minutes and then subjected to 30 reaction cycles of denaturation at 98°C for 10 seconds, and each of annealing and extension at 68°C for 6 minutes. An about 4.1-kb DNA fragment amplified was isolated by agarose gel electrophoresis, followed by extraction and purification from the gel using MagExtractor PCR&Gel cleanup(Toyobo Co., Ltd.). The obtained DNA was digested with restriction enzymes SphI and BamHI, and then purified using MagExtractor PCR&Gel cleanup (Toyobo Co., Ltd.).
A pUC18 plasmid (Toyobo Co., Ltd.) was digested with restriction enzymes SphI and BamHI, and then purified using MagExtractor PCR&Gel cleanup (Toyobo Co., Ltd.).
The about 4.1-kb DNA fragment and the digested plasmid were ligated, and the resulting ligation solution was used for transformation of E. coli (Competent high DH5α, Toyobo Co., Ltd.). For the ligation reaction, a Ligation high (Toyobo Co., Ltd.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using MagExtractor plasmid (Toyobo Co., Ltd.), the plasmid into which the DNA fragment encoding human collagen Type I α2 had been inserted (which may be referred to hereinafter as pUC18-HsCOL1A2) was isolated from the cultured cells to give pUC18-HsCOL1A2.

### (1-12) Cloning of a DNA encoding human collagen Type III α1 (Construction of pUC19-HsCOL3A1)

The oligonucleotides 27 and 28 mentioned below were synthesized. To these oligonucleotides, a phosphate group was added at their 5' ends using T4 polynucleotide kinase (Toyobo Co., Ltd.). Then, a DNA fragment encoding human collagen Type III α1 was amplified by PCR using the phosphorylated oligonucleotides 27 and 28 as primers and Human brain cDNAs (Toyobo Co., Ltd.) as a template.
The oligonucleotides 27 and 28 used were:
(a) oligonucleotide 27: GGCTGAGTTTTATGACGGGC (SEQ ID NO:27), and
(b) oligonucleotide 28: GACAAGATTAGAACAAGAGG (SEQ ID NO:28).
The composition of the reaction solutions is given as follows:
(a) cDNA solution, 5 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD -plus- (Toyobo Co., Ltd.), 5 µl;
(f) KOD-plus- DNA polymerase (1 U/µl, Toyobo Co., Ltd.), 1 µl;
(g) sterile distilled water 29 µl.
The PCR was conducted under conditions where the reaction solution was heated at 94°C for 2 minutes and then subjected to 35 reaction cycles of denaturation at 98°C for 20 seconds, annealing at 58°C for 10 seconds, and extension at 74°C for 5 minutes.
An about 4.6-kb DNA fragment resulted from the PCR was purified using MagExtractor PCR&Gel cleanup (Toyobo Co., Ltd.).
A pUC19 plasmid (Toyobo Co., Ltd.) was digested with a restriction enzyme SmaI, purified using MagExtractor PCR&Gel cleanup (Toyobo Co., Ltd.), and then dephosphorylated with an alkaline phosphatase, and purified again using MagExtractor PCR&Gel cleanup (Toyobo Co., Ltd.).
The about 4.6-kb DNA fragment and the dephosphorylated plasmid were ligated, and the resulting ligation solution was used for transformation of E. coli (Competent high DH5α, Toyobo Co., Ltd.). For the ligation reaction, a Ligation high (Toyobo Co., Ltd.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using MagExtractor plasmid (Toyobo Co., Ltd.), the plasmid into which the DNA fragment encoding human collagen Type I α1 had been inserted (which may be referred to hereinafter as pUC19-HsCOL3A1) was isolated from the cultured cells to give pUC19-HsCOL3A1.

### (1-13) Preparation and cloning of a Zeocyn-resistance cassette (Construction of pUC57-ZeoR)

A Zeocyn-resistance cassette indicated by SEQ ID NO:112 was prepared by an assembly PCR method. The DNA fragment prepared was inserted into the EcoRV site of a pUC57 plasmid to be cloned. The resulting plasmid is referred to as pUC57-ZeoR.

### (1-14) Preparation and cloning of a synthetic DNA encoding an about 13-kDa FK506-binding protein (Construction of pUC57-YFKBP13A)

A DNA fragment encoding an about 13-kDa FK506-binding protein (FKBP13A) indicated by SEQ ID NO:113 was prepared by an assembly PCR method. The synthetic DNA fragment prepared was inserted into the EcoRV site of a pUC57 plasmid to be cloned. The resulting plasmid is referred to as pUC57-YFKBP13A.

### (1-15) Preparation and cloning of a synthetic DNA encoding an about 19-kDa FK506-binding protein (Construction of pUC57-YFKBP19)

A DNA fragment encoding an about 19-kDa FK506-binding protein (FKBP19) indicated by SEQ ID NO:78 was prepared by an assembly PCR method. The synthetic DNA fragment prepared was inserted into the EcoRV site of a pUC57 plasmid to be cloned. The resulting plasmid is referred to as pUC57-YFKBP19.

### (1-16) Preparation and cloning of a synthetic DNA encoding an about 23-kDa FK506-binding protein (Construction of pUC57-YFKBP23)

A synthetic DNA fragment encoding an about 23-kDa FK506-binding protein (FKBP23) indicated by SEQ ID NO:76 was prepared by an assembly PCR method. The synthetic DNA fragment prepared was inserted into the EcoRV site of a pUC57 plasmid to be cloned. The resulting plasmid is referred to as pUC57-YFKBP23.

### (1-17) Preparation and cloning of a synthetic DNA encoding an about 63-kDa FK506-binding protein (Construction of pUC57-YFKBP63)

A synthetic DNA fragment encoding an about 63-kDa FK506-binding protein (FKBP63) indicated by SEQ ID NO:114 was prepared by an assembly PCR method. The synthetic DNA fragment prepared was inserted into the EcoRV site of a pUC57 plasmid to be cloned. The resulting plasmid is referred to as pUC57-YFKBP63.

### (1-18) Preparation and cloning of a synthetic DNA encoding an about 65-kDa FK506-binding protein (Construction of pUC57-YFKBP65)

A synthetic DNA fragment encoding an about 65-kDa FK506-binding protein (FKBP65) indicated by SEQ ID NO:115 was prepared by an assembly PCR method. The synthetic DNA fragment prepared was inserted into the EcoRV site of a pUC57 plasmid to be cloned. The resulting plasmid is referred to as pUC57-YFKBP65.

### (1-19) Preparation and cloning of a synthetic DNA encoding human Type I α1 collagen (Construction of pUC57-YHsCOL1A1)

A synthetic DNA fragment encoding human Type I α1 collagen indicated by SEQ ID NO:98 was prepared by an assembly PCR method. The synthetic DNA fragment prepared was inserted into the EcoRV site of a pUC57 plasmid to be cloned. The resulting plasmid is referred to as pUC57-YHsCOL1A1.

### (1-20) Preparation and cloning of a synthetic DNA encoding human Type I α2 collagen (Construction of pUC57-YHsCOL1A2)

A synthetic DNA fragment encoding human Type I α2 collagen indicated by SEQ ID NO:100 was prepared by an assembly PCR method. The synthetic DNA fragment prepared was inserted into the EcoRV site of a pUC57 plasmid to be cloned. The resulting plasmid is referred to as pUC57-YHsCOL1A2.

### (1-21) Preparation and cloning of a synthetic DNA encoding human Type II α1 collagen (Construction of pUC57-YHsCOL2A1)

A synthetic DNA fragment encoding human Type II α1 collagen indicated by SEQ ID NO:102 was prepared by an assembly PCR method. The synthetic DNA fragment prepared was inserted into the EcoRV site of a pUC57 plasmid to be cloned. The resulting plasmid is referred to as pUC57-YHsCOL2A1.

### Example 2 (Construction of plasmids for introducing expression cassettes)

(2-1) Preparation of a plasmid (pEXP-A-P4HBsig(-)Alrev) for introducing an expression cassette for a prolyl 4-hydroxylase α1 subunit (P4Hα1) and an expression cassette for a prolyl 4-hydroxylase β subunit (P4Hβ)

### (2-1-1) Construction of pSN003

The oligonucleotides 29 and 30 mentioned below were synthesized. A DNA fragment which had a restriction enzyme recognition sequence added at each end of the AOX1 terminator was amplified by PCR using the oligonucleotides 29 and 30 as primers and the plasmid named as pAOX1Term-TOPO (see, Example (1-7)) as a template.
The oligonucleotides 29 and 30 used were:
(a) oligonucleotide 29: TCGACTAGTTTAGACATGACTGTTCCTCAGTTCAA (SEQ ID NO:29), and
(b) oligonucleotide 30: AACTGCAGGCACAAACGAACGTCTCACTTA (SEQ ID NO:30).
The composition of the reaction solutions is given as follows:
(a) plasmid solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl (1 U);
(g) sterile distilled water 33 µl.
The PCR was conducted under conditions where the reaction solution was heated at 94°C for 2 minutes and then subjected to 25 cycles of denaturation at 94°C for 15 seconds, annealing at 60°C for 30 seconds, and extension at 68°C for 1 minute, followed by additionally keeping the reaction solution at 68°C for 5 minutes.
An about 0.3-kb DNA fragment resulted from the PCR was digested with restriction enzymes SpeI and PstI, and then isolated by agarose gel electrophoresis, followed by extraction and purification from the gel using MinElute Gel Extraction Kit (QIAGEN).
A pBluescriptII KS(+) plasmid (Stratagene) was digested with restriction enzymes SpeI and PstI, and then isolated by agarose gel electrophoresis, followed by extraction and purification from the gel using MinElute Gel Extraction Kit (QIAGEN).
The about 0.3-kb DNA fragment and the digested plasmid were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA having the restriction enzyme recognition sequence added at each end of the AOX1 terminator had been inserted (which may be referred to hereinafter as pSN003) was isolated from the cultured cells to give pSN003.

### (2-1-2) Construction of pSN004

The plasmid named as pAOX1Pro+15aa-TOPO (see, Example (1-3)) was digested with a restriction enzyme Eco52I. Then, an about 1.0-kb DNA fragment comprising the AOX1 promoter was isolated by agarose gel electrophoresis, followed by extraction and purification from the gel using MinElute Gel Extraction Kit (QIAGEN).
The plasmid named as pSN003 (see, Example (2-1-1)) was digested with a restriction enzyme Eco52I, dephosphorylated with an alkaline phosphatase, and then purified using MinElute Reaction Cleanup Kit (QIAGEN).
The about 1.0-kb DNA fragment and the dephosphorylated plasmid were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the about 1.0-kb DNA fragment comprising the AOX1 promoter had been inserted (which may be referred to hereinafter as pSN004) was isolated from the cultured cells to give pSN004.

### (2-1-3) Construction of pSN005

The oligonucleotides 31 and 32 mentioned below were synthesized:
(a) oligonucleotide 31: TATTCGAAACGCATATGTGACCGGCAGACTAGTGG (SEQ ID NO:31), and
(b) oligonucleotide 32: CCACTAGTCTGCCGGTCACATATGGGTTTCGAATA (SEQ ID NO:32).
A solution having the composition mentioned below was prepared and kept at 98°C for 5 minutes, at 50°C for 50 minutes, and then at 37°C for 1 hour:
(a) oligonucleotide 31 (50 pmol/µl), 5 µl;
(b) oligonucleotide 32 (50 pmol/µl), 5 µl;
(c) Tris-HCl (100 mM), 10 µl;
(d) MgCl₂ (100 mM), 10 µl;
(e) dithiothreitol (10 mM), 10 µl;
(f) sterile distilled water 60 µl.
A DNA linker in which the oligonucleotides 31 and 32 had been annealed was digested with restriction enzymes BspT104I and SpeI. Next, the mixture solution was extracted with phenol:chloroform:isoamyl alcohol (25:24:1) and then subjected to ethanol precipitation to purify the DNA linker (Linker 1).
Additionally, the plasmid named as pSN004 (see, Example (2-1-2)) was digested with restriction enzymes BspT104I and SpeI. An about 4.3-kb DNA fragment was separated and purified by agarose gel electrophoresis.
The DNA linker (Linker 1) and the about 4.3-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA linker (Linker 1) had been inserted (which may be referred to hereinafter as pSN005) was isolated from the cultured cells to give pSN005.

### (2-1-4) Construction of pSN015

The oligonucleotides 33 and 34 mentioned below were synthesized. A DNA fragment which had a restriction enzyme recognition sequence added at each end of the DNA encoding the signal sequence and the pro sequence of the α factor gene was amplified by PCR using the oligonucleotides 33 and 34 as primers and the plasmid named as pafactor-TOPO (see, Example (1-9)) as a template.
The oligonucleotides 33 and 34 used were:
(a) oligonucleotide 33: GGTTCGAAACGATGAGATTTCCTTCAATTTTTACT (SEQ ID NO:33), and
(b) oligonucleotide 34: TCGACTAGTAGCTTCAGCCTCTCTTTTATCC (SEQ ID NO:34).
The composition of the reaction solutions is given as follows:
(a) plasmid solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 3 minutes and then subjected to 10 cycles of denaturation at 94°C for 15 seconds, annealing at 60°C for 30 seconds, and extension at 68°C for 1 minute and then 15 cycles of denaturation at 94°C for 15 seconds, and each of annealing and extension at 68°C for 1 minute, followed by additionally keeping the reaction solution at 68°C for 5 minutes.
An about 0.3-kb DNA fragment resulted from the PCR was digested with restriction enzymes BspT104I and SpeI, and then isolated by agarose gel electrophoresis, followed by extraction and purification from the gel using MinElute Gel Extraction Kit (QIAGEN).
The plasmid named as pSN005 (see, Example (2-1-3)) was digested with restriction enzymes BspT104I and SpeI. Then, an about 4.2-kb DNA fragment was separated by agarose gel electrophoresis, followed by extraction and purification from the gel using MinElute Gel Extraction Kit (QIAGEN).
The about 0.3-kb DNA fragment and the about 4.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA having the restriction enzyme recognition sequence added at each end of the DNA encoding the signal sequence and the pro sequence of the α factor gene had been inserted (which may be referred to hereinafter as pSN015) was isolated from the cultured cells to give pSN015.

### (2-1-5) Construction of pSN020

A cDNA clone encoding human prolyl 4-hydroxylase subunit (P4Hβ) (Clone ID: 3848651) was purchased from Invitrogen. The oligonucleotides 35 and 36 mentioned below were synthesized. A DNA fragment which had a restriction enzyme recognition sequence added at each end of the DNA encoding a P4Hβ having no signal sequence was amplified by PCR using the oligonucleotides 35 and 36 as primers and the P4Hβ cDNA clone as a template.
The oligonucleotides 35 and 36 used were:
(a) oligonucleotide 35: TTACTAGTGACGCCCCCGAGGAGGA (SEQ ID NO:35), and
(b) oligonucleotide 36: TTACTAGTTTACAGTTCATCTTTCACAGCTTTCTG (SEQ ID NO:36).
The composition of the reaction solutions is given as follows:
(a) plasmid solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 3 minutes and then subjected to 10 cycles of denaturation at 94°C for 15 seconds, annealing at 60°C for 30 seconds, and extension at 68°C for 2 minutes and then 15 cycles of denaturation at 94°C for 15 seconds, and each of annealing and extension at 68°C for 2 minutes followed by additionally keeping the reaction solution at 68°C for 5 minutes.
An about 1.5-kb DNA fragment resulted from the PCR was digested with a restriction enzyme SpeI, and then isolated by agarose gel electrophoresis, followed by extraction and purification from the gel using MinElute Gel Extraction Kit (QIAGEN).
The plasmid named as pSN015 (see, Example (2-1-4)) was digested with a restriction enzyme SpeI, dephosphorylated with an alkaline phosphatase, and then purified using MinElute Reaction Cleanup Kit (QIAGEN).
The about 1.5-kb DNA fragment and the dephosphorylated plasmid were ligated, and the resulting ligation solution was used for transformation of E. coli (Competent high JM109, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequence added at each end of the DNA encoding the P4Hβ having no signal sequence had been inserted (which may be referred to hereinafter as pSN020) was isolated from the cultured cells to give pSN020.

### (2-1-6) Construction of pP4Hbsig(-)rev-TOPO

The oligonucleotides 37 and 38 mentioned below were synthesized. A DNA fragment which had a restriction enzyme recognition sequence added at each end of the expression cassette for a prolyl 4-hydroxylase β subunit having the α-factor signal and pro sequences was amplified by PCR using the oligonucleotides 37 and 38 as primers and the plasmid named as pSN020 (see, Example (2-1-5)) as a template.
The oligonucleotides 37 and 38 used were:
(a) oligonucleotide 37: AACCGCGGTCTAACATCCAAAGACGAAAGGTTGAA (SEQ ID NO:37), and
(b) oligonucleotide 38: AACCCGGGGCACAAACGAACGTCTCACTTAATCTT (SEQ ID NO:38).
The composition of the reaction solutions is given as follows:
(a) plasmid solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 3 minutes and then subjected to 10 cycles of denaturation at 94°C for 15 seconds, annealing at 60°C for 30 seconds, and extension at 68°C for 3.5 minutes and then 15 cycles of denaturation at 94°C for 15 seconds, and each of annealing and extension at 68°C for 3.5 minutes, followed by additionally keeping the reaction solution at 68°C for 5 minutes.
An about 3.0-kb DNA fragment resulted from the PCR was isolated by agarose gel electrophoresis, followed by extraction and purification from the gel using MinElute Gel Extraction Kit (QIAGEN). The about 3.0-kb DNA fragment purified was ligated into the "PCR Product insertion site" of a pCR-BluntII-TOPO plasmid (Invitrogen), and the resulting ligation solution was used for transformation of E. coli (Competent high JM109, Toyobo Co., Ltd.). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the DNA fragment having the restriction enzyme recognition sequence added at each end of the expression cassette for the prolyl 4-hydroxylase β subunit having the α-factor signal and pro sequences had been inserted (which may be referred to hereinafter as pP4HBsig(-)rev-TOPO) was isolated from the cultured cells to give pP4HBsig(-)rev-TOPO.

### (2-1-7) Construction of pSN007

The oligonucleotides 39 and 40 mentioned below were synthesized:
(a) oligonucleotide 39: TATTCGAAACGACGCGTGTCAGCTAGCACTAGTGC (SEQ ID NO:39), and
(b) oligonucleotide 40: GCACTAGTGCTAGCTGACACGCGTCGTTTCGAATA (SEQ ID NO:40).
A solution having the composition mentioned below was prepared and kept at 98°C for 5 minutes, at 50°C for 50 minutes, and then at 37°C for 1 hour:
(a) oligonucleotide 39 (50 pmol/µl), 5 µl;
(b) oligonucleotide 40 (50 pmol/µl), 5 µl;
(c) Tris-HCl (100 mM), 10 µl;
(d) MgCl₂ (100 mM), 10 µl;
(e) dithiothreitol (10mM), 10 µl;
(f) sterile distilled water 60 µl.
A DNA linker in which the oligonucleotides 39 and 40 had been annealed was digested with restriction enzymes BspT104I and SpeI. Next, the mixture solution was extracted with phenol:chloroform:isoamyl alcohol (25:24:1) and then subjected to ethanol precipitation to purify the DNA linker (Linker 3).
Additionally, the plasmid named as pSN004 (see, Example (2-1-2)) was digested with restriction enzymes BspT104I and SpeI. An about 4.2-kb DNA fragment was separated and purified by agarose gel electrophoresis.
The DNA linker (Linker 3) and the about 4.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA linker (Linker 3) had been inserted (which may be referred to hereinafter as pSN007) was isolated from the cultured cells to give pSN007.

### (2-1-8) Construction of pSN017

A cDNA clone encoding human prolyl 4-hydroxylase α1 subunit (P4Hα1) (Clone ID: 4797051) was purchased from Invitrogen. The oligonucleotides 41 and 42 mentioned below were synthesized. A DNA fragment which had a restriction enzyme recognition sequence added at each end of the DNA encoding P4Hα1 was amplified by PCR using the oligonucleotides 41 and 42 as primers and the P4Hα1 cDNA clone as a template.
The oligonucleotides 41 and 42 used were:
(a) oligonucleotide 41: TATTCGAAACGATGATCTGGTATATATTAATTATA (SEQ ID NO:41), and
(b) oligonucleotide 42: TTGCTAGCTCATTCCAATTCTGACAACGTACAAGG (SEQ ID NO:42).
The composition of the reaction solutions is given as follows:
(a) plasmid solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 3 minutes and then subjected to 10 cycles of denaturation at 94°C for 15 seconds, annealing at 60°C for 30 seconds, and extension at 68°C for 2 minutes and then 15 cycles of denaturation at 94°C for 15 seconds, and each of annealing and extension at 68°C for 2 minutes, followed by additionally keeping the reaction solution at 68°C for 5 minutes.
An about 1.6-kb DNA fragment resulted from the PCR was digested with restriction enzymes BspT104I and SpeI, and then isolated by agarose gel electrophoresis, followed by extraction and purification from the gel using MinElute Gel Extraction Kit (QIAGEN).
The plasmid named as pSN007 (see, Example (2-1-7)) was digested with restriction enzymes BspT104I and SpeI. Then, an about 4.2-kb DNA fragment was separated by agarose gel electrophoresis, followed by extraction and purification from the gel using MinElute Gel Extraction Kit (QIAGEN).
The about 1.6-kb DNA fragment and the about 4.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequence added at each end of the DNA encoding P4Hα1 had been inserted (which may be referred to hereinafter as pSN017) was isolated from the cultured cells to give pSN017.

### (2-1-9) Construction of pP4HA1-SmaI-TOPO

The oligonucleotides 43 and 38 mentioned below were synthesized. A DNA fragment which had a restriction enzyme recognition sequence added at each end of the expression cassette for a prolyl 4-hydroxylase α1 subunit was amplified by PCR using the oligonucleotides 43 and 38 as primers and the plasmid named as pSN017 (see, Example (2-1-8)) as a template.
The oligonucleotides 43 and 38 used were:
(a)oligonucleotide 43: AACCCGGGTCTAACATCCAAAGACGAAAGGTTGAA (SEQ ID NO:43), and
(b)oligonucleotide 38: AACCCGGGGCACAAACGAACGTCTCACTTAATCTT (SEQ ID NO:38).
The composition of the reaction solutions is given as follows:
(a) plasmid solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
   (e)10x PCR buffer for KOD-plus-(Toyobo Co., Ltd.), 5µ1;
(f) KOD-plus- DNA polymerase (1 U/µl, Toyobo Co., Ltd.), 1 µl;
(g) sterile distilled water 33µl.
The PCR was conducted under conditions where the reaction solution was heated at 94°C for 3 minutes and then subjected to 10 cycles of denaturation at 94°C for 15 seconds, annealing at 60°C for 30 seconds, and extension at 68°C for 3.5 minutes and then 15 cycles of denaturation at 94°C for 15 seconds, and each of annealing and extension at 68°C for 3.5 minutes, followed by additionally keeping the reaction solution at 68°C for 5 minutes.
An about 2.8-kb DNA fragment resulted from the PCR was isolated by agarose gel electrophoresis, followed by extraction and purification from the gel using MinElute Gel Extraction Kit (QIAGEN). The about 2.8-kb DNA fragment was ligated into the "PCR Product insertion site" of a pCR-BluntII-TOPO plasmid (Invitrogen), and the resulting ligation solution was used for transformation of E. coli (Competent high JM109, Toyobo Co., Ltd.). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the DNA fragment having the restriction enzyme recognition sequences added at each end of the expression cassette for the prolyl 4-hydroxylase α1 subunit had been inserted (which may be referred to hereinafter as ) was isolated from the cultured cells to give pP4HA1-SmaI-TOPO.

### (2-1-10) Construction of pSN023

The oligonucleotides 44 and 45 mentioned below were synthesized. A DNA fragment which had a restriction enzyme recognition sequence added at each end of the DNA encoding ARG4 was amplified by PCR using the oligonucleotides 44 and 45 as primers and the plasmid named as pARG4-TOPO (see, Example (1-2)) as a template.
The oligonucleotides 44 and 45 used were:
(a) oligonucleotide 44: AACTCGAGACGAAAATATGGTACCTGCCCT (SEQ ID NO:44), and
(b) oligonucleotide 45: CCATCGATACAGAGGTATCATCCAATGATTCC (SEQ ID NO:45).
The composition of the reaction solutions is given as follows:
(a) plasmid solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 3 minutes and then subjected to 10 cycles of denaturation at 94°C for 15 seconds, annealing at 60°C for 30 seconds, and extension at 68°C for 2 minutes and then 15 cycles of denaturation at 94°C for 15 seconds, and each of annealing and extension at 68°C for 2 minutes, followed by additionally keeping the reaction solution at 68°C for 5 minutes.
An about 2.2-kb DNA fragment resulted from the PCR was digested with restriction enzymes XhoI and ClaI, and then isolated by agarose gel electrophoresis, followed by extraction and purification from the gel using MinElute Gel Extraction Kit (QIAGEN).
A pBluescriptII KS(+) plasmid (Stratagene) was digested with restriction enzymes XhoI and ClaI, and then isolated by agarose gel electrophoresis, followed by extraction and purification from the gel using MinElute Gel Extraction Kit (QIAGEN).
The about 2.2-kb DNA fragment and the digested plasmid were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequence added at each end of the DNA encoding ARG4 had been inserted (which may be referred to hereinafter as pSN023) was isolated from the cultured cells to give pSN023.

### (2-1-11) Construction of pEXP-A-P4Hbsig(-)rev

The plasmid named as pP4Hbsig(-)rev-TOPO (see, Example (2-1-6)) was digested with restriction enzymes SacII and Cfr9I. An about 3.0-kb DNA fragment which had the restriction enzyme recognition sequence added at each end of the expression cassette for the prolyl 4-hydroxylase β subunit having the α-factor signal and pro sequences was isolated by agarose gel electrophoresis, followed by extraction and purification from the gel using MinElute Gel Extraction Kit (QIAGEN).
The plasmid named as pSN023 (see, Example (2-1-10) was digested with restriction enzymes SacI and Cfr9I. Then, an about 5.2-kb DNA fragment was isolated by agarose gel electrophoresis, followed by extraction and purification from the gel using MinElute Gel Extraction Kit (QIAGEN).
The about 3.0-kb DNA fragment and the about 5.2-kb DNA fragment were ligated, and the resulting ligation solution was used for transformation of E. coli (Competent high JM109, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequence added at each end of the expression cassette for the prolyl 4-hydroxylase β subunit having the α-factor signal and the pro sequences had been inserted (which may be referred to hereinafter as pEXP-A-P4Hbsig(-)rev) was isolated from the cultured cells to give pEXP-A-P4Hbsig(-)rev.

### (2-1-12) Construction of pEXP-A-P4Hbsig(-)A1rev

The plasmid named as pP4HA1-SmaI-TOPO (see, Example (2-1-9)) was digested with a restriction enzyme Cfr9I. An about 2.8-kb DNA fragment which had the restriction enzyme recognition sequence added at each end of the expression cassette for the prolyl 4-hydroxylase α1 subunit was isolated by agarose gel electrophoresis, followed by extraction and purification from the gel using MinElute Gel Extraction Kit (QIAGEN).
The plasmid named as pEXP-A-P4Hbsig(-)rev (see, Example (2-1-11)) was digested with a restriction enzyme Cfr9I, dephosphorylated with an alkaline phosphatase, and then purified using MinElute Reaction Cleanup Kit (QIAGEN).
The about 3.0-kb DNA fragment and the dephosphorylated plasmid were ligated, and the resulting ligation solution was used for transformation of E. coli (Competent high JM109, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequences added at each end of the expression cassette for the prolyl 4-hydroxylase α1 subunit had been inserted (which may be referred to hereinafter as pEXP-A-P4Hbsig(-)A1rev) was isolated from the cultured cells to give pEXP-A-P4Hbsig(-)A1rev.

(2-2) Preparation of plasmids for introducing an expression cassette for an FK506 binding protein

### (2-2-1) Construction of pTS011

A cDNA clone encoding heat shock protein 47 (Hsp47) (Clone ID: 3030138) was purchased from Invitrogen. The oligonucleotides 46 and 47 mentioned below were synthesized. A DNA fragment which had a restriction enzyme recognition sequence added at each end of the DNA encoding HSP47 was amplified by PCR using the oligonucleotides 46 and 47 as primers and the HSP47 DNA clone as a template.
The oligonucleotides 46 and 47 used were:
(a) oligonucleotide 46: TATTCGAAACGATGCGCTCCCTCCTGCTTCTC (SEQ ID NO:46), and
(b) oligonucleotide 47: TTACTAGTTATAACTCGTCTCGCATCTTGTCACC (SEQ ID NO:47).
The composition of the reaction solutions is given as follows:
(a) DNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 3 minutes and then subjected to 5 cycles of denaturation at 94°C for 15 seconds, annealing at 60°C for 30 seconds, and extension at 68°C for 1.5 minutes and then 20 cycles of denaturation at 94°C for 15 seconds, and each of annealing and extension at 68°C for 1.5 minutes, followed by additionally keeping the reaction solution at 68°C for 1.5 minutes.
An about 1.3-kb DNA fragment resulted from the PCR was digested with restriction enzymes BspT104I and SpeI, and then isolated by agarose gel electrophoresis, followed by extraction and purification from the gel using MinElute Gel Extraction Kit (QIAGEN).
The plasmid named as pSN005 (see, Example (2-1-3)) was digested with restriction enzymes BspT104I and SpeI. Then, an about 4.2-kb DNA fragment was separated by agarose gel electrophoresis, followed by extraction and purification from the gel using MinElute Gel Extraction Kit (QIAGEN).
The about 1.3-kb DNA fragment and the about 4.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequence added at each end of the DNA encoding HSP47 had been inserted (which may be referred to hereinafter as pTS011) was isolated from the cultured cells to give pTS011.

### (2-2-2) Construction of pEXP-A-HSP47native signal

The plasmid named as pTS011 (see, Example (2-2-1)) was digested with restriction enzymes BspT104I and SpeI. An about 1.3-kb DNA fragment which had the restriction enzyme recognition sequence at each end of the DNA encoding HSP47 was separated by agarose gel electrophoresis, followed by extraction and purification from the gel using MinElute Gel Extraction Kit (QIAGEN).
The plasmid named as pEXP-A-P4Hbsig(-)rev (see, Example (2-1-11)) was digested with restriction enzymes BspT104I and SpeI. Then, an about 6.3-kb DNA fragment was separated by agarose gel electrophoresis, followed by extraction and purification from the gel using MinElute Gel Extraction Kit (QIAGEN).
The about 1.3-kb DNA fragment and the about 6.3-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequence added at each end of the DNA encoding HSP47 had been inserted (which may be referred to hereinafter as pEXP-A-HSP47native signal) was isolated from the cultured cells to give pEXP-A-HSP47native signal.

### (2-2-3) Construction of pEXP-A-HSP47native signal ZeoR2rev

The oligonucleotides 48 and 49 mentioned below were synthesized. A DNA fragment which had a restriction enzyme recognition sequence added at each end of the Zeocyn-resistance cassette was amplified by PCR using the oligonucleotides 48 and 49 as primers and the plasmid named as pUC57-ZeoR (see, Example (1-13)) as a template.
The oligonucleotides 48 and 49 used were:
(a) oligonucleotide 48: TTATCGATCCCACACACCATAGCTTCA (SEQ ID NO:48), and
(b) oligonucleotide 49: TGATCGATAGCTTGCAAATTAAAGCCTTC (SEQ ID NO:49).
The composition of the reaction solutions is given as follows:
(a) cDNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 5 cycles of denaturation at 94°C for 15 seconds, annealing at 60°C for 30 seconds, and extension at 68°C for 1.5 minutes and then 20 cycles of denaturation at 94°C for 15 seconds, and each of annealing and extension at 68°C for 1.5 minutes, followed by additionally keeping the reaction solution at 68°C for 1.5 minutes.
An about 1.2-kb DNA fragment resulted from the PCR was digested with a restriction enzyme ClaI. Then, the digested, about 1.2-kb DNA fragment was isolated by agarose gel electrophoresis, followed by extraction and purification from the gel using MinElute Gel Extraction Kit (QIAGEN).
The plasmid named as pEXP-A-HSP47native signal (see, Example (2-2-2)) was digested with a restriction enzyme ClaI, and then dephosphorylated with an alkaline phosphatase, and purified using MinElute Reaction Cleanup Kit (QIAGEN).
The digested, about 1.2-kb DNA fragment and the dephosphorylated plasmid were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin and 25 µg/ml of Zeocyn, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequence added at each end of the Zeocyn-resistance cassette had been inserted (which may be referred to hereinafter as pEXP-A-HSP47native signal ZeoR2rev) was isolated from the cultured cells to give pEXP-A-HSP47native signal ZeoR2rev.

### (2-2-4) Construction of pEXP-A-FKBP13A ZeoR

The plasmid named as pUC57-YFKBP13A (see, Example (1-14)) was digested with restriction enzymes BspT104I and SpeI. An about 0.4-kb DNA fragment encoding FKBP13A was separated by agarose gel electrophoresis, followed by extraction and purification from the gel using MinElute Gel Extraction Kit (QIAGEN).
The plasmid named as pEXP-A-HSP47native signal ZeoR2rev (see, Example (2-2-3)) was digested with restriction enzymes BspT104I and SpeI. Then, an about 6.4-kb DNA fragment was separated by agarose gel electrophoresis, followed by extraction and purification from the gel using MinElute Gel Extraction Kit (QIAGEN).
The about 0.4-kb DNA fragment and the about 6.4-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding FKBP13A had been inserted (which may be referred to hereinafter as pEXP-A-FKBP13A ZeoR) was isolated from the cultured cells to give pEXP-A-FKBP13A ZeoR.

### (2-2-5) Construction of pEXP-A-FKBP19 ZeoR

The plasmid named as pUC57-YFKBP19 (see, Example (1-15)) was digested with restriction enzymes BspT104I and SpeI. An about 0.6-kb DNA fragment encoding FKBP19 was separated by agarose gel electrophoresis, followed by extraction and purification from the gel using MinElute Gel Extraction Kit (QIAGEN).
The plasmid named as pEXP-A-HSP47native signal ZeoR2rev (see, Example (2-2-3)) was digested with restriction enzymes BspT104I and SpeI. Then, an about 6.4-kb DNA fragment was separated by agarose gel electrophoresis, followed by extraction and purification from the gel using MinElute Gel Extraction Kit (QIAGEN).
The about 0.6-kb DNA fragment and the about 6.4-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding FKBP19 had been inserted (which may be referred to hereinafter as pEXP-A-FKBP19 ZeoR) was isolated from the cultured cells to give pEXP-A-FKBP19 ZeoR.

### (2-2-6) Construction of pEXP-A-FKBP23 ZeoR

The plasmid named as pUC57-YFKBP23 (see, Example (1-16)) was digested with restriction enzymes BspT104I and SpeI. An about 0.7-kb DNA fragment encoding FKBP23 was separated by agarose gel electrophoresis, followed by extraction and purification from the gel using MinElute Gel Extraction Kit (QIAGEN).
The plasmid named as pEXP-A-HSP47native signal ZeoR2rev (see, Example (2-2-3)) was digested with restriction enzymes BspT104I and SpeI. Then, an about 6.4-kb DNA fragment was separated by agarose gel electrophoresis, followed by extraction and purification from the gel using MinElute Gel Extraction Kit (QIAGEN).
The about 0.7-kb DNA fragment and the about 6.4-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding FKBP23 had been inserted (which may be referred to hereinafter as pEXP-A-FKBP23 ZeoR) was isolated from the cultured cells to give pEXP-A-FKBP23 ZeoR.

### (2-2-7) Construction of pEXP-A-FKBP63 ZeoR

The plasmid named as pUC57-YFKBP63 (see, Example (1-17)) was digested with restriction enzymes BspT104I and SpeI. An about 1.7-kb DNA fragment encoding FKBP63 was separated by agarose gel electrophoresis, followed by extraction and purification from the gel using MinElute Gel Extraction Kit (QIAGEN).
The plasmid named as pEXP-A-HSP47native signal ZeoR2rev (see, Example (2-2-3)) was digested with restriction enzymes BspT104I and SpeI. Then, an about 6.4-kb DNA fragment was separated by agarose gel electrophoresis, followed by extraction and purification from the gel using MinElute Gel Extraction Kit (QIAGEN).
The about 1.7-kb DNA fragment and the about 6.4-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding FKBP63 had been inserted (which may be referred to hereinafter as pEXP-A-FKBP63 ZeoR) was isolated from the cultured cells to give pEXP-A-FKBP63 ZeoR.

### (2-2-8) Construction of pEXP-A-FKBP65 ZeoR

The plasmid named as pUC57-YFKBP65 (see, Example (1-18)) was digested with restriction enzymes BspT104I and SpeI. An about 1.8-kb DNA fragment encoding FKBP19 was separated by agarose gel electrophoresis, followed by extraction and purification from the gel using MinElute Gel Extraction Kit (QIAGEN).
The plasmid named as pEXP-A-HSP47native signal ZeoR2rev (see, Example (2-2-3)) was digested with restriction enzymes BspT104I and SpeI. Then, an about 6.4-kb DNA fragment was separated by agarose gel electrophoresis, followed by extraction and purification from the gel using MinElute Gel Extraction Kit (QIAGEN).
The about 1.8-kb DNA fragment and the about 6.4-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding FKBP65 had been inserted (which may be referred to hereinafter as pEXP-A-FKBP65 ZeoR) was isolated from the cultured cells to give pEXP-A-FKBP65 ZeoR.

### (2-2-9) Construction of pEXP-A-PER3 Pro-FKBP23 ZeoR

The plasmid named as pCR-BII-PER3 Pro SacII-Psp1406I(-) (see, Example (1-4)) was digested with restriction enzymes Psp1406I and SacII. Then, an about 1.0-kb DNA fragment which had the restriction enzyme recognition sequence added at each end of the PER3 promoter was separated by agarose gel electrophoresis, followed by extraction and purification from the gel using MinElute Gel Extraction Kit (QIAGEN).
The plasmid named as pEXP-A-FKBP23 ZeoR (see, Example (2-2-6)) was digested with restriction enzymes BspT104I and SacII. An about 7.2-kb DNA fragment was separated by agarose gel electrophoresis, followed by extraction and purification from the gel using MinElute Gel Extraction Kit (QIAGEN).
The about 1.0-kb DNA fragment and the about 7.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA having the restriction enzyme recognition sequence at each end of the PER3 promoter had been inserted (which may be referred to hereinafter as pEXP-A-PER3 Pro-FKBP23 ZeoR) was isolated from the cultured cells to give pEXP-A-PER3 Pro-FKBP23 ZeoR.

### (2-2-10) Construction of pEXP-A-AOX2 Pro-FKBP23 ZeoR

The plasmid named as pCR-BII-AOX2 Pro SacII-Psp1046I(-) (see, Example (1-5)) was digested with restriction enzymes Psp1406I and SacII. Then, an about 1.1-kb DNA fragment which had the restriction enzyme recognition sequence added at each end of the AOX2 promoter was separated by agarose gel electrophoresis, followed by extraction and purification from the gel using MinElute Gel Extraction Kit (QIAGEN).
The plasmid named as pEXP-A-FKBP23 ZeoR (see, Example (2-2-6)) was digested with restriction enzymes BspT104I and SacII. An about 7.2-kb DNA fragment was separated by agarose gel electrophoresis, followed by extraction and purification from the gel using MinElute Gel Extraction Kit (QIAGEN).
The about 1.1-kb DNA fragment and the about 7.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA having the restriction enzyme recognition sequence at each end of the AOX2 promoter had been inserted (which may be referred to hereinafter as pEXP-A-AOX2 Pro-FKBP23 ZeoR) was isolated from the cultured cells to give pEXP-A-AOX2 Pro-FKBP23 ZeoR.

### (2-2-11) Construction of pEXP-A-FLD1 Pro-FKBP23 ZeoR

The plasmid named as pCR-BII-FLD1 Pro SacII-Psp1406I(+) (see, Example (1-6)) was digested with restriction enzymes Psp1406I and SacII. Then, an about 0.9-kb DNA fragment which had the restriction enzyme recognition sequence added at each end of the FLD1 promoter was separated by agarose gel electrophoresis, followed by extraction and purification from the gel using MinElute Gel Extraction Kit (QIAGEN).
The plasmid named as pEXP-A-FKBP23 ZeoR (see, Example (2-2-6)) was digested with restriction enzymes BspT104I and SacII. An about 7.2-kb DNA fragment was separated by agarose gel electrophoresis, followed by extraction and purification from the gel using MinElute Gel Extraction Kit (QIAGEN).
The about 0.9-kb DNA fragment and the about 7.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA having the restriction enzyme recognition sequence at each end of the FLD1 promoter had been inserted (which may be referred to hereinafter as pEXP-A-FLD1 Pro-FKBP23 ZeoR) was isolated from the cultured cells to give pEXP-A-FLD1 Pro-FKBP23 ZeoR.

(2-3) Preparation of a plasmid (pEXP-HA-YHsCOLIA2-1A1) for introducing an expression cassette for human collagen Type I α1 and an expression cassette for human collagen Type I α2

### (2-3-1) Construction of pSN001

The oligonucleotides 51 and 52 mentioned below were synthesized. A DNA fragment which had a restriction enzyme recognition sequence added at each end of the HIS4 was amplified by PCR using the oligonucleotides 50 and 51 as primers and the plasmid named as pHIS4-TOPO (see, Example (1-1)) as a template.
The oligonucleotides 50 and 51 used were:
(a) oligonucleotide 50: GGAAGCTTGATCTCCTGATGACTGACTCACTG (SEQ ID NO:50), and
(b) oligonucleotide 51: CCCTGCAGTAATTAAATAAGTCCCAGTTTCTCCA (SEQ ID NO:51).
The composition of the reaction solutions is given as follows:
(a) plasmid solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 3 minutes and then subjected to 5 cycles of denaturation at 94°C for 15 seconds, annealing at 60°C for 30 seconds, and extension at 68°C for 2 minutes and then 20 cycles of denaturation at 94°C for 15 seconds, and each of annealing and extension at 68°C for 2 minutes, followed by additionally keeping the reaction solution at 68°C for 5 minutes.
An about 2.6-kb DNA fragment resulted from the PCR was digested with restriction enzymes HindIII and PstI, and then isolated by agarose gel electrophoresis, followed by extraction and purification from the gel using MinElute Gel Extraction Kit (QIAGEN).
A pBluescriptII KS(+) plasmid (Stratagene) was digested with restriction enzymes HindIII and PstI. Then, an about 3.0-kb DNA fragment was isolated by agarose gel electrophoresis, followed by extraction and purification from the gel using MinElute Gel Extraction Kit (QIAGEN).
The about 2.6-kb DNA fragment and the about 3.0-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA having the restriction enzyme recognition sequence at each end of the HIS4 had been inserted (which may be referred to hereinafter as pSN001) was isolated from the cultured cells to give pSN001.

### (2-3-2) Construction of pSN002

The oligonucleotides 52 and 53 mentioned below were synthesized. A DNA fragment which had a restriction enzyme recognition sequence added at each end of the 3'-downstream non-coding region of AOX1 was amplified by PCR using the oligonucleotides 52 and 53 as primers and the plasmid named as pAOXI-3'-TOPO (see, Example (1-8)) as a template.
The oligonucleotides 52 and 53 used were:
(a) oligonucleotide 52: GCATCGATTCGAGTATCTATGATTGGAAGTATGG (SEQ ID NO:52), and
(b) oligonucleotide 53: AAGGGCCCGATCTTGAGATAAATTTCACGTTTAAA (SEQ ID NO:53).
The composition of the reaction solutions is given as follows:
(a) plasmid solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 3 minutes and then subjected to 5 cycles of denaturation at 94°C for 15 seconds, annealing at 55°C for 30 seconds, and extension at 68°C for 2 minutes and then 20 cycles of denaturation at 94°C for 15 seconds, and each of annealing and extension at 68°C for 2 minutes, followed by additionally keeping the reaction solution at 68°C for 5 minutes.
An about 0.8-kb DNA fragment resulted from the PCR was digested with restriction enzymes ClaI and ApaI, and then isolated by agarose gel electrophoresis, followed by extraction and purification from the gel using MinElute Gel Extraction Kit (QIAGEN).
The plasmid named as pSN001 (see, Example (2-3-1)) was digested with restriction enzymes ClaI and ApaI. Then, an about 5.6-kb DNA fragment was isolated by agarose gel electrophoresis, followed by extraction and purification from the gel using MinElute Gel Extraction Kit (QIAGEN).
The about 0.8-kb DNA fragment and the about 5.6-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA having the restriction enzyme recognition sequence added at each end of the 3'-downstream non-coding region of AOX1 had been inserted (which may be referred to hereinafter as pSN002) was isolated from the cultured cells to give pSN002.

### (2-3-3) Construction of pSN006

The oligonucleotides 54 and 55 mentioned below were synthesized:
(a) oligonucleotide 54: TATTCGAAACGCATATGGTACCGGCAGACTAGTGG (SEQ ID NO:54), and
(b) oligonucleotide 55: CCACTAGTCGCCTAGGCGACATATGGTTTCGAATA (SEQ ID NO:55).
A solution having the composition mentioned below was prepared and kept at 98°C for 5 minutes, at 50°C for 50 minutes, and then at 37°C for 1 hour:
(a) oligonucleotide 54 (50 pmol/µl), 5 µl;
(b) oligonucleotide 55 (50 pmol/µl), 5 µl;
(c) Tris-HCl (100 mM), 10 µl;
(d) MgCl₂ (100 mM), 10 µl;
(e) dithiothreitol (10 mM), 10 µl;
(f) sterile distilled water 60 µl.
A DNA linker in which the oligonucleotides 54 and 55 had been annealed was digested with restriction enzymes BspT104I and SpeI. Next, the mixture solution was extracted with phenol:chloroform:isoamyl alcohol (25:24:1) and then subjected to ethanol precipitation to purify the DNA linker (Linker 2).
Additionally, the plasmid named as pSN004 (see, Example (2-1-2)) was digested with restriction enzymes BspT104I and SpeI. An about 4.2-kb DNA fragment was separated and purified by agarose gel electrophoresis.
The DNA linker (Linker 2) and the about 4.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA linker (Linker 2) had been inserted (which may be referred to hereinafter as pSN006) was isolated from the cultured cells to give pSN006.

### (2-3-4) Construction of pEXH002

The plasmid named as pSN006 (see, Example (2-3-3)) was digested with restriction enzymes Eco52I and PstI. An about 1.3-kb DNA fragment comprising the AOX1 promoter and the AOX1 terminator was isolated by agarose gel electrophoresis, followed by extraction and purification from the gel using MinElute Gel Extraction Kit (QIAGEN).
The plasmid named as pSN002 (see, Example (2-3-1)) was digested with restriction enzymes Eco52I and PstI. An about 6.3-kb DNA fragment was isolated by agarose gel electrophoresis, followed by extraction and purification from the gel using MinElute Gel Extraction Kit (QIAGEN).
The about 1.3-kb DNA fragment and the about 6.3-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment comprising the AOX1 promoter and the AOX1 terminator had been inserted (which may be referred to hereinafter as pEXH002) was isolated from the cultured cells to give pEXH002.

### (2-3-5) Construction of PTS001

The plasmid named as pUC57-YHsCOL1A1 (see, Example (1-19)) was digested with restriction enzymes BspT104I and SpeI. An about 4.4-kb DNA fragment encoding human collagen Type I α1 was separated by agarose gel electrophoresis, followed by extraction and purification from the gel using MinElute Gel Extraction Kit (QIAGEN).
The plasmid named as pSN006 (see, Example (2-3-3)) was digested with restriction enzymes BspT104I and SpeI. An about 4.2-kb DNA fragment was separated by agarose gel electrophoresis, followed by extraction and purification from the gel using MinElute Gel Extraction Kit (QIAGEN).
The about 4.4-kb DNA fragment and the about 4.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding human collagen Type I α1 had been inserted (which may be referred to hereinafter as PTS001) was isolated from the cultured cells to give PTS001.

### (2-3-6) Construction of pEXP-HA-YHsCOL1A1

The plasmid named as pTS001 (see, Example (2-3-5)) was digested with restriction enzymes Eco52I and SpeI. An about 5.3-kb DNA fragment encoding the AOX1 promoter and human collagen Type I α1 was isolated by agarose gel electrophoresis, followed by extraction and purification from the gel using MinElute Gel Extraction Kit (QIAGEN).
The plasmid named as pEXH002 (see, Example (2-3-4)) was digested with restriction enzymes Eco52I and SpeI. An about 6.6-kb DNA fragment was isolated by agarose gel electrophoresis, followed by extraction and purification from the gel using MinElute Gel Extraction Kit (QIAGEN).
The about 5.3-kb DNA fragment and the about 6.6-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding the AOX1 promoter and human collagen Type I α1 had been inserted (which may be referred to hereinafter as pEXP-HA-YHsCOL1A1) was isolated from the cultured cells to give pEXP-HA-YHsCOL1A1.

### (2-3-7) Construction of pTS002

The plasmid named as pUC57-YHsCOL1A2 (see, Example (1-20)) was digested with restriction enzymes BspT104I and SpeI. An about 4.1-kb DNA fragment encoding human collagen Type I α2 was separated by agarose gel electrophoresis, followed by extraction and purification from the gel using MinElute Gel Extraction Kit (QIAGEN).
The plasmid named as pSN006 (see, Example (2-3-3)) was digested with restriction enzymes BspT104I and SpeI. An about 4.2-kb DNA fragment was separated by agarose gel electrophoresis, followed by extraction and purification from the gel using MinElute Gel Extraction Kit (QIAGEN).
The about 4.1-kb DNA fragment and the about 4.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding human collagen Type I α2 had been inserted (which may be referred to hereinafter as pTS002) was isolated from the cultured cells to give pTS002.

### (2-3-8) Construction of pYHsCOL1A2 Exp unit-Eco52I

The oligonucleotides 56 and 57 mentioned below were synthesized. A DNA fragment which had a restriction enzyme recognition sequence added at each end of the expression cassette for human collagen Type I α2 was amplified by PCR using the oligonucleotides 56 and 57 as primers and the plasmid named as pTS002 (see, Example (2-3-7)) as a template.
The oligonucleotides 56 and 57 used were:
(a) oligonucleotide 56: AACGGCCGTCTAACATCCAAAGACGAAAGGTTGAA (SEQ ID NO:56), and
(b) oligonucleotide 57: AACGGCCGGCACAAACGAACGTCTCACTTAATCTT (SEQ ID NO:57).
The composition of the reaction solutions is given as follows:
(a) plasmid solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 3 minutes and then subjected to 5 cycles of denaturation at 94°C for 15 seconds, annealing at 60°C for 30 seconds, and extension at 68°C for 6 minutes and then 18 cycles of denaturation at 94°C for 15 seconds, and each of annealing and extension at 68°C for 6 minutes, followed by additionally keeping the reaction solution at 68°C for 5 minutes.
An about 5.4-kb DNA fragment resulted from the PCR was isolated by agarose gel electrophoresis, followed by extraction and purification from the gel using MinElute Gel Extraction Kit (QIAGEN). The about 5.4-kb DNA fragment purified was ligated into the "PCR Product insertion site" of a pCR-BluntII-TOPO plasmid (Invitrogen), and the resulting ligation solution was used for transformation of E. coli (Competent high DH5α, Toyobo Co., Ltd.). For the ligation reaction, a Zero-Blunt TOPO PCR cloning kit (Invitrogen) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the DNA fragment having the restriction enzyme recognition sequences added at each end of the expression cassette for human collagen Type I α2 had been inserted (which may be referred to hereinafter as pYHsCOL1A2 Exp unit-Eco52I) was isolated from the cultured cells to give pYHsCOL1A2 Exp unit-Eco52I.

### (2-3-9) Construction of pEXP-HA-YHsCOL1A2-1A1

The plasmid named as pYHsCOL1A2 Exp unit-Eco52I (see, Example (2-3-8)) was digested with a restriction enzyme Eco52I. An about 5.4-kb DNA fragment which had the restriction enzyme recognition sequence added at each end of the expression cassette for human collagen Type I α2 was separated by agarose gel electrophoresis, followed by extraction and purification from the gel using MinElute Gel Extraction Kit (QIAGEN).
The plasmid named as pEXP-HA-YHsCOL1A1 (see, Example (2-3-6)) was digested with a restriction enzyme Eco52I, dephosphorylated with an alkaline phosphatase, and then purified using MinElute Reaction Cleanup Kit (QIAGEN).
The about 5.4-kb DNA fragment and the dephosphorylated plasmid were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequences added at each end of the expression cassette for human collagen Type I α2 had been inserted (which may be referred to hereinafter as pEXP-HA-YHsCOL1A2-1A1) was isolated from the cultured cells to give pEXP-HA-YHsCOL1A2-1A1.

(2-4) Preparation of a plasmid (pEXP-HA-YHsCOL1A2-1A1 N60C15) for introducing an expression cassette for a fusion polypeptide which consists of the N-terminal non-helix region, 60 N-terminal residues of the helix region, 15 C-terminal residues of the helix region, and the C-terminal non-helix region of human collagen Type I α1 and an expression cassette for a fusion polypeptide which consists of the N-terminal non-helix region, 60 N-terminal residues of the helix region, 15 C-terminal residues of the helix region, and the C-terminal non-helix region of human collagen Type I α2

### (2-4-1) Construction of pAT021

The oligonucleotides 58 and 59 mentioned below were synthesized. A fragment containing the expression cassette for a fusion polypeptide of the N-terminal non-helix region, 60 N-terminal residues of the helix region, 15 C-terminal residues of the helix region, and the C-terminal non-helix region of human collagen Type I α1, and a portion of the vector plasmid was amplified by PCR using the oligonucleotides 58 and 59 as primers and the plasmid named as pTS001 (see, Example (2-3-5)) as a template.
The oligonucleotides 58 and 59 used were:
(a) oligonucleotide 58: CGGCTTACCAGCCTCGC (SEQ ID NO:58), and
(b) oligonucleotide 59: GGACCACCAGGGCCGC (SEQ ID NO:59).
The composition of the reaction solutions is given as follows:
(a) cDNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 25 cycles of denaturation at 94°C for 15 seconds and each of annealing and extension at 68°C for 6 minutes, followed by additionally keeping the reaction solution at 68°C for 6 minutes.
An about 5.8-kb DNA fragment resulted from the PCR was purified using MinElute PCR Purification Kit (QIAGEN). To the DNA fragment obtained, a phosphate group was added at the 5' end using T4 polynucleotide kinase (Takara Bio Inc.). Then, the phosphorylated, about 5.8-kb DNA fragment was separated by agarose gel electrophoresis, followed by extraction and purification from the gel using MinElute Gel Extraction Kit (QIAGEN).
The phosphorylated, about 5.8-kb DNA fragment was self-ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid which comprised the expression cassette for the fusion polypeptide consisting of the N-terminal non-helix region, 60 N-terminal residues of the helix region, 15 C-terminal residues of the helix region, and the C-terminal non-helix region of human collagen Type I α1 (which may be referred to hereinafter as pAT021) was purified from the cultured cells to give pAT021.

### (2-4-2) Construction of pEXP-HA-YHsCOL1A1 N60C15

The plasmid named as pAT021 (see, Example (2-4-1)) was digested with restriction enzymes Eco52I and SpeI. An about 2.5-kb DNA fragment encoding the AOX1 promoter and the fusion polypeptide of the N-terminal non-helix region, 60 N-terminal residues of the helix region, 15 C-terminal residues of the helix region, and the C-terminal non-helix region of human collagen Type I α1 was isolated by agarose gel electrophoresis, followed by extraction and purification from the gel using MinElute Gel Extraction Kit (QIAGEN).
The plasmid named as pEXH002 (see, Example (2-3-4)) was digested with restriction enzymes Eco52I and SpeI. An about 6.6-kb DNA fragment was isolated by agarose gel electrophoresis, followed by extraction and purification from the gel using MinElute Gel Extraction Kit (QIAGEN).
The about 2.5-kb DNA fragment and the about 6.6-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding the AOX1 promoter and the fusion polypeptide of the N-terminal non-helix region, 60 N-terminal residues of the helix region, 15 C-terminal residues of the helix region, and the C-terminal non-helix region of human collagen Type I α1 had been inserted (which may be referred to hereinafter as pEXP-HA-YHsCOL1A1 N60C15) was isolated from the cultured cells to give pEXP-HA-YHsCOL1A1 N60C15.

### (2-4-3) Construction of pYHsCOL1A2 N60C15 Exp unit-Eco52I

The oligonucleotides 60 and 61 mentioned below were synthesized. A fragment containing the expression cassette for a fusion polypeptide consisting of the N-terminal non-helix region, 60 N-terminal residues of the helix region, 15 C-terminal residues of the helix region, and the C-terminal non-helix region of human collagen Type I α2, and a portion of the vector plasmid was amplified by PCR using the oligonucleotides 60 and 61 as primers and the plasmid named as pYHsCOL1A2 Exp unit-Eco52I (see, Example (2-3-8)) as a template.
The oligonucleotides 60 and 61 used were:
(a) oligonucleotide 60: GGGTTTACCTGGGTGGCCG (SEQ ID NO:60), and
(b) oligonucleotide 61: GGACCTCCTGGCCCACC (SEQ ID NO:61).
The composition of the reaction solutions is given as follows:
(a) cDNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 25 cycles of denaturation at 94°C for 15 seconds and each of annealing and extension at 68°C for 6 minutes, followed by additionally keeping the reaction solution at 68°C for 6 minutes.
An about 6.1-kb DNA fragment resulted from the PCR was purified using MinElute PCR Purification Kit (QIAGEN). To the DNA fragment obtained, a phosphate group was added at the 5' end using T4 polynucleotide kinase (Takara Bio Inc.). Then, the phosphorylated, about 6.1-kb DNA fragment was separated by agarose gel electrophoresis, followed by extraction and purification from the gel using MinElute Gel Extraction Kit (QIAGEN).
The phosphorylated, about 6.1-kb DNA fragment was self-ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid which comprised the expression cassette for the fusion polypeptide consisting of the N-terminal non-helix region, 60 N-terminal residues of the helix region, 15 C-terminal residues of the helix region, and the C-terminal non-helix region of human collagen Type I α2 (which may be referred to hereinafter as pYHsCOL1A2 N60C15 Exp unit-Eco52I) was purified from the cultured cells to give pYHsCOL1A2 N60C15 Exp unit-Eco52I.

### (2-4-4) Construction of pEXP-HA-YHsCOL1A2-1A1 N60C15

The plasmid named as pYHsCOL1A2 N60C15 Exp unit-Eco52I (see, Example (2-4-3)) was digested with a restriction enzyme Eco52I. An about 2.3-kb DNA fragment which had the restriction enzyme recognition sequence added at each end of the expression cassette for the fusion polypeptide consisting of the N-terminal non-helix region, 60 N-terminal residues of the helix region, 15 C-terminal residues of the helix region, and the C-terminal non-helix region of human collagen Type I α2 was separated by agarose gel electrophoresis, followed by extraction and purification from the gel using MinElute Gel Extraction Kit (QIAGEN).
The plasmid named as pEXH-HA-YHsCOL1A1 N60C15 (see, Example (2-4-2)) was digested with a restriction enzyme Eco52I, dephosphorylated with an alkaline phosphatase, and then purified using MinElute Reaction Cleanup Kit (QIAGEN).
The about 2.3-kb DNA fragment and the dephosphorylated plasmid were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequence added at each end of the expression cassette for the fusion polypeptide consisting of the N-terminal non-helix region, 60 N-terminal residues of the helix region, 15 C-terminal residues of the helix region, and the C-terminal non-helix region of human collagen Type I α2 had been inserted (which may be referred to hereinafter as pEXP-HA-YHsCOL1A2-1A1 N60C15) was isolated from the cultured cells to give pEXP-HA-YHsCOL1A1 N60C15.

(2-5) Preparation of a plasmid (pEXP-HA-YHsCOL1A2-1A1 M500X2) for introducing an expression cassette for a fusion polypeptide which consists of the N-terminal non-helix region, 27 N-terminal residues of the helix region, a dimer made of 522 central residues of the helix region, 15 C-terminal residues of the helix region, and the C-terminal non-helix region of human collagen Type I α1 and an expression cassette for a fusion polypeptide which consists of the N-terminal non-helix region, 27 N-terminal residues of the helix region, a dimer made of 522 central residues of the helix region, 15 C-terminal residues of the helix region, and the C-terminal non-helix region of human collagen Type I α2

### (2-5-1) Construction of pAT017

The oligonucleotides 62 and 63 mentioned below were synthesized. A DNA fragment encoding the 522 central residues of the helix region of human collagen Type I α1 was amplified by PCR using the oligonucleotides 62 and 63 as primers and the plasmid named as pTS001 (see, Example (2-3-5)) as a template.
The oligonucleotides 62 and 63 used were:
(a) oligonucleotide 62: GGTCCACAGGGTCCAGGAG (SEQ ID NO:62), and
(b) oligonucleotide 63: ATCAGCTCCTGGTGATCCCTTTTC (SEQ ID NO:63).
The composition of the reaction solutions is given as follows:
(a) cDNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 25 cycles of denaturation at 94°C for 15 seconds and each of annealing and extension at 68°C for 1 minute 40 seconds, followed by additionally keeping the reaction solution at 68°C for 1 minute 40 seconds.
An about 1.6-kb DNA fragment resulted from the PCR was purified using MinElute PCR Purification Kit (QIAGEN). To the DNA fragment obtained, a phosphate group was added at the 5' end using T4 polynucleotide kinase (Takara Bio Inc.). Then, the phosphorylated, about 1.6-kb DNA fragment was separated by agarose gel electrophoresis, followed by extraction and purification from the gel using MinElute Gel Extraction Kit (QIAGEN).
The oligonucleotides 59 and 64 mentioned below were synthesized. A region which was composed of the AOX1 promoter, a DNA fragment encoding the N-terminal non-helix region and 27 N-terminal residues of the helix region of human collagen Type I α1, a DNA fragment encoding the C-terminal non-helix region and 15 C-terminal residues of the helix region of human collagen Type I α1, and a portion of the vector plasmid was amplified by PCR using the oligonucleotides 59 and 64 as primers and the plasmid named as pTS001 (see, Example (2-3-5)) as a template.
The oligonucleotides 59 and 64 used were:
(a) oligonucleotide 59: GGACCACCAGGGCCGC (SEQ ID NO:59), and
(b) oligonucleotide 64: TGGTGGACCTTGAAAACCCTG (SEQ ID NO:64).
The composition of the reaction solutions is given as follows:
(a) cDNA solution (10 ng/ul), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 25 cycles of denaturation at 94°C for 15 seconds and each of annealing and extension at 68°C for 6 minutes, followed by additionally keeping the reaction solution at 68°C for 6 minutes.
An about 5.7-kb DNA fragment resulted from the PCR was purified using MinElute PCR Purification Kit (QIAGEN). The DNA fragment obtained was dephosphorylated with an alkaline phosphatase. Then, the dephosphorylated, about 5.7-kb DNA fragment was separated by agarose gel electrophoresis, followed by extraction and purification from the gel using MinElute Gel Extraction Kit (QIAGEN).
The phosphorylated, about 1.6-kb DNA fragment and the dephosphorylated, about 5.7-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding the 522 central residues of the helix region of human collagen Type I α1 had been inserted (which may be referred to hereinafter as pAT017) was isolated from the cultured cells to give pAT017.

### (2-5-2) Construction of pAT027

The oligonucleotides 65 and 66 mentioned below were synthesized. A DNA fragment encoding the 522 central residues of the helix region of human collagen Type I α1 was amplified by PCR using the oligonucleotides 65 and 66 as primers and the plasmid named as pBlue-HsCOL1A1 (see, Example (1-10)) as a template.
The oligonucleotides 65 and 66 used were:
(a) oligonucleotide 65: GGACCCCAGGGCCCCG (SEQ ID NO:65), and
(b) oligonucleotide 66: ATCAGCACCAGGGGATCCTTTC (SEQ ID NO:66).
The composition of the reaction solutions is given as follows:
(a) cDNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 25 cycles of denaturation at 94°C for 15 seconds and each of annealing and extension at 68°C for 1 minute 40 seconds, followed by additionally keeping the reaction solution at 68°C for 1 minute 40 seconds.
An about 1.6-kb DNA fragment resulted from the PCR was purified using MinElute PCR Purification Kit (QIAGEN). To the DNA fragment obtained, a phosphate group was added at the 5' end using T4 polynucleotide kinase (Takara Bio Inc.). Then, the phosphorylated, about 1.6-kb DNA fragment was separated by agarose gel electrophoresis, followed by extraction and purification from the gel using MinElute Gel Extraction Kit (QIAGEN).
The oligonucleotides 63 and 59 mentioned below were synthesized. A DNA fragment which was composed of the AOX1 promoter, a DNA fragment encoding the N-terminal non-helix region and 27 N-terminal residues of the helix region of human collagen Type I α1, a DNA fragment encoding the 522 central residues of the helix region of human collagen Type I α1, a DNA fragment encoding the C-terminal non-helix region and 15 C-terminal residues of the helix region of human collagen Type I α1, the AOX1 terminator, and a portion of the vector plasmid was amplified by PCR using the oligonucleotides 63 and 59 as primers and the plasmid named as pAT017 (see, Example (2-5-1)) as a template.
The oligonucleotides 63 and 59 used were:
(a) oligonucleotide 63: ATCAGCTCCTGGTGATCCCTTTTC (SEQ ID NO:63), and
(b) oligonucleotide 59: GGACCACCAGGGCCGC (SEQ ID NO:59).
The composition of the reaction solutions is given as follows:
(a) cDNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 25 cycles of denaturation at 94°C for 15 seconds and each of annealing and extension at 68°C for 7 minutes 40 seconds, followed by additionally keeping the reaction solution at 68°C for 7 minutes 40 seconds.
An about 7.3-kb DNA fragment resulted from the PCR was purified using MinElute PCR Purification Kit (QIAGEN). The DNA fragment obtained was dephosphorylated with an alkaline phosphatase. Then, the dephosphorylated, about 7.3-kb DNA fragment was separated by agarose gel electrophoresis, followed by extraction and purification from the gel using MinElute Gel Extraction Kit (QIAGEN).
The phosphorylated, about 1.6-kb DNA fragment and the dephosphorylated, about 7.3-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding the 522 central residues of the helix region of human collagen Type I α1 had been inserted (which may be referred to hereinafter as pAT027) was isolated from the cultured cells to give pAT027.

### (2-5-3) Construction of pEXP-HA-HsCOL1A1 M500X2

The plasmid named as pAT027 (see, Example (2-5-2)) was digested with restriction enzymes Eco52I and SpeI. An about 5.6-kb DNA fragment consisting of the AOX1 promoter and the DNA fragment encoding the fusion polypeptide of the N-terminal non-helix region, 27 N-terminal residues of the helix region, a dimer made of the 522 central residues of the helix region, 15 C-terminal residues of the helix region and the C-terminal non-helix region of human collagen Type I α1 was isolated by agarose gel electrophoresis, followed by extraction and purification from the gel using MinElute Gel Extraction Kit (QIAGEN).
The plasmid named as pEXH002 (see, Example (2-3-4)) was digested with restriction enzymes Eco52I and SpeI. An about 6.6-kb DNA fragment was isolated by agarose gel electrophoresis, followed by extraction and purification from the gel using MinElute Gel Extraction Kit (QIAGEN).
The about 5.6-kb DNA fragment and the about 6.6-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding the AOX1 promoter and the fusion polypeptide of the N-terminal non-helix region, 27 N-terminal residues of the helix region, a dimer made of the 522 central residues of the helix region, 15 C-terminal residues of the helix region and the C-terminal non-helix region of human collagen Type I α1 had been inserted (which may be referred to hereinafter as pEXP-HA-HsCOL1A1 M500X2) was isolated from the cultured cells to give pEXP-HA-HsCOL1A1 M500X2.

### (2-5-4) Construction of pAT018

The oligonucleotides 67 and 68 mentioned below were synthesized. A DNA fragment encoding the 522 central residues of the helix region of human collagen Type I α2 was amplified by PCR using the oligonucleotides 67 and 68 as primers and the plasmid named as pTS002 (see, Example (2-3-7)) as a template.
The oligonucleotides 67 and 68 used were:
(a) oligonucleotide 67: GGGCCAGTTGGCGCAG (SEQ ID NO:67), and
(b) oligonucleotide 68: TGCTTCTCCAGATGGTCCTTTCTC (SEQ ID NO:68).
The composition of the reaction solutions is given as follows:
(a) cDNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 25 cycles of denaturation at 94°C for 15 seconds and each of annealing and extension at 68°C for 1 minute 40 seconds, followed by additionally keeping the reaction solution at 68°C for 1 minute 40 seconds.
An about 1.6-kb DNA fragment resulted from the PCR was purified using MinElute PCR Purification Kit (QIAGEN). To the DNA fragment obtained, a phosphate group was added at the 5' end using T4 polynucleotide kinase (Takara Bio Inc.). Then, the phosphorylated, about 1.6-kb DNA fragment was separated by agarose gel electrophoresis, followed by extraction and purification from the gel using MinElute Gel Extraction Kit (QIAGEN).
The oligonucleotides 61 and 69 mentioned below were synthesized. A DNA fragment which was composed of the AOX1 promoter, a DNA fragment encoding the N-terminal non-helix region and 27 N-terminal residues of the helix region of human collagen Type I α2, a DNA fragment encoding the C-terminal non-helix region and 15 C-terminal residues of the helix region of human collagen Type I α2, the AOX1 terminator, and a portion of the vector plasmid was amplified by PCR using the oligonucleotides 61 and 69 as primers and the plasmid named as pTS002 (see, Example (2-3-7)) as a template.
The oligonucleotides 61 and 69 used were:
(a) oligonucleotide 61: GGACCTCCTGGCCCACC (SEQ ID NO:61), and
(b) oligonucleotide 69: TGCGGGTCCTTGGAATC (SEQ ID NO:69).
The composition of the reaction solutions is given as follows:
(a) cDNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 25 cycles of denaturation at 94°C for 15 seconds and each of annealing and extension at 68°C for 6 minutes, followed by additionally keeping the reaction solution at 68°C for 6 minutes.
An about 5.4-kb DNA fragment resulted from the PCR was purified using MinElute PCR Purification Kit (QIAGEN). The DNA fragment obtained was dephosphorylated with an alkaline phosphatase. Then, the dephosphorylated, about 5.4-kb DNA fragment was separated by agarose gel electrophoresis, followed by extraction and purification from the gel using MinElute Gel Extraction Kit (QIAGEN).
The phosphorylated, about 1.6-kb DNA fragment and the dephosphorylated, about 5.4-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding the 522 central residues of the helix region of human collagen Type I α2 had been inserted (which may be referred to hereinafter as pAT018) was isolated from the cultured cells to give pAT018.

### (2-5-5) Construction pAT018-Eco52I

The plasmid named as pAT018 (see, Example (2-5-4)) was digested with restriction enzymes AccIII and PmeI. An about 2.6-kb fragment comprising a 3' region of the AOX1 promoter, an DNA fragment encoding the N-terminal non-helix region and 27 N-terminal residues of the helix region of human collagen Type I α2 and an DNA fragment encoding the 522 central residues of the helix region of human collagen Type I α2 was isolated by agarose gel electrophoresis, followed by extraction and purification from the gel using MinElute Gel Extraction Kit (QIAGEN).
The plasmid named as pYHsCOL1A2 Exp unit-Eco52I (see, Example (2-3-8)) was digested with restriction enzymes AccIII and PmeI. An about 4.9-kb DNA fragment was separated by agarose gel electrophoresis, followed by extraction and purification from the gel using MinElute Gel Extraction Kit (QIAGEN).
The about 2.6-kb DNA fragment and the about 4.9-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of kanamycin, the E. coli cells which had been transformed were inoculated and cultured. A colony formed on the agar medium was inoculated into LB medium containing 50 µg/ml of kanamycin and incubated with shaking (37°C, 17 hours). Then, using QIAprep Spin Miniprep Kit (QIAGEN), the plasmid into which the fragment comprising the 3' region of the AOX1 promoter, the DNA fragment encoding the N-terminal non-helix region and 27 N-terminal residues of the helix region of human collagen Type I α2 and the DNA fragment encoding 522 central residues of the helix region of human collagen Type I α2 had been inserted (which may be referred to hereinafter as pAT018-Eco52I) was isolated from the cultured cells to give pAT018-Eco52I.

### (2-5-6) Construction of pAT028-Eco52I

The oligonucleotides 70 and 71 mentioned below were synthesized. A DNA fragment encoding the 522 central residues of the helix region of human collagen Type I α2 was amplified by PCR using the oligonucleotides 70 and 71 as primers and the plasmid named as pUC18-HsCOL1A2 (see, Example (1-11)) as a template.
The oligonucleotides 70 and 71 used were:
(a) oligonucleotide 70: GGCCCTGTTGGTGCTGC (SEQ ID NO:70), and
(b) oligonucleotide 71: AGCCTCTCCAGAGGGACCCTT (SEQ ID NO:71).
The composition of the reaction solutions is given as follows:
(a) cDNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 25 cycles of denaturation at 94°C for 15 seconds and each of annealing and extension at 68°C for 1 minute 40 seconds, followed by additionally keeping the reaction solution at 68°C for 1 minute 40 seconds.
An about 1.6-kb DNA fragment resulted from the PCR was purified using MinElute PCR Purification Kit (QIAGEN). To the DNA fragment obtained, a phosphate group was added at the 5' end using T4 polynucleotide kinase (Takara Bio Inc.). Then, the phosphorylated, about 1.6-kb DNA fragment was separated by agarose gel electrophoresis, followed by extraction and purification from the gel using MinElute Gel Extraction Kit (QIAGEN).
The oligonucleotides 68 and 61 mentioned below were synthesized. A DNA fragment which was composed of the AOX1 promoter, a DNA fragment encoding the N-terminal non-helix region and 27 N-terminal residues of the helix region of human collagen Type I α2, a DNA fragment encoding the 522 central residues of the helix region of human collagen Type I α2, a DNA fragment encoding the C-terminal non-helix region and 15 C-terminal residues of the helix region of human collagen Type I α2, the AOX1 terminator and a portion of the vector plasmid was amplified by PCR using the oligonucleotides 68 and 61 as primers and the plasmid named as pAT018-Eco52I (see, Example (2-5-5)) as a template.
The oligonucleotides 68 and 61 used were:
(a) oligonucleotide 68: TGCTTCTCCAGATGGTCCTTTCTC (SEQ ID NO:68), and
(b) oligonucleotide 61: GGACCTCCTGGCCCACC (SEQ ID NO:61).
The composition of the reaction solutions is given as follows:
(a) DNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 25 cycles of denaturation at 94°C for 15 seconds and each of annealing and extension at 68°C for 7 minutes 40 seconds, followed by additionally keeping the reaction solution at 68°C for 7 minutes 40 seconds.
An about 7.6-kb DNA fragment resulted from the PCR was purified using MinElute PCR Purification Kit (QIAGEN). The DNA fragment obtained was dephosphorylated with an alkaline phosphatase. Then, the dephosphorylated, about 7.3-kb DNA fragment was separated by agarose gel electrophoresis, followed by extraction and purification from the gel using MinElute Gel Extraction Kit (QIAGEN).
The phosphorylated, about 1.6-kb DNA fragment and the dephosphorylated, about 7.6-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding the 522 central residues of the helix region of human collagen Type I α2 had been inserted (which may be referred to hereinafter as pAT028-Eco52I) was isolated from the cultured cells to give pAT028-Eco52I.

### (2-5-7) Construction of pEXP-HA-HsCOL1A2-1A1 M500X2

The plasmid named as pAT028-Eco52I (see, Example (2-5-6)) was digested with a restriction enzyme Eco52I. An about 5.6-kb DNA fragment which comprised the expression cassette for the fusion polypeptide consisting of the N-terminal non-helix region, 27 N-terminal residues of the helix region, a dimer made of the 522 central residues of the helix region, 15 C-terminal residues of the helix region and the C-terminal non-helix region of human collagen Type I α2 was separated by agarose gel electrophoresis, followed by extraction and purification from the gel using MinElute Gel Extraction Kit (QIAGEN).
The plasmid named as pEXP-HA-HsCOL1A M500X2 (see, Example (2-5-3)) was digested with a restriction enzyme Eco52I, dephosphorylated with an alkaline phosphatase, and then purified using MinElute Reaction Cleanup Kit (QIAGEN).
The about 5.6-kb DNA fragment and the dephosphorylated plasmid were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment comprising the expression cassette for the fusion polypeptide consisting of the N-terminal non-helix region, 27 N-terminal residues of the helix region, a dimer made of the 522 central residues of the helix region, 15 C-terminal residues of the helix region and the C-terminal non-helix region of human collagen Type I α2 had been inserted (which may be referred to hereinafter as pEXP-HA-HsCOL1A2-1A1 M500X2) was purified from the cultured cells to give pEXP-HA-HsCOL1A2-1A1 M500X2.

(2-6) Preparation of a plasmid (pEXP-HA-HsCOL3A1) for introducing an expression cassette for human collagen Type III α1

### (2-6-1) Construction of pSN026

The oligonucleotides 72 and 73 mentioned below were synthesized. A DNA fragment which had a restriction enzyme recognition sequence added at each end of the DNA encoding human collagen Type III α1 was amplified by PCR using the oligonucleotides 72 and 73 as primers and the plasmid named as pUC19-HsCOL3A1 (see, Example (1-12)) as a template. The oligonucleotides 72 and 73 used were:
(a) oligonucleotide 72: TATTCGAAACGATGATGAGCTTTGTGCAAAAGGGG (SEQ ID NO:72), and
(b) oligonucleotide 73: TTACTAGTTTATAAAAAGCAAACAGGGCCAACGT (SEQ ID NO:73).
The composition of the reaction solutions is given as follows:
(a) cDNA solution (10 ng/µl), 1 µl;
(b) dNTPs (mix, 2 mM each), 5 µl;
(c) MgSO₄ (25 mM), 2 µl;
(d) primers (10 pmol/µl), 1.5 µl each;
(e) 10x PCR buffer for KOD-plus- (Toyobo Co., Ltd.), 5 µl;
(f) KOD-plus- DNA polymerase (1 U/µl, Toyobo Co., Ltd.), 1 µl;
(g) sterile distilled water 33 µl.
The PCR was conducted under conditions where the reaction solution was heated at 94°C for 2 minutes and then subjected to 5 cycles of denaturation at 94°C for 15 seconds, annealing at 60°C for 30 seconds, and extension at 68°C for 5 minutes and then 23 cycles of denaturation at 94°C for 15 seconds, and each of annealing and extension at 68°C for 5 minutes, followed by additionally keeping the reaction solution at 68°C for 5 minutes.
An about 4.4-kb DNA fragment resulted from the PCR was digested with restriction enzymes BspT104I and SpeI. Then, the digested, about 4.4-kb DNA fragment was isolated by agarose gel electrophoresis, followed by extraction and purification from the gel using MinElute Gel Extraction Kit (QIAGEN).
The plasmid named as pSN006 (see, Example (2-3-3)) was digested with restriction enzymes BspT104I and SpeI. Then, an about 4.2-kb DNA fragment was separated by agarose gel electrophoresis, followed by extraction and purification from the gel using MinElute Gel Extraction Kit (QIAGEN).
The digested, about 4.4-kb DNA fragment and the about 4.2-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment having the restriction enzyme recognition sequence added at each end of the DNA encoding human collagen Type III α1 had been inserted (which may be referred to hereinafter as pSN026) was isolated from the cultured cells to give pSN026.

### (2-6-2) Construction of pEXP-HA-HsCOL3A1

The plasmid named as pSN026 (see, Example (2-6-1)) was digested with restriction enzymes Eco52I and SpeI. An about 5.3-kb DNA fragment encoding the AOX1 promoter and human collagen Type III α1 was isolated by agarose gel electrophoresis, followed by extraction and purification from the gel using MinElute Gel Extraction Kit (QIAGEN).
The plasmid named as pEXH002 (see, Example (2-3-4)) was digested with restriction enzymes Eco52I and SpeI. An about 6.6-kb DNA fragment was isolated by agarose gel electrophoresis, followed by extraction and purification from the gel using MinElute Gel Extraction Kit (QIAGEN).
The about 5.3-kb DNA fragment and the about 6.6-kb DNA fragment were ligated, and the resulting ligation solution was used for transformation of E. coli (Competent high DH5α, Toyobo Co., Ltd.). For the ligation reaction, a Ligation Kit ver 2.1 (Takara Bio Inc.) was used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding the AOX1 promoter and human collagen Type III α1 had been inserted (which may be referred to hereinafter as pEXP-HA-HsCOL3A1) was isolated from the cultured cells to give pEXP-HA-HsCOL3A1.

(2-7) Preparation of a plasmid (pEXP-HA-YHsCOL2A1) for introducing an expression cassette for human collagen Type II α1

### (2-7-1) Construction of pIM200

The plasmid named as pUC57-YHsCOL2A1 (see, Example (1-21)) was digested with restriction enzymes BspT104I and SpeI. An about 4.4-kb DNA fragment encoding human collagen Type II α1 was separated by agarose gel electrophoresis, followed by extraction and purification from the gel using MinElute Gel Extraction Kit (QIAGEN).
The plasmid named as pSN006 (see, Example (2-3-3)) was digested with restriction enzymes BspT104I and SpeI. An about 4.2-kb DNA fragment was separated by agarose gel electrophoresis, followed by extraction and purification from the gel using MinElute Gel Extraction Kit (QIAGEN).
The about 4.4-kb DNA fragment and the about 4.2-kb DNA fragment are ligated, and the resulting ligation solution is used for transformation of E. coli (Competent high DH5α, Toyobo Co., Ltd.). For the ligation reaction, a Ligation Kit ver 2.1 (Takara Bio Inc.) is used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding human collagen Type II α1 had been inserted (which may be referred to hereinafter as pIM200) was isolated from the cultured cells to give pIM200.

### (2-7-2) Construction of pEXP-HA-YHsCOL2A1

The plasmid named as pIM200 (see, Example (2-7-1)) was digested with restriction enzymes Eco52I and SpeI. An about 5.3-kb DNA fragment encoding the AOX1 promoter and human collagen Type II α1 was isolated by agarose gel electrophoresis, followed by extraction and purification from the gel using MinElute Gel Extraction Kit (QIAGEN).
The plasmid named as pEXH002 (see, Example (2-3-4)) was digested with restriction enzymes Eco52I and SpeI. An about 6.6-kb DNA fragment was isolated by agarose gel electrophoresis, followed by extraction and purification from the gel using MinElute Gel Extraction Kit (QIAGEN).
The about 5.3-kb DNA fragment and the about 6.6-kb DNA fragment are ligated, and the resulting ligation solution is used for transformation of E. coli (Competent high DH5α, Toyobo Co., Ltd.). For the ligation reaction, a Ligation Kit ver 2.1 (Takara Bio Inc.) is used.
On LB agar medium containing 50 µg/ml of ampicillin, the E. coli cells which had been transformed were inoculated and cultured. A colony formed on the agar medium was inoculated into LB medium containing 50 µg/ml of ampicillin and incubated with shaking (37°C, 17 hours). Then, using QIAprep Spin Miniprep Kit (QIAGEN), the plasmid into which the DNA fragment encoding the AOX1 promoter and human collagen Type II α1 had been inserted (which may be referred to hereinafter as pEXP-HA-YHsCOL2A1) was isolated from the cultured cells to give pEXP-HA-YHsCOL2A1.

Example 3 (Preparation of yeasts having expression cassettes introduced therein)

### (3-1) Preparation of a yeast in which an expression cassette for prolyl 4-hydroxylase α1 subunit and an expression cassette for prolyl 4-hydroxylase β subunit are introduced

### (3-1-1) Transformation of yeast

Komagataella pastoris strain PPY12 (ATCC 204163) which was commercially available from the American Type Culture Collection (ATCC) was purchased.
Using an electroporation method, strain PPY12 was transformed with pEXP-A-P4Hbsig(-)A1rev obtained in Example 2-1.
Into 100 ml of YPD liquid medium (1% Yeast Extract, 2% peptone, and 2% glucose), strain PPY12 was inoculated, and cultured at 30°C until the turbidity of cell culture (OD₆₀₀) reached about 10. The cells were collected from the cultured medium and the supernatant was removed. The cells were then suspended in 1M sorbitol at a turbidity of OD₆₆₀ = about 150 to prepare a cell suspension.
Ten (10) µg of pEXP-A-P4Hbsig(-)A1rev (see, Example (2-1-12)) was digested with a restriction enzyme AatII. The DNA was collected through ethanol precipitation and dissolved in 5 µl of 10 mM Tris-HCl to prepare a DNA solution.
A mixture was prepared by combining 100 µl of the cell suspension and 5 µl of the DNA solution. To the mixture, a pulse was applied using a gene transfer device (ECM630, BTX), and then the mixture was spread on MD agar medium (1.34% Yeast Nitrogen Base, 4 × 10⁻⁵% biotin, 2% glucose, 0.005% histidine, 2% agar) and cultured at 30°C for 48 hours. Colonies formed on the MD agar medium were isolated as transformants. Strain 050525-5-3 was obtained as a transformant.

### (3-1-2) Determination of expression of prolyl 4-hydroxylase α1 subunit and prolyl 4-hydroxylase β subunit

Strain 050525-5-3 was inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol, 0.005% histidine] which was prepared in a 500-ml baffled Erlenmeyer flask, and cultured with shaking at 30°C for 24 hours. A cell suspension was prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol, 0.005% histidine] which was prepared in a 500-ml baffled Erlenmeyer flask, at a final concentration of OD₆₆₀ = about 10, and cultured with shaking at 30°C for 72 hours. Every about 12 hours during this period, 50% methanol was added in an amount of 0.5 ml each.
The cells were collected from the resultant culture and the supernatant was removed. After that, the cells were subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption was centrifuged to collect the supernatant of the cell disrupted solution to prepare a soluble fraction.
An aliquot of the soluble fraction was mixed with a sample buffer (Nacalai Tesque., Inc.), and the mixture was kept at 100°C for 5 minutes, followed by electrophoresis on SDS-polyacrylamide gel. The electrophoresis, in which the an ERICA-MP (DRC) was used for electrophoresis apparatus and 5-15% gradient gel (XV PANTERA MP gel, DRC) was used for the electrophoresis gel, was performed at 200 V for 20 minutes. After the electrophoresis was completed, the acrylamide gel was subjected to electro-blotting onto a PVDF membrane (Immobilon-P, Millipore Corp.). The electro-blotting, in which MINICA-MP (DRC) was used for the blotting apparatus, was performed at 40 V for 30 minutes.
A half of the resulting membrane was used to carry out western blotting analysis to detect prolyl 4-hydroxylase α1 subunit. Blocking one (Nacalai Tesque., Inc.) was used for blocking of the membrane, Can Get Signal (Toyobo Co., Ltd.) was used for antibody reactions, 2,000-times diluted anti-rat prolyl 4-hydroxylase α1 subunit mouse antibody (63167, MP Biomedicals) was used for the primary antibody, and 20,000-times diluted anti-mouse IgG-HRP Linked sheep antibody (NA-931, GE Healthcare Bio Science) was used for the secondary antibody. The secondary antibody was detected by chemiluminescence using ECL Western Blotting Detection System (GE Healthcare Bio Science). The chemiluminescence was detected and quantitatively determined with an image analyzer (LAS-3000UVmini, Fujifilm Corporation). The results of western blotting analysis demonstrated that a signal was detected at the position corresponding to the molecular weight of the prolyl 4-hydroxylase α1 subunit, and thus, it was shown that the prolyl 4-hydroxylase α1 subunit was produced.
The other half of the resulting membrane was used to carry out western blotting analysis to detect prolyl 4-hydroxylase β subunit. Blocking one (Nacalai Tesque., Inc.) was used for blocking of the membrane, Can Get Signal (Toyobo Co., Ltd.) was used for antibody reactions, 5,000-times diluted anti-human prolyl 4-hydroxylase β subunit rabbit antibody (SPA-890, Stressgen) was used for the primary antibody, and 20,000-times diluted anti-rabbit IgG-HRP Linked donkey antibody (NA-934, GE Healthcare Bio Science) was used for the secondary antibody. The secondary antibody was detected by chemiluminescence using ECL Western Blotting Detection System (GE Healthcare Bio Science). The chemiluminescence was detected and quantitatively determined with an image analyzer (LAS-3000UVmini, Fujifilm Corporation). The results of western blotting analysis demonstrated that strain 050525-5-3 provided a signal that was detected at the position corresponding to the molecular weight of the prolyl 4-hydroxylase β subunit, and thus, it was shown that the prolyl 4-hydroxylase β subunit was produced.

(3-2) Preparation of a yeast in which an expression cassette for prolyl 4-hydroxylase α1 subunit, an expression cassette for prolyl 4-hydroxylase β subunit, an expression cassette for human collagen Type I α1 and an expression cassette for human collagen Type I α2 are introduced

### (3-2-1) Transformation of yeast

Using an electroporation method, strain 050525-5-3 (see, Example (3-1-1)) was transformed with pEXP-HA-YHsCOL1A2-1A1 obtained in Example 2-3-9.
Into 100 ml of YPD liquid medium (1% Yeast Extract, 2% peptone, and 2% glucose), strain 050525-5-3 was inoculated, and cultured at 30°C until the turbidity of cell culture (OD₆₆₀) reached about 10. The cells were collected from the cultured medium and the supernatant was removed. The cells were then suspended in 1M sorbitol at a turbidity of OD₆₆₀ = about 150 to prepare a cell suspension.
Ten (10) µg of pEXP-HA-YHsCOL1A2-1A1 (see, Example (2-3-9)) was digested with a restriction enzyme XbaI. The DNA was collected through ethanol precipitation and dissolved in 5 µl of 10 mM Tris-HCl to prepare a DNA solution.
A mixture was prepared by combining 100 µl of the cell suspension and 5 µl of the DNA solution. To the mixture, a pulse was applied using a gene transfer device (ECM630, BTX), and then the mixture was spread on MD agar medium (1.34% Yeast Nitrogen Base, 4 × 10⁻⁵% biotin, 2% glucose, 2% agar) and cultured at 30°C for 48 hours. Colonies formed on the MD agar medium were isolated as transformants. Strain 070327-1-11 was obtained as a transformant.

### (3-2-2) Determination of expression of human collagen Type I α1 and human collagen Type I α2

Strain 070327-1-11 (see, Example (3-2-1)) was inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which was prepared in a 500-ml baffled Erlenmeyer flask, and cultured with shaking at 30°C for 24 hours. A cell suspension was prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which was prepared in a 500-ml baffled Erlenmeyer flask, at a final concentration of OD₆₆₀ = about 10, and cultured with shaking at 30°C for 72 hours. Every about 12 hours during this period, 50% methanol was added in an amount of 0.5 ml each.
The cells were collected from the resultant culture and the supernatant was removed. After that, the cells were subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption was centrifuged to collect the supernatant of the cell disrupted solution to prepare a soluble fraction.
An aliquot of the soluble fraction was mixed with a sample buffer (Nacalai Tesque., Inc.), and the mixture was kept at 100°C for 5 minutes, followed by electrophoresis on SDS-polyacrylamide gel. The electrophoresis, in which ERICA-MP (DRC) was used for the electrophoresis apparatus and 5-15% gradient gel (XV PANTERA MP gel, DRC) was used for the electrophoresis gel, was performed at 200 V for 20 minutes. After the electrophoresis was completed, the resulting acrylamide gel was subjected to staining with a CBB staining solution to detect protein bands. The results demonstrated that strain 070327-1-1 provided bands that were detected at the molecular weights corresponding to a procollagen of collagen Type I α1 and corresponding to a procollagen of collagen Type I α2, and thus it was shown that human collagen Type I α1 and human collagen Type I α2 was produced.

(3-3) Preparation of a yeast in which an expression cassette for prolyl 4-hydroxylase α1 subunit, an expression cassette for prolyl 4-hydroxylase β subunit, an expression cassette for a fusion polypeptide which consists of the N-terminal non-helix region, 60 N-terminal residues of the helix region, 15 C-terminal residues of the helix region and the C-terminal non-helix region of human collagen Type I α1, and an expression cassette for a fusion polypeptide which consists of the N-terminal non-helix region, 60 N-terminal residues of the helix region, 15 C-terminal residues of the helix region and the C-terminal non-helix region of human collagen Type I α2 are introduced

### (3-3-1) Transformation of yeast

Using an electroporation method, strain 050525-5-3 (see, Example (3-1-1)) was transformed with pEXP-HA-YHsCOL1A2-1A1 N60C15 obtained in Example 2-4-4.
Into 100 ml of YPD liquid medium (1% Yeast Extract, 2% peptone, and 2% glucose), strain 050525-5-3 (see, Example (3-1-1)) was inoculated, and cultured at 30°C until the turbidity of cell culture (OD₆₆₀) reached about 10. The cells were collected from the cultured medium and the supernatant was removed. The cells were then suspended in 1M sorbitol at a turbidity of OD₆₆₀ = about 150 to prepare a cell suspension.
Ten (10) µg of pEXP-HA-YHsCOL1A2-1A1 N60C15 (see, Example (2-4-4)) was digested with a restriction enzyme XbaI. The DNA was collected through ethanol precipitation and dissolved in 5 µl of 10 mM Tris-HCl to prepare a DNA solution.
A mixture was prepared by combining 100 µl of the cell suspension and 5 µl of the DNA solution. To the mixture, a pulse was applied using a gene transfer device (ECM630, BTX), and then the mixture was spread on MD agar medium (1.34% Yeast Nitrogen Base, 4 × 10⁻⁵% biotin, 2% glucose, 2% agar) and cultured at 30°C for 48 hours. Colonies formed on the MD agar medium were isolated as transformants. Strain 080118-3-3 was obtained as a transformant.

(3-3-2) Determination of expression of a fusion polypeptide which consists of the N-terminal non-helix region, 60 N-terminal residues of the helix region, 15 C-terminal residues of the helix region and the C-terminal non-helix region of human collagen Type I α1 and an expression cassette for a fusion polypeptide which consists of the N-terminal non-helix region, 60 N-terminal residues of the helix region, 15 C-terminal residues of the helix region and the C-terminal non-helix region of human collagen Type I α2
Strain 080118-3-3 (see, Example (3-3-1)) was inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which was prepared in a 500-ml baffled Erlenmeyer flask, and cultured with shaking at 30°C for 24 hours. A cell suspension was prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which was prepared in a 500-ml baffled Erlenmeyer flask, at a final concentration of OD₆₆₀ = about 10, and cultured with shaking at 30°C for 72 hours. Every about 12 hours during this period, 50% methanol was added in an amount of 0.5 ml each.
The cells were collected from the resultant culture and the supernatant was removed. After that, the cells were subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption was centrifuged to collect the supernatant of the cell disrupted solution to prepare a soluble fraction.
An aliquot of the soluble fraction was mixed with a sample buffer (Nacalai Tesque., Inc.), and the mixture was kept at 100°C for 5 minutes, followed by electrophoresis on SDS-polyacrylamide gel. The electrophoresis, in which ERICA-MP (DRC) was used for the electrophoresis apparatus and 5-15% gradient gel (XV PANTERA MP gel, DRC) was used for the electrophoresis gel, was performed at 200 V for 20 minutes. After the electrophoresis was completed, the acrylamide gel was subjected to electro-blotting onto a PVDF membrane (Immobilon-P, Millipore Corp.). The electro-blotting, in which MINICA-MP (DRC) was used for the blotting apparatus, was performed at 40 V for 30 minutes.
A half of the resulting membrane was used to carry out western blotting analysis to detect a procollagen of the fusion polypeptide consisting of the N-terminal non-helix region, 60 N-terminal residues of the helix region, 15 C-terminal residues of the helix region and the C-terminal non-helix region of human collagen Type I α1. Blocking one (Nacalai Tesque., Inc.) was used for blocking of the membrane, Can Get Signal (Toyobo Co., Ltd.) was used for antibody reactions, 2,000-times diluted anti-human procollagen Type I α1 goat antibody (SC-8782, Santa Cruz) was used for the primary antibody, and 2,000-times diluted anti-goat IgG-HRP Linked donkey antibody (SC-2033, Santa Cruz) was used for the secondary antibody. The secondary antibody was detected by chemiluminescence using ECL Western Blotting Detection System (GE Healthcare Bio Science). The chemiluminescence was detected and quantitatively determined with an image analyzer (LAS-3000UVmini, Fujifilm Corporation). The results of western blotting analysis demonstrated that strain 080118-3-3 provided a signal that was detected at the position corresponding to the molecular weight of a procollagen of the fusion polypeptide consisting of the N-terminal non-helix region, 60 N-terminal residues of the helix region, 15 C-terminal residues of the helix region and the C-terminal non-helix region of human collagen Type I α1, and thus, it was shown that the fusion polypeptide consisting of the N-terminal non-helix region, 60 N-terminal residues of the helix region, 15 C-terminal residues of the helix region and the C-terminal non-helix region of human collagen Type I α1 was produced.
The other half of the resulting membrane was used to carry out western blotting analysis to detect a procollagen of the fusion polypeptide consisting of the N-terminal non-helix region, 60 N-terminal residues of the helix region, 15 C-terminal residues of the helix region and the C-terminal non-helix region of human collagen Type I α2. Blocking one (Nacalai Tesque., Inc.) was used for blocking of the membrane, Can Get Signal (Toyobo Co., Ltd.) was used for antibody reactions, 5,000-times diluted anti-human collagen Type I rabbit antibody (ab292, abcam) was used for the primary antibody, and 10,000-times diluted anti-rabbit IgG-HRP Linked donkey antibody (NA-934, GE Healthcare Bio Science) was used for the secondary antibody. The secondary antibody was detected by chemiluminescence using ECL Western Blotting Detection System (GE Healthcare Bio Science). The chemiluminescence was detected and quantitatively determined with an image analyzer (LAS-3000UVmini, Fujifilm Corporation). The results of western blotting analysis demonstrated that strain 080118-3-3 (see, Example (3-7-1)) provided a signal that was detected at the position corresponding to the molecular weight of a procollagen of the fusion polypeptide consisting of the N-terminal non-helix region, 60 N-terminal residues of the helix region, 15 C-terminal residues of the helix region and the C-terminal non-helix region of human collagen Type I α2, and thus, it was shown that the fusion polypeptide consisting of the N-terminal non-helix region, 60 N-terminal residues of the helix region, 15 C-terminal residues of the helix region and the C-terminal non-helix region of human collagen Type I α2 was produced.

(3-4) Preparation of a yeast in which an expression cassette for prolyl 4-hydroxylase α1 subunit, an expression cassette for prolyl 4-hydroxylase β subunit, an expression cassette for a fusion polypeptide which consists of the N-terminal non-helix region, 27 N-terminal residues of the helix region, a dimer made of 522 central residues of the helix region, 15 C-terminal residues of the helix region and the C-terminal non-helix region of human collagen Type I α1, and an expression cassette for a fusion polypeptide which consists of the N-terminal non-helix region, 27 N-terminal residues of the helix region, a dimer made of 522 central residues of the helix region, 15 C-terminal residues of the helix region and the C-terminal non-helix region of human collagen Type I α2 are introduced

### (3-4-1) Transformation of yeast

Using an electroporation method, strain 050525-5-3 (see, Example (3-1-1)) was transformed with pEXP-HA-HsCOL1A2-1A1 M500X2 obtained in Example 2-5-7.
Into 100 ml of YPD liquid medium (1% Yeast Extract, 2% peptone, and 2% glucose), strain 050525-5-3 (see, Example (3-1-1)) was inoculated, and cultured at 30°C until the turbidity of cell culture (OD₆₆₀) reached about 10. The cells were collected from the cultured medium and the supernatant was removed. The cells were then suspended in 1M sorbitol at a turbidity of OD₆₆₀ = about 150 to prepare a cell suspension.
Ten (10) µg of pEXP-HA-HsCOL1A2-1A1 M500X2 (see, Example (2-5-7)) was digested with a restriction enzyme XbaI. The DNA was collected through ethanol precipitation and dissolved in 5 µl of 10 mM Tris-HCl to prepare a DNA solution.
A mixture was prepared by combining 100 µl of the cell suspension and 5 µl of the DNA solution. To the mixture, a pulse was applied using a gene transfer device (ECM630, BTX), and then the mixture was spread on MD agar medium (1.34% Yeast Nitrogen Base, 4 × 10⁻⁵% biotin, 2% glucose, 2% agar) and cultured at 30°C for 48 hours. Colonies formed on the MD agar medium were isolated as transformants. Strain 080801-2-4 was obtained as a transformant.

(3-4-2) Determination of expression of a fusion polypeptide which consists of the N-terminal non-helix region, 27 N-terminal residues of the helix region, a dimer made of 522 central residues of the helix region, 15 C-terminal residues of the helix region and the C-terminal non-helix region of human collagen Type I α1, and of a fusion polypeptide which consists of the N-terminal non-helix region, 27 N-terminal residues of the helix region, a dimer made of 522 central residues of the helix region, 15 C-terminal residues of the helix region and the C-terminal non-helix region of human collagen Type I α2
Strain 080801-2-4 (see, Example (3-4-1)) was inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which was prepared in a 500-ml baffled Erlenmeyer flask, and cultured with shaking at 30°C for 24 hours. A cell suspension was prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which was prepared in a 500-ml baffled Erlenmeyer flask, at a final concentration of OD₆₆₀ = about 10, and cultured with shaking at 30°C for 72 hours. Every about 12 hours during this period, 50% methanol was added in an amount of 0.5 ml each.
The cells were collected from the resultant culture and the supernatant was removed. After that, the cells were subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption was centrifuged to collect the supernatant of the cell disrupted solution to prepare a soluble fraction.
An aliquot of the soluble fraction was mixed with a sample buffer (Nacalai Tesque., Inc.), and the mixture was kept at 100°C for 5 minutes, followed by electrophoresis on SDS-polyacrylamide gel. The electrophoresis, in which ERICA-MP (DRC) was used for the electrophoresis apparatus and 5-15% gradient gel (XV PANTERA MP gel, DRC) was used for the electrophoresis gel, was performed at 200 V for 20 minutes. After the electrophoresis was completed, the acrylamide gel was subjected to electro-blotting onto a PVDF membrane (Immobilon-P, Millipore Corp.). The electro-blotting, in which MINICA-MP (DRC) was used for the blotting apparatus, was performed at 40 V for 30 minutes.
A half of the resulting membrane was used to carry out western blotting analysis to detect a procollagen of the fusion polypeptide consisting of the N-terminal non-helix region, 27 N-terminal residues of the helix region, a dimer made of 522 central residues of the helix region, 15 C-terminal residues of the helix region and the C-terminal non-helix region of human collagen Type I α1. Blocking one (Nacalai Tesque., Inc.) was used for blocking of the membrane, Can Get Signal (Toyobo Co., Ltd.) was used for antibody reactions, 2,000-times diluted anti-human procollagen Type I α1 goat antibody (SC-8782, Santa Cruz) was used for the primary antibody, and 2,000-times diluted anti-goat IgG-HRP Linked donkey antibody (SC-2033, Santa Cruz) was used for the secondary antibody. The secondary antibody was detected by chemiluminescence using ECL Western Blotting Detection System (GE Healthcare Bio Science). The chemiluminescence was detected and quantitatively determined with an image analyzer (LAS-3000UVmini, Fujifilm Corporation). The results of western blotting analysis demonstrated that strain 080801-2-4 provided a signal that was detected at the position corresponding to the molecular weight of a procollagen of the fusion polypeptide consisting of the N-terminal neon-helix region, 27 N-terminal residues of the helix region, a dimer made of 522 central residues of the helix region, 15 C-terminal residues of the helix region and the C-terminal non-helix region of human collagen Type I α1, and thus it was shown that the fusion polypeptide consisting of the N-terminal non-helix region, 27 N-terminal residues of the helix region, a dimer made of 522 central residues of the helix region, 15 C-terminal residues of the helix region and the C-terminal non-helix region of human collagen Type I α1 was produced.
The other half of the resulting membrane was used to carry out western blotting analysis to detect a procollagen of the fusion polypeptide consisting of the N-terminal non-helix region, 27 N-terminal residues of the helix region, a dimer made of 522 central residues of the helix region, 15 C-terminal residues of the helix region and the C-terminal non-helix region of human collagen Type I α2. Blocking one (Nacalai Tesque., Inc.) was used for blocking of the membrane, Can Get Signal (Toyobo Co., Ltd.) was used for antibody reactions, 5,000-times diluted anti-human collagen Type I rabbit antibody (ab292, abcam) was used for the primary antibody, and 10,000-times diluted anti-rabbit IgG-HRP Linked donkey antibody (NA-934, GE Healthcare Bio Science) was used for the secondary antibody. The secondary antibody was detected by chemiluminescence using ECL Western Blotting Detection System (GE Healthcare Bio Science). The chemiluminescence was detected and quantitatively determined with an image analyzer (LAS-3000UVmini, Fujifilm Corporation). The results of western blotting analysis demonstrated that strain 080801-2-4 provided a signal that was detected at the position corresponding to the molecular weight of a procollagen of the fusion polypeptide consisting of the N-terminal non-helix region, 27 N-terminal residues of the helix region, a dimer made of 522 central residues of the helix region, 15 C-terminal residues of the helix region and the C-terminal non-helix region of human collagen Type I α2, and thus, it was shown that the fusion polypeptide consisting of the N-terminal non-helix region, 27 N-terminal residues of the helix region, a dimer made of 522 central residues of the helix region, 15 C-terminal residues of the helix region and the C-terminal non-helix region of human collagen Type I α2 was produced.

(3-5) Preparation of a yeast in which an expression cassette for prolyl 4-hydroxylase α1 subunit, an expression cassette for prolyl 4-hydroxylase β subunit and an expression cassette for human collagen Type III α1 are introduced

### (3-5-1) Transformation of yeast

Using an electroporation method, strain 050525-5-3 (see, Example (3-1-1)) was transformed with pEXP-HA-HsCOL3A1 obtained in Example 2-6-2.
Into 100 ml of YPD liquid medium (1% Yeast Extract, 2% peptone, and 2% glucose), strain 050525-5-3 (see, Example (3-1-1)) was inoculated, and cultured at 30°C until the turbidity of cell culture (OD₆₆₀) reached about 10. The cells were collected from the cultured medium and the supernatant was removed. The cells were then suspended in 1M sorbitol at a turbidity of OD₆₆₀ = about 150 to prepare a cell suspension.
Ten (10) µg of pEXP-HA-HsCOL3A1 (see, Example (2-6-2)) was digested with a restriction enzyme XbaI. The DNA was collected through ethanol precipitation and dissolved in 5 µl of 10 mM Tris-HCl to prepare a DNA solution.
A mixture was prepared by combining 100 µl of the cell suspension and 5 µl of the DNA solution. To the mixture, a pulse was applied using a gene transfer device (ECM630, BTX), and then the mixture was spread on MD agar medium (1.34% Yeast Nitrogen Base, 4 × 10⁻⁵% biotin, 2% glucose, 2% agar) and cultured at 30°C for 48 hours. Colonies formed on the MD agar medium were isolated as transformants. Strain 080917-1-2 was obtained as a transformant.

### (3-5-2) Determination of expression of human collagen Type III α1

Strain 080917-1-2 (see, Example (3-5-1)) was inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which was prepared in a 500-ml baffled Erlenmeyer flask, and cultured with shaking at 30°C for 24 hours. A cell suspension was prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which was prepared in a 500-ml baffled Erlenmeyer flask, at a final concentration of OD₆₆₀ = about 10, and cultured with shaking at 30°C for 72 hours. Every about 12 hours during this period, 50% methanol was added in an amount of 0.5 ml each.
The cells were collected from the resultant culture and the supernatant was removed. After that, the cells were subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption was centrifuged to collect the supernatant of the cell disrupted solution to prepare a soluble fraction.
An aliquot of the soluble fraction was mixed with a sample buffer (Nacalai Tesque., Inc.), and the mixture was kept at 100°C for 5 minutes, followed by electrophoresis on SDS-polyacrylamide gel. The electrophoresis, in which ERICA-MP (DRC) was used for the electrophoresis apparatus and 5-15% gradient gel (XV PANTERA MP gel, DRC) was used for the electrophoresis gel, was performed at 200 V for 20 minutes. After the electrophoresis was completed, the resulting acrylamide gel was subjected to staining with a CBB staining solution to detect protein bands. The results demonstrated that strain 080917-1-2 provided a band that was detected at the molecular weight corresponding to a procollagen of collagen Type III α1, and thus it was shown that human collagen Type III α1 was produced.

(3-6) Preparation of a yeast in which an expression cassette for prolyl 4-hydroxylase α1 subunit, an expression cassette for prolyl 4-hydroxylase β subunit and an expression cassette for human collagen Type II α1 are introduced

### (3-6-1) Transformation of yeast

Using an electroporation method, strain 050525-5-3 (see, Example (3-1-1)) is transformed with pEXP-HA-YHsCOL2A1 obtained in Example 2-7-2.
Into 100 ml of YPD liquid medium (1% Yeast Extract, 2% peptone, and 2% glucose), strain 050525-5-3 (see, Example (3-1-1)) is inoculated, and cultured at 30°C until the turbidity of cell culture (OD₆₆₀) reaches about 10. The cells are collected from the cultured medium and the supernatant is removed. The cells are then suspended in 1M sorbitol at a turbidity of OD₆₆₀ = about 150 to prepare a cell suspension.
Ten (10) µg of pEXP-HA-YHsCOL2A1 (see, Example (2-7-2)) is digested with a restriction enzyme XbaI. The DNA is collected through ethanol precipitation and dissolved in 5 µl of 10 mM Tris-HCl to prepare a DNA solution.
A mixture is prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 2% agar) and cultured at 30°C for 48 hours. Colonies formed on the MD agar medium are isolated as transformants.

### (3-6-2) Determination of expression of human collagen Type II α1

A transformant (see, Example (3-6-1)) is inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which is prepared in a 500-ml baffled Erlenmeyer flask, and cultured with shaking at 30°C for 24 hours. A cell suspension is prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which is prepared in a 500-ml baffled Erlenmeyer flask, at a final concentration of OD₆₆₀ = about 10, and cultured with shaking at 30°C for 72 hours. Every about 12 hours during this period, 50% methanol is added in an amount of 0.5 ml each.
The cells are collected from the resultant culture and the supernatant is removed. After that, the cells are subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption is centrifuged to collect the supernatant of the cell disrupted solution to prepare a soluble fraction.
An aliquot of the soluble fraction is mixed with a sample buffer (Nacalai Tesque., Inc.), and the mixture is kept at 100°C for 5 minutes, followed by electrophoresis on SDS-polyacrylamide gel. The electrophoresis, in which ERICA-MP (DRC) is used for the electrophoresis apparatus and 5-15% gradient gel (XV PANTERA MP gel, DRC) is used for the electrophoresis gel, is performed at 200 V for 20 minutes. After the electrophoresis is completed, the resulting acrylamide gel is subjected to staining with a CBB staining solution to detect protein bands. A band is detected at the molecular weight corresponding to a procollagen of collagen Type II α1, demonstrating that human collagen Type II α1 is produced.

(3-7) Preparation of a yeast in which an expression cassette for prolyl 4-hydroxylase α1 subunit, an expression cassette for prolyl 4-hydroxylase β subunit, an expression cassette for human collagen Type I α1, an expression cassette for human collagen Type I α2 and an expression cassette for FKBP13A are introduced

### (3-7-1) Transformation of yeast

Using an electroporation method, strain 070327-1-11 (see, Example (3-2-1)) was transformed with pEXP-A-FKBP13A ZeoR obtained in Example 2-2-4.
Into 100 ml of YPD liquid medium (1% Yeast Extract, 2% peptone, and 2% glucose), strain 070327-1-11 (see, Example (3-2-1)) was inoculated, and cultured at 30°C until the turbidity of cell culture (OD₆₆₀) reached about 10. The cells were collected from the cultured medium and the supernatant was removed. The cells were then suspended in 1M sorbitol at a turbidity of OD₆₆₀ = about 150 to prepare a cell suspension.
Ten (10) µg of pEXP-A-FKBP13A ZeoR (see, Example (2-2-4)) was digested with a restriction enzyme BglII. The DNA was collected through ethanol precipitation and dissolved in 5 µl of 10 mM Tris-HCl to prepare a DNA solution.
A mixture was prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 300 µg/ml Zeocyn, 2% agar) and cultured at 30°C for 48 hours. Colonies formed on the MD agar medium were isolated as transformants. Strain 081022-2-1 was obtained as a pEXP-A-FKBP13A ZeoR transformant.

### (3-7-2) Determination of expression of FKBP13A

Strain 081022-2-1 (see, Example (3-7-1)) was inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which was prepared in a 500-ml baffled Erlenmeyer flask, and cultured with shaking at 30°C for 24 hours. A cell suspension was prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which was prepared in a 500-ml baffled Erlenmeyer flask, at a final concentration of OD₆₆₀ = about 10, and cultured with shaking at 30°C for 72 hours. Every about 12 hours during this period, 50% methanol was added in an amount of 0.5 ml each.
The cells were collected from the resultant culture and the supernatant was removed. After that, the cells were subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption was centrifuged to collect the supernatant of the cell disrupted solution to prepare a soluble fraction.
An aliquot of the soluble fraction was mixed with a sample buffer (Nacalai Tesque., Inc.), and the mixture was kept at 100°C for 5 minutes, followed by electrophoresis on SDS-polyacrylamide gel. The electrophoresis, in which ERICA-MP (DRC) was used for the electrophoresis apparatus and 5-15% gradient gel (XV PANTERA MP gel, DRC) was used for the electrophoresis gel, was performed at 200 V for 20 minutes. After the electrophoresis was completed, the acrylamide gel was subjected to electro-blotting onto a PVDF membrane (Immobilon-P, Millipore Corp.). The electro-blotting, in which MINICA-MP (DRC) was used for the blotting apparatus, was performed at 40 V for 30 minutes.
The resulting membrane was used to carry out western blotting analysis to detect the FK506 binding protein. Blocking one (Nacalai Tesque., Inc.) was used for blocking of the membrane, Can Get Signal (Toyobo Co., Ltd.) was used for antibody reactions, 2,000-times diluted anti-FKBP13A rabbit antibody (11700-1-AP, ProteinTech Group) was used for the primary antibody, and 10,000-times diluted anti-rabbit IgG-HRP Linked donkey antibody (NA-934, GE Healthcare Bio Science) was used for the secondary antibody. The secondary antibody was detected by chemiluminescence using ECL Western Blotting Detection System (GE Healthcare Bio Science). The chemiluminescence was detected and quantitatively determined with an image analyzer (LAS-3000UVmini, Fujifilm Corporation). The results of western blotting analysis demonstrated that strain 081022-2-1 provided a signal that was detected at the position corresponding to the molecular weight of the FKBP13A, and thus, it was shown that the FKBP13A was produced.

(3-8) Preparation of a yeast in which an expression cassette for prolyl 4-hydroxylase α1 subunit, an expression cassette for prolyl 4-hydroxylase β subunit, an expression cassette for human collagen Type I α1, an expression cassette for human collagen Type I α2 and an expression cassette for FKBP19 are introduced

### (3-8-1) Transformation of yeast

Using an electroporation method, strain 070327-1-11 (see, Example (3-2-1)) was transformed with pEXP-A-FKBP19 ZeoR obtained in Example 2-2-5.
Into 100 ml of YPD liquid medium (1% Yeast Extract, 2% peptone, and 2% glucose), strain 070327-1-11 (see, Example (3-2-1)) was inoculated, and cultured at 30°C until the turbidity of cell culture (OD₆₆₀) reached about 10. The cells were collected from the cultured medium and the supernatant was removed. The cells were then suspended in 1M sorbitol at a turbidity of OD₆₆₀ = about 150 to prepare a cell suspension.
Ten (10) µg of pEXP-A-FKBP19 ZeoR (see, Example (2-2-5)) was digested with a restriction enzyme BglII. The DNA was collected through ethanol precipitation and dissolved in 5 µl of 10 mM Tris-HCl to prepare a DNA solution.
A mixture was prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 300 µg/ml Zeocyn, 2% agar) and cultured at 30°C for 48 hours. Colonies formed on the MD agar medium were isolated as transformants. Strain 081022-1-1 was obtained as a pEXP-A-FKBP19 ZeoR transformant.

### (3-8-2) Determination of expression of FKBP19

Strain 081022-1-1 (see, Example (3-8-1)) was inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which was prepared in a 500-ml baffled Erlenmeyer flask, and cultured with shaking at 30°C for 24 hours. A cell suspension was prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which was prepared in a 500-ml baffled Erlenmeyer flask, at a final concentration of OD₆₆₀ = about 10, and cultured with shaking at 30°C for 72 hours. Every about 12 hours during this period, 50% methanol was added in an amount of 0.5 ml each.
The cells were collected from the resultant culture and the supernatant was removed. After that, the cells were subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption was centrifuged to collect the supernatant of the cell disrupted solution to prepare a soluble fraction.
An aliquot of the soluble fraction was mixed with a sample buffer (Nacalai Tesque., Inc.), and the mixture was kept at 100°C for 5 minutes, followed by electrophoresis on SDS-polyacrylamide gel. The electrophoresis, in which ERICA-MP (DRC) was used for the electrophoresis apparatus and 5-15% gradient gel (XV PANTERA MP gel, DRC) was used for the electrophoresis gel, was performed at 200 V for 20 minutes. After the electrophoresis was completed, the acrylamide gel was subjected to electro-blotting onto a PVDF membrane (Immobilon-P, Millipore Corp.). The electro-blotting, in which MINICA-MP (DRC) was used for the blotting apparatus, was performed at 40 V for 30 minutes.
The resulting membrane was used to carry out western blotting analysis to detect the FK506 binding protein. Blocking one (Nacalai Tesque., Inc.) was used for blocking of the membrane, Can Get Signal (Toyobo Co., Ltd.) was used for antibody reactions, 2,000-times diluted anti-FKBP19 mouse antibody (H00051303-B01, Abnova) was used for the primary antibody, and 10,000-times diluted anti-mouse IgG-HRP Linked sheep antibody (NA-931, GE Healthcare Bio Science) was used for the secondary antibody. The secondary antibody was detected by chemiluminescence using ECL Western Blotting Detection System (GE Healthcare Bio Science). The chemiluminescence was detected and quantitatively determined with an image analyzer (LAS-3000UVmini, Fujifilm Corporation). The results of western blotting analysis demonstrated that strain 081022-1-1 provided a signal that was detected at the position corresponding to the molecular weight of the FKBP19, and thus, it was shown that the FKBP19 was produced.

(3-9) Preparation of a yeast in which an expression cassette for prolyl 4-hydroxylase α1 subunit, an expression cassette for prolyl 4-hydroxylase β subunit, an expression cassette for human collagen Type I α1, an expression cassette for human collagen Type I α2 and an expression cassette for FKBP23 (Part 1) are introduced

### (3-9-1) Transformation of yeast

Using an electroporation method, strain 070327-1-11 (see, Example (3-2-1)) was transformed with pEXP-A-FKBP23 ZeoR obtained in Example 2-2-6.
Into 100 ml of YPD liquid medium (1% Yeast Extract, 2% peptone, and 2% glucose), strain 070327-1-11 (see, Example (3-2-1)) was inoculated, and cultured at 30°C until the turbidity of cell culture (OD₆₆₀) reached about 10. The cells were collected from the cultured medium and the supernatant was removed. The cells were then suspended in 1M sorbitol at a turbidity of OD₆₆₀ = about 150 to prepare a cell suspension.
Ten (10) µg of pEXP-A-FKBP23 ZeoR (see, Example (2-2-6)) was digested with a restriction enzyme BglII. The DNA was collected through ethanol precipitation and dissolved in 5 µl of 10 mM Tris-HCl to prepare a DNA solution.
A mixture was prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 300 µg/ml Zeocyn, 2% agar) and cultured at 30°C for 48 hours. Colonies formed on the MD agar medium were isolated as transformants. Strain 081024-3-1 was obtained as a pEXP-A-FKBP23 ZeoR transformant.

### (3-9-2) Determination of expression of FKBP23

Strain 081024-3-1 (see, Example (3-9-1)) was inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which was prepared in a 500-ml baffled Erlenmeyer flask, and cultured with shaking at 30°C for 24 hours. A cell suspension was prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which was prepared in a 500-ml baffled Erlenmeyer flask, at a final concentration of OD₆₆₀ = about 10, and cultured with shaking at 30°C for 72 hours. Every about 12 hours during this period, 50% methanol was added in an amount of 0.5 ml each.
The cells were collected from the resultant culture and the supernatant was removed. After that, the cells were subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption was centrifuged to collect the supernatant of the cell disrupted solution to prepare a soluble fraction.
An aliquot of the soluble fraction was mixed with a sample buffer (Nacalai Tesque., Inc.), and the mixture was kept at 100°C for 5 minutes, followed by electrophoresis on SDS-polyacrylamide gel. The electrophoresis, in which ERICA-MP (DRC) was used for the electrophoresis apparatus and 5-15% gradient gel (XV PANTERA MP gel, DRC) was used for the electrophoresis gel, was performed at 200 V for 20 minutes. After the electrophoresis was completed, the acrylamide gel was subjected to electro-blotting onto a PVDF membrane (Immobilon-P, Millipore Corp.). The electro-blotting, in which MINICA-MP (DRC) was used for the blotting apparatus, was performed at 40 V for 30 minutes.
The resulting membrane was used to carry out western blotting analysis to detect the FK506 binding protein. Blocking one (Nacalai Tesque., Inc.) was used for blocking of the membrane, Can Get Signal (Toyobo Co., Ltd.) was used for antibody reactions, 2,000-times diluted anti-FKBP23 rabbit antibody (12092-1-AP, ProteinTech Group) was used for the primary antibody, and 10,000-times diluted anti-rabbit IgG-HRP Linked donkey antibody (NA-934, GE Healthcare Bio Science) was used for the secondary antibody. The secondary antibody was detected by chemiluminescence using ECL Western Blotting Detection System (GE Healthcare Bio Science). The chemiluminescence was detected and quantitatively determined with an image analyzer (LAS-3000UVmini, Fujifilm Corporation). The results of western blotting analysis demonstrated that strain 081024-3-1 provided a signal that was detected at the position corresponding to the molecular weight of the FKBP23, and thus, it was shown that the FKBP23 was produced.

(3-10) Preparation of a yeast in which an expression cassette for prolyl 4-hydroxylase α1 subunit, an expression cassette for prolyl 4-hydroxylase β subunit, an expression cassette for human collagen Type I α1, an expression cassette for human collagen Type I α2 and an expression cassette for FKBP63 are introduced

### (3-10-1) Transformation of yeast

Using an electroporation method, strain 070327-1-11 (see, Example (3-2-1)) was transformed with pEXP-A-FKBP63 ZeoR obtained in Example 2-2-7.
Into 100 ml of YPD liquid medium (1% Yeast Extract, 2% peptone, and 2% glucose), strain 070327-1-11 (see, Example (3-2-1)) was inoculated, and cultured at 30°C until the turbidity of cell culture (OD₆₆₀) reached about 10. The cells were collected from the cultured medium and the supernatant was removed. The cells were then suspended in 1M sorbitol at a turbidity of OD₆₆₀ = about 150 to prepare a cell suspension.
Ten (10) µg of pEXP-A-FKBP63 ZeoR (see, Example (2-2-7)) was digested with a restriction enzyme BglII. The DNA was collected through ethanol precipitation and dissolved in 5 µl of 10 mM Tris-HCl to prepare a DNA solution.
A mixture was prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 300 µg/ml Zeocyn, 2% agar) and cultured at 30°C for 48 hours. Colonies formed on the MD agar medium were isolated as transformants. Strain 081024-2-1 was obtained as a pEXP-A-FKBP63 ZeoR transformant.

### (3-10-2) Determination of expression of FKBP63

Strain 081024-2-1 (see, Example (3-10-1)) was inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which was prepared in a 500-ml baffled Erlenmeyer flask, and cultured with shaking at 30°C for 24 hours. A cell suspension was prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which was prepared in a 500-ml baffled Erlenmeyer flask, at a final concentration of OD₆₆₀ = about 10, and cultured with shaking at 30°C for 72 hours. Every about 12 hours during this period, 50% methanol was added in an amount of 0.5 ml each.
The cells were collected from the resultant culture and the supernatant was removed. After that, the cells were subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption was centrifuged to collect the supernatant of the cell disrupted solution to prepare a soluble fraction.
An aliquot of the soluble fraction was mixed with a sample buffer (Nacalai Tesque., Inc.), and the mixture was kept at 100°C for 5 minutes, followed by electrophoresis on SDS-polyacrylamide gel. The electrophoresis, in which ERICA-MP (DRC) was used for the electrophoresis apparatus and 5-15% gradient gel (XV PANTERA MP gel, DRC) was used for the electrophoresis gel, was performed at 200 V for 20 minutes. After the electrophoresis was completed, the resulting acrylamide gels was subjected to staining with a CBB staining solution to detect protein bands. The results demonstrated that strain 081024-2-1 provided a band that was detected at the molecular weight corresponding to the FKBP63, and thus, it was shown that the FKBP63 was produced.

(3-11) Preparation of a yeast in which an expression cassette for prolyl 4-hydroxylase α1 subunit, an expression cassette for prolyl 4-hydroxylase β subunit, an expression cassette for human collagen Type I α1, an expression cassette for human collagen Type I α2 and an expression cassette for FKBP65 are introduced

### (3-11-1) Transformation of yeast

Using an electroporation method, strain 070327-1-11 (see, Example (3-2-1)) was transformed with pEXP-A-FKBP65 ZeoR obtained in Example 2-2-8.
Into 100 ml of YPD liquid medium (1% Yeast Extract, 2% peptone, and 2% glucose), strain 070327-1-11 (see, Example (3-11-1)) was inoculated, and cultured at 30°C until the turbidity of cell culture (OD₆₆₀) reached about 10. The cells were collected from the cultured medium and the supernatant was removed. The cells were then suspended in 1M sorbitol at a turbidity of OD₆₆₀ = about 150 to prepare a cell suspension.
Ten (10) µg of pEXP-A-FKBP65 ZeoR (see, Example (2-2-8)) was digested with a restriction enzyme BglII. The DNA was collected through ethanol precipitation and dissolved in 5 µl of 10 mM Tris-HCl to prepare a DNA solution.
A mixture was prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 300 µg/ml Zeocyn, 2% agar) and cultured at 30°C for 48 hours. Colonies formed on the MD agar medium were isolated as transformants. Strain 081024-1-1 was obtained as a pEXP-A-FKBP65 ZeoR transformant.

### (3-11-2) Determination of expression of FKBP65

Strain 081024-1-1 (see, Example (3-11-1)) was inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which was prepared in a 500-ml baffled Erlenmeyer flask, and cultured with shaking at 30°C for 24 hours. A cell suspension was prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which was prepared in a 500-ml baffled Erlenmeyer flask, at a final concentration of OD₆₆₀ = about 10, and cultured with shaking at 30°C for 72 hours. Every about 12 hours during this period, 50% methanol was added in an amount of 0.5 ml each.
The cells were collected from the resultant culture and the supernatant was removed. After that, the cells were subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption was centrifuged to collect the supernatant of the cell disrupted solution to prepare a soluble fraction.
An aliquot of the soluble fraction was mixed with a sample buffer (Nacalai Tesque., Inc.), and the mixture was kept at 100°C for 5 minutes, followed by electrophoresis on SDS-polyacrylamide gel. The electrophoresis, in which ERICA-MP (DRC) was used for the electrophoresis apparatus and 5-15% gradient gel (XV PANTERA MP gel, DRC) was used for the electrophoresis gel, was performed at 200 V for 20 minutes. After the electrophoresis was completed, the acrylamide gel was subjected to electro-blotting onto a PVDF membrane (Immobilon-P, Millipore Corp.). The electro-blotting, in which MINICA-MP (DRC) was used for the blotting apparatus, was performed at 40 V for 30 minutes.
The resulting membrane was used to carry out western blotting analysis to detect the FK506 binding protein. Blocking one (Nacalai Tesque., Inc.) was used for blocking of the membrane, Can Get Signal (Toyobo Co., Ltd.) was used for antibody reactions, 2,000-times diluted anti-FKBP65 rabbit antibody (12172-1-AP, ProteinTech Group) was used for the primary antibody, and 10,000-times diluted anti-rabbit IgG-HRP Linked donkey antibody (NA-934, GE Healthcare Bio Science) was used for the secondary antibody. The secondary antibody was detected by chemiluminescence using ECL Western Blotting Detection System (GE Healthcare Bio Science). The chemiluminescence was detected and quantitatively determined with an image analyzer (LAS-3000UVmini, Fujifilm Corporation). The results of western blotting analysis demonstrated that strain 081024-1-1 provided a signal that was detected at the position corresponding to the molecular weight of the FKBP65, and thus, it was shown that the FKBP65 was produced.

(3-12) Preparation of a yeast in which an expression cassette for prolyl 4-hydroxylase α1 subunit, an expression cassette for prolyl 4-hydroxylase β subunit, an expression cassette for human collagen Type I α1, an expression cassette for human collagen Type I α2 and an expression cassette for FKBP23 (Part 2) are introduced

### (3-12-1) Transformation of yeast

Using an electroporation method, strain 070327-1-11 (see, Example (3-2-1)) is transformed with pEXP-A-PER3 Pro-FKBP23 ZeoR (see, Example (2-2-9)), pEXP-A-AOX2 Pro-FKBP23 ZeoR (see, Example (2-2-10)) or pEXP-A-FLD1 Pro-FKBP23 ZeoR (see, Example (2-2-11)).
Into 100 ml of YPD liquid medium (1% Yeast Extract, 2% peptone, and 2% glucose), strain 050818-3-1 is inoculated, and cultured at 30°C until the turbidity of cell culture (OD₆₆₀) reaches about 10. The cells are collected from the cultured medium and the supernatant is removed. The cells are then suspended in 1M sorbitol at a turbidity of OD₆₆₀ = about 150 to prepare a cell suspension.
Ten (10) µg of pEXP-A-PER3 Pro-FKBP23 ZeoR (see, Example (2-2-9)), pEXP-A-AOX2 Pro-FKBP23 ZeoR (see, Example (2-2-10)) or pEXP-A-FLD1 Pro-FKBP23 ZeoR (see, Example (2-2-11)) is digested with a restriction enzyme BglII. The DNA is collected through ethanol precipitation and dissolved in 5 µl of 10 mM Tris-HCl to prepare a DNA solution.
A mixture is prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 300 µg/ml Zeocyn, 2% agar) and cultured at 30°C for 48 hours. Colonies formed on the MD agar medium are isolated as transformants.

### (3-12-2) Determination of expression of FKBP23

A transformant (see, Example (3-12-1)) is inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which is prepared in a 500-ml baffled Erlenmeyer flask, and cultured with shaking at 30°C for 24 hours. A cell suspension is prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which is prepared in a 500-ml baffled Erlenmeyer flask, at a final concentration of OD₆₆₀ = about 10, and cultured with shaking at 30°C for 72 hours. Every about 12 hours during this period, 50% methanol is added in an amount of 0.5 ml each.
The cells are collected from the resultant culture and the supernatant is removed. After that, the cells are subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption is centrifuged to collect the supernatant of the cell disrupted solution to prepare a soluble fraction.
An aliquot of the soluble fraction is mixed with a sample buffer (Nacalai Tesque., Inc.), and the mixture is kept at 100°C for 5 minutes, followed by electrophoresis on SDS-polyacrylamide gel. The electrophoresis, in which ERICA-MP (DRC) is used for the electrophoresis apparatus and 5-15% gradient gel (XV PANTERA MP gel, DRC) is used for the electrophoresis gel, is performed at 200 V for 20 minutes. After the electrophoresis is completed, the acrylamide gel is subjected to electro-blotting onto a PVDF membrane (Immobilon-P, Millipore Corp.). The electro-blotting, in which MINICA-MP (DRC) is used for the blotting apparatus, is performed at 40 V for 30 minutes.
The resulting membrane is used to carry out western blotting analysis to detect the FK506 binding protein. Blocking one (Nacalai Tesque., Inc.) is used for blocking of the membrane, Can Get Signal (Toyobo Co., Ltd.) is used for antibody reactions, 2,000-times diluted anti-FKBP23 rabbit antibody (12092-1-AP, ProteinTech Group) is used for the primary antibody, and 10,000-times diluted anti-rabbit IgG-HRP Linked donkey antibody (NA-934, GE Healthcare Bio Science) is used for the secondary antibody. The secondary antibody is detected by chemiluminescence using ECL Western Blotting Detection System (GE Healthcare Bio Science). The chemiluminescence is detected and quantitatively determined with an image analyzer (LAS-3000UVmini, Fujifilm Corporation). A band is detected at the molecular weight corresponding to the FKBP23, demonstrating that the FKBP23 is produced.

(3-13) Preparation of a yeast in which an expression cassette for prolyl 4-hydroxylase α1 subunit; an expression cassette for prolyl 4-hydroxylase β subunit; an expression cassette for a fusion polypeptide which consists of the N-terminal non-helix region, 60 N-terminal residues of the helix region, 15 C-terminal residues of the helix region and the C-terminal non-helix region of human collagen Type I α1; an expression cassette for a fusion polypeptide which consists of the N-terminal non-helix region, 60 N-terminal residues of the helix region, 15 C-terminal residues of the helix region and the C-terminal non-helix region of human collagen Type I α2I α2; and an expression cassette for FKBP23 are introduced

### (3-13-1) Transformation of yeast

Using an electroporation method, strain 080118-3-3 (see, Example (3-3-1)) is transformed with pEXP-A-PER3 Pro-FKBP23 ZeoR (see, Example (2-2-9)), pEXP-A-AOX2 Pro-FKBP23 ZeoR (see, Example (2-2-10)) or pEXP-A-FLD1 Pro-FKBP23 ZeoR (see, Example (2-2-11)).
Into 100 ml of YPD liquid medium (1% Yeast Extract, 2% peptone, and 2% glucose), strain 080118-3-3 (see, Example (3-3-1)) is inoculated, and cultured at 30°C until the turbidity of cell culture (OD₆₆₀) reaches about 10. The cells are collected from the cultured medium and the supernatant is removed. The cells are then suspended in 1M sorbitol at a turbidity of OD₆₆₀ = about 150 to prepare a cell suspension.
Ten (10) µg of pEXP-A-PER3 Pro-FKBP23 ZeoR (see, Example (2-2-9)), pEXP-A-AOX2 Pro-FKBP23 ZeoR (see, Example (2-2-10)) or pEXP-A-FLD1 Pro-FKBP23 ZeoR (see, Example (2-2-11)) is digested with a restriction enzyme BglII. The DNA is collected through ethanol precipitation and dissolved in 5 µl of 10 mM Tris-HCl to prepare a DNA solution.
A mixture is prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 300 µg/ml Zeocyn, 2% agar) and cultured at 30°C for 48 hours. Colonies formed on the MD agar medium are isolated as transformants.

### (3-13-2) Determination of expression of FKBP23

A transformant (see, Example (3-13-1)) is inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which is prepared in a 500-ml baffled Erlenmeyer flask, and cultured with shaking at 30°C for 24 hours. A cell suspension is prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which is prepared in a 500-ml baffled Erlenmeyer flask, at a final concentration of OD₆₆₀ = about 10, and cultured with shaking at 30°C for 72 hours. Every about 12 hours during this period, 50% methanol is added in an amount of 0.5 ml each.
The cells are collected from the resultant culture and the supernatant is removed. After that, the cells are subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption is centrifuged to collect the supernatant of the cell disrupted solution to prepare a soluble fraction.
An aliquot of the soluble fraction is mixed with a sample buffer (Nacalai Tesque., Inc.), and the mixture is kept at 100°C for 5 minutes, followed by electrophoresis on SDS-polyacrylamide gel. The electrophoresis, in which ERICA-MP (DRC) is used for the electrophoresis apparatus and 5-15% gradient gel (XV PANTERA MP gel, DRC) is used for the electrophoresis gel, is performed at 200 V for 20 minutes. After the electrophoresis is completed, the acrylamide gel is subjected to electro-blotting onto a PVDF membrane (Immobilon-P, Millipore Corp.). The electro-blotting, in which MINICA-MP (DRC) is used for the blotting apparatus, is performed at 40 V for 30 minutes.
The resulting membrane is used to carry out western blotting analysis to detect the FKBP23. Blocking one (Nacalai Tesque., Inc.) is used for blocking of the membrane, Can Get Signal (Toyobo Co., Ltd.) is used for antibody reactions, 2,000-times diluted anti-FKBP23 rabbit antibody (12092-1-AP, ProteinTech Group) is used for the primary antibody, and 10,000-times diluted anti-rabbit IgG-HRP Linked donkey antibody (NA-934, GE Healthcare Bio Science) is used for the secondary antibody. The secondary antibody is detected by chemiluminescence using ECL Western Blotting Detection System (GE Healthcare Bio Science). The chemiluminescence is detected and quantitatively determined with an image analyzer (LAS-3000UVmini, Fujifilm Corporation). A band is detected at the molecular weight corresponding to the FKBP23, demonstrating that the FKBP23 is produced.

(3-14) Preparation of a yeast in which an expression cassette for prolyl 4-hydroxylase α1 subunit; an expression cassette for prolyl 4-hydroxylase β subunit; an expression cassette for a fusion polypeptide which consists of the N-terminal non-helix region, 27 N-terminal residues of the helix region, a dimer made of 522 central residues of the helix region, 15 C-terminal residues of the helix region and the C-terminal non-helix region of human collagen Type I α1; an expression cassette for a fusion polypeptide which consists of the N-terminal non-helix region, 27 N-terminal residues of the helix region, a dimer made of 522 central residues of the helix region, 15 C-terminal residues of the helix region and the C-terminal non-helix region of human collagen Type I α2; and an expression cassette for FKBP23 are introduced

### (3-14-1) Transformation of yeast

Using an electroporation method, strain 080801-2-4 (see, Example (3-4-1)) is transformed with pEXP-A-PER3 Pro-FKBP23 ZeoR (see, Example (2-2-9)), pEXP-A-AOX2 Pro-FKBP23 ZeoR (see, Example (2-2-10)) or pEXP-A-FLD1 Pro-FKBP23 ZeoR (see, Example (2-2-11)).
Into 100 ml of YPD liquid medium (1% Yeast Extract, 2% peptone, and 2% glucose), strain 080801-2-4 (see, Example (3-4-1)) is inoculated, and cultured at 30°C until the turbidity of cell culture (OD₆₆₀) reaches about 10. The cells are collected from the cultured medium and the supernatant is removed. The cells are then suspended in 1M sorbitol at a turbidity of OD₆₆₀ = about 150 to prepare a cell suspension.
Ten (10) µg of pEXP-A-PER3 Pro-FKBP23 ZeoR (see, Example (2-2-9)), pEXP-A-AOX2 Pro-FKBP23 ZeoR (see, Example (2-2-10)) or pEXP-A-FLD1 Pro-FKBP23 ZeoR (see, Example (2-2-11)) is digested with a restriction enzyme BglII. The DNA is collected through ethanol precipitation and dissolved in 5 µl of 10 mM Tris-HCl to prepare a DNA solution.
A mixture is prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 300 µg/ml Zeocyn, 2% agar) and cultured at 30°C for 48 hours. Colonies formed on the MD agar medium are isolated as transformants.

### (3-14-2) Determination of expression of FKBP23

A transformant (see, Example (3-14-1)) is inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which is prepared in a 500-ml baffled Erlenmeyer flask, and cultured with shaking at 30°C for 24 hours. A cell suspension is prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which is prepared in a 500-ml baffled Erlenmeyer flask, at a final concentration of OD₆₆₀ = about 10, and cultured with shaking at 30°C for 72 hours. Every about 12 hours during this period, 50% methanol is added in an amount of 0.5 ml each.
The cells are collected from the resultant culture and the supernatant is removed. After that, the cells are subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption is centrifuged to collect the supernatant of the cell disrupted solution to prepare a soluble fraction.
An aliquot of the soluble fraction is mixed with a sample buffer (Nacalai Tesque., Inc.), and the mixture is kept at 100°C for 5 minutes, followed by electrophoresis on SDS-polyacrylamide gel. The electrophoresis, in which ERICA-MP (DRC) is used for the electrophoresis apparatus and 5-15% gradient gel (XV PANTERA MP gel, DRC) is used for the electrophoresis gel, is performed at 200 V for 20 minutes. After the electrophoresis is completed, the acrylamide gel is subjected to electro-blotting onto a PVDF membrane (Immobilon-P, Millipore Corp.). The electro-blotting, in which MINICA-MP (DRC) is used for the blotting apparatus, is performed at 40 V for 30 minutes.
The resulting membrane is used to carry out western blotting analysis to detect the FKBP23. Blocking one (Nacalai Tesque., Inc.) is used for blocking of the membrane, Can Get Signal (Toyobo Co., Ltd.) is used for antibody reactions, 2,000-times diluted anti-FKBP23 rabbit antibody (12092-1-AP, ProteinTech Group) is used for the primary antibody, and 10,000-times diluted anti-rabbit IgG-HRP Linked donkey antibody (NA-934, GE Healthcare Bio Science) is used for the secondary antibody. The secondary antibody is detected by chemiluminescence using ECL Western Blotting Detection System (GE Healthcare Bio Science). The chemiluminescence is detected and quantitatively determined with an image analyzer (LAS-3000UVmini, Fujifilm Corporation). A band is detected at the molecular weight corresponding to the FKBP23, demonstrating that the FKBP23 is produced.

(3-15) Preparation of a yeast in which an expression cassette for prolyl 4-hydroxylase α1 subunit, an expression cassette for prolyl 4-hydroxylase β subunit, an expression cassette for human collagen Type III α1 and an expression cassette for FKBP23 are introduced

### (3-15-1) Transformation of yeast

Using an electroporation method, strain 080917-1-2 (see, Example (3-5-1)) is transformed with pEXP-A-PER3 Pro-FKBP23 ZeoR (see, Example (2-2-9)), pEXP-A-AOX2 Pro-FKBP23 ZeoR (see, Example (2-2-10)) or pEXP-A-FLD1 Pro-FKBP23 ZeoR (see, Example (2-2-11)).
Into 100 ml of YPD liquid medium (1% Yeast Extract, 2% peptone, and 2% glucose), strain 080917-1-2 (see, Example (3-5-1)) is inoculated, and cultured at 30°C until the turbidity of cell culture (OD₆₆₀) reaches about 10. The cells are collected from the cultured medium and the supernatant is removed. The cells are then suspended in 1M sorbitol at a turbidity of OD₆₆₀ = about 150 to prepare a cell suspension.
Ten (10) µg of pEXP-A-PER3 Pro-FKBP23 ZeoR (see, Example (2-2-9)), pEXP-A-AOX2 Pro-FKBP23 ZeoR (see, Example (2-2-10)), or pEXP-A-FLD1 Pro-FKBP23 ZeoR (see, Example (2-2-11)) is digested with a restriction enzyme BglII. The DNA is collected through ethanol precipitation and dissolved in 5 µl of 10 mM Tris-HCl to prepare a DNA solution.
A mixture is prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 300 µg/ml Zeocyn, 2% agar) and cultured at 30°C for 48 hours. Colonies formed on the MD agar medium are isolated as transformants.

### (3-15-2) Determination of expression of FKBP23

A transformant (see, Example (3-15-1)) is inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which is prepared in a 500-ml baffled Erlenmeyer flask, and cultured with shaking at 30°C for 24 hours. A cell suspension is prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which is prepared in a 500-ml baffled Erlenmeyer flask, at a final concentration of OD₆₆₀ = about 10, and cultured with shaking at 30°C for 72 hours. Every about 12 hours during this period, 50% methanol is added in an amount of 0.5 ml each.
The cells are collected from the resultant culture and the supernatant is removed. After that, the cells are subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption is centrifuged to collect the supernatant of the cell disrupted solution to prepare a soluble fraction.
An aliquot of the soluble fraction is mixed with a sample buffer (Nacalai Tesque., Inc.), and the mixture is kept at 100°C for 5 minutes, followed by electrophoresis on SDS-polyacrylamide gel. The electrophoresis, in which ERICA-MP (DRC) is used for the electrophoresis apparatus and 5-15% gradient gel (XV PANTERA MP gel, DRC) is used for the electrophoresis gel, is performed at 200 V for 20 minutes. After the electrophoresis is completed, the acrylamide gel is subjected to electro-blotting onto a PVDF membrane (Immobilon-P, Millipore Corp.). The electro-blotting, in which MINICA-MP (DRC) is used for the blotting apparatus, is performed at 40 V for 30 minutes.
The resulting membrane is used to carry out western blotting analysis to detect the FKBP23. Blocking one (Nacalai Tesque., Inc.) is used for blocking of the membrane, Can Get Signal (Toyobo Co., Ltd.) is used for antibody reactions, 2,000-times diluted anti-FKBP23 rabbit antibody (12092-1-AP, ProteinTech Group) is used for the primary antibody, and 10,000-times diluted anti-rabbit IgG-HRP Linked donkey antibody (NA-934, GE Healthcare Bio Science) is used for the secondary antibody. The secondary antibody is detected by chemiluminescence using ECL Western Blotting Detection System (GE Healthcare Bio Science). The chemiluminescence is detected and quantitatively determined with an image analyzer (LAS-3000UVmini, Fujifilm Corporation). A band is detected at the molecular weight corresponding to the FKBP23, demonstrating that the FKBP23 is produced.

(3-16) Preparation of a yeast in which an expression cassette for prolyl 4-hydroxylase α1 subunit, an expression cassette for prolyl 4-hydroxylase β subunit, an expression cassette for human collagen Type II α1 and an expression cassette for FKBP23 are introduced

### (3-16-1) Transformation of yeast

Using an electroporation method, a yeast having introduced therein an expression cassette for prolyl 4-hydroxylase α1 subunit, an expression cassette for prolyl 4-hydroxylase β subunit, and an expression cassette for human collagen Type II α1 (see, Example (3-6-1)) is transformed with pEXP-A-PER3 Pro-FKBP23 ZeoR (see, Example (2-2-9)), pEXP-A-AOX2 Pro-FKBP23 ZeoR (see, Example (2-2-10)) or pEXP-A-FLD1 Pro-FKBP23 ZeoR (see, Example (2-2-11)).
Into 100 ml of YPD liquid medium (1% Yeast Extract, 2% peptone, and 2% glucose), strain 080917-1-2 (see, Example (3-5-1)) is inoculated, and cultured at 30°C until the turbidity of cell culture (OD₆₆₀) reaches about 10. The cells are collected from the cultured medium and the supernatant is removed. The cells are then suspended in 1M sorbitol at a turbidity of OD₆₆₀ = about 150 to prepare a cell suspension.
Ten (10) µg of pEXP-A-PER3 Pro-FKBP23 ZeoR (see, Example (2-2-9)), pEXP-A-AOX2 Pro-FKBP23 ZeoR (see, Example (2-2-10)) or pEXP-A-FLD1 Pro-FKBP23 ZeoR (see, Example (2-2-11)) is digested with a restriction enzyme BglII. The DNA is collected through ethanol precipitation and dissolved in 5 µl of 10 mM Tris-HCl to prepare a DNA solution.
A mixture is prepared by combining 100 µl of the cell suspension and 5 µl of the DNA solution. To the mixture, a pulse is applied using a gene transfer device (ECM630, BTX), and then the mixture is spread on MD agar medium (1.34% Yeast Nitrogen Base, 4 × 10⁻⁵% biotin, 2% glucose, 300 µg/ml Zeocyn, 2% agar) and cultured at 30°C for 48 hours. Colonies formed on the MD agar medium are isolated as transformants.

(3-16-2) Determination of expression of FKBP23
A transformant (see, Example (3-16-1)) is inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which is prepared in a 500-ml baffled Erlenmeyer flask, and cultured with shaking at 30°C for 24 hours. A cell suspension is prepared from the resultant culture, inoculated in 50 ml of BMM medium [100 mM potassium phosphate buffer (pH 6.0), 1.34% Yeast Nitrogen Base (Difco), 4 × 10⁻⁵% biotin, 0.5% methanol] which is prepared in a 500-ml baffled Erlenmeyer flask, at a final concentration of OD₆₆₀ = about 10, and cultured with shaking at 30°C for 72 hours. Every about 12 hours during this period, 50% methanol is added in an amount of 0.5 ml each.
The cells are collected from the resultant culture and the supernatant is removed. After that, the cells are subjected to cell disruption using a Multi-beads Shocker (Yasui Kikai Corporation) under conditions for yeast cell disruption (glass beads of a diameter of 0.5 mm, 2,500 rpm, 40 minutes). The solution resulting from the cell disruption is centrifuged to collect the supernatant of the cell disrupted solution to prepare a soluble fraction.
An aliquot of the soluble fraction is mixed with a sample buffer (Nacalai Tesque., Inc.), and the mixture is kept at 100°C for 5 minutes, followed by electrophoresis on SDS-polyacrylamide gel. The electrophoresis, in which ERICA-MP (DRC) is used for the electrophoresis apparatus and 5-15% gradient gel (XV PANTERA MP gel, DRC) is used for the electrophoresis gel, is performed at 200 V for 20 minutes. After the electrophoresis is completed, the acrylamide gel is subjected to electro-blotting onto a PVDF membrane (Immobilon-P, Millipore Corp.). The electro-blotting, in which MINICA-MP (DRC) is used for the blotting apparatus, is performed at 40 V for 30 minutes.
The resulting membrane is used to carry out western blotting analysis to detect the FKBP23. Blocking one (Nacalai Tesque., Inc.) is used for blocking of the membrane, Can Get Signal (Toyobo Co., Ltd.) is used for antibody reactions, 2,000-times diluted anti-FKBP23 rabbit antibody (12092-1-AP, ProteinTech Group) is used for the primary antibody, and 10,000-times diluted anti-rabbit IgG-HRP Linked donkey antibody (NA-934, GE Healthcare Bio Science) is used for the secondary antibody. The secondary antibody is detected by chemiluminescence using ECL Western Blotting Detection System (GE Healthcare Bio Science). The chemiluminescence is detected and quantitatively determined with an image analyzer (LAS-3000UVmini, Fujifilm Corporation). A band is detected at the molecular weight corresponding to the FKBP23, demonstrating that the FKBP23 is produced.

Example 4 (Preparation of collagen) (Part 1)

(4-1) Culturing of yeast and preparation of cells (Part 1)
Strains 070327-1-11 (see, Example (3-2-1)), 081022-2-1 (see, Example (3-7-1)), 081022-1-1 (see, Example (3-8-1)), 081024-3-1 (see, Example (3-9-1)), 081024-2-1 (see, Example (3-10-1)) and 081024-1-1 (see, Example (3-11-1)) were each inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which was prepared in a 500-ml baffled Erlenmeyer flask, and cultured with shaking at 30°C for 24 hours of pre-culture.
In a 3-L jar fermentor (Model BMS-03P1, Able Corporation), 0.8 L of Basal salt medium was prepared. The pre-cultured medium was inoculated so as to give a final cell concentration equal to OD₆₆₀ = about 1.25, and then main culture under agitation and aeration was started.
The main culture was started with setting the culture temperature to 30°C, the maximum air flow rate to 0.8 vvm (0.8 L), and the agitation rate to 800 rpm. After all the available glycerol in the medium was consumed and the oxygen consumption was decreased, feeding of a glycerol feed medium (50% glycerol, 1.2% PMT1 solution) was started at a rate of about 15 g/h. The glycerol fed-batch culture was stopped when the OD₆₆₀ of the cultured medium achieved about 400. Subsequently, feeding of a methanol feed medium (98.6% (w/v) methanol, 1.2% PMT1 solution) was started for methanol fed-batch culture. After the methanol fed-batch culture was started, the culture temperature was changed to 32°C and the dissolved oxygen concentration was controlled to be about 8 ppm. Throughout the culture period, the culture medium was maintained at a constant pH of about 5.0 using 28% ammonia water and 4M phosphoric acid. After about 136 hours of culture, the cultured medium yielded a cell mass of 1696 g for strain 070327-1-11, 1671 g for strain 081022-2-1, 1639 g for strain 081022-1-1, 1590 g for strain 081024-3-1, 1674 g for strain 081024-2-1, and 1599 g for strain 081024-1-1. The composition of the medium used herein is given as follows:
(Composition of Basal salt medium)
   (a) 85% H₃PO₄, 26.7 mL/L;
   (b) CaSO₄-2H₂O, 0.93 g/L;
   (c) K₂SO₄, 18.2 g/L;
   (d) MgSO₄-7H₂O, 14.9 g/L;
   (e) KOH, 4.13 g/L;
   (f) glycerol, 40 g/L.
   After the above components were mixed, the mixture was subjected to autoclave sterilization. After adjusted to pH 5.0 with 28% ammonia water, 2 ml of PMT1 solution and 1 ml of a solution of defoaming agent in methanol (12.5% Adekanol LG295S) were added per liter of the medium.
(Composition of PMT1 Solution)
   (a) CuSO₄-5H₂O 6.0 g/L;
   (b) KI, 0.8 g/L;
   (c) MnSO₄-H₂O 3.0 g/L;
   (d) Na₂MoO₄-2H₂O 0.2 g/L;
   (e) H₃BO₃, 0.2 g;
   (f) CaSO₄-2H₂O 0.5 g/L;
   (g) ZnCl₂, 20 g/L;
   (h) FeSO₄-7H₂O, 65 g/L;
   (i) biotin, 0.2 g/L;
   (j) conc. sulfuric acid, 5 mL/L.
   The cultured medium was centrifuged at 5,000 rpm at 4°C for 10 minutes to collect the cells. To remove the medium components, the cells were suspended in potassium phosphate buffer (50 mM, pH 6.0), and then centrifuged to collect the cells. These procedures were repeated twice in total to prepare the cells. A cell mass of 632.6 g was obtained for strain 070327-1-11, 650.2 g for strain 081022-2-1, 627.6 g for strain 081022-1-1, 578.3 g for strain 081024-3-1, 638.7 g for strain 081024-2-1 and 614.3 g for strain 081024-1-1.

### (4-2) Extraction and purification of collagen (Part 1)

Extraction and purification of respective collagens was carried out by disrupting 600 g of the cells of strain 070327-1-11 (see, Example (4-1)), 600 g of the cells of strain 081022-2-1 (see, Example (4-1)), 600 g of the cells of strain 081022-1-1 (see, Example (4-1)), 600 g of the cells of strain 081024-3-1 (see, Example (4-1)), 600 g of the strain 081024-2-1 (see, Example (4-1)) and 600 g of the cells of strain 081024-1-1 (see, Example (4-1)). The cell disruption was conducted using DYNO-MILL Type KDL-A (Willy A. Bachofen AG).
The cells of each strain were suspended at a concentration of 40% (W/W) in potassium phosphate buffer (50 mM, pH 6.0) to prepare a suspension. Using a feed pump, the suspension was applied at a rate of 4,000 g/hour into the DYNO-MILL which had been set to conditions for yeast cell disruption.
Potassium phosphate buffer (50 mM, pH 6.0) was added so as to reduce the concentration of the cell disrupted solution to half its current concentration. Concentrated hydrochloric acid was used at a final concentration of 0.2 N to adjust the pH to be highly acidic (i.e., a pH of 1.5 or less). After pepsin (P7000, SIGMA) was added to be at a final concentration of 5 mg/ml, the cell disrupted solution was incubated at 4°C with stirring. After about 96 hours, the cell disrupted solution was centrifuged at 9,000 rpm at 4°C for 30 minutes, and the insoluble fraction was removed to prepare the supernatant. The resulting supernatant was adjusted to a pH of about 10 by adding 10 N NaOH, and then incubated at 4°C with stirring. After about 16 hours, the supernatant was centrifuged at 9,000 rpm at 4°C for 30 minutes, and the insoluble fraction was removed to prepare the supernatant.
To the resulting supernatant, acetic acid was added to be at a final concentration of 0.5 M. The mixture was adjusted to a pH of 3.0 using concentrated hydrochloric acid, and then incubated at 4°C with stirring. After about 16 hours, the incubated supernatant was centrifuged at 9,000 rpm at 4°C for 30 minutes, and the insoluble fraction was removed to prepare the supernatant. NaCl was dissolved at a final concentration of 1 M in the resulting supernatant, which was then incubated at 4°C with stirring. After about 16 hours, the incubated supernatant was centrifuged at 9,000 rpm at 4°C for 30 minutes to collect the insoluble fraction. 0.1 N HCl was added to the insoluble fraction, which was then incubated at 4°C with stirring to prepare a dissolved solution of the insoluble fraction. After about 16 hours, the dissolved solution was centrifuged at 9,000 rpm at 4°C for 30 minutes, and the insoluble fraction was removed prepare the supernatant.
To the resulting supernatant, 1 M potassium phosphate buffer (pH 7.4) was added to be at a final concentration of 0.05 M, and then the pH was adjusted to a pH of about 7.4 using 10 N NaOH. After NaCl was dissolved at a final concentration of 4 M, the mixture was incubated at 4°C with stirring. After about 16 hours, the supernatant was centrifuged at 9,000 rpm at 4°C for 30 minutes to collect the insoluble fraction. 0.1 N HCl was added to the insoluble fraction so that the weight of the added 0.1 N HCl was 2.8-times that of the insoluble fraction, and then the mixture was incubated at 4°C with stirring. After about 16 hours, the mixture was centrifuged at 9,000 rpm at 4°C for 30 minutes to collect the insoluble fraction. To the insoluble fraction, 0.1 N HCl was added, and the mixture was incubated at 4°C with stirring to prepare a dissolved solution of the insoluble fraction. After about 16 hours, the dissolved solution was centrifuged at 9,000 rpm at 4°C for 30 minutes, and the insoluble fraction was removed to prepare the supernatant.
To the resulting supernatant, acetic acid was added to be at a final concentration of 0.5 M, and then the pH was adjusted to a pH of about 3.0 using concentrated hydrochloric acid. NaCl was dissolved at a final concentration of 2 M in the pH-adjusted supernatant, which was then incubated at 4°C with stirring. After about 16 hours, the supernatant was centrifuged at 9,000 rpm at 4°C for 30 minutes to collect the insoluble fraction. 0.1 N HCl was added to the insoluble fraction, which was then incubated at 4°C with stirring to prepare a dissolved solution of the insoluble fraction. After about 16 hours, the dissolved solution was centrifuged at 9,000 rpm at 4°C for 30 minutes, and the insoluble fraction was removed to prepare the supernatant.
The resulting supernatant was placed in a dialysis tube (Spectra/Por 132665, SPECTUM LABORATORIES), dialyzed three times against a 10-times volume of a solution of 1 mM HCl, and then filtered through a 0.22 µm filter (Millex GP, Millipore Corp.). Solutions of respective purified collagens were obtained: 67.6 g of a purified collagen was obtained from the cells of strain 070327-1-11, 86.4 g of a purified collagen from the cells of strain 081022-2-1, 98.8 g of a purified collagen purified collagen from the cells of strain 081022-1-1, 42.6 g of a purified collagen from the cells of strain 081024-3-1, 49.4 g of a purified collagen from the cells of strain 081024-2-1, and 72.6 g of a purified collagen from the cells of strain 081024-1-1.

Example 5 (Analysis of purified collagen solutions) (Part 1)

### (5-1) Determination of the concentration of collagen type I in purified collagen solutions) (Part 1)

Human collagen Type I was obtained from FibroGen. A BCA protein assay kit (Thermo Fisher Scientific) was used and the human collagen Type I was used as standard to determine the concentration of human collagen Type I in the purified collagen solutions. The concentration of collagen Type I was 4.89 mg/ml in the purified collagen solution from the cells of strain 070327-1-11 (see, Example (4-1)), 5.64 mg/ml in the purified collagen solution from the cells of strain 081022-2-1 (see, Example (4-1)), 4.88 mg/ml in the purified collagen solution from the cells of strain 081022-1-1 (see, Example (4-1)), 3.72 mg/ml in the purified collagen solution from the cells of strain 081024-3-1 (see, Example (4-1)), 7.39 mg/ml in the purified collagen solution from the cells of strain 081024-2-1 (see, Example (4-1)), and 4.68 mg/ml in the purified collagen solution from the cells of strain 081024-1-1 (see, Example (4-1)).

### (5-2) Quantitative determination of the number of neutral sugars by phenol-sulfuric acid method (Part 1)

Aqueous mannose solutions of 6.25 µg/ml, 12.5 µg/ml, 25.0 µg/ml, 50.0 µg/ml, 100 µg/ml were prepared. Into a glass test tube, 0.2 g of each of the mannose solutions or the purified collagen solutions was dispensed. To the test tubes, 0.2 ml of a solution of 5% (w/w) phenol in water was added and mixed, followed by dropwise addition of 1 ml concentrated sulfuric acid. The test tubes were allowed to stand at room temperature for 40 minutes for color development, and then their absorbance (OD₄₉₀) was measured. A standard curve which was prepared from the absorbances of the mannose solutions was used to calculate, as a value in terms of mannose, the concentration of neutral sugars in the purified collagen solutions. From the obtained value, the number of neutral sugars per collagen Type I molecule was calculated. Calculation was carried out using the molecular weights of mannose and collagen Type I as 180 and 318800, respectively. The number of neutral sugars was 15.4 for the purified collagen solution from the cells of strain 070327-1-11 (see, Example (4-1)), 18.1 for the purified collagen solution from the cells of strain 081022-2-1 (see, Example (4-1)), 10.5 for the purified collagen solution from the cells of strain 081022-1-1 (see, Example (4-1)), 8.7 for the purified collagen solution from the cells of strain 081024-3-1 (see, Example (4-1)), 15.9 for the purified collagen solution from the cells of strain 081024-2-1 (see, Example (4-1)), and 14.7 for the purified collagen solution from the cells of strain 081024-1-1 (see, Example (4-1)).

Example 6 (Preparation of collagen) (Part 2)

### (6-1) Culturing of yeast and preparation of cells (Part 2)

A transformant [see, Example (3-12-1), Example (3-14-1), Example (3-15-1), and Example (3-16-1)] is inoculated in 100 ml of BMGY medium [1% Yeast Extract, 2% peptone, 100 mM potassium phosphate buffer (pH 6.0), 1.34% Yeast Nitrogen Base (Difco), 4 × 10⁻⁵% biotin, 1% glycerol] which is prepared in a 500-ml baffled Erlenmeyer flask, and cultured with shaking at 30°C for 24 hours of pre-culture.
In a 3-L jar fermentor (Model BMS-03P1, Able Corporation), 0.8 L of Basal salt medium is prepared. The pre-cultured medium is inoculated so as to give a final cell concentration equal to OD₆₆₀ = about 1.25, and then main culture under agitation and aeration is started.
The main culture is started with setting the culture temperature to 30°C, the maximum air flow rate to 0.8 vvm (0.8 L), and the agitation rate to 800 rpm. After all the available glycerol in the medium is consumed and the oxygen consumption is decreased, feeding of a glycerol feed medium (50% glycerol, 1.2% PMT1 solution) is started at a rate of about 15 g/h. The glycerol fed-batch culture is stopped when the OD₆₆₀ of the cultured medium achieves about 130. Subsequently, feeding of a methanol feed medium (98.6% (w/v) methanol, 1.2% PMT1 solution) is started for methanol fed-batch culture. After the methanol fed-batch culture is started, the culture temperature is changed to 32°C and the dissolved oxygen concentration is controlled to be about 8 ppm. Throughout the culture period, the culture medium is maintained at a constant pH of about 5.0 using 28% ammonia water and 4M phosphoric acid. After about 136 hours of culture, the cultured medium is obtained. The composition of the medium used herein is given as follows:
(Composition of Basal salt medium)
   (a) 85% H₃PO₄, 26.7 mL/L;
   (b) CaSO₄-2H₂O 0.93 g/L;
   (c) K₂SO₄, 18.2g/L;
   (d) MgSO₄-7H₂O, 14.9 g/L;
   (e) KOH, 4.13 g/L;
   (f) glycerol, 40 g/L.
   After the above components are mixed, the mixture is subjected to autoclave sterilization. After adjusted to pH 5.0 with 28% ammonia water, 2 ml of PMT1 solution and 1 ml of a solution of defoaming agent in methanol (12.5% Adekanol LG295S) is added per liter of the medium.
(Composition of PMT1 Solution)
   (a) CuSO₄-5H₂O, 6.0 g/L;
   (b) KI, 0.8 g/L;
   (c) MnSO₄-H₂O, 3.0 g/L;
   (d) Na₂MoO₄-2H₂O, 0.2 g/L;
   (e) H₃BO₃, 0.2 g;
   (f) CaSO₄-2H₂O 0.5 g/L;
   (g) ZnCl₂, 20 g/L;
   (h) FeSO₄-7H₂O, 65 g/L;
   (i) biotin, 0.2 g/L;
(j) conc. sulfuric acid, 5 mL/L.
The cultured medium is centrifuged at 5,000 rpm at 4°C for 10 minutes to collect the cells. To remove the medium components, the cells are suspended in potassium phosphate buffer (50 mM, pH 6.0), and then centrifuged to collect the cells. These procedures are repeated twice in total to prepare the cells of strain 050818-3-1.

### (6-2) Extraction and purification of collagen (Part 2)

Extraction and purification of collagen is carried out by disrupting the cells of the transformant [see, Example (3-12-1), Example (3-13-1), Example (3-14-1), Example (3-15-1), and Example (3-16-1)]. The cell disruption is conducted using DYNO-MILL Type KDL-A (Willy A. Bachofen AG).
The cells of each strain are suspended at a concentration of 20% (W/W) in potassium phosphate buffer (50 mM, pH 6.0) to prepare a suspension. Using a feed pump, the suspension is applied at a rate of 4,000 g/hour into the DYNO-MILL which has been set to conditions for yeast cell disruption.
Potassium phosphate buffer (50 mM, pH 6.0) is added so as to reduce the concentration of the cell disrupted solution to half its current concentration. Concentrated hydrochloric acid is used at a final concentration of 0.2 N to adjust the pH to be highly acidic (i.e., a pH of 1.5 or less). After pepsin (P7000, SIGMA) is added to be at a final concentration of 5 mg/ml, the cell disrupted solution is incubated at 4°C with stirring. After about 96 hours, the cell disrupted solution is centrifuged at 9,000 rpm at 4°C for 30 minutes, and the insoluble fraction is removed to prepare the supernatant. The resulting supernatant is adjusted to a pH of about 10 by adding 10 N NaOH, and then incubated at 4°C with stirring. After about 16 hours, the supernatant is centrifuged at 9,000 rpm at 4°C for 30 minutes, and the insoluble fraction is removed to prepare the supernatant.
To the resulting supernatant, acetic acid is added to be at a final concentration of 0.5 M. The mixture is adjusted to a pH of 3.0 using concentrated hydrochloric acid, and then is incubated at 4°C with stirring. After about 16 hours, the incubated supernatant is centrifuged at 9,000 rpm at 4°C for 30 minutes, and the insoluble fraction is removed to prepare the supernatant. NaCl is dissolved at a final concentration of 1 M in the resulting supernatant, which is then incubated at 4°C with stirring. After about 16 hours, the incubated supernatant is centrifuged at 9,000 rpm at 4°C for 30 minutes to collect the insoluble fraction. 0.1 N HCl is added to the insoluble fraction, which is then incubated at 4°C with stirring to prepare a dissolved solution of the insoluble fraction. After about 16 hours, the dissolved solution is centrifuged at 9,000 rpm at 4°C for 30 minutes, and the insoluble fraction is removed to prepare the supernatant.
To the resulting supernatant, 1 M potassium phosphate buffer (pH 7.4) is added to be at a final concentration of 0.05 M, and then the pH is adjusted to a pH of about 7.4 using 10 N NaOH. After NaCl is dissolved at a final concentration of 4 M, the mixture is incubated at 4°C with stirring. After about 16 hours, the supernatant is centrifuged at 9,000 rpm at 4°C for 30 minutes to collect the insoluble fraction. 0.1 N HCl is added to the insoluble fraction, which is then incubated at 4°C with stirring to prepare a dissolved solution of the insoluble fraction. After about 16 hours, the solution is centrifuged at 9,000 rpm at 4°C for 30 minutes, and the insoluble fraction is removed to prepare the supernatant.
To the resulting supernatant, acetic acid is added to be at a final concentration of 0.5 M, and then the pH is adjusted to a pH of about 3.0 using concentrated hydrochloric acid. NaCl is dissolved at a final concentration of 2 M in the pH-adjusted supernatant, which is then incubated at 4°C with stirring. After about 16 hours, the supernatant is centrifuged at 9,000 rpm at 4°C for 30 minutes to collect the insoluble fraction. 0.1 N HCl is added to the insoluble fraction, which is then incubated at 4°C with stirring to prepare a dissolved solution of the insoluble fraction.
The resulting supernatant is placed in a dialysis tube (Spectra/Por 132665, SPECTUM LABORATORIES), dialyzed three times against a 10-times volume of a solution of 1 mM HCl, and then filtered through a 0.22 µm filter (Millex GP, Millipore Corp.) to obtain a purified collagen solution.

Example 7 (Analysis of purified collagen solutions) (Part 2)

### (7-1) Determination of the concentration of collagen in purified collagen solutions) (Part 2)

Human collagen Type I is obtained from FibroGen. A BCA protein assay kit (Thermo Fisher Scientific) is used and the human collagen Type I is used as standard to determine the concentration of collagen in the purified collagen solutions (see, Example (6-2)).

### (7-2) Quantitative determination of the number of neutral sugars by phenol-sulfuric acid method (Part 2)

Aqueous mannose solutions of 6.25 µg/ml, 12.5 µg/ml, 25.0 µg/ml, 50.0 µg/ml, 100 µg/ml are prepared. Into a glass test tube, 0.2 g of each of the mannose solutions or the purified collagen solutions (see, Example (6-2)) is dispensed. To the test tubes, 0.2 ml of a solution of 5% (w/w) phenol in water is added and mixed, followed by dropwise addition of 1 ml concentrated sulfuric acid. The test tubes are allowed to stand at room temperature for 40 minutes for color development, and then their absorbance (OD₄₉₀) is measured. A standard curve which is prepared from the absorbances of the mannose solutions is used to calculate, as values in terms of mannose, the concentration of neutral sugars in the purified collagen solutions. From the obtained value, the number of neutral sugars per collagen molecule is calculated.

### Industrial Applicability

According to the present invention, there can be provided, for example, a transformant which produces a glycine repeat sequence protein which is usable as a high performance versatile material that is more commercially valuable for pharmaceuticals, industrial products, cosmetics, foods etc., in a state where its inherent physical properties are not impaired, and a process for producing a glycine repeat sequence protein using the transformant.

### Sequence Listing Free Text

The nucleotide sequence set forth in SEQ ID NO:1 relates to a primer for cloning of his4.
The nucleotide sequence set forth in SEQ ID NO:2 relates to a primer for cloning of his4.
The nucleotide sequence set forth in SEQ ID NO:3 relates to a primer for cloning of arg4.
The nucleotide sequence set forth in SEQ ID NO:4 relates to a primer for cloning of arg4.
The nucleotide sequence set forth in SEQ ID NO:5 relates to a primer for cloning of the aox1 promoter.
The nucleotide sequence set forth in SEQ ID NO:6 relates to a primer for cloning of the aox1 promoter.
The nucleotide sequence set forth in SEQ ID NO:7 relates to a primer for cloning of the per3 promoter.
The nucleotide sequence set forth in SEQ ID NO:8 relates to a primer for cloning of the per3 promoter.
The nucleotide sequence set forth in SEQ ID NO:9 relates to a primer for cloning of the per3 promoter, which has a restriction enzyme site added thereto.
The nucleotide sequence set forth in SEQ ID NO:10 relates to a primer for cloning of the per3 promoter, which has a restriction enzyme site added thereto.
The nucleotide sequence set forth in SEQ ID NO:11 relates to a primer for cloning of the aox2 promoter, which has a restriction enzyme site added thereto.
The nucleotide sequence set forth in SEQ ID NO:12 relates to a primer for cloning of the aox2 promoter, which has a restriction enzyme site added thereto.
The nucleotide sequence set forth in SEQ ID NO:13 relates to a primer for cloning of the fld1 promoter.
The nucleotide sequence set forth in SEQ ID NO:14 relates to a primer for cloning of the fld1 promoter.
The nucleotide sequence set forth in SEQ ID NO:15 relates to a primer for cloning of the fld1 promoter, which has a restriction enzyme site added thereto.
The nucleotide sequence set forth in SEQ ID NO:16 relates to a primer for cloning of the fld1 promoter, which has a restriction enzyme site added thereto.
The nucleotide sequence set forth in SEQ ID NO:17 relates to a primer for cloning of the aox1 terminator.
The nucleotide sequence set forth in SEQ ID NO:18 relates to a primer for cloning of the aox1 terminator.
The nucleotide sequence set forth in SEQ ID NO:19 relates to a primer for cloning of an aox1 3' non-coding region.
The nucleotide sequence set forth in SEQ ID NO:20 relates to a primer for cloning of an aox1 3' non-coding region.
The nucleotide sequence set forth in SEQ ID NO:21 relates to a primer for cloning of α factor.
The nucleotide sequence set forth in SEQ ID NO:22 relates to a primer for cloning of α factor.
The nucleotide sequence set forth in SEQ ID NO:23 relates to a primer for cloning of human col1a1.
The nucleotide sequence set forth in SEQ ID NO:24 relates to a primer for cloning of human col1a1.
The nucleotide sequence set forth in SEQ ID NO:25 relates to a primer for cloning of human col1a2.
The nucleotide sequence set forth in SEQ ID NO:26 relates to a primer for cloning of human col1a2.
The nucleotide sequence set forth in SEQ ID NO:27 relates to a primer for cloning of human col3a1.
The nucleotide sequence set forth in SEQ ID NO:28 relates to a primer for cloning of human col3a1.
The nucleotide sequence set forth in SEQ ID NO:29 relates to a primer for cloning of the aox1 terminator, which has a restriction enzyme site added thereto.
The nucleotide sequence set forth in SEQ ID NO:30 relates to a primer for cloning of the aox1 terminator, which has a restriction enzyme site added thereto.
The nucleotide sequence set forth in SEQ ID NO:31 relates to Linker 1 for the construction of psn005.
The nucleotide sequence set forth in SEQ ID NO:32 relates to Linker 1 for the construction of psn005.
The nucleotide sequence set forth in SEQ ID NO:33 relates to a primer for the construction of an expression vector that contains a DNA encoding the signal sequence and pro sequence of the α gene, which has a restriction enzyme site added thereto.
The nucleotide sequence set forth in SEQ ID NO:34 relates to a primer for the construction of an expression vector that contains a DNA encoding the signal sequence and pro sequence of the α gene, which has a restriction enzyme site added thereto.
The nucleotide sequence set forth in SEQ ID NO:35 relates to a primer for cloning of p4hb, which has a restriction enzyme site added thereto.
The nucleotide sequence set forth in SEQ ID NO:36 relates to a primer for cloning of p4hb, which has a restriction enzyme site added thereto.
The nucleotide sequence set forth in SEQ ID NO:37 relates to a primer for cloning of an expression unit, which has a restriction enzyme site added thereto.
The nucleotide sequence set forth in SEQ ID NO:38 relates to a primer for cloning of an expression unit, which has a restriction enzyme site added thereto.
The nucleotide sequence set forth in SEQ ID NO:39 relates to Linker 3 for the construction of psn007.
The nucleotide sequence set forth in SEQ ID NO:40 relates to Linker 3 for the construction of psn007.
The nucleotide sequence set forth in SEQ ID NO:41 relates to a primer for cloning of p4ha1, which has a restriction enzyme site added thereto.
The nucleotide sequence set forth in SEQ ID NO:42 relates to a primer for cloning of p4ha1, which has a restriction enzyme site added thereto.
The nucleotide sequence set forth in SEQ ID NO:43 relates to a primer for cloning of an expression unit, which has a restriction enzyme site added thereto.
The nucleotide sequence set forth in SEQ ID NO:44 relates to a primer for cloning of arg4, which has a restriction enzyme site added thereto.
The nucleotide sequence set forth in SEQ ID NO:45 relates to a primer for cloning of arg4, which has a restriction enzyme site added thereto.
The nucleotide sequence set forth in SEQ ID NO:46 relates to a primer for cloning of the hsp47 gene, which has a restriction enzyme site added thereto.
The nucleotide sequence set forth in SEQ ID NO:47 relates to a primer for cloning of the hsp47 gene, which has a restriction enzyme site added thereto.
The nucleotide sequence set forth in SEQ ID NO:48 relates to a primer for cloning of the zeor gene, which has a restriction enzyme site added thereto.
The nucleotide sequence set forth in SEQ ID NO:49 relates to a primer for cloning of the zeor gene, which has a restriction enzyme site added thereto.
The nucleotide sequence set forth in SEQ ID NO:50 relates to a primer for cloning of his4, which has a restriction enzyme site added thereto.
The nucleotide sequence set forth in SEQ ID NO:51 relates to a primer for cloning of his4, which has a restriction enzyme site added thereto.
The nucleotide sequence set forth in SEQ ID NO:52 relates to a primer for cloning of an aox1 3' non-coding region, which has a restriction enzyme site added thereto.
The nucleotide sequence set forth in SEQ ID NO:53 relates to a primer for cloning of an aox1 3' non-coding region, which has a restriction enzyme site added thereto.
The nucleotide sequence set forth in SEQ ID NO:54 relates to Linker 3 for the construction of psn006.
The nucleotide sequence set forth in SEQ ID NO:55 relates to Linker 3 for the construction of psn006.
The nucleotide sequence set forth in SEQ ID NO:56 relates to a primer for cloning of an expression unit, which has a restriction enzyme site added thereto.
The nucleotide sequence set forth in SEQ ID NO:57 relates to a primer for cloning of an expression unit, which has a restriction enzyme site added thereto.
The nucleotide sequence set forth in SEQ ID NO:58 relates to a primer for subcloning of human col1a1.
The nucleotide sequence set forth in SEQ ID NO:59 relates to a primer for subcloning of human col1a1.
The nucleotide sequence set forth in SEQ ID NO:60 relates to a primer for subcloning of human col1a2.
The nucleotide sequence set forth in SEQ ID NO:61 relates to a primer for subcloning of human col1a2.
The nucleotide sequence set forth in SEQ ID NO:62 relates to a primer for subcloning of human col1a1.
The nucleotide sequence set forth in SEQ ID NO:63 relates to a primer for subcloning of human col1a1.
The nucleotide sequence set forth in SEQ ID NO:64 relates to a primer for subcloning of human col1a1.
The nucleotide sequence set forth in SEQ ID NO:65 relates to a primer for subcloning of human col1a1.
The nucleotide sequence set forth in SEQ ID NO:66 relates to a primer for subcloning of human col1a1.
The nucleotide sequence set forth in SEQ ID NO:67 relates to a primer for subcloning of human col1a2.
The nucleotide sequence set forth in SEQ ID NO:68 relates to a primer for subcloning of human col1a2.
The nucleotide sequence set forth in SEQ ID NO:69 relates to a primer for subcloning of human col1a2.
The nucleotide sequence set forth in SEQ ID NO:70 relates to a primer for subcloning of human col1a2.
The nucleotide sequence set forth in SEQ ID NO:71 relates to a primer for subcloning of human col1a2.
The nucleotide sequence set forth in SEQ ID NO:72 relates to a primer for subcloning of human col3a1, which has a restriction enzyme site added thereto.
The nucleotide sequence set forth in SEQ ID NO:73 relates to a primer for subcloning of human col3a1, which has a restriction enzyme site added thereto.
The nucleotide sequence set forth in SEQ ID NO:76 relates to a synthetic DNA of the ORF of human FKBP23, which has codons optimized for yeast.
The nucleotide sequence set forth in SEQ ID NO:78 relates to a synthetic DNA of the ORF of human FKBP19, which has codons optimized for yeast.
The nucleotide sequence set forth in SEQ ID NO:98 relates to a synthetic DNA of the ORF of human collagen, which has codons optimized for yeast.
The nucleotide sequence set forth in SEQ ID NO:100 relates to a synthetic DNA of the ORF of human collagen Type I α2 precursor, which has codons optimized for yeast.
The nucleotide sequence set forth in SEQ ID NO:102 relates to a synthetic DNA of the ORF of human collagen Type II α1 precursor, which has codons optimized for yeast.
The nucleotide sequence set forth in SEQ ID NO:104 relates to a synthetic DNA of the ORF of human collagen Type III α1 precursor, which has codons optimized for yeast.
The nucleotide sequence set forth in SEQ ID NO:112 relates to a synthetic DNA of the ORF of human FKBP13A, which has codons optimized for yeast.
The nucleotide sequence set forth in SEQ ID NO:113 relates to a synthetic DNA of the ORF of human FKBP63, which has codons optimized for yeast.
The nucleotide sequence set forth in SEQ ID NO:114 relates to a synthetic DNA of the ORF of human FKBP65, which has codons optimized for yeast.
The nucleotide sequence set forth in SEQ ID NO:115 relates to a synthetic DNA for a Zeocyn(TM)-resistance cassette.

## Claims

1. A transformant in which all of the following polynucleotides (1), (2) and (3) are transfected into a microbial cell:
(1) a polynucleotide comprising a nucleotide sequence encoding the amino acid sequence of an FK506 binding protein that is capable of binding to FK506 and has a molecular weight of 15,000 or more and 60,000 or less;
(2) a polynucleotide comprising a nucleotide sequence encoding the amino acid sequence of a prolyl hydroxylase; and
(3) a polynucleotide comprising a nucleotide sequence encoding the amino acid sequence of a glycine repeat sequence protein having the following characteristics (A) and (B):
<characteristic (A)> the polypeptide chain of the glycine repeat sequence protein having a continuous, Gly-Xaa-Yaa repeating sequence, and
<characteristic (B)> an imino acid being contained in the continuous, Gly-Xaa-Yaa repeating sequence of the polypeptide chain of the glycine repeat sequence protein,
wherein Xaa and Yaa each represent any amino acid.

2. The transformant according to claim 1, which produces all of the proteins encoded by the polynucleotides (1), (2) and (3) within the cell.

3. The transformant according to claim 1 or 2, wherein the FK506 binding protein is FKBP23 or FKBP19.

4. The transformant according to claim 1 or 2, wherein the FK506 binding protein is FKBP23.

5. The transformant according to any one of claims 1 to 4, wherein the polynucleotide comprising a nucleotide sequence encoding the amino acid sequence of the FK506 binding protein is linked to downstream of a yeast derived promoter.

6. The transformant according to any one of claims 1 to 5, which further produces a lysyl hydroxylase within the cell.

7. The transformant according to claim 6, wherein the lysyl hydroxylase is at least one lysyl hydroxylase selected from lysyl hydroxylase 1 and lysyl hydroxylase 3.

8. The transformant according to any one of claims 1 to 5, which further produces Hsp47 within the cell.

9. The transformant according to any one of claims 1 to 8, wherein the prolyl hydroxylase is prolyl 4-hydroxylase.

10. The transformant according to any one of claims 1 to 8, wherein the prolyl hydroxylase is an enzyme comprising a prolyl 4-hydroxylase α subunit and a prolyl 4-hydroxylase β subunit.

11. The transformant according to claim 10, wherein a yeast α-factor prepro sequence is fused at the amino terminus of the prolyl 4-hydroxylase β subunit.

12. The transformant according to claim 10 or 11, wherein the prolyl 4-hydroxylase α subunit is an α1 subunit, an α2 subunit, or an α3 subunit.

13. The transformant according to any one of claims 1 to 12, wherein at least one polynucleotide comprising a nucleotide sequence encoding the amino acid sequence of the prolyl hydroxylase protein is linked to downstream of a yeast derived promoter.

14. The transformant according to any one of claims 1 to 13, wherein the glycine repeat sequence protein having the characteristics (A) and (B) is at least one collagen selected from among collagens of Type I to Type XXIX.

15. The transformant according to any one of claims 1 to 13, wherein the glycine repeat sequence protein having the characteristics (A) and (B) is at least one collagen selected from among collagen Type I, collagen Type II, and collagen Type III.

16. The transformant according to any one of claims 1 to 15, wherein the microbial cell is an eukaryote cell.

17. The transformant according to claim 16, wherein the eukaryote cell is a yeast cell.

18. The transformant according to claim 17, wherein the yeast cell is a methanol-utilizing yeast cell.

19. The transformant according to claim 18, wherein the methanol-utilizing yeast cell is Komagataella pastoris.

20. A glycine repeat sequence protein having the characteristics (A) and (B) which is obtainable by being produced by the transformant according to any one of claims 1 to 19.

21. A process for producing a glycine repeat sequence protein, comprising:
a first step of transfecting all of the following polynucleotides (1), (2) and (3) into a microbial cell:
(1) a polynucleotide comprising a nucleotide sequence encoding the amino acid sequence of an FK506 binding protein that is capable of binding to FK506 and has a molecular weight of 15,000 or more and 60,000 or less,
(2) a polynucleotide comprising a nucleotide sequence encoding the amino acid sequence of a prolyl hydroxylase, and
(3) a polynucleotide comprising a nucleotide sequence encoding the amino acid sequence of a glycine repeat sequence protein having the following characteristics (A) and (B):
<characteristic (A)> the polypeptide chain of the glycine repeat sequence protein having a continuous, Gly-Xaa-Yaa repeating sequence, and
<characteristic (B)> an imino acid being contained in the continuous, Gly-Xaa-Yaa repeating sequence of the polypeptide chain of the glycine repeat sequence protein,
wherein Xaa and Yaa each represent any amino acid;
a second step of culturing the transformant resulting from the first step, thereby producing the glycine repeat sequence protein; and
a third step of collecting the glycine repeat sequence protein produced in the second step.

22. A glycine repeat sequence protein produced by the process according to claim 21.


SEQUENCE LISTING

<110>  SUMITOMO CHEMICAL COMPANY, LIMITED
       Dainippon Sumitomo Pharma Co., Ltd.
       KOKEN CO., LTD.

<120>  Transformant with the ability to produce protein having glycin repeat
sequnce

<130>  670192

<150>  JP2010-057484
<151>  2010-03-15

<160>  121

<170>  PatentIn version 3.1

<210>  1
<211>  30
<212>  DNA
<213>  Artificial Sequence

<220>
<223>  primer for cloning of his4

<400>  1
gatctcctga tgactgactc actgataata                                        30


<210>  2
<211>  30
<212>  DNA
<213>  Artificial Sequence

<220>
<223>  primer for cloning of his4

<400>  2
taattaaata agtcccagtt tctccatacg                                        30


<210>  3
<211>  25
<212>  DNA
<213>  Artificial Sequence

<220>
<223>  primer for cloning of arg4

<400>  3
acgaaaatat ggtacctgcc ctcac                                             25


<210>  4
<211>  30
<212>  DNA
<213>  Artificial Sequence

<220>
<223>  primer for cloning of arg4

<400>  4
gttctatcta cccgaggaaa ccgatacata                                        30

<210>  5
<211>  27
<212>  DNA
<213>  Artificial Sequence

<220>
<223>  primer for cloning of aox1 promoter

<400>  5
agatctaaca tccaaagacg aaaggtt                                          27


<210>  6
<211>  29
<212>  DNA
<213>  Artificial Sequence

<220>
<223>  primer for cloning of aox1 promoter

<400>  6
atccaccacc tagaactagg atatcaaac                                        29


<210>  7
<211>  25
<212>  DNA
<213>  Artificial Sequence

<220>
<223>  primer for cloning of per3 promoter

<400>  7
ctaagggtct cactggtgtt tcagc                                            25


<210>  8
<211>  24
<212>  DNA
<213>  Artificial Sequence

<220>
<223>  primer for cloning of per3 promoter

<400>  8
agttccttgc aactgtagtg gtcg                                             24


<210>  9
<211>  27
<212>  DNA
<213>  Artificial Sequence

<220>
<223>  primer for cloning of per3 promoter with addition of restriction
       enzyme site

<400>  9
aaccgcggct cgtcactatc gtcgttg                                          27


<210>  10
<211>  35
<212>  DNA

<213>  Artificial Sequence

<220>
<223>  primer for cloning of per3 promoter with addition of restriction
       enzyme site

<400>  10
cgaacgttac ctgaagatag gtaaaaaaaa attgc                                35


<210>  11
<211>  32
<212>  DNA
<213>  Artificial Sequence

<220>
<223>  primer for cloning of aox2 promoter with addition of restriction
       enzyme site

<400>  11
aaccgcggct agtagaactt tgacatctgc ta                                  32


<210>  12
<211>  31
<212>  DNA
<213>  Artificial Sequence

<220>
<223>  primer for cloning of aox2 promoter with addition of restriction
       enzyme site

<400>  12
cgaacgtttt gatttgtttg tggggattta g                                   31


<210>  13
<211>  24
<212>  DNA
<213>  Artificial Sequence

<220>
<223>  primer for cloning of fld1 promoter

<400>  13
gcagtgttgg ctaacgtcta ttcg                                           24


<210>  14
<211>  23
<212>  DNA
<213>  Artificial Sequence

<220>
<223>  primer for cloning of fld1 promoter

<400>  14
acttcatggg ctcttggagg aag                                            23


<210>  15
<211>  31
<212>  DNA
<213>  Artificial Sequence

<220>
<223>  primer for cloning of fld1 promoter with addition of restriction
       enzyme site

<400>  15
aaccgcggcc tgaataccgt aacatagtga c                                    31


<210>  16
<211>  33
<212>  DNA
<213>  Artificial Sequence

<220>
<223>  primer for cloning of fld1 promoter with addition of restriction
       enzyme site

<400>  16
cgaacgtttc aagaattgta tgaacaagca aag                                  33


<210>  17
<211>  26
<212>  DNA
<213>  Artificial Sequence

<220>
<223>  primer for cloning of aox1 terminator

<400>  17
ccttagacat gactgttcct cagttc                                          26


<210>  18
<211>  24
<212>  DNA
<213>  Artificial Sequence

<220>
<223>  primer for cloning of aox1 terminator

<400>  18
gcacaaacga acgtctcact taat                                            24


<210>  19
<211>  30
<212>  DNA
<213>  Artificial Sequence

<220>
<223>  primer for cloning of aox1 3' non coding region

<400>  19
tcgagtatct atgattggaa gtatgggaat                                      30


<210>  20
<211>  30
<212>  DNA
<213>  Artificial Sequence

<220>
<223>  primer for cloning of aox1 3' non coding region

<400>  20
gatcttgaga taaatttcac gtttaaaatc                                        30


<210>  21
<211>  32
<212>  DNA
<213>  Artificial Sequence

<220>
<223>  primer for cloning of alpha factor

<400>  21
tcaaacaaga agattacaaa ctatcaattt ca                                     32


<210>  22
<211>  29
<212>  DNA
<213>  Artificial Sequence

<220>
<223>  primer for cloning of alpha factor

<400>  22
gtacgagcta aaagtacagt gggaacaaa                                         29


<210>  23
<211>  27
<212>  DNA
<213>  Artificial Sequence

<220>
<223>  primer for cloning of human col1a1

<400>  23
cagccacaaa gagtctacat gtctagg                                           27


<210>  24
<211>  20
<212>  DNA
<213>  Artificial Sequence

<220>
<223>  primer for cloning of human col1a1

<400>  24
aggttgggat ggagggagtt                                                   20


<210>  25
<211>  37
<212>  DNA
<213>  Artificial Sequence

<220>
<223>  primer for cloning of human col1a2

<400>  25
gccaagcttg catgctcagc tttgtggata cgcggac                                37


<210>  26

<211>  44
<212>  DNA
<213>  Artificial Sequence

<220>
<223>  primer for cloning of human col1a2

<400>  26
cggtacccgg ggatccttat ttgaaacaga ctgggccaat gtcc                        44


<210>  27
<211>  20
<212>  DNA
<213>  Artificial Sequence

<220>
<223>  primer for cloning of human col3a1

<400>  27
ggctgagttt tatgacgggc                                                   20


<210>  28
<211>  20
<212>  DNA
<213>  Artificial Sequence

<220>
<223>  primer for cloning of human col3a1

<400>  28
gacaagatta gaacaagagg                                                   20


<210>  29
<211>  35
<212>  DNA
<213>  Artificial Sequence

<220>
<223>  primer for cloning of aox1 terminator with addition of restrictio
       n enzyme site

<400>  29
tcgactagtt tagacatgac tgttcctcag ttcaa                                  35


<210>  30
<211>  30
<212>  DNA
<213>  Artificial Sequence

<220>
<223>  primer for cloning of aox1 terminator with addition of restrictio
       n enzyme site

<400>  30
aactgcaggc acaaacgaac gtctcactta                                        30


<210>  31
<211>  35
<212>  DNA
<213>  Artificial Sequence

<220>
<223>  linker 1 for construction of psn005

<400>  31
tattcgaaac gcatatgtga ccggcagact agtgg                                35


<210>  32
<211>  35
<212>  DNA
<213>  Artificial Sequence

<220>
<223>  linker 1 for construction of psn005

<400>  32
ccactagtct gccggtcaca tatgggtttc gaata                                35


<210>  33
<211>  35
<212>  DNA
<213>  Artificial Sequence

<220>
<223>  primer for construction of expression vector of alpha pre pro seq
       uence with addition of restriction enzyme site

<400>  33
ggttcgaaac gatgagattt ccttcaattt ttact                                35


<210>  34
<211>  31
<212>  DNA
<213>  Artificial Sequence

<220>
<223>  primer for construction of expression vector of alpha pre pro seq
       uence with addition of restriction enzyme site

<400>  34
tcgactagta gcttcagcct ctcttttatc c                                    31


<210>  35
<211>  25
<212>  DNA
<213>  Artificial Sequence

<220>
<223>  primer for cloning of p4hb with addition of restriction enzyme si
       te

<400>  35
ttactagtga cgcccccgag gagga                                           25


<210>  36
<211>  35
<212>  DNA
<213>  Artificial Sequence

<220>

<223>  primer for cloning of p4hb with addition of restriction enzyme si
      te

<400>  36
ttactagttt acagttcatc tttcacagct ttctg                                35


<210>  37
<211>  35
<212>  DNA
<213>  Artificial Sequence

<220>
<223>  primer for cloning of expression unit with addition of restrictio
      n enzyme site

<400>  37
aaccgcggtc taacatccaa agacgaaagg ttgaa                                35


<210>  38
<211>  35
<212>  DNA
<213>  Artificial Sequence

<220>
<223>  primer for cloning of expression unit with addition of restrictio
      n enzyme site

<400>  38
aacccggggc acaaacgaac gtctcactta atctt                                35


<210>  39
<211>  35
<212>  DNA
<213>  Artificial Sequence

<220>
<223>  linker 3 for construction of psn007

<400>  39
tattcgaaac gacgcgtgtc agctagcact agtgc                                35


<210>  40
<211>  35
<212>  DNA
<213>  Artificial Sequence

<220>
<223>  linker 3 for construction of psn007

<400>  40
gcactagtgc tagctgacac gcgtcgtttc gaata                                35


<210>  41
<211>  35
<212>  DNA
<213>  Artificial Sequence

<220>
<223>  primer for cloning of p4ha1 with addition of restriction enzyme s
      ite

<400>  41
tattcgaaac gatgatctgg tatatattaa ttata                                35


<210>  42
<211>  35
<212>  DNA
<213>  Artificial Sequence

<220>
<223>  primer for cloning of p4ha1 with addition of restriction enzyme s
       ite

<400>  42
ttgctagctc attccaattc tgacaacgta caagg                                35


<210>  43
<211>  35
<212>  DNA
<213>  Artificial Sequence

<220>
<223>  primer for cloning of expression unit with addition of restrictio
       n enzyme site

<400>  43
aacccgggtc taacatccaa agacgaaagg ttgaa                                35


<210>  44
<211>  30
<212>  DNA
<213>  Artificial Sequence

<220>
<223>  primer for cloning of arg4 with addition of restriction enzyme si
       te

<400>  44
aactcgagac gaaaatatgg tacctgccct                                      30


<210>  45
<211>  32
<212>  DNA
<213>  Artificial Sequence

<220>
<223>  primer for cloning of arg4 with addition of restriction enzyme si
       te

<400>  45
ccatcgatac agaggtatca tccaatgatt cc                                   32


<210>  46
<211>  32
<212>  DNA
<213>  Artificial Sequence

<220>
<223>  primer for cloning of hsp47 gene with addition of restriction enz
       yme site

<400>  46
tattcgaaac gatgcgctcc ctcctgcttc tc                                    32


<210>  47
<211>  34
<212>  DNA
<213>  Artificial Sequence

<220>
<223>  primer for cloning of hsp47 gene with addition of restriction enz
       yme site

<400>  47
ttactagtta taactcgtct cgcatcttgt cacc                                  34


<210>  48
<211>  27
<212>  DNA
<213>  Artificial Sequence

<220>
<223>  primer for cloning of zeor gene with addition of restriction enzy
       me site

<400>  48
ttatcgatcc cacacaccat agcttca                                          27


<210>  49
<211>  29
<212>  DNA
<213>  Artificial Sequence

<220>
<223>  primer for cloning of zeor gene with addition of restriction enzy
       me site

<400>  49
tgatcgatag cttgcaaatt aaagccttc                                        29


<210>  50
<211>  32
<212>  DNA
<213>  Artificial Sequence

<220>
<223>  primer for cloning of his4 with addition of restriction enzyme si
       te

<400>  50
ggaagcttga tctcctgatg actgactcac tg                                    32


<210>  51
<211>  34
<212>  DNA
<213>  Artificial Sequence

<220>
<223>  primer for cloning of his4 with addition of restriction enzyme si
       te

<400>  51
ccctgcagta attaaataag tcccagtttc tcca                                34


<210>  52
<211>  34
<212>  DNA
<213>  Artificial Sequence

<220>
<223>  primer for cloning of aox1 3' non coding region with addition of
       restriction enzyme site

<400>  52
gcatcgattc gagtatctat gattggaagt atgg                                34


<210>  53
<211>  35
<212>  DNA
<213>  Artificial Sequence

<220>
<223>  primer for cloning of aox1 3' non coding region with addition of
       restriction enzyme site

<400>  53
aagggcccga tcttgagata aatttcacgt ttaaa                               35


<210>  54
<211>  35
<212>  DNA
<213>  Artificial Sequence

<220>
<223>  linker 3 for construction of psn006

<400>  54
tattcgaaac gcatatggta ccggcagact agtgg                               35


<210>  55
<211>  35
<212>  DNA
<213>  Artificial Sequence

<220>
<223>  linker 3 for construction of psn006

<400>  55
ccactagtcg cctaggcgac atatggtttc gaata                               35


<210>  56
<211>  35
<212>  DNA
<213>  Artificial Sequence

<220>
<223>  primer for cloning of expression unit with addition of restrictio
       n enzyme site

<400>  56

aacggccgtc taacatccaa agacgaaagg ttgaa                                35

<210>  57
<211>  35
<212>  DNA
<213>  Artificial Sequence

<220>
<223>  primer for cloning of expression unit with addition of restrictio
       n enzyme site

<400>  57
aacggccggc acaaacgaac gtctcactta atctt                                35

<210>  58
<211>  17
<212>  DNA
<213>  Artificial Sequence

<220>
<223>  primer for subcloning of human col1a1

<400>  58
cggcttacca gcctcgc                                                    17

<210>  59
<211>  16
<212>  DNA
<213>  Artificial Sequence

<220>
<223>  primer for subcloning of human col1a1

<400>  59
ggaccaccag ggccgc                                                     16

<210>  60
<211>  19
<212>  DNA
<213>  Artificial Sequence

<220>
<223>  primer for subcloning of human col1a2

<400>  60
gggtttacct gggtggccg                                                  19

<210>  61
<211>  17
<212>  DNA
<213>  Artificial Sequence

<220>
<223>  primer for subcloning of human col1a2

<400>  61
ggacctcctg gcccacc                                                    17

<210>  62

<211>  19
<212>  DNA
<213>  Artificial Sequence

<220>
<223>  primer for subcloning of human col1a1

<400>  62
ggtccacagg gtccaggag                                                    19


<210>  63
<211>  24
<212>  DNA
<213>  Artificial Sequence

<220>
<223>  primer for subcloning of human col1a1

<400>  63
atcagctcct ggtgatccct tttc                                              24


<210>  64
<211>  21
<212>  DNA
<213>  Artificial Sequence

<220>
<223>  primer for subcloning of human col1a1

<400>  64
tggtggacct tgaaaaccct g                                                 21


<210>  65
<211>  16
<212>  DNA
<213>  Artificial Sequence

<220>
<223>  primer for subcloning of human col1a1

<400>  65
ggaccccagg gccccg                                                       16


<210>  66
<211>  22
<212>  DNA
<213>  Artificial Sequence

<220>
<223>  primer for subcloning of human col1a1

<400>  66
atcagcacca ggggatcctt tc                                                22


<210>  67
<211>  16
<212>  DNA
<213>  Artificial Sequence

<220>

<223>  primer for subcloning of human col1a2

<400>  67
gggccagttg gcgcag                                                    16


<210>  68
<211>  24
<212>  DNA
<213>  Artificial Sequence

<220>
<223>  primer for subcloning of human col1a2

<400>  68
tgcttctcca gatggtcctt tctc                                           24


<210>  69
<211>  17
<212>  DNA
<213>  Artificial Sequence

<220>
<223>  primer for subcloning of human col1a2

<400>  69
tgcgggtcct tggaatc                                                   17


<210>  70
<211>  17
<212>  DNA
<213>  Artificial Sequence

<220>
<223>  primer for subcloning of human col1a2

<400>  70
ggccctgttg gtgctgc                                                   17


<210>  71
<211>  21
<212>  DNA
<213>  Artificial Sequence

<220>
<223>  primer for subcloning of human col1a2

<400>  71
agcctctcca gagggaccct t                                              21


<210>  72
<211>  35
<212>  DNA
<213>  Artificial Sequence

<220>
<223>  primer for cloning of human col3a1 with addition of restriction e
       nzyme site

<400>  72
tattcgaaac gatgatgagc tttgtgcaaa agggg                               35

<210>  73
<211>  34
<212>  DNA
<213>  Artificial Sequence

<220>
<223>  primer for cloning of human col3a1 with addition of restriction e
       nzyme site

<400>  73
ttactagttt ataaaaagca aacagggcca acgt                                34


<210>  74
<211>  222
<212>  PRT
<213>  Homo sapiens

<400>  74

Met Pro Lys Thr Met His Phe Leu Phe Arg Phe Ile Val Phe Phe Tyr
1               5                   10                  15

Leu Trp Gly Leu Phe Thr Ala Gln Arg Gln Lys Lys Glu Glu Ser Thr
            20                  25                  30

Glu Glu Val Lys Ile Glu Val Leu His Arg Pro Glu Asn Cys Ser Lys
        35                  40                  45

Thr Ser Lys Lys Gly Asp Leu Leu Asn Ala His Tyr Asp Gly Tyr Leu
    50                  55                  60

Ala Lys Asp Gly Ser Lys Phe Tyr Cys Ser Arg Thr Gln Asn Glu Gly
65                  70                  75                  80

His Pro Lys Trp Phe Val Leu Gly Val Gly Gln Val Ile Lys Gly Leu
                85                  90                  95

Asp Ile Ala Met Thr Asp Met Cys Pro Gly Glu Lys Arg Lys Val Val
            100                 105                 110

Ile Pro Pro Ser Phe Ala Tyr Gly Lys Glu Gly Tyr Ala Glu Gly Lys
        115                 120                 125

Ile Pro Pro Asp Ala Thr Leu Ile Phe Glu Ile Glu Leu Tyr Ala Val
    130                 135                 140

Thr Lys Gly Pro Arg Ser Ile Glu Thr Phe Lys Gln Ile Asp Met Asp
145                 150                 155                 160

Asn Asp Arg Gln Leu Ser Lys Ala Glu Ile Asn Leu Tyr Leu Gln Arg
                165                 170                 175

Glu Phe Glu Lys Asp Glu Lys Pro Arg Asp Lys Ser Tyr Gln Asp Ala
            180                 185                 190

Val Leu Glu Asp Ile Phe Lys Lys Asn Asp His Asp Gly Asp Gly Phe
        195                 200                 205

Ile Ser Pro Lys Glu Tyr Asn Val Tyr Gln His Asp Glu Leu
    210                 215                 220

<210>  75
<211>  201
<212>  PRT
<213>  Homo sapiens

<400>  75

Met Thr Leu Arg Pro Ser Leu Leu Pro Leu His Leu Leu Leu Leu Leu
1               5                   10                  15

Leu Leu Ser Ala Ala Val Cys Arg Ala Glu Ala Gly Leu Glu Thr Glu
            20                  25                  30

Ser Pro Val Arg Thr Leu Gln Val Glu Thr Leu Val Glu Pro Pro Glu
        35                  40                  45

Pro Cys Ala Glu Pro Ala Ala Phe Gly Asp Thr Leu His Ile His Tyr
    50                  55                  60

Thr Gly Ser Leu Val Asp Gly Arg Ile Ile Asp Thr Ser Leu Thr Arg
65                  70                  75                  80

Asp Pro Leu Val Ile Glu Leu Gly Gln Lys Gln Val Ile Pro Gly Leu
                85                  90                  95

Glu Gln Ser Leu Leu Asp Met Cys Val Gly Glu Lys Arg Arg Ala Ile
            100                 105                 110

Ile Pro Ser His Leu Ala Tyr Gly Lys Arg Gly Phe Pro Pro Ser Val
        115                 120                 125

Pro Ala Asp Ala Val Val Gln Tyr Asp Val Glu Leu Ile Ala Leu Ile
    130                 135                 140

Arg Ala Asn Tyr Trp Leu Lys Leu Val Lys Gly Ile Leu Pro Leu Val
145                 150                 155                 160

Gly Met Ala Met Val Pro Ala Leu Leu Gly Leu Ile Gly Tyr His Leu
                165                 170                 175

Tyr Arg Lys Ala Asn Arg Pro Lys Val Ser Lys Lys Lys Leu Lys Glu

180                 185                 190

Glu Lys Arg Asn Lys Ser Lys Lys Lys
        195                 200

<210>  76
<211>  669
<212>  DNA
<213>  Artificial Sequence

<220>
<223>  synthesized DNA of human FKBP23 ORF having codon optimized for yea
       st

<400>  76
atgccaaaga ctatgcactt tctatttcga tttatcgtgt tcttttatct ctggggacta      60

ttcactgccc agcgtcaaaa aaaggaagag agtaccgagg aagttaagat tgaagttctg     120

cacaggcctg aaaattgctc caagacgagc aagaaaggcg atctgcttaa tgcccattac     180

gatgggtact tggctaagga tggttcaaaa ttctactgtt ctcgtaccca aaacgaggga     240

cacccaaagt ggtttgtctt gggtgtcgga caggttatta aaggtttgga tatcgctatg     300

acagatatgt gtcccggcga aaagagaaaa gttgttatac cgccctcttt tgcttatgga     360

aaagagggtt atgccgaagg taaaatacct cctgacgcta cattgatctt cgagattgaa     420

ttatatgctg tcactaaggg accaagatcg attgaaactt tcaaacaaat tgacatggat     480

aacgacagac aactttccaa ggcagaaatt aacctttacc tgcaaagaga attcgagaaa     540

gacgagaaac ctagagacaa gtcataccaa gacgcagtgt tggaagatat ctttaagaag     600

aatgaccatg atggtgatgg ttttatttct ccaaaagagt ataacgtata ccagcatgat     660

gagttataa                                                             669

<210>  77
<211>  669
<212>  DNA
<213>  Homo sapiens

<400>  77
atgccaaaaa ccatgcattt cttattcaga ttcattgttt tcttttatct gtggggcctt      60

tttactgctc agagacaaaa gaaagaggag agcaccgaag aagtgaaaat agaagttttg     120

catcgtccag aaaactgctc taagacaagc aagaagggag acctactaaa tgcccattat     180

gacggctacc tggctaaaga cggctcgaaa ttctactgca gccggacaca aaatgaaggc     240

caccccaaat ggtttgttct tggtgttggg caagtcataa aaggcctaga cattgctatg     300

acagatatgt gccctggaga aaagcgaaaa gtagttatac ccccttcatt tgcatacgga     360

aaggaaggct atgcagaagg caagattcca ccggatgcta cattgatttt tgagattgaa     420

ctttatgctg tgaccaaagg accacggagc attgagacat ttaaacaaat agacatggac     480

aatgacaggc agctctctaa agccgagata aacctctact tgcaaaggga atttgaaaaa     540

gatgagaagc cacgtgacaa gtcatatcag gatgcagttt tagaagatat ttttaagaag 600

aatgaccatg atggtgatgg cttcatttct cccaaggaat acaatgtata ccaacacgat 660

gaactatag 669

<210> 78
<211> 606
<212> DNA
<213> Artificial Sequence

<220>
<223> synthesized DNA of human FKBP19 ORF having codon optimized for yea
      st

<400> 78
atgactttga gaccatcctt actaccttta catctattgt tactcctgct cttgtctgct 60

gctgtgtgcc gtgctgaagc tgggcttgaa acagagtcac cagtgcgaac ccttcaagtc 120

gagaccctgg ttgaacctcc ggaaccttgt gccgaaccag ccgcttttgg tgatacgctc 180

catatccact atactggatc tttggtcgat ggacgaatta tcgacacatc ccttactagg 240

gaccctttgg ttattgaatt gggtcagaaa caagttattc ctggcttgga gcaaagtctg 300

ttggatatgt gtgtaggtga gaagcgtaga gcaataatcc catcacactt ggcatacggt 360

aaaagaggtt tccccccaag tgttcctgcc gacgccgtcg tacagtacga tgtcgagcta 420

attgcattga ttagagctaa ctattggtta aagcttgtga aaggtattct gccacttgtt 480

ggcatggcta tggttccagc cctattagga ttgatcggat accatctgta tagaaaagca 540

aatagaccca aggtttctaa gaagaaattg aaagaagaga agaggaacaa gagcaagaag 600

aaataa 606

<210> 79
<211> 606
<212> DNA
<213> Homo sapiens

<400> 79
atgaccctgc gcccctcact cctcccgctc catctgctgc tgctgctgct gctcagtgcg 60

gcggtgtgcc gggctgaggc tgggctcgaa accgaaagtc ccgtccggac cctccaagtg 120

gagaccctgg tggagccccc agaaccatgt gccgagcccg ctgcttttgg agacacgctt 180

cacatacact acacgggaag cttggtagat ggacgtatta ttgacacctc cctgaccaga 240

gaccctctgg ttatagaact tggccaaaag caggtgattc caggtctgga gcagagtctt 300

ctcgacatgt gtgtgggaga gaagcgaagg gcaatcattc cttctcactt ggcctatgga 360

aaacggggat ttccaccatc tgtcccagcg gatgcagtgg tgcagtatga cgtggagctg 420

attgcactaa tccgagccaa ctactggcta aagctggtga agggcatttt gcctctggta 480

gggatggcca tggtgccagc cctcctgggc ctcattgggt atcacctata cagaaaggcc 540

aatagaccca aagtctccaa aaagaagctc aaggaagaga aacgaaacaa gagcaaaaag     600

aaataa                                                                606

<210>  80
<211>  534
<212>  PRT
<213>  Homo sapiens

<400>  80

Met Ile Trp Tyr Ile Leu Ile Ile Gly Ile Leu Leu Pro Gln Ser Leu
1               5                   10                  15

Ala His Pro Gly Phe Phe Thr Ser Ile Gly Gln Met Thr Asp Leu Ile
            20                  25                  30

His Thr Glu Lys Asp Leu Val Thr Ser Leu Lys Asp Tyr Ile Lys Ala
        35                  40                  45

Glu Glu Asp Lys Leu Glu Gln Ile Lys Lys Trp Ala Glu Lys Leu Asp
    50                  55                  60

Arg Leu Thr Ser Thr Ala Thr Lys Asp Pro Glu Gly Phe Val Gly His
65                  70                  75                  80

Pro Val Asn Ala Phe Lys Leu Met Lys Arg Leu Asn Thr Glu Trp Ser
                85                  90                  95

Glu Leu Glu Asn Leu Val Leu Lys Asp Met Ser Asp Gly Phe Ile Ser
            100                 105                 110

Asn Leu Thr Ile Gln Arg Gln Tyr Phe Pro Asn Asp Glu Asp Gln Val
        115                 120                 125

Gly Ala Ala Lys Ala Leu Leu Arg Leu Gln Asp Thr Tyr Asn Leu Asp
    130                 135                 140

Thr Asp Thr Ile Ser Lys Gly Asn Leu Pro Gly Val Lys His Lys Ser
145                 150                 155                 160

Phe Leu Thr Ala Glu Asp Cys Phe Glu Leu Gly Lys Val Ala Tyr Thr
                165                 170                 175

Glu Ala Asp Tyr Tyr His Thr Glu Leu Trp Met Glu Gln Ala Leu Arg
            180                 185                 190

Gln Leu Asp Glu Gly Glu Ile Ser Thr Ile Asp Lys Val Ser Val Leu
        195                 200                 205

Asp Tyr Leu Ser Tyr Ala Val Tyr Gln Gln Gly Asp Leu Asp Lys Ala

210                 215                 220

Leu Leu Leu Thr Lys Lys Leu Leu Glu Leu Asp Pro Glu His Gln Arg
225                 230                 235                 240

Ala Asn Gly Asn Leu Lys Tyr Phe Glu Tyr Ile Met Ala Lys Glu Lys
                245                 250                 255

Asp Val Asn Lys Ser Ala Ser Asp Asp Gln Ser Asp Gln Lys Thr Thr
            260                 265                 270

Pro Lys Lys Lys Gly Val Ala Val Asp Tyr Leu Pro Glu Arg Gln Lys
        275                 280                 285

Tyr Glu Met Leu Cys Arg Gly Glu Gly Ile Lys Met Thr Pro Arg Arg
    290                 295                 300

Gln Lys Lys Leu Phe Cys Arg Tyr His Asp Gly Asn Arg Asn Pro Lys
305                 310                 315                 320

Phe Ile Leu Ala Pro Ala Lys Gln Glu Asp Glu Trp Asp Lys Pro Arg
                325                 330                 335

Ile Ile Arg Phe His Asp Ile Ile Ser Asp Ala Glu Ile Glu Ile Val
            340                 345                 350

Lys Asp Leu Ala Lys Pro Arg Leu Arg Arg Ala Thr Ile Ser Asn Pro
        355                 360                 365

Ile Thr Gly Asp Leu Glu Thr Val His Tyr Arg Ile Ser Lys Ser Ala
    370                 375                 380

Trp Leu Ser Gly Tyr Glu Asn Pro Val Val Ser Arg Ile Asn Met Arg
385                 390                 395                 400

Ile Gln Asp Leu Thr Gly Leu Asp Val Ser Thr Ala Glu Glu Leu Gln
                405                 410                 415

Val Ala Asn Tyr Gly Val Gly Gly Gln Tyr Glu Pro His Phe Asp Phe
            420                 425                 430

Ala Arg Lys Asp Glu Pro Asp Ala Phe Lys Glu Leu Gly Thr Gly Asn
        435                 440                 445

Arg Ile Ala Thr Trp Leu Phe Tyr Met Ser Asp Val Ser Ala Gly Gly
    450                 455                 460

Ala Thr Val Phe Pro Glu Val Gly Ala Ser Val Trp Pro Lys Lys Gly
465                 470                 475                 480

Thr Ala Val Phe Trp Tyr Asn Leu Phe Ala Ser Gly Glu Gly Asp Tyr
                485                 490                 495

Ser Thr Arg His Ala Ala Cys Pro Val Leu Val Gly Asn Lys Trp Val
            500                 505                 510

Ser Asn Lys Trp Leu His Glu Arg Gly Gln Glu Phe Arg Arg Pro Cys
        515                 520                 525

Thr Leu Ser Glu Leu Glu
    530

<210>  81
<211>  533
<212>  PRT
<213>  Homo sapiens

<400>  81

Met Lys Leu Trp Val Ser Ala Leu Leu Met Ala Trp Phe Gly Val Leu
1               5                   10                  15

Ser Cys Val Gln Ala Glu Phe Phe Thr Ser Ile Gly His Met Thr Asp
            20                  25                  30

Leu Ile Tyr Ala Glu Lys Glu Leu Val Gln Ser Leu Lys Glu Tyr Ile
        35                  40                  45

Leu Val Glu Glu Ala Lys Leu Ser Lys Ile Lys Ser Trp Ala Asn Lys
    50                  55                  60

Met Glu Ala Leu Thr Ser Lys Ser Ala Ala Asp Ala Glu Gly Tyr Leu
65                  70                  75                  80

Ala His Pro Val Asn Ala Tyr Lys Leu Val Lys Arg Leu Asn Thr Asp
                85                  90                  95

Trp Pro Ala Leu Glu Asp Leu Val Leu Gln Asp Ser Ala Ala Gly Phe
            100                 105                 110

Ile Ala Asn Leu Ser Val Gln Arg Gln Phe Phe Pro Thr Asp Glu Asp
        115                 120                 125

Glu Ile Gly Ala Ala Lys Ala Leu Met Arg Leu Gln Asp Thr Tyr Arg
    130                 135                 140

Leu Asp Pro Gly Thr Ile Ser Arg Gly Glu Leu Pro Gly Thr Lys Tyr
145                 150                 155                 160

Gln Ala Met Leu Ser Val Asp Asp Cys Phe Gly Met Gly Arg Ser Ala
                165                 170                 175

Tyr Asn Glu Gly Asp Tyr Tyr His Thr Val Leu Trp Met Glu Gln Val
            180                 185                 190

Leu Lys Gln Leu Asp Ala Gly Glu Glu Ala Thr Thr Thr Lys Ser Gln
        195                 200                 205

Val Leu Asp Tyr Leu Ser Tyr Ala Val Phe Gln Leu Gly Asp Leu His
    210                 215                 220

Arg Ala Leu Glu Leu Thr Arg Arg Leu Leu Ser Leu Asp Pro Ser His
225                 230                 235                 240

Glu Arg Ala Gly Gly Asn Leu Arg Tyr Phe Glu Gln Leu Leu Glu Glu
                245                 250                 255

Glu Arg Glu Lys Thr Leu Thr Asn Gln Thr Glu Ala Glu Leu Ala Thr
            260                 265                 270

Pro Glu Gly Ile Tyr Glu Arg Pro Val Asp Tyr Leu Pro Glu Arg Asp
        275                 280                 285

Val Tyr Glu Ser Leu Cys Arg Gly Glu Gly Val Lys Leu Thr Pro Arg
    290                 295                 300

Arg Gln Lys Arg Leu Phe Cys Arg Tyr His His Gly Asn Arg Ala Pro
305                 310                 315                 320

Gln Leu Leu Ile Ala Pro Phe Lys Glu Glu Asp Glu Trp Asp Ser Pro
                325                 330                 335

His Ile Val Arg Tyr Tyr Asp Val Met Ser Asp Glu Glu Ile Glu Arg
            340                 345                 350

Ile Lys Glu Ile Ala Lys Pro Lys Leu Ala Arg Ala Thr Val Arg Asp
        355                 360                 365

Pro Lys Thr Gly Val Leu Thr Val Ala Ser Tyr Arg Val Ser Lys Ser
    370                 375                 380

Ser Trp Leu Glu Glu Asp Asp Asp Pro Val Val Ala Arg Val Asn Arg
385                 390                 395                 400

Arg Met Gln His Ile Thr Gly Leu Thr Val Lys Thr Ala Glu Leu Leu
                405                 410                 415

Gln Val Ala Asn Tyr Gly Val Gly Gly Gln Tyr Glu Pro His Phe Asp

420                 425                 430

Phe Ser Arg Arg Pro Phe Asp Ser Gly Leu Lys Thr Glu Gly Asn Arg
        435                 440                 445

Leu Ala Thr Phe Leu Asn Tyr Met Ser Asp Val Glu Ala Gly Gly Ala
    450                 455                 460

Thr Val Phe Pro Asp Leu Gly Ala Ala Ile Trp Pro Lys Lys Gly Thr
465                 470                 475                 480

Ala Val Phe Trp Tyr Asn Leu Leu Arg Ser Gly Glu Gly Asp Tyr Arg
                485                 490                 495

Thr Arg His Ala Ala Cys Pro Val Leu Val Gly Cys Lys Trp Val Ser
            500                 505                 510

Asn Lys Trp Phe His Glu Arg Gly Gln Glu Phe Leu Arg Pro Cys Gly
        515                 520                 525

Ser Thr Glu Val Asp
    530

<210>  82
<211>  544
<212>  PRT
<213>  Homo sapiens

<400>  82

Met Gly Pro Gly Ala Arg Leu Ala Ala Leu Leu Ala Val Leu Ala Leu
1               5                   10                  15

Gly Thr Gly Asp Pro Glu Arg Ala Ala Ala Arg Gly Asp Thr Phe Ser
            20                  25                  30

Ala Leu Thr Ser Val Ala Arg Ala Leu Ala Pro Glu Arg Arg Leu Leu
        35                  40                  45

Gly Leu Leu Arg Arg Tyr Leu Arg Gly Glu Glu Ala Arg Leu Arg Asp
    50                  55                  60

Leu Thr Arg Phe Tyr Asp Lys Val Leu Ser Leu His Glu Asp Ser Thr
65                  70                  75                  80

Thr Pro Val Ala Asn Pro Leu Leu Ala Phe Thr Leu Ile Lys Arg Leu
                85                  90                  95

Gln Ser Asp Trp Arg Asn Val Val His Ser Leu Glu Ala Ser Glu Asn
            100                 105                 110

Ile Arg Ala Leu Lys Asp Gly Tyr Glu Lys Val Glu Gln Asp Leu Pro
        115                 120                 125

Ala Phe Glu Asp Leu Glu Gly Ala Ala Arg Ala Leu Met Arg Leu Gln
    130                 135                 140

Asp Val Tyr Met Leu Asn Val Lys Gly Leu Ala Arg Gly Val Phe Gln
145                 150                 155                 160

Arg Val Thr Gly Ser Ala Ile Thr Asp Leu Tyr Ser Pro Lys Arg Leu
                165                 170                 175

Phe Ser Leu Thr Gly Asp Asp Cys Phe Gln Val Gly Lys Val Ala Tyr
            180                 185                 190

Asp Met Gly Asp Tyr Tyr His Ala Ile Pro Trp Leu Glu Glu Ala Val
        195                 200                 205

Ser Leu Phe Arg Gly Ser Tyr Gly Glu Trp Lys Thr Glu Asp Glu Ala
    210                 215                 220

Ser Leu Glu Asp Ala Leu Asp His Leu Ala Phe Ala Tyr Phe Arg Ala
225                 230                 235                 240

Gly Asn Val Ser Cys Ala Leu Ser Leu Ser Arg Glu Phe Leu Leu Tyr
                245                 250                 255

Ser Pro Asp Asn Lys Arg Met Ala Arg Asn Val Leu Lys Tyr Glu Arg
            260                 265                 270

Leu Leu Ala Glu Ser Pro Asn His Val Val Ala Glu Ala Val Ile Gln
        275                 280                 285

Arg Pro Asn Ile Pro His Leu Gln Thr Arg Asp Thr Tyr Glu Gly Leu
    290                 295                 300

Cys Gln Thr Leu Gly Ser Gln Pro Thr Leu Tyr Gln Ile Pro Ser Leu
305                 310                 315                 320

Tyr Cys Ser Tyr Glu Thr Asn Ser Asn Ala Tyr Leu Leu Leu Gln Pro
                325                 330                 335

Ile Arg Lys Glu Val Ile His Leu Glu Pro Tyr Ile Ala Leu Tyr His
            340                 345                 350

Asp Phe Val Ser Asp Ser Glu Ala Gln Lys Ile Arg Glu Leu Ala Glu
        355                 360                 365

Pro Trp Leu Gln Arg Ser Val Val Ala Ser Gly Glu Lys Gln Leu Gln
    370                 375                 380

Val Glu Tyr Arg Ile Ser Lys Ser Ala Trp Leu Lys Asp Thr Val Asp
385                 390                 395                 400

Pro Lys Leu Val Thr Leu Asn His Arg Ile Ala Ala Leu Thr Gly Leu
                405                 410                 415

Asp Val Arg Pro Pro Tyr Ala Glu Tyr Leu Gln Val Val Asn Tyr Gly
            420                 425                 430

Ile Gly Gly His Tyr Glu Pro His Phe Asp His Ala Thr Ser Pro Ser
        435                 440                 445

Ser Pro Leu Tyr Arg Met Lys Ser Gly Asn Arg Val Ala Thr Phe Met
    450                 455                 460

Ile Tyr Leu Ser Ser Val Glu Ala Gly Gly Ala Thr Ala Phe Ile Tyr
465                 470                 475                 480

Ala Asn Leu Ser Val Pro Val Val Arg Asn Ala Ala Leu Phe Trp Trp
                485                 490                 495

Asn Leu His Arg Ser Gly Glu Gly Asp Ser Asp Thr Leu His Ala Gly
            500                 505                 510

Cys Pro Val Leu Val Gly Asp Lys Trp Val Ala Asn Lys Trp Ile His
        515                 520                 525

Glu Tyr Gly Gln Glu Phe Arg Arg Pro Cys Ser Ser Ser Pro Glu Asp
    530                 535                 540

<210>  83
<211>  508
<212>  PRT
<213>  Homo sapiens

<400>  83

<210>  84
<211>  1605
<212>  DNA
<213>  Homo sapiens

<400>  84
atgatctggt atatattaat tataggaatt ctgcttcccc agtctttggc tcatccaggc      60

ttttttactt caattggtca gatgactgat ttgatccata ctgagaaaga tctggtgact     120

tctctgaaag attatattaa ggcagaagag gacaagttag aacaaataaa aaaatgggca     180

gagaagttag atcggctaac tagtacagcg acaaaagatc cagaaggatt tgttgggcat     240

ccagtaaatg cattcaaatt aatgaaacgt ctgaatactg agtggagtga gttggagaat     300

ctggtcctta aggatatgtc agatggcttt atctctaacc taaccattca gagacagtac 360

tttcctaatg atgaagatca ggttggggca gccaaagctc tgttacgtct ccaggatacc 420

tacaatttgg atacagatac catctcaaag ggtaatcttc caggagtgaa acacaaatct 480

tttctaacgg ctgaggactg ctttgagttg ggcaaagtgg cctatacaga agcagattat 540

taccatacgg aactgtggat ggaacaagcc ctaaggcaac tggatgaagg cgagatttct 600

accatagata aagtctctgt tctagattat ttgagctatg cggtatatca gcagggagac 660

ctggataagg cacttttgct cacaaagaag cttcttgaac tagatcctga acatcagaga 720

gctaatggta acttaaaata ttttgagtat ataatggcta aagaaaaaga tgtcaataag 780

tctgcttcag atgaccaatc tgatcagaaa actacaccaa agaaaaaagg ggttgctgtg 840

gattacctgc cagagagaca gaagtacgaa atgctgtgcc gtggggaggg tatcaaaatg 900

acccctcgga gacagaaaaa actcttttgc cgctaccatg atggaaaccg taatcctaaa 960

tttattctgg ctccagctaa acaggaggat gaatgggaca agcctcgtat tattcgcttc 1020

catgatatta tttctgatgc agaaattgaa atcgtcaaag acctagcaaa accaaggctg 1080

aggcgagcca ccatttcaaa cccaataaca ggagacttgg agacggtaca ttacagaatt 1140

agcaaaagtg cctggctctc tggctatgaa aatcctgtgg tgtctcgaat taatatgaga 1200

atacaagatc taacaggact agatgtttcc acagcagagg aattacaggt agcaaattat 1260

ggagttggag gacagtatga accccatttt gactttgcac ggaaagatga gccagatgct 1320

ttcaaagagc tggggacagg aaatagaatt gctacatggc tgttttatat gagtgatgtg 1380

tctgcaggag gagccactgt ttttcctgaa gttggagcta gtgtttggcc caaaaaagga 1440

actgctgttt tctggtataa tctgtttgcc agtggagaag gagattatag tacacggcat 1500

gcagcctgtc cagtgctagt tggcaacaaa tgggtatcca ataaatggct ccatgaacgt 1560

ggacaagaat ttcgaagacc ttgtacgttg tcagaattgg aatga 1605

<210> 85
<211> 1602
<212> DNA
<213> Homo sapiens

<400> 85
   atgaaactct gggtgtctgc attgctgatg gcctggtttg gtgtcctgag ctgtgtgcag 60
   gccgaattct tcacctctat tgggcacatg actgacctga tttatgcaga gaaagagctg 120
   gtgcagtctc tgaaagagta catccttgtg gaggaagcca agctttccaa gattaagagc 180
   tgggccaaca aaatggaagc cttgactagc aagtcagctg ctgatgctga gggctacctg 240
   gctcaccctg tgaatgccta caaactggtg aagcggctaa acacagactg gcctgcgctg 300
   gaggaccttg tcctgcagga ctcagctgca ggttttatcg ccaacctctc tgtgcagcgg 360
   cagttcttcc ccactgatga ggacgagata ggagctgcca aagccctgat gagacttcag 420
   gacacataca ggctggaccc aggcacaatt tccagagggg aacttccagg aaccaagtac 480
   caggcaatgc tgagtgtgga tgactgcttt gggatgggcc gctcggccta caatgaaggg 540
   gactattatc atacggtgtt gtggatggag caggtgctaa agcagcttga tgccggggag 600
   gaggccacca caaccaagtc acaggtgctg gactacctca gctatgctgt cttccagttg 660
   ggtgatctgc accgtgccct ggagctcacc cgccgcctgc tctcccttga cccaagccac 720
   gaacgagctg gagggaatct gcggtacttt gagcagttat tggaggaaga gagagaaaaa 780
   acgttaacaa atcagacaga agctgagcta gcaaccccag aaggcatcta tgagaggcct 840
   gtggactacc tgcctgagag ggatgtttac gagagcctct gtcgtgggga gggtgtcaaa 900
   ctgacacccc gtagacagaa gaggcttttc tgtaggtacc accatggcaa cagggcccca 960
   cagctgctca ttgccccctt caaagaggag gacgagtggg acagcccgca catcgtcagg 1020
   tactacgatg tcatgtctga tgaggaaatc gagaggatca aggagatcgc aaaacctaaa 1080
   cttgcacgag ccaccgttcg tgatcccaag acaggagtcc tcactgtcgc cagctaccgg 1140
   gtttccaaaa gctcctggct agaggaagat gatgaccctg ttgtggcccg agtaaatcgt 1200
   cggatgcagc atatcacagg gttaacagta aagactgcag aattgttaca ggttgcaaat 1260
   tatggagtgg gaggacagta tgaaccgcac ttcgacttct ctaggcgacc ttttgacagc 1320
   ggcctcaaaa cagaggggaa taggttagcg acgtttctta actacatgag tgatgtagaa 1380
   gctggtggtg ccaccgtctt ccctgatctg ggggctgcaa tttggcctaa gaagggtaca 1440
   gctgtgttct ggtacaacct cttgcggagc ggggaaggtg actaccgaac aagacatgct 1500
   gcctgccctg tgcttgtggg ctgcaagtgg gtctccaata agtggttcca tgaacgagga 1560
   caggagttct tgagaccttg tggatcaaca gaagttgact ga 1602

<210> 86
<211> 1635
<212> DNA
<213> Homo sapiens

<400> 86
atgggtcctg gggcgcggct ggcggcgctg ctggcggtgc tggcgctcgg gacaggagac 60

ccagaaaggg ctgcggctcg gggcgacacg ttctcggcgc tgaccagcgt ggcgcgcgcc 120

ctggcgcccg agcgccggct gctggggctg ctgaggcggt acctgcgcgg ggaggaggcg 180

cggctgcggg acctgactag attctacgac aaggtacttt ctttgcatga ggattcaaca 240

acccctgtgg ctaaccctct gcttgcattt actctcatca aacgcctgca gtctgactgg 300

aggaatgtgg tacatagtct ggaggccagt gagaacatcc gagctctgaa ggatggctat 360

gagaaggtgg agcaagacct tccagccttt gaggaccttg agggagcagc aagggccctg 420

atgcggctgc aggacgtgta catgctcaat gtgaaaggcc tggcccgagg tgtctttcag 480

agagtcactg gctctgccat cactgacctg tacagcccca aacggctctt ttctctcaca 540

ggggatgact gcttccaagt tggcaaggtg gcctatgaca tgggggatta ttaccatgcc 600

attccatggc tggaggaggc tgtcagtctc ttccgaggat cttacggaga gtggaagaca 660

gaggatgagg caagtctaga agatgccttg gatcacttgg cctttgctta tttccgggca 720

ggaaatgttt cgtgtgccct cagcctctct cgggagtttc ttctctacag cccagataat 780

aagaggatgg ccaggaatgt cttgaaatat gaaaggctct tggcagagag ccccaaccac 840

gtggtagctg aggctgtcat ccagaggccc aatatacccc acctgcagac cagagacacc 900

tacgaggggc tatgtcagac cctgggttcc cagcccactc tctaccagat ccctagcctc 960

tactgttcct atgagaccaa ttccaacgcc tacctgctgc tccagcccat ccggaaggag 1020

gtcatccacc tggagcccta cattgctctc taccatgact tcgtcagtga ctcagaggct 1080

cagaaaatta gagaacttgc agaaccatgg ctacagaggt cagtggtggc atcaggggag 1140

aagcagttac aagtggagta ccgcatcagc aaaagtgcct ggctgaagga cactgttgac 1200

ccaaaactgg tgaccctcaa ccaccgcatt gctgccctca caggccttga tgtccggcct 1260

ccctatgcag agtatctgca ggtggtgaac tatggcatcg gaggacacta tgagcctcac 1320

tttgaccatg ctacgtcacc aagcagcccc ctctacagaa tgaagtcagg aaaccgagtt 1380

gcaacattta tgatctatct gagctcggtg gaagctggag gagccacagc cttcatctat 1440

gccaacctca gcgtgcctgt ggttaggaat gcagcactgt tttggtggaa cctgcacagg 1500

agtggtgaag ggacagtgac cacacttcat gctggctgtc ctgtcctggt gggagataag 1560

tgggtggcca acaagtggat acatgagtat ggacaggaat tccgcagacc ctgcagctcc 1620

agccctgaag actga 1635

<210> 87
<211> 1527
<212> DNA
<213> Homo sapiens

<400> 87
atgctgcgcc gcgctctgct gtgcctggcc gtggccgccc tggtgcgcgc cgacgccccc 60

gaggaggagg accacgtcct ggtgctgcgg aaaagcaact tcgcggaggc gctggcggcc 120

cacaagtacc tgctggtgga gttctatgcc ccttggtgtg gccactgcaa ggctctggcc 180

cctgagtatg ccaaagccgc tgggaagctg aaggcagaag gttccgagat caggttggcc 240

aaggtggacg ccacggagga gtctgacctg gcccagcagt acggcgtgcg cggctatccc 300

accatcaagt tcttcaggaa tggagacacg gcttccccca aggaatatac agctggcaga 360

gaggctgatg acatcgtgaa ctggctgaag aagcgcacgg gcccggctgc caccaccctg 420

cctgacggcg cagctgcaga gtccttggtg gagtccagcg aggtggctgt catcggcttc 480

ttcaaggacg tggagtcgga ctctgccaag cagtttttgc aggcagcaga ggccatcgat 540

gacataccat ttgggatcac ttccaacagt gacgtgttct ccaaatacca gctcgacaaa 600

gatggggttg tcctctttaa gaagtttgat gaaggccgga acaactttga aggggaggtc 660

accaaggaga acctgctgga ctttatcaaa cacaaccagc tgccccttgt catcgagttc 720

accgagcaga cagccccgaa gatttttgga ggtgaaatca agactcacat cctgctgttc 780

ttgcccaaga gtgtgtctga ctatgacggc aaactgagca acttcaaaac agcagccgag 840

agcttcaagg gcaagatcct gttcatcttc atcgacagcg accacaccga caaccagcgc 900

atcctcgagt tctttggcct gaagaaggaa gagtgcccgg ccgtgcgcct catcaccctg 960

gaggaggaga tgaccaagta caagcccgaa tcggaggagc tgacggcaga gaggatcaca 1020

gagttctgcc accgcttcct ggagggcaaa atcaagcccc acctgatgag ccaggagctg 1080

ccggaggact gggacaagca gcctgtcaag gtgcttgttg ggaagaactt tgaagacgtg 1140

gcttttgatg agaaaaaaaa cgtctttgtg gagttctatg ccccatggtg tggtcactgc 1200

aaacagttgg ctcccatttg ggataaactg ggagagacgt acaaggacca tgagaacatc 1260

gtcatcgcca agatggactc gactgccaac gaggtggagg ccgtcaaagt gcacagcttc 1320

cccacactca agttctttcc tgccagtgcc gacaggacgg tcattgatta caacggggaa 1380

cgcacgctgg atggttttaa gaaattcctg gagagcggtg gccaggatgg ggcaggggat 1440

gatgacgatc tcgaggacct ggaagaagca gaggagccag acatggagga agacgatgat 1500

cagaaagctg tgaaagatga actgtaa 1527

<210> 88
<211> 1464
<212> PRT
<213> Homo sapiens

<400> 88

<210> 89
<211> 1366
<212> PRT
<213> Homo sapiens

<400> 89

<210> 90
<211> 1487
<212> PRT
<213> Homo sapiens

<400> 90

<210>  91
<211>  1466
<212>  PRT
<213>  Homo sapiens

<400>  91

Met Met Ser Phe Val Gln Lys Gly Ser Trp Leu Leu Leu Ala Leu Leu
1               5                   10                  15

His Pro Thr Ile Ile Leu Ala Gln Gln Glu Ala Val Glu Gly Gly Cys

20                  25                  30

Ser His Leu Gly Gln Ser Tyr Ala Asp Arg Asp Val Trp Lys Pro Glu
        35                  40                  45

Pro Cys Gln Ile Cys Val Cys Asp Ser Gly Ser Val Leu Cys Asp Asp
    50                  55                  60

Ile Ile Cys Asp Asp Gln Glu Leu Asp Cys Pro Asn Pro Glu Ile Pro
65                  70                  75                  80

Phe Gly Glu Cys Cys Ala Val Cys Pro Gln Pro Pro Thr Ala Pro Thr
                85                  90                  95

Arg Pro Pro Asn Gly Gln Gly Pro Gln Gly Pro Lys Gly Asp Pro Gly
            100                 105                 110

Pro Pro Gly Ile Pro Gly Arg Asn Gly Asp Pro Gly Ile Pro Gly Gln
        115                 120                 125

Pro Gly Ser Pro Gly Ser Pro Gly Pro Pro Gly Ile Cys Glu Ser Cys
    130                 135                 140

Pro Thr Gly Pro Gln Asn Tyr Ser Pro Gln Tyr Asp Ser Tyr Asp Val
145                 150                 155                 160

Lys Ser Gly Val Ala Val Gly Gly Leu Ala Gly Tyr Pro Gly Pro Ala
                165                 170                 175

Gly Pro Pro Gly Pro Pro Gly Pro Pro Gly Thr Ser Gly His Pro Gly
            180                 185                 190

Ser Pro Gly Ser Pro Gly Tyr Gln Gly Pro Pro Gly Glu Pro Gly Gln
        195                 200                 205

Ala Gly Pro Ser Gly Pro Pro Gly Pro Pro Gly Ala Ile Gly Pro Ser
    210                 215                 220

Gly Pro Ala Gly Lys Asp Gly Glu Ser Gly Arg Pro Gly Arg Pro Gly
225                 230                 235                 240

Glu Arg Gly Leu Pro Gly Pro Pro Gly Ile Lys Gly Pro Ala Gly Ile
                245                 250                 255

Pro Gly Phe Pro Gly Met Lys Gly His Arg Gly Phe Asp Gly Arg Asn
            260                 265                 270

Gly Glu Lys Gly Glu Thr Gly Ala Pro Gly Leu Lys Gly Glu Asn Gly
        275                 280                 285

Leu Pro Gly Glu Asn Gly Ala Pro Gly Pro Met Gly Pro Arg Gly Ala
    290                 295                 300

Pro Gly Glu Arg Gly Arg Pro Gly Leu Pro Gly Ala Ala Gly Ala Arg
305                 310                 315                 320

Gly Asn Asp Gly Ala Arg Gly Ser Asp Gly Gln Pro Gly Pro Pro Gly
                325                 330                 335

Pro Pro Gly Thr Ala Gly Phe Pro Gly Ser Pro Gly Ala Lys Gly Glu
            340                 345                 350

Val Gly Pro Ala Gly Ser Pro Gly Ser Asn Gly Ala Pro Gly Gln Arg
        355                 360                 365

Gly Glu Pro Gly Pro Gln Gly His Ala Gly Ala Gln Gly Pro Pro Gly
    370                 375                 380

Pro Pro Gly Ile Asn Gly Ser Pro Gly Gly Lys Gly Glu Met Gly Pro
385                 390                 395                 400

Ala Gly Ile Pro Gly Ala Pro Gly Leu Met Gly Ala Arg Gly Pro Pro
                405                 410                 415

Gly Pro Ala Gly Ala Asn Gly Ala Pro Gly Leu Arg Gly Gly Ala Gly
            420                 425                 430

Glu Pro Gly Lys Asn Gly Ala Lys Gly Glu Pro Gly Pro Arg Gly Glu
        435                 440                 445

Arg Gly Glu Ala Gly Ile Pro Gly Val Pro Gly Ala Lys Gly Glu Asp
    450                 455                 460

Gly Lys Asp Gly Ser Pro Gly Glu Pro Gly Ala Asn Gly Leu Pro Gly
465                 470                 475                 480

Ala Ala Gly Glu Arg Gly Ala Pro Gly Phe Arg Gly Pro Ala Gly Pro
                485                 490                 495

Asn Gly Ile Pro Gly Glu Lys Gly Pro Ala Gly Glu Arg Gly Ala Pro
            500                 505                 510

Gly Pro Ala Gly Pro Arg Gly Ala Ala Gly Glu Pro Gly Arg Asp Gly
        515                 520                 525

Val Pro Gly Gly Pro Gly Met Arg Gly Met Pro Gly Ser Pro Gly Gly
    530                 535                 540

Pro Gly Ser Asp Gly Lys Pro Gly Pro Pro Gly Ser Gln Gly Glu Ser
545                 550                 555                 560

Gly Arg Pro Gly Pro Pro Gly Pro Ser Gly Pro Arg Gly Gln Pro Gly
                565                 570                 575

Val Met Gly Phe Pro Gly Pro Lys Gly Asn Asp Gly Ala Pro Gly Lys
            580                 585                 590

Asn Gly Glu Arg Gly Gly Pro Gly Gly Pro Gly Pro Gln Gly Pro Pro
        595                 600                 605

Gly Lys Asn Gly Glu Thr Gly Pro Gln Gly Pro Pro Gly Pro Thr Gly
    610                 615                 620

Pro Gly Gly Asp Lys Gly Asp Thr Gly Pro Pro Gly Pro Gln Gly Leu
625                 630                 635                 640

Gln Gly Leu Pro Gly Thr Gly Gly Pro Pro Gly Glu Asn Gly Lys Pro
                645                 650                 655

Gly Glu Pro Gly Pro Lys Gly Asp Ala Gly Ala Pro Gly Ala Pro Gly
            660                 665                 670

Gly Lys Gly Asp Ala Gly Ala Pro Gly Glu Arg Gly Pro Pro Gly Leu
        675                 680                 685

Ala Gly Ala Pro Gly Leu Arg Gly Gly Ala Gly Pro Pro Gly Pro Glu
    690                 695                 700

Gly Gly Lys Gly Ala Ala Gly Pro Pro Gly Pro Pro Gly Ala Ala Gly
705                 710                 715                 720

Thr Pro Gly Leu Gln Gly Met Pro Gly Glu Arg Gly Gly Leu Gly Ser
                725                 730                 735

Pro Gly Pro Lys Gly Asp Lys Gly Glu Pro Gly Gly Pro Gly Ala Asp
            740                 745                 750

Gly Val Pro Gly Lys Asp Gly Pro Arg Gly Pro Thr Gly Pro Ile Gly
        755                 760                 765

Pro Pro Gly Pro Ala Gly Gln Pro Gly Asp Lys Gly Glu Gly Gly Ala
    770                 775                 780

Pro Gly Leu Pro Gly Ile Ala Gly Pro Arg Gly Ser Pro Gly Glu Arg
785                 790                 795                 800

Gly Glu Thr Gly Pro Pro Gly Pro Ala Gly Phe Pro Gly Ala Pro Gly
                805                 810                 815

Gln Asn Gly Glu Pro Gly Gly Lys Gly Glu Arg Gly Ala Pro Gly Glu
            820                 825                 830

Lys Gly Glu Gly Gly Pro Pro Gly Val Ala Gly Pro Pro Gly Gly Ser
        835                 840                 845

Gly Pro Ala Gly Pro Pro Gly Pro Gln Gly Val Lys Gly Glu Arg Gly
    850                 855                 860

Ser Pro Gly Gly Pro Gly Ala Ala Gly Phe Pro Gly Ala Arg Gly Leu
865                 870                 875                 880

Pro Gly Pro Pro Gly Ser Asn Gly Asn Pro Gly Pro Pro Gly Pro Ser
                885                 890                 895

Gly Ser Pro Gly Lys Asp Gly Pro Pro Gly Pro Ala Gly Asn Thr Gly
            900                 905                 910

Ala Pro Gly Ser Pro Gly Val Ser Gly Pro Lys Gly Asp Ala Gly Gln
        915                 920                 925

Pro Gly Glu Lys Gly Ser Pro Gly Ala Gln Gly Pro Pro Gly Ala Pro
    930                 935                 940

Gly Pro Leu Gly Ile Ala Gly Ile Thr Gly Ala Arg Gly Leu Ala Gly
945                 950                 955                 960

Pro Pro Gly Met Pro Gly Pro Arg Gly Ser Pro Gly Pro Gln Gly Val
                965                 970                 975

Lys Gly Glu Ser Gly Lys Pro Gly Ala Asn Gly Leu Ser Gly Glu Arg
            980                 985                 990

Gly Pro Pro Gly Pro Gln Gly Leu  Pro Gly Leu Ala Gly  Thr Ala Gly
        995                 1000                 1005

Glu Pro  Gly Arg Asp Gly Asn  Pro Gly Ser Asp Gly  Leu Pro Gly
    1010                 1015                 1020

Arg Asp  Gly Ser Pro Gly Gly  Lys Gly Asp Arg Gly  Glu Asn Gly
    1025                 1030                 1035

Ser Pro  Gly Ala Pro Gly Ala  Pro Gly His Pro Gly  Pro Pro Gly
    1040                 1045                 1050

Pro Val  Gly Pro Ala Gly Lys  Ser Gly Asp Arg Gly  Glu Ser Gly

1055                1060                1065

Pro Ala Gly Pro Ala Gly Ala Pro Gly Pro Ala Gly Ser Arg Gly
    1070                1075                1080

Ala Pro Gly Pro Gln Gly Pro Arg Gly Asp Lys Gly Glu Thr Gly
    1085                1090                1095

Glu Arg Gly Ala Ala Gly Ile Lys Gly His Arg Gly Phe Pro Gly
    1100                1105                1110

Asn Pro Gly Ala Pro Gly Ser Pro Gly Pro Ala Gly Gln Gln Gly
    1115                1120                1125

Ala Ile Gly Ser Pro Gly Pro Ala Gly Pro Arg Gly Pro Val Gly
    1130                1135                1140

Pro Ser Gly Pro Pro Gly Lys Asp Gly Thr Ser Gly His Pro Gly
    1145                1150                1155

Pro Ile Gly Pro Pro Gly Pro Arg Gly Asn Arg Gly Glu Arg Gly
    1160                1165                1170

Ser Glu Gly Ser Pro Gly His Pro Gly Gln Pro Gly Pro Pro Gly
    1175                1180                1185

Pro Pro Gly Ala Pro Gly Pro Cys Cys Gly Gly Val Gly Ala Ala
    1190                1195                1200

Ala Ile Ala Gly Ile Gly Gly Glu Lys Ala Gly Gly Phe Ala Pro
    1205                1210                1215

Tyr Tyr Gly Asp Glu Pro Met Asp Phe Lys Ile Asn Thr Asp Glu
    1220                1225                1230

Ile Met Thr Ser Leu Lys Ser Val Asn Gly Gln Ile Glu Ser Leu
    1235                1240                1245

Ile Ser Pro Asp Gly Ser Arg Lys Asn Pro Ala Arg Asn Cys Arg
    1250                1255                1260

Asp Leu Lys Phe Cys His Pro Glu Leu Lys Ser Gly Glu Tyr Trp
    1265                1270                1275

Val Asp Pro Asn Gln Gly Cys Lys Leu Asp Ala Ile Lys Val Phe
    1280                1285                1290

Cys Asn Met Glu Thr Gly Glu Thr Cys Ile Ser Ala Asn Pro Leu
    1295                1300                1305

Asn Val Pro Arg Lys His Trp Trp Thr Asp Ser Ser Ala Glu Lys
    1310                1315                1320

Lys His Val Trp Phe Gly Glu Ser Met Asp Gly Gly Phe Gln Phe
    1325                1330                1335

Ser Tyr Gly Asn Pro Glu Leu Pro Glu Asp Val Leu Asp Val Gln
    1340                1345                1350

Leu Ala Phe Leu Arg Leu Leu Ser Ser Arg Ala Ser Gln Asn Ile
    1355                1360                1365

Thr Tyr His Cys Lys Asn Ser Ile Ala Tyr Met Asp Gln Ala Ser
    1370                1375                1380

Gly Asn Val Lys Lys Ala Leu Lys Leu Met Gly Ser Asn Glu Gly
    1385                1390                1395

Glu Phe Lys Ala Glu Gly Asn Ser Lys Phe Thr Tyr Thr Val Leu
    1400                1405                1410

Glu Asp Gly Cys Thr Lys His Thr Gly Glu Trp Ser Lys Thr Val
    1415                1420                1425

Phe Glu Tyr Arg Thr Arg Lys Ala Val Arg Leu Pro Ile Val Asp
    1430                1435                1440

Ile Ala Pro Tyr Asp Ile Gly Gly Pro Asp Gln Glu Phe Gly Val
    1445                1450                1455

Asp Val Gly Pro Val Cys Phe Leu
    1460                1465

<210>  92
<211>  525
<212>  PRT
<213>  Homo sapiens

<400>  92

Met Phe Ser Phe Val Asp Leu Arg Leu Leu Leu Leu Leu Ala Ala Thr
1               5                   10                  15

Ala Leu Leu Thr His Gly Gln Glu Glu Gly Gln Val Glu Gly Gln Asp
            20                  25                  30

Glu Asp Ile Pro Pro Ile Thr Cys Val Gln Asn Gly Leu Arg Tyr His
        35                  40                  45

Asp Arg Asp Val Trp Lys Pro Glu Pro Cys Arg Ile Cys Val Cys Asp
    50                  55                  60

Asn Gly Lys Val Leu Cys Asp Asp Val Ile Cys Asp Glu Thr Lys Asn
65                  70                  75                  80

Cys Pro Gly Ala Glu Val Pro Glu Gly Glu Cys Cys Pro Val Cys Pro
                85                  90                  95

Asp Gly Ser Glu Ser Pro Thr Asp Gln Glu Thr Thr Gly Val Glu Gly
            100                 105                 110

Pro Lys Gly Asp Thr Gly Pro Arg Gly Pro Arg Gly Pro Ala Gly Pro
        115                 120                 125

Pro Gly Arg Asp Gly Ile Pro Gly Gln Pro Gly Leu Pro Gly Pro Pro
    130                 135                 140

Gly Pro Pro Gly Pro Pro Gly Pro Pro Gly Leu Gly Gly Asn Phe Ala
145                 150                 155                 160

Pro Gln Leu Ser Tyr Gly Tyr Asp Glu Lys Ser Thr Gly Gly Ile Ser
                165                 170                 175

Val Pro Gly Pro Met Gly Pro Ser Gly Pro Arg Gly Leu Pro Gly Pro
            180                 185                 190

Pro Gly Ala Pro Gly Pro Gln Gly Phe Gln Gly Pro Pro Gly Glu Pro
        195                 200                 205

Gly Glu Pro Gly Ala Ser Gly Pro Met Gly Pro Arg Gly Pro Pro Gly
    210                 215                 220

Pro Pro Gly Lys Asn Gly Asp Asp Gly Glu Ala Gly Lys Pro Gly Pro
225                 230                 235                 240

Pro Gly Pro Pro Gly Pro Pro Gly Pro Pro Gly Pro Pro Ser Ala Gly
                245                 250                 255

Phe Asp Phe Ser Phe Leu Pro Gln Pro Pro Gln Glu Lys Ala His Asp
            260                 265                 270

Gly Gly Arg Tyr Tyr Arg Ala Asp Asp Ala Asn Val Val Arg Asp Arg
        275                 280                 285

Asp Leu Glu Val Asp Thr Thr Leu Lys Ser Leu Ser Gln Gln Ile Glu
    290                 295                 300

Asn Ile Arg Ser Pro Glu Gly Ser Arg Lys Asn Pro Ala Arg Thr Cys

305                 310                 315                 320

Arg Asp Leu Lys Met Cys His Ser Asp Trp Lys Ser Gly Glu Tyr Trp
                325                 330                 335

Ile Asp Pro Asn Gln Gly Cys Asn Leu Asp Ala Ile Lys Val Phe Cys
            340                 345                 350

Asn Met Glu Thr Gly Glu Thr Cys Val Tyr Pro Thr Gln Pro Ser Val
        355                 360                 365

Ala Gln Lys Asn Trp Tyr Ile Ser Lys Asn Pro Lys Asp Lys Arg His
    370                 375                 380

Val Trp Phe Gly Glu Ser Met Thr Asp Gly Phe Gln Phe Glu Tyr Gly
385                 390                 395                 400

Gly Gln Gly Ser Asp Pro Ala Asp Val Ala Ile Gln Leu Thr Phe Leu
                405                 410                 415

Arg Leu Met Ser Thr Glu Ala Ser Gln Asn Ile Thr Tyr His Cys Lys
            420                 425                 430

Asn Ser Val Ala Tyr Met Asp Gln Gln Thr Gly Asn Leu Lys Lys Ala
        435                 440                 445

Leu Leu Leu Gln Gly Ser Asn Glu Ile Glu Ile Arg Ala Glu Gly Asn
    450                 455                 460

Ser Arg Phe Thr Tyr Ser Val Thr Val Asp Gly Cys Thr Ser His Thr
465                 470                 475                 480

Gly Ala Trp Gly Lys Thr Val Ile Glu Tyr Lys Thr Thr Lys Thr Ser
                485                 490                 495

Arg Leu Pro Ile Ile Asp Val Ala Pro Leu Asp Val Gly Ala Pro Asp
            500                 505                 510

Gln Glu Phe Gly Phe Asp Val Gly Pro Val Cys Phe Leu
        515                 520                 525

<210>  93
<211>  427
<212>  PRT
<213>  Homo sapiens

<400>  93

Met Leu Ser Phe Val Asp Thr Arg Thr Leu Leu Leu Leu Ala Val Thr
1               5                   10                  15

Leu Cys Leu Ala Thr Cys Gln Ser Leu Gln Glu Glu Thr Val Arg Lys
            20                  25                  30

Gly Pro Ala Gly Asp Arg Gly Pro Arg Gly Glu Arg Gly Pro Pro Gly
        35                  40                  45

Pro Pro Gly Arg Asp Gly Glu Asp Gly Pro Thr Gly Pro Pro Gly Pro
    50                  55                  60

Pro Gly Pro Pro Gly Pro Pro Gly Leu Gly Gly Asn Phe Ala Ala Gln
65                  70                  75                  80

Tyr Asp Gly Lys Gly Val Gly Leu Gly Pro Gly Pro Met Gly Leu Met
                85                  90                  95

Gly Pro Arg Gly Pro Pro Gly Ala Ala Gly Ala Pro Gly Pro Gln Gly
            100                 105                 110

Phe Gln Gly Pro Ala Gly Glu Pro Gly Glu Pro Gly Gln Thr Gly Pro
        115                 120                 125

Ala Gly Ala Arg Gly Pro Ala Gly Pro Pro Gly Lys Ala Gly Glu Asp
    130                 135                 140

Gly His Pro Gly Lys Pro Gly Pro Pro Gly Pro Pro Gly Pro Pro Gly
145                 150                 155                 160

Pro Pro Gly Val Ser Gly Gly Gly Tyr Asp Phe Gly Tyr Asp Gly Asp
                165                 170                 175

Phe Tyr Arg Ala Asp Gln Pro Arg Ser Ala Pro Ser Leu Arg Pro Lys
            180                 185                 190

Asp Tyr Glu Val Asp Ala Thr Leu Lys Ser Leu Asn Asn Gln Ile Glu
        195                 200                 205

Thr Leu Leu Thr Pro Glu Gly Ser Arg Lys Asn Pro Ala Arg Thr Cys
    210                 215                 220

Arg Asp Leu Arg Leu Ser His Pro Glu Trp Ser Ser Gly Tyr Tyr Trp
225                 230                 235                 240

Ile Asp Pro Asn Gln Gly Cys Thr Met Asp Ala Ile Lys Val Tyr Cys
                245                 250                 255

Asp Phe Ser Thr Gly Glu Thr Cys Ile Arg Ala Gln Pro Glu Asn Ile
            260                 265                 270

Pro Ala Lys Asn Trp Tyr Arg Ser Ser Lys Asp Lys Lys His Val Trp
        275                 280                 285

Leu Gly Glu Thr Ile Asn Ala Gly Ser Gln Phe Glu Tyr Asn Val Glu
    290                 295                 300

Gly Val Thr Ser Lys Glu Met Ala Thr Gln Leu Ala Phe Met Arg Leu
305                 310                 315                 320

Leu Ala Asn Tyr Ala Ser Gln Asn Ile Thr Tyr His Cys Lys Asn Ser
                325                 330                 335

Ile Ala Tyr Met Asp Glu Glu Thr Gly Asn Leu Lys Lys Ala Val Ile
            340                 345                 350

Leu Gln Gly Ser Asn Asp Val Glu Leu Val Ala Glu Gly Asn Ser Arg
        355                 360                 365

Phe Thr Tyr Thr Val Leu Val Asp Gly Cys Ser Lys Lys Thr Asn Glu
    370                 375                 380

Trp Gly Lys Thr Ile Ile Glu Tyr Lys Thr Asn Lys Pro Ser Arg Leu
385                 390                 395                 400

Pro Phe Leu Asp Ile Ala Pro Leu Asp Ile Gly Gly Ala Asp Gln Glu
                405                 410                 415

Phe Phe Val Asp Ile Gly Pro Val Cys Phe Lys
            420                 425

<210>  94
<211>  1014
<212>  PRT
<213>  Bos taurus cattle

<400>  94

Met Phe Ser Phe Val Asp Leu Arg Leu Leu Leu Leu Leu Ala Ala Thr
1               5                   10                  15

Ala Leu Leu Thr His Gly Gln Glu Glu Gly Gln Val Glu Gly Gln Asp
            20                  25                  30

Glu Asp Ile Pro Pro Ile Thr Cys Val Gln Asn Gly Leu Arg Tyr His
        35                  40                  45

Asp Arg Asp Val Trp Lys Pro Glu Pro Cys Arg Ile Cys Val Cys Asp
    50                  55                  60

Asn Gly Lys Val Leu Cys Asp Asp Val Ile Cys Asp Glu Thr Lys Asn
65                  70                  75                  80

Cys Pro Gly Ala Glu Val Pro Glu Gly Glu Cys Cys Pro Val Cys Pro
                85                  90                  95

Asp Gly Ser Glu Ser Pro Thr Asp Gln Glu Thr Thr Gly Val Glu Gly
            100                 105                 110

Pro Lys Gly Asp Thr Gly Pro Arg Gly Pro Arg Gly Pro Ala Gly Pro
        115                 120                 125

Pro Gly Arg Asp Gly Ile Pro Gly Gln Pro Gly Leu Pro Gly Pro Pro
    130                 135                 140

Gly Pro Pro Gly Pro Pro Gly Pro Pro Gly Leu Gly Gly Asn Phe Ala
145                 150                 155                 160

Pro Gln Leu Ser Tyr Gly Tyr Asp Glu Lys Ser Thr Gly Gly Ile Ser
                165                 170                 175

Val Pro Gly Pro Met Gly Pro Ser Gly Pro Arg Gly Leu Pro Gly Pro
            180                 185                 190

Pro Gly Ala Pro Gly Pro Gln Gly Phe Gln Gly Pro Pro Gly Pro Gln
        195                 200                 205

Gly Pro Gly Gly Pro Pro Gly Pro Lys Gly Asn Ser Gly Glu Pro Gly
    210                 215                 220

Ala Pro Gly Ser Lys Gly Asp Thr Gly Ala Lys Gly Glu Pro Gly Pro
225                 230                 235                 240

Val Gly Val Gln Gly Pro Pro Gly Pro Ala Gly Glu Glu Gly Lys Arg
                245                 250                 255

Gly Ala Arg Gly Glu Pro Gly Pro Thr Gly Leu Pro Gly Pro Pro Gly
            260                 265                 270

Glu Arg Gly Gly Pro Gly Ser Arg Gly Phe Pro Gly Ala Asp Gly Val
        275                 280                 285

Ala Gly Pro Lys Gly Pro Ala Gly Glu Arg Gly Ser Pro Gly Pro Ala
    290                 295                 300

Gly Pro Lys Gly Ser Pro Gly Glu Ala Gly Arg Pro Gly Glu Ala Gly
305                 310                 315                 320

Leu Pro Gly Ala Lys Gly Leu Thr Gly Ser Pro Gly Ser Pro Gly Pro
                325                 330                 335

Asp Gly Lys Thr Gly Pro Pro Gly Pro Ala Gly Gln Asp Gly Arg Pro
            340                 345                 350

Gly Pro Pro Gly Pro Pro Gly Ala Arg Gly Gln Ala Gly Val Met Gly
        355                 360                 365

Phe Pro Gly Pro Lys Gly Ala Ala Gly Glu Pro Gly Lys Ala Gly Glu
    370                 375                 380

Arg Gly Val Pro Gly Pro Pro Gly Ala Val Gly Pro Ala Gly Lys Asp
385                 390                 395                 400

Gly Glu Ala Gly Ala Gln Gly Pro Pro Gly Pro Ala Gly Pro Ala Gly
                405                 410                 415

Glu Arg Gly Glu Gln Gly Pro Ala Gly Ser Pro Gly Phe Gln Gly Leu
            420                 425                 430

Pro Gly Pro Ala Gly Pro Pro Gly Glu Ala Gly Lys Pro Gly Glu Gln
        435                 440                 445

Gly Val Pro Gly Asp Leu Gly Ala Pro Gly Pro Ser Gly Ala Arg Gly
    450                 455                 460

Glu Arg Gly Phe Pro Gly Glu Arg Gly Val Gln Gly Pro Pro Gly Pro
465                 470                 475                 480

Ala Gly Pro Arg Gly Ala Asn Gly Ala Pro Gly Asn Asp Gly Ala Lys
                485                 490                 495

Gly Asp Ala Gly Ala Pro Gly Ala Pro Gly Ser Gln Gly Ala Pro Gly
            500                 505                 510

Leu Gln Gly Met Pro Gly Glu Arg Gly Ala Ala Gly Leu Pro Gly Pro
        515                 520                 525

Lys Gly Asp Arg Gly Asp Ala Gly Pro Lys Gly Ala Asp Gly Ser Pro
    530                 535                 540

Gly Lys Asp Gly Val Arg Gly Leu Thr Gly Pro Ile Gly Pro Pro Gly
545                 550                 555                 560

Pro Ala Gly Ala Pro Gly Asp Lys Gly Glu Ser Gly Pro Ser Gly Pro
                565                 570                 575

Ala Gly Pro Thr Gly Ala Arg Gly Ala Pro Gly Asp Arg Gly Glu Pro
            580                 585                 590

Gly Pro Pro Gly Pro Ala Gly Phe Ala Gly Pro Pro Gly Ala Asp Gly
        595                 600                 605

Gln Pro Gly Ala Lys Gly Glu Pro Gly Asp Ala Gly Ala Lys Gly Asp
    610                 615                 620

Ala Gly Pro Pro Gly Pro Ala Gly Pro Ala Gly Pro Pro Gly Pro Ile
625                 630                 635                 640

Gly Asn Val Gly Ala Pro Gly Ala Lys Gly Ala Arg Gly Ser Ala Gly
                645                 650                 655

Pro Pro Gly Ala Thr Gly Phe Pro Gly Ala Ala Gly Arg Val Gly Pro
            660                 665                 670

Pro Gly Pro Ser Gly Asn Ala Gly Pro Pro Gly Pro Pro Gly Pro Ala
        675                 680                 685

Gly Lys Glu Gly Gly Lys Gly Pro Arg Gly Glu Thr Gly Pro Ala Gly
    690                 695                 700

Arg Pro Gly Glu Val Gly Pro Pro Gly Pro Pro Gly Pro Ala Gly Glu
705                 710                 715                 720

Lys Gly Ser Pro Gly Ala Asp Gly Pro Pro Gly Pro Pro Gly Pro Pro
                725                 730                 735

Gly Pro Pro Gly Pro Pro Ser Ala Gly Phe Asp Phe Ser Phe Leu Pro
            740                 745                 750

Gln Pro Pro Gln Glu Lys Ala His Asp Gly Gly Arg Tyr Tyr Arg Ala
        755                 760                 765

Asp Asp Ala Asn Val Val Arg Asp Arg Asp Leu Glu Val Asp Thr Thr
    770                 775                 780

Leu Lys Ser Leu Ser Gln Gln Ile Glu Asn Ile Arg Ser Pro Glu Gly
785                 790                 795                 800

Ser Arg Lys Asn Pro Ala Arg Thr Cys Arg Asp Leu Lys Met Cys His
                805                 810                 815

Ser Asp Trp Lys Ser Gly Glu Tyr Trp Ile Asp Pro Asn Gln Gly Cys
            820                 825                 830

Asn Leu Asp Ala Ile Lys Val Phe Cys Asn Met Glu Thr Gly Glu Thr
        835                 840                 845

Cys Val Tyr Pro Thr Gln Pro Ser Val Ala Gln Lys Asn Trp Tyr Ile

850                 855                 860

Ser Lys Asn Pro Lys Asp Lys Arg His Val Trp Phe Gly Glu Ser Met
865                 870                 875                 880

Thr Asp Gly Phe Gln Phe Glu Tyr Gly Gly Gln Gly Ser Asp Pro Ala
                885                 890                 895

Asp Val Ala Ile Gln Leu Thr Phe Leu Arg Leu Met Ser Thr Glu Ala
            900                 905                 910

Ser Gln Asn Ile Thr Tyr His Cys Lys Asn Ser Val Ala Tyr Met Asp
        915                 920                 925

Gln Gln Thr Gly Asn Leu Lys Lys Ala Leu Leu Leu Gln Gly Ser Asn
    930                 935                 940

Glu Ile Glu Ile Arg Ala Glu Gly Asn Ser Arg Phe Thr Tyr Ser Val
945                 950                 955                 960

Thr Val Asp Gly Cys Thr Ser His Thr Gly Ala Trp Gly Lys Thr Val
                965                 970                 975

Ile Glu Tyr Lys Thr Thr Lys Thr Ser Arg Leu Pro Ile Ile Asp Val
            980                 985                 990

Ala Pro Leu Asp Val Gly Ala Pro  Asp Gln Glu Phe Gly  Phe Asp Val
        995                 1000                1005

Gly Pro  Val Cys Phe Leu
    1010

<210>  95
<211>  916
<212>  PRT
<213>  Homo sapiens

<400>  95

Met Leu Ser Phe Val Asp Thr Arg Thr Leu Leu Leu Leu Ala Val Thr
1               5                   10                  15

Leu Cys Leu Ala Thr Cys Gln Ser Leu Gln Glu Glu Thr Val Arg Lys
            20                  25                  30

Gly Pro Ala Gly Asp Arg Gly Pro Arg Gly Glu Arg Gly Pro Pro Gly
        35                  40                  45

Pro Pro Gly Arg Asp Gly Glu Asp Gly Pro Thr Gly Pro Pro Gly Pro
    50                  55                  60

Pro Gly Pro Pro Gly Pro Pro Gly Leu Gly Gly Asn Phe Ala Ala Gln
65                  70                  75                  80

Tyr Asp Gly Lys Gly Val Gly Leu Gly Pro Gly Pro Met Gly Leu Met
                85                  90                  95

Gly Pro Arg Gly Pro Pro Gly Ala Ala Gly Ala Pro Gly Pro Gln Gly
            100                 105                 110

Phe Gln Gly Pro Ala Gly Pro Val Gly Ala Ala Gly Ala Thr Gly Ala
        115                 120                 125

Arg Gly Leu Val Gly Glu Pro Gly Pro Ala Gly Ser Lys Gly Glu Ser
    130                 135                 140

Gly Asn Lys Gly Glu Pro Gly Ser Ala Gly Pro Gln Gly Pro Pro Gly
145                 150                 155                 160

Pro Ser Gly Glu Glu Gly Lys Arg Gly Pro Asn Gly Glu Ala Gly Ser
                165                 170                 175

Ala Gly Pro Pro Gly Pro Pro Gly Leu Arg Gly Ser Pro Gly Ser Arg
            180                 185                 190

Gly Leu Pro Gly Ala Asp Gly Arg Ala Gly Val Met Gly Pro Pro Gly
        195                 200                 205

Ser Arg Gly Ala Ser Gly Pro Ala Gly Val Arg Gly Pro Asn Gly Asp
    210                 215                 220

Ala Gly Arg Pro Gly Glu Pro Gly Leu Met Gly Pro Arg Gly Leu Pro
225                 230                 235                 240

Gly Ser Pro Gly Asn Ile Gly Pro Ala Gly Lys Glu Gly Pro Val Gly
                245                 250                 255

Leu Pro Gly Ile Asp Gly Arg Pro Gly Pro Ile Gly Pro Ala Gly Ala
            260                 265                 270

Arg Gly Glu Pro Gly Asn Ile Gly Phe Pro Gly Pro Lys Gly Pro Thr
        275                 280                 285

Gly Asp Pro Gly Lys Asn Gly Asp Lys Gly His Ala Gly Leu Ala Gly
    290                 295                 300

Ala Arg Gly Ala Pro Gly Pro Asp Gly Asn Asn Gly Ala Gln Gly Pro
305                 310                 315                 320

Pro Gly Pro Gln Gly Val Gln Gly Gly Lys Gly Glu Gln Gly Pro Ala
                325                 330                 335

Gly Pro Pro Gly Phe Gln Gly Leu Pro Gly Pro Ser Gly Pro Ala Gly
            340                 345                 350

Glu Val Gly Lys Pro Gly Glu Arg Gly Leu His Gly Glu Phe Gly Leu
        355                 360                 365

Pro Gly Pro Ala Gly Pro Arg Gly Glu Arg Gly Pro Pro Gly Glu Ser
    370                 375                 380

Gly Ala Ala Gly Pro Thr Gly Pro Ile Gly Ser Arg Gly Pro Ser Gly
385                 390                 395                 400

Pro Pro Gly Pro Asp Gly Asn Lys Gly Glu Pro Gly Val Val Gly Ala
                405                 410                 415

Val Gly Thr Ala Gly Pro Ser Gly Pro Ser Gly Leu Pro Gly Glu Arg
            420                 425                 430

Gly Ala Ala Gly Ile Pro Gly Gly Lys Gly Glu Lys Gly Glu Pro Gly
        435                 440                 445

Leu Arg Gly Glu Ile Gly Asn Pro Gly Arg Asp Gly Ala Arg Gly Ala
    450                 455                 460

Pro Gly Ala Val Gly Ala Pro Gly Pro Ala Gly Ala Thr Gly Asp Arg
465                 470                 475                 480

Gly Glu Ala Gly Ala Ala Gly Pro Ala Gly Pro Ala Gly Pro Arg Gly
                485                 490                 495

Ser Pro Gly Glu Arg Gly Glu Val Gly Pro Ala Gly Pro Asn Gly Phe
            500                 505                 510

Ala Gly Pro Ala Gly Ala Ala Gly Gln Pro Gly Ala Lys Gly Glu Arg
        515                 520                 525

Gly Ala Lys Gly Pro Lys Gly Glu Asn Gly Val Val Gly Pro Thr Gly
    530                 535                 540

Pro Val Gly Ala Ala Gly Pro Ala Gly Pro Asn Gly Pro Pro Gly Pro
545                 550                 555                 560

Ala Gly Ser Arg Gly Asp Gly Gly Pro Pro Gly Met Thr Gly Phe Pro
                565                 570                 575

Gly Ala Ala Gly Arg Thr Gly Pro Pro Gly Pro Ser Gly Ile Ser Gly

580                 585                 590

Pro Pro Gly Pro Pro Gly Pro Ala Gly Lys Glu Gly Leu Arg Gly Pro
        595                 600                 605

Arg Gly Asp Gln Gly Pro Val Gly Arg Thr Gly Glu Val Gly Ala Val
    610                 615                 620

Gly Pro Pro Gly Phe Ala Gly Glu Lys Gly Pro Ser Gly Glu Ala Gly
625                 630                 635                 640

Pro Pro Gly Pro Pro Gly Pro Pro Gly Pro Pro Gly Val Ser Gly Gly
                645                 650                 655

Gly Tyr Asp Phe Gly Tyr Asp Gly Asp Phe Tyr Arg Ala Asp Gln Pro
            660                 665                 670

Arg Ser Ala Pro Ser Leu Arg Pro Lys Asp Tyr Glu Val Asp Ala Thr
        675                 680                 685

Leu Lys Ser Leu Asn Asn Gln Ile Glu Thr Leu Leu Thr Pro Glu Gly
    690                 695                 700

Ser Arg Lys Asn Pro Ala Arg Thr Cys Arg Asp Leu Arg Leu Ser His
705                 710                 715                 720

Pro Glu Trp Ser Ser Gly Tyr Tyr Trp Ile Asp Pro Asn Gln Gly Cys
                725                 730                 735

Thr Met Asp Ala Ile Lys Val Tyr Cys Asp Phe Ser Thr Gly Glu Thr
            740                 745                 750

Cys Ile Arg Ala Gln Pro Glu Asn Ile Pro Ala Lys Asn Trp Tyr Arg
        755                 760                 765

Ser Ser Lys Asp Lys Lys His Val Trp Leu Gly Glu Thr Ile Asn Ala
    770                 775                 780

Gly Ser Gln Phe Glu Tyr Asn Val Glu Gly Val Thr Ser Lys Glu Met
785                 790                 795                 800

Ala Thr Gln Leu Ala Phe Met Arg Leu Leu Ala Asn Tyr Ala Ser Gln
                805                 810                 815

Asn Ile Thr Tyr His Cys Lys Asn Ser Ile Ala Tyr Met Asp Glu Glu
            820                 825                 830

Thr Gly Asn Leu Lys Lys Ala Val Ile Leu Gln Gly Ser Asn Asp Val
        835                 840                 845

Glu Leu Val Ala Glu Gly Asn Ser Arg Phe Thr Tyr Thr Val Leu Val
    850                 855                 860

Asp Gly Cys Ser Lys Lys Thr Asn Glu Trp Gly Lys Thr Ile Ile Glu
865                 870                 875                 880

Tyr Lys Thr Asn Lys Pro Ser Arg Leu Pro Phe Leu Asp Ile Ala Pro
                885                 890                 895

Leu Asp Ile Gly Gly Ala Asp Gln Glu Phe Phe Val Asp Ile Gly Pro
            900                 905                 910

Val Cys Phe Lys
        915

<210>  96
<211>  1536
<212>  PRT
<213>  Homo sapiens

<400>  96

Met Phe Ser Phe Val Asp Leu Arg Leu Leu Leu Leu Leu Ala Ala Thr
1               5                   10                  15

Ala Leu Leu Thr His Gly Gln Glu Glu Gly Gln Val Glu Gly Gln Asp
            20                  25                  30

Glu Asp Ile Pro Pro Ile Thr Cys Val Gln Asn Gly Leu Arg Tyr His
        35                  40                  45

Asp Arg Asp Val Trp Lys Pro Glu Pro Cys Arg Ile Cys Val Cys Asp
    50                  55                  60

Asn Gly Lys Val Leu Cys Asp Asp Val Ile Cys Asp Glu Thr Lys Asn
65                  70                  75                  80

Cys Pro Gly Ala Glu Val Pro Glu Gly Glu Cys Cys Pro Val Cys Pro
                85                  90                  95

Asp Gly Ser Glu Ser Pro Thr Asp Gln Glu Thr Thr Gly Val Glu Gly
            100                 105                 110

Pro Lys Gly Asp Thr Gly Pro Arg Gly Pro Arg Gly Pro Ala Gly Pro
        115                 120                 125

Pro Gly Arg Asp Gly Ile Pro Gly Gln Pro Gly Leu Pro Gly Pro Pro
    130                 135                 140

Gly Pro Pro Gly Pro Pro Gly Pro Pro Gly Leu Gly Gly Asn Phe Ala
145                 150                 155                 160

Pro Gln Leu Ser Tyr Gly Tyr Asp Glu Lys Ser Thr Gly Gly Ile Ser
                165                 170                 175

Val Pro Gly Pro Met Gly Pro Ser Gly Pro Arg Gly Leu Pro Gly Pro
            180                 185                 190

Pro Gly Ala Pro Gly Pro Gln Gly Phe Gln Gly Pro Pro Gly Pro Gln
        195                 200                 205

Gly Pro Gly Gly Pro Pro Gly Pro Lys Gly Asn Ser Gly Glu Pro Gly
    210                 215                 220

Ala Pro Gly Ser Lys Gly Asp Thr Gly Ala Lys Gly Glu Pro Gly Pro
225                 230                 235                 240

Val Gly Val Gln Gly Pro Pro Gly Pro Ala Gly Glu Glu Gly Lys Arg
                245                 250                 255

Gly Ala Arg Gly Glu Pro Gly Pro Thr Gly Leu Pro Gly Pro Pro Gly
            260                 265                 270

Glu Arg Gly Gly Pro Gly Ser Arg Gly Phe Pro Gly Ala Asp Gly Val
        275                 280                 285

Ala Gly Pro Lys Gly Pro Ala Gly Glu Arg Gly Ser Pro Gly Pro Ala
    290                 295                 300

Gly Pro Lys Gly Ser Pro Gly Glu Ala Gly Arg Pro Gly Glu Ala Gly
305                 310                 315                 320

Leu Pro Gly Ala Lys Gly Leu Thr Gly Ser Pro Gly Ser Pro Gly Pro
                325                 330                 335

Asp Gly Lys Thr Gly Pro Pro Gly Pro Ala Gly Gln Asp Gly Arg Pro
            340                 345                 350

Gly Pro Pro Gly Pro Pro Gly Ala Arg Gly Gln Ala Gly Val Met Gly
        355                 360                 365

Phe Pro Gly Pro Lys Gly Ala Ala Gly Glu Pro Gly Lys Ala Gly Glu
    370                 375                 380

Arg Gly Val Pro Gly Pro Pro Gly Ala Val Gly Pro Ala Gly Lys Asp
385                 390                 395                 400

Gly Glu Ala Gly Ala Gln Gly Pro Pro Gly Pro Ala Gly Pro Ala Gly

405                 410                 415

Glu Arg Gly Glu Gln Gly Pro Ala Gly Ser Pro Gly Phe Gln Gly Leu
            420                 425                 430

Pro Gly Pro Ala Gly Pro Pro Gly Glu Ala Gly Lys Pro Gly Glu Gln
        435                 440                 445

Gly Val Pro Gly Asp Leu Gly Ala Pro Gly Pro Ser Gly Ala Arg Gly
    450                 455                 460

Glu Arg Gly Phe Pro Gly Glu Arg Gly Val Gln Gly Pro Pro Gly Pro
465                 470                 475                 480

Ala Gly Pro Arg Gly Ala Asn Gly Ala Pro Gly Asn Asp Gly Ala Lys
                485                 490                 495

Gly Asp Ala Gly Ala Pro Gly Ala Pro Gly Ser Gln Gly Ala Pro Gly
            500                 505                 510

Leu Gln Gly Met Pro Gly Glu Arg Gly Ala Ala Gly Leu Pro Gly Pro
        515                 520                 525

Lys Gly Asp Arg Gly Asp Ala Gly Pro Lys Gly Ala Asp Gly Ser Pro
    530                 535                 540

Gly Lys Asp Gly Val Arg Gly Leu Thr Gly Pro Ile Gly Pro Pro Gly
545                 550                 555                 560

Pro Ala Gly Ala Pro Gly Asp Lys Gly Glu Ser Gly Pro Ser Gly Pro
                565                 570                 575

Ala Gly Pro Thr Gly Ala Arg Gly Ala Pro Gly Asp Arg Gly Glu Pro
            580                 585                 590

Gly Pro Pro Gly Pro Ala Gly Phe Ala Gly Pro Pro Gly Ala Asp Gly
        595                 600                 605

Gln Pro Gly Ala Lys Gly Glu Pro Gly Asp Ala Gly Ala Lys Gly Asp
    610                 615                 620

Ala Gly Pro Pro Gly Pro Ala Gly Pro Ala Gly Pro Pro Gly Pro Ile
625                 630                 635                 640

Gly Asn Val Gly Ala Pro Gly Ala Lys Gly Ala Arg Gly Ser Ala Gly
                645                 650                 655

Pro Pro Gly Ala Thr Gly Phe Pro Gly Ala Ala Gly Arg Val Gly Pro
            660                 665                 670

Pro Gly Pro Ser Gly Asn Ala Gly Pro Pro Gly Pro Pro Gly Pro Ala
        675                 680                 685

Gly Lys Glu Gly Gly Lys Gly Pro Arg Gly Glu Thr Gly Pro Ala Gly
    690                 695                 700

Arg Pro Gly Glu Val Gly Pro Pro Gly Pro Pro Gly Pro Ala Gly Glu
705                 710                 715                 720

Lys Gly Ser Pro Gly Ala Asp Gly Pro Gln Gly Pro Gly Gly Pro Pro
                725                 730                 735

Gly Pro Lys Gly Asn Ser Gly Glu Pro Gly Ala Pro Gly Ser Lys Gly
            740                 745                 750

Asp Thr Gly Ala Lys Gly Glu Pro Gly Pro Val Gly Val Gln Gly Pro
        755                 760                 765

Pro Gly Pro Ala Gly Glu Glu Gly Lys Arg Gly Ala Arg Gly Glu Pro
    770                 775                 780

Gly Pro Thr Gly Leu Pro Gly Pro Pro Gly Glu Arg Gly Gly Pro Gly
785                 790                 795                 800

Ser Arg Gly Phe Pro Gly Ala Asp Gly Val Ala Gly Pro Lys Gly Pro
                805                 810                 815

Ala Gly Glu Arg Gly Ser Pro Gly Pro Ala Gly Pro Lys Gly Ser Pro
            820                 825                 830

Gly Glu Ala Gly Arg Pro Gly Glu Ala Gly Leu Pro Gly Ala Lys Gly
        835                 840                 845

Leu Thr Gly Ser Pro Gly Ser Pro Gly Pro Asp Gly Lys Thr Gly Pro
    850                 855                 860

Pro Gly Pro Ala Gly Gln Asp Gly Arg Pro Gly Pro Pro Gly Pro Pro
865                 870                 875                 880

Gly Ala Arg Gly Gln Ala Gly Val Met Gly Phe Pro Gly Pro Lys Gly
                885                 890                 895

Ala Ala Gly Glu Pro Gly Lys Ala Gly Glu Arg Gly Val Pro Gly Pro
            900                 905                 910

Pro Gly Ala Val Gly Pro Ala Gly Lys Asp Gly Glu Ala Gly Ala Gln
        915                 920                 925

Gly Pro Pro Gly Pro Ala Gly Pro Ala Gly Glu Arg Gly Glu Gln Gly
    930                 935                 940

Pro Ala Gly Ser Pro Gly Phe Gln Gly Leu Pro Gly Pro Ala Gly Pro
945                 950                 955                 960

Pro Gly Glu Ala Gly Lys Pro Gly Glu Gln Gly Val Pro Gly Asp Leu
                965                 970                 975

Gly Ala Pro Gly Pro Ser Gly Ala Arg Gly Glu Arg Gly Phe Pro Gly
            980                 985                 990

Glu Arg Gly Val Gln Gly Pro Pro  Gly Pro Ala Gly Pro  Arg Gly Ala
        995                 1000                 1005

Asn Gly  Ala Pro Gly Asn Asp  Gly Ala Lys Gly Asp  Ala Gly Ala
    1010                 1015                 1020

Pro Gly  Ala Pro Gly Ser Gln  Gly Ala Pro Gly Leu  Gln Gly Met
    1025                 1030                 1035

Pro Gly  Glu Arg Gly Ala Ala  Gly Leu Pro Gly Pro  Lys Gly Asp
    1040                 1045                 1050

Arg Gly  Asp Ala Gly Pro Lys  Gly Ala Asp Gly Ser  Pro Gly Lys
    1055                 1060                 1065

Asp Gly  Val Arg Gly Leu Thr  Gly Pro Ile Gly Pro  Pro Gly Pro
    1070                 1075                 1080

Ala Gly  Ala Pro Gly Asp Lys  Gly Glu Ser Gly Pro  Ser Gly Pro
    1085                 1090                 1095

Ala Gly  Pro Thr Gly Ala Arg  Gly Ala Pro Gly Asp  Arg Gly Glu
    1100                 1105                 1110

Pro Gly  Pro Pro Gly Pro Ala  Gly Phe Ala Gly Pro  Pro Gly Ala
    1115                 1120                 1125

Asp Gly  Gln Pro Gly Ala Lys  Gly Glu Pro Gly Asp  Ala Gly Ala
    1130                 1135                 1140

Lys Gly  Asp Ala Gly Pro Pro  Gly Pro Ala Gly Pro  Ala Gly Pro
    1145                 1150                 1155

Pro Gly  Pro Ile Gly Asn Val  Gly Ala Pro Gly Ala  Lys Gly Ala
    1160                 1165                 1170

Arg Gly Ser Ala Gly Pro Pro Gly Ala Thr Gly Phe Pro Gly Ala
    1175                1180                1185

Ala Gly Arg Val Gly Pro Pro Gly Pro Ser Gly Asn Ala Gly Pro
    1190                1195                1200

Pro Gly Pro Pro Gly Pro Ala Gly Lys Glu Gly Gly Lys Gly Pro
    1205                1210                1215

Arg Gly Glu Thr Gly Pro Ala Gly Arg Pro Gly Glu Val Gly Pro
    1220                1225                1230

Pro Gly Pro Pro Gly Pro Ala Gly Glu Lys Gly Ser Pro Gly Ala
    1235                1240                1245

Asp Gly Pro Pro Gly Pro Pro Gly Pro Pro Gly Pro Pro Gly Pro
    1250                1255                1260

Pro Ser Ala Gly Phe Asp Phe Ser Phe Leu Pro Gln Pro Pro Gln
    1265                1270                1275

Glu Lys Ala His Asp Gly Gly Arg Tyr Tyr Arg Ala Asp Asp Ala
    1280                1285                1290

Asn Val Val Arg Asp Arg Asp Leu Glu Val Asp Thr Thr Leu Lys
    1295                1300                1305

Ser Leu Ser Gln Gln Ile Glu Asn Ile Arg Ser Pro Glu Gly Ser
    1310                1315                1320

Arg Lys Asn Pro Ala Arg Thr Cys Arg Asp Leu Lys Met Cys His
    1325                1330                1335

Ser Asp Trp Lys Ser Gly Glu Tyr Trp Ile Asp Pro Asn Gln Gly
    1340                1345                1350

Cys Asn Leu Asp Ala Ile Lys Val Phe Cys Asn Met Glu Thr Gly
    1355                1360                1365

Glu Thr Cys Val Tyr Pro Thr Gln Pro Ser Val Ala Gln Lys Asn
    1370                1375                1380

Trp Tyr Ile Ser Lys Asn Pro Lys Asp Lys Arg His Val Trp Phe
    1385                1390                1395

Gly Glu Ser Met Thr Asp Gly Phe Gln Phe Glu Tyr Gly Gly Gln
    1400                1405                1410

Gly Ser Asp Pro Ala Asp Val Ala Ile Gln Leu Thr Phe Leu Arg

1415                    1420                    1425

Leu Met  Ser Thr Glu Ala Ser  Gln Asn Ile Thr Tyr  His Cys Lys
    1430                    1435                    1440

Asn Ser  Val Ala Tyr Met Asp  Gln Gln Thr Gly Asn  Leu Lys Lys
    1445                    1450                    1455

Ala Leu  Leu Leu Gln Gly Ser  Asn Glu Ile Glu Ile  Arg Ala Glu
    1460                    1465                    1470

Gly Asn  Ser Arg Phe Thr Tyr  Ser Val Thr Val Asp  Gly Cys Thr
    1475                    1480                    1485

Ser His  Thr Gly Ala Trp Gly  Lys Thr Val Ile Glu  Tyr Lys Thr
    1490                    1495                    1500

Thr Lys  Thr Ser Arg Leu Pro  Ile Ile Asp Val Ala  Pro Leu Asp
    1505                    1510                    1515

Val Gly  Ala Pro Asp Gln Glu  Phe Gly Phe Asp Val  Gly Pro Val
    1520                    1525                    1530

Cys Phe  Leu
    1535

<210>  97
<211>  1438
<212>  PRT
<213>  Homo sapiens

<400>  97

Met Leu Ser Phe Val Asp Thr Arg Thr Leu Leu Leu Leu Ala Val Thr
1               5                   10                  15

Leu Cys Leu Ala Thr Cys Gln Ser Leu Gln Glu Glu Thr Val Arg Lys
            20                  25                  30

Gly Pro Ala Gly Asp Arg Gly Pro Arg Gly Glu Arg Gly Pro Pro Gly
        35                  40                  45

Pro Pro Gly Arg Asp Gly Glu Asp Gly Pro Thr Gly Pro Pro Gly Pro
    50                  55                  60

Pro Gly Pro Pro Gly Pro Pro Gly Leu Gly Gly Asn Phe Ala Ala Gln
65                  70                  75                  80

Tyr Asp Gly Lys Gly Val Gly Leu Gly Pro Gly Pro Met Gly Leu Met
                85                  90                  95

Gly Pro Arg Gly Pro Pro Gly Ala Ala Gly Ala Pro Gly Pro Gln Gly
        100                 105                 110

Phe Gln Gly Pro Ala Gly Pro Val Gly Ala Ala Gly Ala Thr Gly Ala
    115                 120                 125

Arg Gly Leu Val Gly Glu Pro Gly Pro Ala Gly Ser Lys Gly Glu Ser
    130                 135                 140

Gly Asn Lys Gly Glu Pro Gly Ser Ala Gly Pro Gln Gly Pro Pro Gly
145                 150                 155                 160

Pro Ser Gly Glu Glu Gly Lys Arg Gly Pro Asn Gly Glu Ala Gly Ser
                165                 170                 175

Ala Gly Pro Pro Gly Pro Pro Gly Leu Arg Gly Ser Pro Gly Ser Arg
            180                 185                 190

Gly Leu Pro Gly Ala Asp Gly Arg Ala Gly Val Met Gly Pro Pro Gly
        195                 200                 205

Ser Arg Gly Ala Ser Gly Pro Ala Gly Val Arg Gly Pro Asn Gly Asp
    210                 215                 220

Ala Gly Arg Pro Gly Glu Pro Gly Leu Met Gly Pro Arg Gly Leu Pro
225                 230                 235                 240

Gly Ser Pro Gly Asn Ile Gly Pro Ala Gly Lys Glu Gly Pro Val Gly
                245                 250                 255

Leu Pro Gly Ile Asp Gly Arg Pro Gly Pro Ile Gly Pro Ala Gly Ala
            260                 265                 270

Arg Gly Glu Pro Gly Asn Ile Gly Phe Pro Gly Pro Lys Gly Pro Thr
        275                 280                 285

Gly Asp Pro Gly Lys Asn Gly Asp Lys Gly His Ala Gly Leu Ala Gly
    290                 295                 300

Ala Arg Gly Ala Pro Gly Pro Asp Gly Asn Asn Gly Ala Gln Gly Pro
305                 310                 315                 320

Pro Gly Pro Gln Gly Val Gln Gly Gly Lys Gly Glu Gln Gly Pro Ala
                325                 330                 335

Gly Pro Pro Gly Phe Gln Gly Leu Pro Gly Pro Ser Gly Pro Ala Gly
            340                 345                 350

Glu Val Gly Lys Pro Gly Glu Arg Gly Leu His Gly Glu Phe Gly Leu
        355                 360                 365

Pro Gly Pro Ala Gly Pro Arg Gly Glu Arg Gly Pro Pro Gly Glu Ser
    370                 375                 380

Gly Ala Ala Gly Pro Thr Gly Pro Ile Gly Ser Arg Gly Pro Ser Gly
385                 390                 395                 400

Pro Pro Gly Pro Asp Gly Asn Lys Gly Glu Pro Gly Val Val Gly Ala
                405                 410                 415

Val Gly Thr Ala Gly Pro Ser Gly Pro Ser Gly Leu Pro Gly Glu Arg
            420                 425                 430

Gly Ala Ala Gly Ile Pro Gly Gly Lys Gly Glu Lys Gly Glu Pro Gly
        435                 440                 445

Leu Arg Gly Glu Ile Gly Asn Pro Gly Arg Asp Gly Ala Arg Gly Ala
    450                 455                 460

Pro Gly Ala Val Gly Ala Pro Gly Pro Ala Gly Ala Thr Gly Asp Arg
465                 470                 475                 480

Gly Glu Ala Gly Ala Ala Gly Pro Ala Gly Pro Ala Gly Pro Arg Gly
                485                 490                 495

Ser Pro Gly Glu Arg Gly Glu Val Gly Pro Ala Gly Pro Asn Gly Phe
            500                 505                 510

Ala Gly Pro Ala Gly Ala Ala Gly Gln Pro Gly Ala Lys Gly Glu Arg
        515                 520                 525

Gly Ala Lys Gly Pro Lys Gly Glu Asn Gly Val Val Gly Pro Thr Gly
    530                 535                 540

Pro Val Gly Ala Ala Gly Pro Ala Gly Pro Asn Gly Pro Pro Gly Pro
545                 550                 555                 560

Ala Gly Ser Arg Gly Asp Gly Gly Pro Pro Gly Met Thr Gly Phe Pro
                565                 570                 575

Gly Ala Ala Gly Arg Thr Gly Pro Pro Gly Pro Ser Gly Ile Ser Gly
            580                 585                 590

Pro Pro Gly Pro Pro Gly Pro Ala Gly Lys Glu Gly Leu Arg Gly Pro
        595                 600                 605

Arg Gly Asp Gln Gly Pro Val Gly Arg Thr Gly Glu Val Gly Ala Val

610                 615                 620

Gly Pro Pro Gly Phe Ala Gly Glu Lys Gly Pro Ser Gly Glu Ala Gly
625                 630                 635                 640

Pro Val Gly Ala Ala Gly Ala Thr Gly Ala Arg Gly Leu Val Gly Glu
                645                 650                 655

Pro Gly Pro Ala Gly Ser Lys Gly Glu Ser Gly Asn Lys Gly Glu Pro
            660                 665                 670

Gly Ser Ala Gly Pro Gln Gly Pro Pro Gly Pro Ser Gly Glu Glu Gly
        675                 680                 685

Lys Arg Gly Pro Asn Gly Glu Ala Gly Ser Ala Gly Pro Pro Gly Pro
    690                 695                 700

Pro Gly Leu Arg Gly Ser Pro Gly Ser Arg Gly Leu Pro Gly Ala Asp
705                 710                 715                 720

Gly Arg Ala Gly Val Met Gly Pro Pro Gly Ser Arg Gly Ala Ser Gly
                725                 730                 735

Pro Ala Gly Val Arg Gly Pro Asn Gly Asp Ala Gly Arg Pro Gly Glu
            740                 745                 750

Pro Gly Leu Met Gly Pro Arg Gly Leu Pro Gly Ser Pro Gly Asn Ile
        755                 760                 765

Gly Pro Ala Gly Lys Glu Gly Pro Val Gly Leu Pro Gly Ile Asp Gly
    770                 775                 780

Arg Pro Gly Pro Ile Gly Pro Ala Gly Ala Arg Gly Glu Pro Gly Asn
785                 790                 795                 800

Ile Gly Phe Pro Gly Pro Lys Gly Pro Thr Gly Asp Pro Gly Lys Asn
                805                 810                 815

Gly Asp Lys Gly His Ala Gly Leu Ala Gly Ala Arg Gly Ala Pro Gly
            820                 825                 830

Pro Asp Gly Asn Asn Gly Ala Gln Gly Pro Pro Gly Pro Gln Gly Val
        835                 840                 845

Gln Gly Gly Lys Gly Glu Gln Gly Pro Ala Gly Pro Pro Gly Phe Gln
    850                 855                 860

Gly Leu Pro Gly Pro Ser Gly Pro Ala Gly Glu Val Gly Lys Pro Gly
865                 870                 875                 880

Glu Arg Gly Leu His Gly Glu Phe Gly Leu Pro Gly Pro Ala Gly Pro
                885                 890                 895

Arg Gly Glu Arg Gly Pro Pro Gly Glu Ser Gly Ala Ala Gly Pro Thr
            900                 905                 910

Gly Pro Ile Gly Ser Arg Gly Pro Ser Gly Pro Pro Gly Pro Asp Gly
        915                 920                 925

Asn Lys Gly Glu Pro Gly Val Val Gly Ala Val Gly Thr Ala Gly Pro
    930                 935                 940

Ser Gly Pro Ser Gly Leu Pro Gly Glu Arg Gly Ala Ala Gly Ile Pro
945                 950                 955                 960

Gly Gly Lys Gly Glu Lys Gly Glu Pro Gly Leu Arg Gly Glu Ile Gly
                965                 970                 975

Asn Pro Gly Arg Asp Gly Ala Arg Gly Ala Pro Gly Ala Val Gly Ala
            980                 985                 990

Pro Gly Pro Ala Gly Ala Thr Gly  Asp Arg Gly Glu Ala  Gly Ala Ala
        995                 1000                1005

Gly Pro  Ala Gly Pro Ala Gly  Pro Arg Gly Ser Pro  Gly Glu Arg
    1010                1015                1020

Gly Glu  Val Gly Pro Ala Gly  Pro Asn Gly Phe Ala  Gly Pro Ala
    1025                1030                1035

Gly Ala  Ala Gly Gln Pro Gly  Ala Lys Gly Glu Arg  Gly Ala Lys
    1040                1045                1050

Gly Pro  Lys Gly Glu Asn Gly  Val Val Gly Pro Thr  Gly Pro Val
    1055                1060                1065

Gly Ala  Ala Gly Pro Ala Gly  Pro Asn Gly Pro Pro  Gly Pro Ala
    1070                1075                1080

Gly Ser  Arg Gly Asp Gly Gly  Pro Pro Gly Met Thr  Gly Phe Pro
    1085                1090                1095

Gly Ala  Ala Gly Arg Thr Gly  Pro Pro Gly Pro Ser  Gly Ile Ser
    1100                1105                1110

Gly Pro  Pro Gly Pro Pro Gly  Pro Ala Gly Lys Glu  Gly Leu Arg
    1115                1120                1125

Gly Pro Arg Gly Asp Gln Gly Pro Val Gly Arg Thr Gly Glu Val
    1130                1135                1140

Gly Ala Val Gly Pro Pro Gly Phe Ala Gly Glu Lys Gly Pro Ser
    1145                1150                1155

Gly Glu Ala Gly Pro Pro Gly Pro Pro Gly Pro Pro Gly Pro Pro
    1160                1165                1170

Gly Val Ser Gly Gly Gly Tyr Asp Phe Gly Tyr Asp Gly Asp Phe
    1175                1180                1185

Tyr Arg Ala Asp Gln Pro Arg Ser Ala Pro Ser Leu Arg Pro Lys
    1190                1195                1200

Asp Tyr Glu Val Asp Ala Thr Leu Lys Ser Leu Asn Asn Gln Ile
    1205                1210                1215

Glu Thr Leu Leu Thr Pro Glu Gly Ser Arg Lys Asn Pro Ala Arg
    1220                1225                1230

Thr Cys Arg Asp Leu Arg Leu Ser His Pro Glu Trp Ser Ser Gly
    1235                1240                1245

Tyr Tyr Trp Ile Asp Pro Asn Gln Gly Cys Thr Met Asp Ala Ile
    1250                1255                1260

Lys Val Tyr Cys Asp Phe Ser Thr Gly Glu Thr Cys Ile Arg Ala
    1265                1270                1275

Gln Pro Glu Asn Ile Pro Ala Lys Asn Trp Tyr Arg Ser Ser Lys
    1280                1285                1290

Asp Lys Lys His Val Trp Leu Gly Glu Thr Ile Asn Ala Gly Ser
    1295                1300                1305

Gln Phe Glu Tyr Asn Val Glu Gly Val Thr Ser Lys Glu Met Ala
    1310                1315                1320

Thr Gln Leu Ala Phe Met Arg Leu Leu Ala Asn Tyr Ala Ser Gln
    1325                1330                1335

Asn Ile Thr Tyr His Cys Lys Asn Ser Ile Ala Tyr Met Asp Glu
    1340                1345                1350

Glu Thr Gly Asn Leu Lys Lys Ala Val Ile Leu Gln Gly Ser Asn
    1355                1360                1365

Asp Val Glu Leu Val Ala Glu Gly Asn Ser Arg Phe Thr Tyr Thr
    1370                1375                1380

Val Leu Val Asp Gly Cys Ser Lys Lys Thr Asn Glu Trp Gly Lys
    1385                1390                1395

Thr Ile Ile Glu Tyr Lys Thr Asn Lys Pro Ser Arg Leu Pro Phe
    1400                1405                1410

Leu Asp Ile Ala Pro Leu Asp Ile Gly Gly Ala Asp Gln Glu Phe
    1415                1420                1425

Phe Val Asp Ile Gly Pro Val Cys Phe Lys
    1430                1435

<210> 98
<211> 4395
<212> DNA
<213> Artificial Sequence

<220>
<223> synthesized DNA of human collagen ORF having codon optimized for y
east

<400> 98
atgttttctt tcgtagattt gcgactcctc ttgttgcttg ctgctaccgc tctccttact 60

catggacagg aagaaggcca ggtggaagga caggacgaag acattcctcc tataacttgt 120

gtgcagaacg gactgaggta tcatgacaga gatgtgtgga agccagagcc ttgtcgaata 180

tgcgtttgtg acaacggaaa ggtgctatgt gatgatgtta tctgtgatga gacaaagaac 240

tgtcctggcg ctgaagttcc agagggagaa tgttgtccag tttgtcctga tggatcggag 300

tcacccactg atcaagagac tacgggagta gaaggtccaa aaggtgacac tggtcccaga 360

ggcccaagag gacctgccgg cccccctggt agggatggca ttcctggtca acccggccta 420

ccaggccccc caggtccacc tggcccccca ggaccccctg gtttaggagg taacttcgct 480

cctcaactgt cttacggtta cgatgaaaag tctactggtg gaatctctgt tcctggacca 540

atgggtccgt ctggtccaag gggtctacct ggtcctcccg gtgcacctgg tccgcagggt 600

tttcaaggtc caccaggtga accgggagaa ccgggagctt caggtccaat gggtcccagg 660

ggtcctcctg gccctcctgg aaagaacggc gatgatggcg aggctggtaa gccgggaaga 720

cctggtgaaa gaggcccacc tggaccacag ggtgctagag gtcttccagg aaccgcagga 780

ttgcctggta tgaagggaca ccgtggcttc agtggtttgg acggagctaa aggagatgca 840

ggtccggctg gtcctaaagg tgagcccggt tccccaggtg aaaacggtgc tcccggccaa 900

atgggaccac gaggactgcc aggcgagaga ggtagaccag gcgctccagg tccagctgga 960

gctagaggaa atgatggtgc cactggtgca gcaggacctc ccggaccaac tgggcccgct 1020

ggacctcctg gttttccagg agcagttgga gcaaagggag aggctggtcc tcaagggcct 1080

agaggaagcg aaggacctca aggggttagg ggtgaaccag gtcctccagg accggctggc 1140
gctgcaggcc cagctggaaa tcccggagct gacggtcaac caggtgctaa aggagctaac 1200
ggtgctccag ggatcgcagg agctcccggt tttcctggtg ctaggggacc cagtggtcca 1260
cagggtccag gaggtcctcc tggacctaaa ggaaatagtg gagaaccagg agctccaggg 1320
agtaaaggag acactggtgc taagggagag ccaggaccag tgggggttca aggtcctccc 1380
ggacccgctg gagaagaggg aaaaagaggt gcacgtggtg agcctggtcc tactggtctg 1440
ccaggtccac ctggggaaag gggtggtcct gggtcaagag gcttccccgg tgctgacgga 1500
gttgctggtc cgaaaggacc agccggagag cgaggatccc caggtcctgc aggccctaag 1560
gggtcccctg gtgaggcagg aagaccgggt gaggctggtt tgcccggtgc caaaggtctc 1620
acgggctccc caggctctcc tggcccagac ggcaaaactg gcccacctgg ccctgccgga 1680
caagacggaa gacctggacc tccaggacca cctggtgcca gaggtcaggc cggtgtgatg 1740
gggtttccag gtcccaaggg tgctgcaggt gaaccaggga aagccggtga acgaggtgtc 1800
cccggtcctc caggtgctgt tgggccggct ggaaaggatg gtgaggccgg tgcacaaggg 1860
cctccaggcc ctgctggtcc tgccggtgaa agaggagaac agggaccagc cggttctcct 1920
ggctttcaag gtctgcccgg acctgccggg cctccaggag aagctggtaa accaggagaa 1980
caaggtgtcc caggagattt gggagcccct gggccctcag gcgctagagg tgagagggga 2040
tttcctggag aacgtggtgt acaaggtccc ccgggtccag caggtccgag gggagctaat 2100
ggagctccag gaaacgacgg tgctaaaggc gacgcaggtg ctccaggagc accaggatca 2160
cagggtgccc caggtttgca aggcatgcca ggggaaagag gagcagcagg acttccaggc 2220
cccaaaggcg acaggggtga cgccggtcca aaaggggccg atggttctcc tggcaaagac 2280
ggcgttcgag gcttgacagg tccgatcggt ccacctggac ccgccggtgc accaggtgat 2340
aagggagaat ctggtccttc aggccctgcc ggtccaaccg ggctagaggg cgctcctggg 2400
gatagaggtg aacccggtcc accagggcca gccggcttcg ctggaccacc aggtgctgat 2460
ggacagcccg gagctaaggg tgagccaggt gatgctggtg ctaaaggtga cgctggacct 2520
ccgggtcccg caggacctgc tggtccccct gggccaattg gtaatgttgg tgccccagga 2580
gcaaaaggag ccagaggttc tgcaggccca ccaggagcca caggattccc cggtgctgct 2640
ggaagagtcg gaccaccggg tccatccggg aacgccggac ctccaggtcc tccgggtcct 2700
gctggtaaag aaggaggtaa gggtccaaga ggtgaaacag ggcctgctgg ccgtcctggt 2760
gaagtcggac ctcctggccc acccggtcca gctggggaaa agggatcacc aggagctgat 2820
ggtcctgcag gtgctcctgg aacccctggt cctcaaggca tcgctggtca aagaggagtc 2880
gtggggttgc cagggcaacg aggtgagcga ggatttccag gattaccagg tccatctgga 2940
gagcccggta agcagggtcc ctcgggagcc agtggtgagc gtggtccacc cggacctatg 3000

gggcctcctg ggctagctgg gccaccaggt gagtctggtc gtgagggagc acctggagcc 3060

gaaggatcac cagggaggga tggctcacct ggagccaagg gtgatagagg cgaaaccgga 3120

cccgcaggcc ctcctggtgc acctggagca ccaggagccc ccggtccggt aggtccagca 3180

ggcaagtcgg gtgacagagg agaaactggt ccagctggtc cagccggccc agttggacct 3240

gtgggtgccc gtggtccagc aggaccacaa ggtccaagag gggataaggg tgaaactggt 3300

gagcagggag acagggggat taagggacat agaggctttt caggtttaca aggtccgcct 3360

gggcctccag gcagcccagg ggagcagggg ccgagtggtg catccggtcc agccggacca 3420

cgtgggccac ctggttctgc tggtgctcct ggtaaagacg gactaaacgg tttgccggga 3480

ccaataggcc cacccgggcc aagagggcgt acaggtgacg ccggacctgt cggaccacca 3540

gggccgcctg gtcctcctgg tccacctggt ccgcccagtg caggattcga cttttccttc 3600

ttaccccaac cgccacagga aaaggcacac gatggtggta gatattaccg tgctgatgat 3660

gccaatgtcg tgagagatag agacttagaa gttgatacga cattaaaatc actttctcaa 3720

caaattgaga acattagatc tccggagggt agcagaaaga acccggccag gacttgccgt 3780

gatcttaaga tgtgccactc cgattggaag tcgggagagt actggatcga tcctaatcaa 3840

ggttgtaact tggatgcaat aaaagtcttc tgtaacatgg agacaggaga aacttgcgtt 3900

tacccaactc aaccaagtgt tgcacagaaa aattggtata tttccaagaa tccaaaggat 3960

aagcgtcatg tttggtttgg agaatctatg accgatggct ttcaattcga gtatggaggt 4020

cagggtagcg acccagctga tgtcgctatt caattgacct ttttgagact tatgtccaca 4080

gaagcttcgc aaaacattac atatcactgt aagaatagcg tagcctacat ggaccaacag 4140

actggtaatc tgaaaaaggc cctgttgttg caaggcagta atgaaatcga aattcgtgcc 4200

gaaggaaatt ccagattcac atactctgtt accgttgacg gctgcacctc tcataccggt 4260

gcctggggta aaactgtaat tgagtataaa acgacaaaga cctcccgtct tcctatcatt 4320

gatgttgctc cattagatgt cggtgcccct gaccaggagt ttggttttga cgtaggcccc 4380

gtctgcttcc tgtaa 4395

<210> 99
<211> 4395
<212> DNA
<213> Homo sapiens

<400> 99
atgttcagct ttgtggacct ccggctcctg ctcctcttag cggccaccgc cctcctgacg 60

cacggccaag aggaaggcca agtcgagggc caagacgaag acatcccacc aatcacctgc 120

gtacagaacg gcctcaggta ccatgaccga gacgtgtgga aacccgagcc ctgccggatc 180

tgcgtctgcg acaacggcaa ggtgttgtgc gatgacgtga tctgtgacga gaccaagaac 240

tgccccggcg ccgaagtccc cgagggcgag tgctgtcccg tctgccccga cggctcagag 300

tcacccaccg accaagaaac caccggcgtc gagggaccca agggagacac tggcccccga 360
ggcccaaggg gacccgcagg cccccctggc cgagatggca tccctggaca gcctggactt 420
cccggacccc ccggaccccc cggacctccc ggaccccctg gcctcggagg aaactttgct 480
ccccagctgt cttatggcta tgatgagaaa tcaaccggag gaatttccgt gcctggcccc 540
atgggtccct ctggtcctcg tggtctccct ggcccccctg gtgcacctgg tccccaaggc 600
ttccaaggtc cccctggtga gcctggcgag cctggagctt caggtcccat gggtccccga 660
ggtcccccag gtccccctgg aaagaatgga gatgatgggg aagctggaaa acctggtcgt 720
cctggtgagc gtgggcctcc tgggcctcag ggtgctcgag gattgcccgg aacagctggc 780
ctccctggaa tgaagggaca cagaggtttc agtggtttgg atggtgccaa gggagatgct 840
ggtcctgctg gtcctaaggg tgagcctggc agccctggtg aaaatggagc tcctggtcag 900
atgggccccc gtggcctgcc tggtgagaga ggtcgccctg gagcccctgg ccctgctggt 960
gctcgtggaa atgatggtgc tactggtgct gccgggcccc ctggtcccac cggccccgct 1020
ggtcctcctg gcttccctgg tgctgttggt gctaagggtg aagctggtcc ccaagggccc 1080
cgaggctctg aaggtcccca gggtgtgcgt ggtgagcctg gcccccctgg ccctgctggt 1140
gctgctggcc ctgctggaaa ccctggtgct gatggacagc ctggtgctaa aggtgccaat 1200
ggtgctcctg gtattgctgg tgctcctggc ttccctggtg cccgaggccc ctctggaccc 1260
cagggccccg gcggccctcc tggtcccaag ggtaacagcg gtgaacctgg tgctcctggc 1320
agcaaaggag acactggtgc taagggagag cctggccctg ttggtgttca aggaccccct 1380
ggccctgctg gagaggaagg aaagcgagga gctcgaggtg aacccggacc cactggcctg 1440
cccggacccc ctggcgagcg tggtggacct ggtagccgtg gtttccctgg cgcagatggt 1500
gttgctggtc ccaagggtcc cgctggtgaa cgtggttctc ctggccctgc tggccccaaa 1560
ggatctcctg gtgaagctgg tcgtcccggt gaagctggtc tgcctggtgc caagggtctg 1620
actggaagcc ctggcagccc tggtcctgat ggcaaaactg gcccccctgg tcccgccggt 1680
caagatggtc gccccggacc cccaggccca cctggtgccc gtggtcaggc tggtgtgatg 1740
ggattccctg gacctaaagg tgctgctgga gagcccggca aggctggaga gcgaggtgtt 1800
cccggacccc ctggcgctgt cggtcctgct ggcaaagatg gagaggctgg agctcaggga 1860
ccccctggcc ctgctggtcc cgctggcgag agaggtgaac aaggccctgc tggctccccc 1920
ggattccagg gtctccctgg tcctgctggt cctccaggtg aagcaggcaa acctggtgaa 1980
cagggtgttc ctggagacct tggcgcccct ggcccctctg gagcaagagg cgagagaggt 2040
ttccctggcg agcgtggtgt gcaaggtccc cctggtcctg ctggtccccg aggggccaac 2100
ggtgctcccg gcaacgatgg tgctaagggt gatgctggtg cccctggagc tcccggtagc 2160
cagggcgccc ctggccttca gggaatgcct ggtgaacgtg gtgcagctgg tcttccaggg 2220
cctaagggtg acagaggtga tgctggtccc aaaggtgctg atggctctcc tggcaaagat 2280

ggcgtccgtg gtctgaccgg ccccattggt cctcctggcc ctgctggtgc ccctggtgac 2340

aagggtgaaa gtggtcccag cggccctgct ggtcccactg gagctcgtgg tgcccccgga 2400

gaccgtggtg agcctggtcc ccccggccct gctggctttg ctggcccccc tggtgctgac 2460

ggccaacctg gtgctaaagg cgaacctggt gatgctggtg ctaaaggcga tgctggtccc 2520

cctggccctg ccggacccgc tggaccccct ggccccattg gtaatgttgg tgctcctgga 2580

gccaaaggtg ctcgcggcag cgctggtccc cctggtgcta ctggtttccc tggtgctgct 2640

ggccgagtcg gtcctcctgg cccctctgga aatgctggac cccctggccc tcctggtcct 2700

gctggcaaag aaggcggcaa aggtccccgt ggtgagactg gccctgctgg acgtcctggt 2760

gaagttggtc cccctggtcc ccctggccct gctggcgaga aaggatcccc tggtgctgat 2820

ggtcctgctg gtgctcctgg tactcccggg cctcaaggta ttgctggaca gcgtggtgtg 2880

gtcggcctgc ctggtcagag aggagagaga ggcttccctg gtcttcctgg cccctctggt 2940

gaacctggca aacaaggtcc ctctggagca agtggtgaac gtggtccccc tggtcccatg 3000

ggcccccctg gattggctgg accccctggt gaatctggac gtgagggggc tcctggtgcc 3060

gaaggttccc ctggacgaga cggttctcct ggcgccaagg gtgaccgtgg tgagaccggc 3120

cccgctggac cccctggtgc tcctggtgct cctggtgccc ctggccccgt tggccctgct 3180

ggcaagagtg gtgatcgtgg tgagactggt cctgctggtc ccgccggtcc tgtcggccct 3240

gttggcgccc gtggccccgc cggaccccaa ggcccccgtg gtgacaaggg tgagacaggc 3300

gaacagggcg acagaggcat aaagggtcac cgtggcttct ctggcctcca gggtccccct 3360

ggccctcctg gctctcctgg tgaacaaggt ccctctggag cctctggtcc tgctggtccc 3420

cgaggtcccc ctggctctgc tggtgctcct ggcaaagatg gactcaacgg tctccctggc 3480

cccattgggc cccctggtcc tcgcggtcgc actggtgatg ctggtcctgt tggtcccccc 3540

ggccctcctg gacctcctgg tccccctggt cctcccagcg ctggtttcga cttcagcttc 3600

ctgccccagc cacctcaaga gaaggctcac gatggtggcc gctactaccg ggctgatgat 3660

gccaatgtgg ttcgtgaccg tgacctcgag gtggacacca ccctcaagag cctgagccag 3720

cagatcgaga acatccggag cccagagggc agccgcaaga accccgcccg cacctgccgt 3780

gacctcaaga tgtgccactc tgactggaag agtggagagt actggattga ccccaaccaa 3840

ggctgcaacc tggatgccat caaagtcttc tgcaacatgg agactggtga gacctgcgtg 3900

taccccactc agcccagtgt ggcccagaag aactggtaca tcagcaagaa ccccaaggac 3960

aagaggcatg tctggttcgg cgagagcatg accgatggat tccagttcga gtatggcggc 4020

cagggctccg accctgccga tgtggccatc cagctgacct tcctgcgcct gatgtccacc 4080

gaggcctccc agaacatcac ctaccactgc aagaacagcg tggcctacat ggaccagcag 4140

actggcaacc tcaagaaggc cctgctcctc cagggctcca acgagatcga gatccgcgcc 4200

gagggcaaca gccgcttcac ctacagcgtc actgtcgatg gctgcacgag tcacaccgga 4260

gcctggggca agacagtgat tgaatacaaa accaccaaga cctcccgcct gcccatcatc 4320

gatgtggccc ccttggacgt tggtgcccca gaccaggaat tcggcttcga cgttggccct 4380

gtctgcttcc tgtaa 4395

<210> 100
<211> 4101
<212> DNA
<213> Artificial Sequence

<220>
<223> synthesized DNA of human collagen type I alpha 2 precursor ORF hav
ing codon optimized for yeast

<400> 100
atgctctctt tcgttgatac tagaactcta ttgctgttag cagttacttt gtgtttagca 60

acctgtcaat ccttgcaaga agaaaccgtt agaaaaggtc ctgcaggaga tagaggtcca 120

agaggcgaga gagggccccc gggaccccca gggcgagatg gtgaggatgg tccgacaggt 180

ccaccaggac ctcctggacc tcccggacct ccgggactcg gtggtaactt cgctgctcaa 240

tatgatggta aaggcgttgg tcttggaccg ggaccaatgg gtctgatggg tcctagagga 300

ccaccagggg cagcaggggc acctggtccc caaggattcc aaggacccgc aggggaaccc 360

ggggagccag gacagacagg acctgcaggg gctagaggac ccgccggtcc accaggtaag 420

gctggtgagg acggccaccc aggtaaaccc ggtcgtcccg gtgaaagggg agtagttggt 480

ccacagggtg caagaggttt tccaggaacc ccaggattgc ctggatttaa gggaatacgt 540

ggtcataacg gtcttgatgg ccttaaaggt caacctggag ccccaggtgt aaaaggagaa 600

cccggcgctc ccggggaaaa cggaacacca ggtcagaccg gagcccgagg tttgccagga 660

gaacgaggac gagttggtgc tcccggtcct gcaggtgcta gaggctccga tggatcggtt 720

ggtccagtgg gaccagctgg cccaataggt agtgcaggtc ctccaggttt ccctggagct 780

cctggaccta agggagaaat aggggctgtg ggcaatgcag gacccgctgg gcctgctgga 840

ccccgtggcg aagttggttt acccggattg agtggtcctg ttggtcctcc aggtaaccct 900

ggcgctaatg gtctaactgg tgccaagggt gctgcaggac ttccaggtgt agccggagct 960

cctgggttgc caggtcctcg tggtatccca gggccagttg gcgcagctgg tgctacggga 1020

gctaggggtc tagtgggcga accaggtcca gcagggtcta agggtgaatc cggtaacaag 1080

ggtgaacctg gaagtgccgg tcctcaggga cctcctggtc catcagggga ggaaggaaag 1140

agaggcccta atggcgaggc tggtagtgca ggaccaccgg gtcctcctgg attgagaggt 1200

tcccccgggt ctcgtggact acctggtgcc gacggtagag ctggcgttat gggtcctccc 1260

ggaagcagag gagcttctgg acccgcaggt gtcaggggtc caaacggtga tgctggaagg 1320

ccaggtgaac caggattgat gggtccacgt ggattgcccg gctctcctgg aaacattgga 1380

cctgccggta aagaaggccc tgtgggtttg ccaggtattg atggcagacc aggtccaatc 1440
ggcccagcag gcgctagagg agagcctggt aacataggat tccctggtcc taagggacca 1500
actggtgatc caggcaagaa cggtgataag ggtcatgccg ggctagctgg ggctagaggt 1560
gcacctggcc cagatgggaa caacggagcc caagggccac cgggtccaca gggggtacaa 1620
ggtggcaagg gcgaacaggg tccagctggc ccgcctggtt ttcagggact tccgggtcct 1680
agcggtcccg ctggagaagt tggaaagcct ggtgaacgtg gactacatgg agaattcgga 1740
ctgcctggtc ctgctggtcc acgtggagaa agaggtcctc ccggggagag tggagctgct 1800
ggtcctaccg gaccaattgg gtcacgtgga ccctccggtc ctccaggacc agatgggaat 1860
aagggtgaac caggagtcgt aggggcagtt ggtacagctg gcccttccgg tccttcgggc 1920
cttccaggag aaagaggagc cgccggaatc cctggtggta aaggcgaaaa aggagaacct 1980
ggtttaaggg gtgagattgg aaatccaggt agagatggtg cccgaggagc cccaggtgcc 2040
gtcggtgctc ccgggccagc cggagccact ggcgataggg gagaagccgg agctgctgga 2100
ccagccggtc cagctggacc aagaggatcc ccgggtgaga gaggagaagt tgggccagcc 2160
ggtccgaacg gtttcgccgg accagccggt gcagccggtc aaccaggtgc taagggcgag 2220
agaggtgcta aaggccctaa aggtgagaat ggtgtcgttg gccccactgg gcctgttgga 2280
gcagctggac ccgctggtcc aaacggacca cccggtcctg ctggatcgag gggtgacggt 2340
ggtccaccag gtatgacggg atttccaggt gcagctggta gaactggtcc ccctggtccg 2400
tccgggattt caggtccacc tgggcctcca ggacctgctg gaaaagaggg cttgaggggt 2460
ccgagaggag atcaaggccc tgtaggaaga accggggaag ttggtgccgt tggacccccg 2520
ggttttgctg ggagaaaggg accatctgga gaagcaggta cagcaggacc acctggtact 2580
ccaggaccgc agggtctctt aggtgcaccc ggtatcttgg gcctgcccgg atctcgtggc 2640
gagcgaggat tacctggtgt ggcaggtgct gtgggtgagc ccggaccact gggtatcgct 2700
ggccctccag gtgcaagagg accccctggt gccgtcggat ctccaggtgt aaatggagct 2760
cccggagagg ccggtaggga cggtaacccg gggaacgatg gccctcctgg aagagatggt 2820
caaccggggc ataaggagaa gagaggctac cctggtaata tcggtcctgt cggtgctgct 2880
ggtgccccag gaccacatgg tccggttgga cctgctggta agcacggtaa tcgaggagag 2940
actggtccgt caggtcctgt tgggccagca ggtgccgtgg gacctagggg tccatcgggg 3000
ccacaaggaa tccgaggtga taaaggtgag cctggcgaaa aaggcccgag aggattgcca 3060
ggactcaagg gtcacaacgg tttgcagggt ttgccaggca ttgccggtca tcacggggac 3120
caaggtgctc ctggatctgt cggcccagct ggtcctcgtg gaccagctgg accatctggc 3180
ccagcaggta aggatggtag aacaggccac ccaggcactg ttggacctgc aggtattaga 3240
ggcccacagg gacaccaagg tccagccgga cctcctggcc caccaggtcc tccgggtcca 3300
cctggagttt caggcggagg ttacgacttt ggatatgacg gagattttta tagagctgat 3360

caacctcgtt cagccccctc tttgagacca aaagactatg aagtggacgc tacattgaag 3420

agtctcaaca atcagatcga aaccttgctt actccggaag gttcaagaaa aaatcctgct 3480

aggacctgca gagacctgag actttctcat ccagagtggt cttccggcta ttattggatt 3540

gatcctaacc aaggctgtac aatggatgct attaaggtct actgcgactt ttccaccggt 3600

gagacatgta taagagctca acctgaaaac attcctgcta aaaattggta cagatcaagt 3660

aaagacaaaa agcatgtgtg gttgggtgaa actattaatg ccggctctca gtttgaatac 3720

aatgtcgagg gagtcacttc taaagagatg gctacgcaac ttgctttcat gcgtttatta 3780

gccaattacg ccagtcaaaa tatcacgtac cactgcaaga atagcattgc ttacatggac 3840

gaggagactg gtaaccttaa aaaggccgtt attctgcaag gtagcaatga cgtagagctg 3900

gtcgctgaag gtaactcacg attcacttat actgtcctgg ttgacggttg ttcaaaaaag 3960

accaacgaat ggggaaaaac gattatcgag tacaagacaa ataagccaag caggctgcct 4020

tttttagaca ttgcaccact agacataggc ggggctgatc aggaattttt cgtggacatt 4080

ggtcccgtct gttttaagta a 4101

<210> 101
<211> 4101
<212> DNA
<213> Homo sapiens

<400> 101
atgctcagct ttgtggatac gcggactttg ttgctgcttg cagtaacctt atgcctagca 60

acatgccaat ctttacaaga ggaaaccgta agaaagggcc cagccggaga tagaggacca 120

cgtggagaaa ggggtccacc aggcccccca ggcagagatg gtgaagatgg tcccacaggc 180

cctcctggtc cacctggtcc tcctggcccc cctggtctcg gtgggaactt tgctgctcag 240

tatgacggaa aaggagttgg acttggccct ggaccaatgg gcttaatggg acctagaggc 300

ccacctggtg cagctggagc cccaggccct caaggtttcc aaggacctgc tggtgagcct 360

ggtgaacctg gtcaaactgg tcctgcaggt gctcgtggtc cagctggccc tcctggcaag 420

gctggtgaag atggtcaccc tggaaaaccc ggacgacctg gtgagagagg agttgttgga 480

ccacagggtg ctcgtggttt ccctggaact cctggacttc ctggcttcaa aggcattagg 540

ggacacaatg gtctggatgg attgaaggga cagcccggtg ctcctggtgt gaagggtgaa 600

cctggtgccc ctggtgaaaa tggaactcca ggtcaaacag gagcccgtgg gcttcctggt 660

gagagaggac gtgttggtgc ccctggccca gctggtgccc gtggcagtga tggaagtgtg 720

ggtcccgtgg gtcctgctgg tcccattggg tctgctggcc ctccaggctt cccaggtgcc 780

cctggcccca agggtgaaat tggagctgtt ggtaacgctg gtcctgctgg tcccgccggt 840

ccccgtggtg aagtgggtct tccaggcctc tccggccccg ttggacctcc tggtaatcct 900

ggagcaaacg gccttactgg tgccaagggt gctgctggcc ttcccggcgt tgctggggct 960

cccggcctcc ctggaccccg cggtattcct ggccctgttg gtgctgccgg tgctactggt 1020
gccagaggac ttgttggtga gcctggtcca gctggctcca aaggagagag cggtaacaag 1080
ggtgagcccg gctctgctgg gccccaaggt cctcctggtc ccagtggtga agaaggaaag 1140
agaggcccta atggggaagc tggatctgcc ggccctccag gacctcctgg gctgagaggt 1200
agtcctggtt ctcgtggtct tcctggagct gatggcagag ctggcgtcat gggccctcct 1260
ggtagtcgtg gtgcaagtgg ccctgctgga gtccgaggac ctaatggaga tgctggtcgc 1320
cctggggagc ctggtctcat gggacccaga ggtcttcctg gttcccctgg aaatatcggc 1380
cccgctggaa aagaaggtcc tgtcggcctc cctggcatcg acggcaggcc tggcccaatt 1440
ggcccagctg gagcaagagg agagcctggc aacattggat tccctggacc caaaggcccc 1500
actggtgatc ctggcaaaaa cggtgataaa ggtcatgctg gtcttgctgg tgctcggggt 1560
gctccaggtc ctgatggaaa caatggtgct cagggacctc ctggaccaca gggtgttcaa 1620
ggtggaaaag gtgaacaggg tcccgctggt cctccaggct tccagggtct gcctggcccc 1680
tcaggtcccg ctggtgaagt tggcaaacca ggagaaaggg gtctccatgg tgagtttggt 1740
ctccctggtc ctgctggtcc aagaggggaa cgcggtcccc caggtgagag tggtgctgcc 1800
ggtcctactg gtcctattgg aagccgaggt ccttctggac ccccagggcc tgatggaaac 1860
aagggtgaac ctggtgttgt tggtgctgtg ggcactgctg gtccatctgg tcctagtgga 1920
ctcccaggag agaggggtgc tgctggcata cctggaggca agggagaaaa gggtgaacct 1980
ggtctcagag gtgaaattgg taaccctggc agagatggtg ctcgtggtgc tcctggtgct 2040
gtaggtgccc ctggtcctgc tggagccaca ggtgaccggg gcgaagctgg ggctgctggt 2100
cctgctggtc ctgctggtcc tcggggaagc cctggtgaac gtggtgaggt cggtcctgct 2160
ggccccaatg gatttgctgg tcctgctggt gctgctggtc aacctggtgc taaaggagaa 2220
agaggagcca aagggcctaa gggtgaaaac ggtgttgttg gtcccacagg ccccgttgga 2280
gctgctggcc cagctggtcc aaatggtccc cccggtcctg ctggaagtcg tggtgatgga 2340
ggcccccctg gtatgactgg tttccctggt gctgctggac ggactggtcc cccaggaccc 2400
tctggtattt ctggccctcc tggtccccct ggtcctgctg ggaaagaagg gcttcgtggt 2460
cctcgtggtg accaaggtcc agttggccga actggagaag taggtgcagt tggtccccct 2520
ggcttcgctg gtgagaaggg tccctctgga gaggctggta ctgctggacc tcctggcact 2580
ccaggtcctc agggtcttct tggtgctcct ggtattctgg gtctccctgg ctcgagaggt 2640
gaacgtggtc taccaggtgt tgctggtgct gtgggtgaac ctggtcctct tggcattgcc 2700
ggccctcctg gggcccgtgg tcctcctggt gctgtgggta gtcctggagt caacggtgct 2760
cctggtgaag ctggtcgtga tggcaaccct gggaacgatg gtcccccagg tcgcgatggt 2820
caacccggac acaagggaga gcgcggttac cctggcaata ttggtcccgt tggtgctgca 2880

ggtgcacctg gtcctcatgg ccccgtgggt cctgctggca aacatggaaa ccgtggtgaa 2940

actggtcctt ctggtcctgt tggtcctgct ggtgctgttg gcccaagagg tcctagtggc 3000

ccacaaggca ttcgtggcga taagggagag cccggtgaaa aggggcccag aggtcttcct 3060

ggcttaaagg gacacaatgg attgcaaggt ctgcctggta tcgctggtca ccatggtgat 3120

caaggtgctc ctggctccgt gggtcctgct ggtcctaggg gccctgctgg tccttctggc 3180

cctgctggaa aagatggtcg cactggacat cctggtacag ttggacctgc tggcattcga 3240

ggccctcagg gtcaccaagg ccctgctggc ccccctggtc cccctggccc tcctggacct 3300

ccaggtgtaa gcggtggtgg ttatgacttt ggttacgatg gagacttcta cagggctgac 3360

cagcctcgct cagcaccttc tctcagaccc aaggactatg aagttgatgc tactctgaag 3420

tctctcaaca accagattga gacccttctt actcctgaag gctctagaaa gaacccagct 3480

cgcacatgcc gtgacttgag actcagccac ccagagtgga gcagtggtta ctactggatt 3540

gaccctaacc aaggatgcac tatggatgct atcaaagtat actgtgattt ctctactggc 3600

gaaacctgta tccgggccca acctgaaaac atcccagcca agaactggta taggagctcc 3660

aaggacaaga aacacgtctg gctaggagaa actatcaatg ctggcagcca gtttgaatat 3720

aatgtagaag gagtgacttc caaggaaatg gctacccaac ttgccttcat gcgcctgctg 3780

gccaactatg cctctcagaa catcacctac cactgcaaga acagcattgc atacatggat 3840

gaggagactg gcaacctgaa aaaggctgtc attctacagg gctctaatga tgttgaactt 3900

gttgctgagg gcaacagcag gttcacttac actgttcttg tagatggctg ctctaaaaag 3960

acaaatgaat ggggaaagac aatcattgaa tacaaaacaa ataagccatc acgcctgccc 4020

ttccttgata ttgcaccttt ggacatcggt ggtgctgacc aggaattctt tgtggacatt 4080

ggcccagtct gtttcaaata a 4101

<210> 102
<211> 4464
<212> DNA
<213> Artificial Sequence

<220>
<223> synthesized DNA of human collagen type II alpha 1 precursor ORF ha
ving codon optimized for yeast

<400> 102
atgatacgat taggcgcccc acagactttg gttttactta cattgcttgt ggctgccgta 60

ctaaggtgtc aaggtcaaga tgttcaagaa gctgggtcct gtgtgcaaga tggtcaaaga 120

tacaatgaca aggatgtctg gaagcccgag ccgtgtagga tctgcgtttg tgataccggc 180

actgtgctgt gtgatgatat tatctgcgaa gatgtcaaag attgcttgtc cccggagatc 240

ccttttggtg agtgttgtcc gatctgtcct acggatttgg caactgcttc gggacaaccg 300

gggcctaaag gacaaaaagg agaaccgggt gatattaagg acatcgtcgg tccgaaaggt 360

ccccccggtc cgcagggtcc cgctggtgag caaggaccga gaggtgacag gggagataag 420
ggggaaaagg gagctccggg gcctagagga cgtgacggcg agccaggtac ccctggtaat 480
ccgggacctc caggaccgcc cggacctccg ggtcccccag gtctaggagg caacttcgct 540
gctcaaatgg ctggtgggtt cgacgagaaa gctggaggtg cccagttggg cgttatgcaa 600
gggccgatgg gtccgatggg gcctcgtgga cctccagggc cggctggcgc acccggacct 660
cagggttttc agggaaatcc gggtgaacca ggtgagcccg gtgttagcgg tccgatgggt 720
ccacgtggcc ctcctggtcc ccctggtaaa ccaggagacg atggagaggc aggaaagccg 780
ggaaaggctg gtgaacgagg tccaccgggc ccacaaggtg caagaggttt tcccggtact 840
ccaggtttgc cgggagttaa gggacaccga ggatacccag gattggacgg agcaaaggga 900
gaggctgggg ctcccggtgt taaaggagag tctggttctc ctggtgaaaa tggttcgccg 960
ggcccaatgg gaccccgagg attgccgggc gagcgtggac gtactggacc agccggagct 1020
gcaggagcca gaggaaacga tggtcagcct ggaccggctg gtcctcccgg tcctgttggc 1080
ccagccggcg gtccaggctt cccaggcgct ccaggggcca aaggcgaagc tggcccaacc 1140
ggcgcacgtg gacctgaggg cgctcagggt cccagagggg agcctggtac cccaggtagt 1200
cccggaccag ctggtgcttc aggtaatcct ggtacagatg gtattccagg tgccaagggt 1260
tcagccggag ctcctggtat agccggtgcc cctggatttc caggtccaag gggtccacca 1320
ggaccccaag gggcaacagg acctttgggt ccaaagggac aaactggaga acccggtatt 1380
gctggtttta aaggtgagca agggcctaag ggcgaaccag gaccagctgg gccacagggt 1440
gctcccggac ctgcaggtga ggaaggtaaa cgtggagcta ggggtgagcc aggtggcgtg 1500
ggaccaattg gaccacccgg tgagagggga gcaccaggta acagaggttt cccagggcag 1560
gatggactgg ctggtcccaa aggagcacca ggagaaagag gaccatcagg tcttgccggt 1620
cctaagggag ctaacggaga tcccggtaga ccaggtgaac caggcttgcc tggtgccaga 1680
ggacttactg gtagacccgg tgatgccggt cctcaaggta aggtgggccc ctctggtgca 1740
cccggagaag atggcagacc aggaccacca ggacctcagg gtgcaagagg gcagccagga 1800
gttatgggct ttccaggccc caaaggtgct aacggagaac ctggtaaagc cggtgaaaag 1860
ggtctgcctg gcgcacctgg ccttcgtggg ctacccggca aggacggaga aacaggtgcc 1920
gctggacctc ccggccctgc cggtcccgcc ggtgagcgtg gtgaacaagg agccccagga 1980
ccatctgggt ttcaaggtct gcctgggcct ccaggtccac caggtgaggg tggaaagcca 2040
ggagatcagg gagttccagg tgaggctgga gcccctggtt tggttgggcc aaggggagaa 2100
cgtggtttcc ctggcgaaag gggtagtcca ggtgcccaag gcttacaggg tcctagagga 2160
cttccaggta ctcctggaac tgacggacct aaaggagcca gtggtcctgc cggacctcct 2220
ggcgctcaag ggccaccagg gctccaagga atgcctggag aaagaggagc agcaggaatc 2280
gctggaccta agggtgatag aggtgacgta ggagaaaagg gtccagaagg tgcccctgga 2340

aaggatggtg gtagaggctt aactggtcct attggaccac ctgggccagc aggcgcaaac 2400
ggagagaaag gtgaggttgg tcctccagga cccgctggtt ccgctggggc acgaggtgct 2460
ccaggggaaa gaggtgaaac aggaccccct ggtcctgctg gctttgcagg gccacctggt 2520
gctgacggac aacctggagc taaaggcgag caaggtgaag ctggtcaaaa gggagatgcc 2580
ggcgctcctg gaccacaagg tccatctggt gccccaggtc cacagggtcc aaccggtgtt 2640
actggtccaa aaggagctag aggtgcacag ggaccacctg gtgcaacggg gttcccaggt 2700
gcagccggta gagttggtcc acccggctct aatggtaacc caggccctcc tggtcctcct 2760
ggaccttccg gtaaagatgg tcccaaggga gcccgtggtg attccggtcc ccctggaagg 2820
gctggtgagc caggattgca aggacctgca gggcctcccg gagaaaaggg tgaacctggt 2880
gatgacggac catctggagc tgagggtcca cctgggcctc agggattagc aggccaaaga 2940
ggtatcgttg gacttcctgg acagagaggc gagaggggtt tccctggact cccaggtcca 3000
tccggggaac ctggtaagca gggcgcacct ggtgctagcg gggacagagg tcctccaggc 3060
ccagtcggac cacctggact caccggacct gccggagaac caggaagaga aggatcccca 3120
ggtgcagacg gacctccagg tcgagacggc gccgccggtg tcaaagggga tcgaggagaa 3180
actggagccg tcggagctcc aggagcccct ggacctcctg gatctcctgg tccagcaggc 3240
cctaccggaa agcaaggcga cagaggagaa gccggtgctc agggtcctat gggcccttca 3300
ggtcctgctg gtgctagagg tatccagggt cctcaaggac caagaggtga taaaggggaa 3360
gctggggaac caggtgagag aggcctgaaa gggcatagag gatttactgg tctgcaagga 3420
ttgccaggac ctcctggccc ttcgggggat cagggtgcca gcgggccagc aggtccaagc 3480
ggtccccgag gacccccagg tcctgtcggt ccatccggca aggatggagc taatggaatt 3540
cccggtccca ttgggccccc tggcccaaga ggcagatctg gtgagacagg tcctgcaggt 3600
ccacccggta acccaggacc acccggtcca cctggtccac caggcccagg cattgacatg 3660
tcagctttcg ctggtttggg tccacgagaa aagggacccg acccattgca atacatgcgt 3720
gcagatcaag ccgcaggagg gctcagacag cacgatgcag aggttgacgc aacactaaaa 3780
tctctgaaca accaaataga atcgattagg tcacctgagg gatccagaaa gaatccagct 3840
agaacttgtc gagacttgaa attatgtcac ccagagtgga aatctggaga ttattggata 3900
gatccaaacc aaggctgcac cttggatgct atgaaggtat tttgtaatat ggagacaggc 3960
gaaacttgtg tatacccaaa cccagctaat gtgcctaaga aaaattggtg gtcatctaag 4020
agtaaagaaa agaaacatat ctggtttggt gagactatta acggtggctt ccatttcagt 4080
tacggcgacg acaatctagc acccaacact gctaacgtgc aaatgacgtt tttacgactg 4140
ttgagtacag aaggttccca gaatattacc tatcactgta aaaattctat agcctattta 4200
gacgaagctg caggcaatct gaagaaagct ttgcttattc aaggttcaaa cgatgtagaa 4260

attagggcag aaggtaactc aagatttaca tacacagcct tgaaagacgg gtgcactaag 4320

catacgggta aatggggtaa gactgtcatc gagtatagaa gtcagaagac ctctcgtctt 4380

cctattatcg acattgctcc aatggatata ggcggaccag agcaagaatt cggagtagac 4440

attggaccag tctgctttct ataa 4464

<210> 103
<211> 4464
<212> DNA
<213> Homo sapiens

<400> 103
atgattcgcc tcggggctcc ccagacgctg gtgctgctga cgctgctcgt cgccgctgtc 60

cttcggtgtc agggccagga tgtccaggag gctggcagct gtgtgcagga tgggcagagg 120

tataatgata aggatgtgtg gaagccggag ccctgccgga tctgtgtctg tgacactggg 180

actgtcctct gcgacgacat aatctgtgaa gacgtgaaag actgcctcag ccctgagatc 240

cccttcggag agtgctgccc catctgccca actgacctcg ccactgccag tgggcaacca 300

ggaccaaagg gacagaaagg agaacctgga gacatcaagg atattgtagg acccaaagga 360

cctcctgggc ctcagggacc tgcaggggaa caaggaccca gaggggatcg tggtgacaaa 420

ggtgaaaaag gtgcccctgg acctcgtggc agagatggag aacctgggac ccctggaaat 480

cctggccccc ctggtcctcc cggcccccct ggtccccctg gtcttggtgg aaactttgct 540

gcccagatgg ctggaggatt tgatgaaaag gctggtggcg cccagttggg agtaatgcaa 600

ggaccaatgg gccccatggg acctcgagga cctccaggcc ctgcaggtgc tcctgggcct 660

caaggatttc aaggcaatcc tggtgaacct ggtgaacctg gtgtctctgg tcccatgggt 720

ccccgtggtc ctcctggtcc ccctggaaag cctggtgatg atggtgaagc tggaaaacct 780

ggaaaagctg gtgaaagggg tccgcctggt cctcagggtg ctcgtggttt cccaggaacc 840

ccaggccttc ctggtgtcaa aggtcacaga ggttatccag gcctggacgg tgctaaggga 900

gaggcgggtg ctcctggtgt gaagggtgag agtggttccc cgggtgagaa cggatctccg 960

ggcccaatgg gtcctcgtgg cctgcctggt gaaagaggac ggactggccc tgctggcgct 1020

gcgggtgccc gaggcaacga tggtcagcca ggccccgcag ggcctccggg tcctgtcggt 1080

cctgctggtg gtcctggctt ccctggtgct cctggagcca agggtgaagc cggccccact 1140

ggtgcccgtg gtcctgaagg tgctcaaggt cctcgcggtg aacctggtac tcctgggtcc 1200

cctgggcctg ctggtgcctc cggtaaccct ggaacagatg gaattcctgg agccaaagga 1260

tctgctggtg ctcctggcat tgctggtgct cctggcttcc ctgggccacg gggccctcct 1320

ggccctcaag gtgcaactgg tcctctgggc ccgaaaggtc agacgggtga acctggtatt 1380

gctggcttca aaggtgaaca aggccccaag ggagaacctg gccctgctgg cccccaggga 1440

gcccctggac ccgctggtga agaaggcaag agaggtgccc gtggagagcc tggtggcgtt 1500

gggcccatcg gtccccctgg agaaagaggt gctcccggca accgcggttt cccaggtcaa 1560
gatggtctgg caggtcccaa gggagcccct ggagagcgag ggcccagtgg tcttgctggc 1620
cccaagggag ccaacggtga ccctggccgt cctggagaac ctggccttcc tggagcccgg 1680
ggtctcactg gccgccctgg tgatgctggt cctcaaggca aagttggccc ttctggagcc 1740
cctggtgaag atggtcgtcc tggacctcca ggtcctcagg gggctcgtgg gcagcctggt 1800
gtcatgggtt tccctggccc caaaggtgcc aacggtgagc ctggcaaagc tggtgagaag 1860
ggactgcctg gtgctcctgg tctgaggggt cttcctggca aagatggtga gacaggtgct 1920
gcaggacccc ctggccctgc tggacctgct ggtgaacgag gcgagcaggg tgctcctggg 1980
ccatctgggt tccagggact tcctggccct cctggtcccc caggtgaagg tggaaaacca 2040
ggtgaccagg gtgttcccgg tgaagctgga gcccctggcc tcgtgggtcc caggggtgaa 2100
cgaggtttcc caggtgaacg tggctctccc ggtgcccagg gcctccaggg tccccgtggc 2160
ctccccggca ctcctggcac tgatggtccc aaaggtgcat ctggcccagc aggcccccct 2220
ggggctcagg gccctccagg tcttcaggga atgcctggcg agaggggagc agctggtatc 2280
gctgggccca aaggcgacag gggtgacgtt ggtgagaaag gccctgaggg agcccctgga 2340
aaggatggtg gacgaggcct gacaggtccc attggccccc ctggcccagc tggtgctaat 2400
ggcgagaagg gagaagttgg acctcctggt cctgcaggaa gtgctggtgc tcgtggcgct 2460
ccgggtgaac gtggagagac tgggcccccc ggaccagcgg gatttgctgg gcctcctggt 2520
gctgatggcc agcctggggc caagggtgag caaggagagg ccggccagaa aggcgatgct 2580
ggtgcccctg gtcctcaggg cccctctgga gcacctgggc ctcagggtcc tactggagtg 2640
actggtccta aaggagcccg aggtgcccaa ggccccccgg gagccactgg attccctgga 2700
gctgctggcc gcgttggacc cccaggctcc aatggcaacc ctggaccccc tggtccccct 2760
ggtccttctg gaaaagatgg tcccaaaggt gctcgaggag acagcggccc ccctggccga 2820
gctggtgaac ccggcctcca aggtcctgct ggaccccctg gcgagaaggg agagcctgga 2880
gatgacggtc cctctggtgc cgaaggtcca ccaggtcccc agggtctggc tggtcagaga 2940
ggcatcgtcg gtctgcctgg gcaacgtggt gagagaggat tccctggctt gcctggcccg 3000
tcgggtgagc ccggcaagca gggtgctcct ggagcatctg gagacagagg tcctcctggc 3060
cccgtgggtc ctcctggcct gacgggtcct gcaggtgaac ctggacgaga gggaagcccc 3120
ggtgctgatg gcccccctgg cagagatggc gctgctggag tcaagggtga tcgtggtgag 3180
actggtgctg tgggagctcc tggagcccct gggccccctg gctcccctgg ccccgctggt 3240
ccaactggca agcaaggaga cagaggagaa gctggtgcac aaggccccat gggaccctca 3300
ggaccagctg gagcccgggg aatccagggt cctcaaggcc ccagaggtga caaaggagag 3360
gctggagagc ctggcgagag aggcctgaag ggacaccgtg gcttcactgg tctgcagggt 3420
ctgcccggcc ctcctggtcc ttctggagac caaggtgctt ctggtcctgc tggtccttct 3480

ggccctagag gtcctcctgg ccccgtcggt ccctctggca aagatggtgc taatggaatc 3540

cctggcccca ttgggcctcc tggtccccgt ggacgatcag gcgaaaccgg ccctgctggt 3600

cctcctggaa atcctggacc ccctggtcct ccaggtcccc ctggccctgg catcgacatg 3660

tccgcctttg ctggcttagg cccgagagag aagggccccg accccctgca gtacatgcgg 3720

gccgaccagg cagccggtgg cctgagacag catgacgccg aggtggatgc cacactcaag 3780

tccctcaaca accagattga gagcatccgc agccccgagg gctcccgcaa gaaccctgct 3840

cgcacctgca gagacctgaa actctgccac cctgagtgga agagtggaga ctactggatt 3900

gaccccaacc aaggctgcac cttggacgcc atgaaggttt tctgcaacat ggagactggc 3960

gagacttgcg tctaccccaa tccagcaaac gttcccaaga agaactggtg gagcagcaag 4020

agcaaggaga agaaacacat ctggtttgga gaaaccatca atggtggctt ccatttcagc 4080

tatggagatg acaatctggc tcccaacact gccaacgtcc agatgacctt cctacgcctg 4140

ctgtccacgg aaggctccca gaacatcacc taccactgca agaacagcat tgcctatctg 4200

gacgaagcag ctggcaacct caagaaggcc ctgctcatcc agggctccaa tgacgtggag 4260

atccgggcag agggcaatag caggttcacg tacactgccc tgaaggatgg ctgcacgaaa 4320

cataccggta agtggggcaa gactgttatc gagtaccggt cacagaagac ctcacgcctc 4380

cccatcattg acattgcacc catggacata ggagggcccg agcaggaatt cggtgtggac 4440

atagggccgg tctgcttctt gtaa 4464

<210> 104
<211> 4401
<212> DNA
<213> Artificial Sequence

<220>
<223> synthesized DNA of human collagen type III alpha 1 precursor ORF h
      aving codon optimized for yeast

<400> 104
   atgatgagct tcgttcaaaa aggatcgtgg ctcctcctgg ctctcttgca cccgaccatt 60
   attttggctc aacaagaggc cgtggagggg gggtgtagcc atctcggaca aagctacgca 120
   gatcgagatg tttggaagcc cgagccctgt caaatatgcg tttgtgatag tggtagcgtt 180
   ctatgcgacg acatcatctg cgatgaccaa gagcttgatt gtccgaatcc agaaattcct 240
   tttggagaat gttgtgctgt gtgcccgcag ccacctaccg cacctaccag gccgcctaat 300
   ggtcaaggcc cacagggtcc aaaaggtgac cctggtccgc ctggaatccc tggacgtaat 360
   ggagatccgg gaattccagg tcaacccgga tcaccgggaa gcccaggccc tccaggaatc 420
   tgcgagtctt gtccaaccgg accgcaaaat tacagtccgc aatacgacag ctatgatgtc 480
   aagtcagggg ttgccgtggg gggattggca gggtacccag gtcctgcagg accgcctggt 540
   ccgccaggtc ccccaggaac atccggccac ccaggaagcc cagggtcgcc gggttatcag 600
   ggaccaccgg gtgagccagg ccaggcaggt ccgtctggtc ctccgggtcc gcccggtgct 660
   atcggaccat ctggaccggc tggtaaagat ggggaatccg gtcgtccggg tcgtccgggt 720
   gagcgtggac ttcctggtcc accgggtatt aagggtccgg ctggaatacc tggtttccct 780
   ggcatgaaag gccatagagg tttcgacggt aggaacggtg agaagggaga gactggtgct 840
   cctggtctta agggtgaaaa tggattgcca ggagaaaatg gtgcaccggg acctatggga 900
   ccgagaggcg caccgggaga gagaggaaga ccaggtttac ccggagcagc cggtgcacgt 960
   ggtaacgacg gagcacgtgg ctctgatggt cagcctggtc ctcctggacc tcccggtacc 1020
   gccgggttcc ctggttctcc gggtgccaaa ggagaggtcg gtcctgctgg atctcccggt 1080
   tctaatggcg ccccaggtca gagaggagaa cctggtcccc aaggtcacgc tggcgctcaa 1140
   ggccctcctg gaccaccagg tatcaacggt tctcctggag gcaagggtga gatgggccct 1200
   gctggtatac caggggcccc aggacttatg ggagctagag gacccccagg tcctgctggt 1260
   gctaacggtg ccccaggatt gcgtggtgga gctggtgaac cagggaaaaa tggagctaaa 1320
   ggagaacctg gcctagaggt tgaaagaggt gaagcaggta tccctggcgt tcctggtgct 1380
   aagggagaag atggaaaaga tgggtcgcca ggggagccag gcgcaaacgg tttacccggt 1440
   gctgctggag agagaggagc tccaggcttt aggggtccag ctggtccaaa cggtattccc 1500
   ggtgaaaaag gtccagccgg tgaacgagga gcaccaggtc cagccggtcc aagaggtgca 1560
   gcaggtgaac caggtcgaga cggagtccca ggtggtcccg gaatgagggg aatgccaggt 1620
   tcgccaggtg gacccggctc agacggaaag ccaggtccac caggtagtca aggagagtca 1680
   gggcgaccag gaccaccagg accatccggt cctcgtgggc agcccggtgt tatgggtttc 1740
   cctggtccca agggcaatga tggcgctcct ggtaagaatg gtgagagagg agggcctggc 1800
   ggtcccggtc cccagggccc tccaggcaag aacggggaaa ctggtccaca gggacctcca 1860
   ggaccaaccg gtccaggagg agacaaagga gatacaggac caccaggtcc acaaggtttg 1920
   caaggattac caggaacggg tgggccacca ggggagaacg gcaagcctgg agaaccaggt 1980
   cctaagggtg atgctggtgc acctggtgct cctggcggaa aaggtgatgc tggagctcct 2040
   ggggaaagag gacctcctgg attggctggg gcaccaggac tgagaggtgg tgctggccct 2100
   ccagggcctg aaggaggaaa aggtgccgct ggaccacccg gtcctccagg cgcagccgga 2160
   actcctggtc tacaggggat gcctggtgaa agaggtggtc ttggtagtcc tgggcctaaa 2220
   ggcgacaaag gtgaaccagg aggtcctggt gctgatggtg ttccaggtaa ggacggccca 2280
   cgaggaccta cgggtccaat cggtcctcca ggacctgctg gtcagccagg tgataaaggg 2340
   gaaggaggtg cacccggttt acctggtatt gccggacctc gaggtagtcc tggtgagagg 2400
   ggagaaactg gcccacctgg gcctgctggt tttccaggtg cccctggtca gaacggagaa 2460
   cctggtggta agggggagag aggtgcacca ggagaaaagg gtgaaggagg cccacccgga 2520
   gttgcaggac ctccaggagg atctgggcca gctggtccac caggacctca aggtgtaaaa 2580
   ggtgagcgag gatcgccagg tggtccaggt gccgcaggct ttccaggggc aagggggtta 2640
   cccggacccc ctggttccaa tggcaaccca ggacctcccg gtccatccgg atcccctggg 2700
   aaggatggtc cccctggtcc cgctggtaac acaggtgctc ctggttctcc cggcgtctca 2760
   ggacccaaag gggatgctgg tcaaccaggt gaaaagggat ccccaggtgc tcaaggtccc 2820
   ccaggtgcac cagggccctt gggtattgct ggaattacag gtgctagagg tttggctggt 2880
   ccacccggca tgccaggtcc taggggatct cctggaccac agggagttaa gggcgagtcc 2940
   ggaaaaccag gtgccaacgg actttcaggt gaaaggggtc ccccaggccc tcaaggcctg 3000
   ccaggattag ccggaactgc tggagaaccc ggacgtgatg gaaacccagg ttctgacggt 3060
   ttgcccggta gggatggaag tccaggaggc aagggcgaca gaggagagaa tggatcacca 3120
   ggagcccctg gagcaccagg acatccagga ccacctggcc ctgtaggtcc agccggtaaa 3180
   tcaggagaca gaggagaatc aggtcccgcc ggtccagctg gcgcccctgg ccccgctgga 3240
   tcaagaggtg ccccaggtcc tcaaggacca agaggcgata aaggagaaac cggagaaaga 3300
   ggtgctgctg gaattaaggg ccacagaggt ttccctggga accctggagc cccagggagt 3360
   ccaggtcctg caggtcagca aggggccatt ggtagtccag gaccagccgg tcccagaggt 3420
   cctgtgggac catctggtcc tccagggaag gatggcactt ctggtcaccc cggccctatc 3480
   ggtccccctg gaccaagagg caacagaggc gagaggggct cagagggatc ccctggtcat 3540
   cctggacaac ctggccctcc agggcctccc ggcgctccag gtccatgctg tggaggtgtc 3600
   ggtgctgctg ctattgctgg catcggaggt gaaaaagccg gtggatttgc cccttattat 3660
   ggcgacgaac caatggattt caagattaac actgacgaaa ttatgacatc tttgaagtcc 3720
   gtaaacggtc aaatagagtc cttgatttca cctgatggtt ctcgtaagaa cccagcccga 3780
   aactgtcgtg atcttaaatt ttgccatcct gaattgaaga gtggtgagta ctgggtggac 3840
   cctaatcaag gttgtaagct agacgccata aaagtgttct gtaatatgga aactggagag 3900
   acttgtattt ccgccaaccc cttgaatgtg cctaggaaac attggtggac cgattcctca 3960
   gctgagaaga agcatgtttg gtttggtgaa tctatggacg ggggctttca gttttcctac 4020
   ggcaatcctg aactaccaga ggatgtcctg gacgttcaac tggccttcct gagattattg 4080
   tccagtagag cctctcaaaa catcacatac cactgtaaga attcaattgc ctatatggac 4140
   caggcctctg ggaatgtcaa aaaggctctg aagctgatgg gcagtaatga aggcgaattt 4200
   aaagcagagg gcaactctaa atttacttac actgttcttg aagatggctg tacaaaacat 4260
   actggtgaat ggtctaagac ggtattcgag tatagaacta gaaaagcagt tagattgcct 4320
   atagttgata tcgcccctta tgatattgga ggtcccgacc aagagtttgg tgtcgatgta 4380
   ggaccagttt gtttcctata a 4401

<210> 105
<211> 4401
<212> DNA
<213> Homo sapiens

<400> 105
atgatgagct ttgtgcaaaa ggggagctgg ctacttctcg ctctgcttca tcccactatt 60

attttggcac aacaggaagc tgttgaagga ggatgttccc atcttggtca gtcctatgcg 120

gatagagatg tctggaagcc agaaccatgc caaatatgtg tctgtgactc aggatccgtt 180

ctctgcgatg acataatatg tgacgatcaa gaattagact gccccaaccc agaaattcca 240

tttggagaat gttgtgcagt ttgcccacag cctccaactg ctcctactcg ccctcctaat 300

ggtcaaggac ctcaaggccc caagggagat ccaggccctc ctggtattcc tgggagaaat 360

ggtgaccctg gtattccagg acaaccaggg tcccctggtt ctcctggccc ccctggaatc 420

tgtgaatcat gccctactgg tcctcagaac tattctcccc agtatgattc atatgatgtc 480

aagtctggag tagcagtagg aggactcgca ggctatcctg gaccagctgg ccccccaggc 540

cctcccggtc cccctggtac atctggtcat cctggttccc ctggatctcc aggataccaa 600

ggaccccctg gtgaacctgg gcaagctggt ccttcaggcc ctccaggacc tcctggtgct 660

ataggtccat ctggtcctgc tggaaaagat ggagaatcag gtagacccgg acgacctgga 720

gagcgaggat tgcctggacc tccaggtatc aaaggtccag ctgggatacc tggattccct 780

ggtatgaaag gacacagagg cttcgatgga cgaaatggag aaaagggtga aacaggtgct 840

cctggattaa agggtgaaaa tggtcttcca ggcgaaaatg gagctcctgg acccatgggt 900

ccaagagggg ctcctggtga gcgaggacgg ccaggacttc ctggggctgc aggtgctcgg 960

ggtaatgacg gtgctcgagg cagtgatggt caaccaggcc ctcctggtcc tcctggaact 1020

gccggattcc ctggatcccc tggtgctaag ggtgaagttg gacctgcagg gtctcctggt 1080

tcaaatggtg cccctggaca aagaggagaa cctggacctc agggacacgc tggtgctcaa 1140

ggtcctcctg gccctcctgg gattaatggt agtcctggtg gtaaaggcga aatgggtccc 1200

gctggcattc ctggagctcc tggactgatg ggagcccggg gtcctccagg accagccggt 1260

gctaatggtg ctcctggact gcgaggtggt gcaggtgagc ctggtaagaa tggtgccaaa 1320

ggagagcccg gaccacgtgg tgaacgcggt gaggctggta ttccaggtgt tccaggagct 1380

aaaggcgaag atggcaagga tggatcacct ggagaacctg gtgcaaatgg gcttccagga 1440

gctgcaggag aaaggggtgc ccctgggttc cgaggacctg ctggaccaaa tggcatccca 1500

ggagaaaagg gtcctgctgg agagcgtggt gctccaggcc ctgcagggcc cagaggagct 1560

gctggagaac ctggcagaga tggcgtccct ggaggtccag gaatgagggg catgcccgga 1620

agtccaggag gaccaggaag tgatgggaaa ccagggcctc ccggaagtca aggagaaagt 1680

ggtcgaccag gtcctcctgg gccatctggt ccccgaggtc agcctggtgt catgggcttc 1740

cccggtccta aaggaaatga tggtgctcct ggtaagaatg gagaacgagg tggccctgga 1800

ggacctggcc ctcagggtcc tcctggaaag aatggtgaaa ctggacctca aggaccccca 1860
gggcctactg ggcctggtgg tgacaaagga gacacaggac cccctggtcc acaaggatta 1920
caaggcttgc ctggtacagg tggtcctcca ggagaaaatg gaaaacctgg ggaaccaggt 1980
ccaaagggtg atgccggtgc acctggagct ccaggaggca agggtgatgc tggtgcccct 2040
ggtgaacgtg gacctcctgg attggcaggg gccccaggac ttagaggtgg agctggtccc 2100
cctggtcccg aaggaggaaa gggtgctgct ggtcctcctg ggccacctgg tgctgctggt 2160
actcctggtc tgcaaggaat gcctggagaa agaggaggtc ttggaagtcc tggtccaaag 2220
ggtgacaagg gtgaaccagg cggcccaggt gctgatggtg tcccagggaa agatggccca 2280
aggggtccta ctggtcctat tggtcctcct ggcccagctg gccagcctgg agataagggt 2340
gaaggtggtg cccccggact tccaggtata gctggacctc gtggtagccc tggtgagaga 2400
ggtgaaactg gccctccagg acctgctggt ttccctggtg ctcctggaca gaatggtgaa 2460
cctggtggta aaggagaaag aggggctccg ggtgagaaag gtgaaggagg ccctcctgga 2520
gttgcaggac cccctggagg ttctggacct gctggtcctc ctggtcccca aggtgtcaaa 2580
ggtgaacgtg gcagtcctgg tggacctggt gctgctggct tccctggtgc tcgtggtctt 2640
cctggtcctc ctggtagtaa tggtaaccca ggacccccag gtcccagcgg ttctccaggc 2700
aaggatgggc ccccaggtcc tgcgggtaac actggtgctc ctggcagccc tggagtgtct 2760
ggaccaaaag gtgatgctgg ccaaccagga gagaagggat cgcctggtgc ccagggccca 2820
ccaggagctc caggcccact tgggattgct gggatcactg gagcacgggg tcttgcagga 2880
ccaccaggca tgccaggtcc taggggaagc cctggccctc agggtgtcaa gggtgaaagt 2940
gggaaaccag gagctaacgg tctcagtgga gaacgtggtc cccctggacc ccagggtctt 3000
cctggtctgg ctggtacagc tggtgaacct ggaagagatg gaaaccctgg atcagatggt 3060
cttccaggcc gagatggatc tcctggtggc aagggtgatc gtggtgaaaa tggctctcct 3120
ggtgcccctg gcgctcctgg tcatccaggc ccacctggtc ctgtcggtcc agctggaaag 3180
agtggtgaca gaggagaaag tggccctgct ggccctgctg gtgctcccgg tcctgctggt 3240
tcccgaggtg ctcctggtcc tcaaggccca cgtggtgaca aaggtgaaac aggtgaacgt 3300
ggagctgctg gcatcaaagg acatcgagga ttccctggta atccaggtgc cccaggttct 3360
ccaggccctg ctggtcagca gggtgcaatc ggcagtccag gacctgcagg ccccagagga 3420
cctgttggac ccagtggacc tcctggcaaa gatggaacca gtggacatcc aggtcccatt 3480
ggaccaccag ggcctcgagg taacagaggt gaaagaggat ctgagggctc cccaggccac 3540
ccagggcaac caggccctcc tggacctcct ggtgcccctg gtccttgctg tggtggtgtt 3600
ggagccgctg ccattgctgg gattggaggt gaaaaagctg gcggttttgc cccgtattat 3660
ggagatgaac caatggattt caaaatcaac accgatgaga ttatgacttc actcaagtct 3720

gttaatggac aaatagaaag cctcattagt cctgatggtt ctcgtaaaaa ccccgctaga 3780

aactgcagag acctgaaatt ctgccatcct gaactcaaga gtggagaata ctgggttgac 3840

cctaaccaag gatgcaaatt ggatgctatc aaggtattct gtaatatgga aactggggaa 3900

acatgcataa gtgccaatcc tttgaatgtt ccacggaaac actggtggac agattctagt 3960

gctgagaaga aacacgtttg gtttggagag tccatggatg gtggttttca gtttagctac 4020

ggcaatcctg aacttcctga agatgtcctt gatgtgcagc tggcattcct tcgacttctc 4080

tccagccgag cttcccagaa catcacatat cactgcaaaa atagcattgc atacatggat 4140

caggccagtg gaaatgtaaa gaaggccctg aagctgatgg ggtcaaatga aggtgaattc 4200

aaggctgaag gaaatagcaa attcacctac acagttctgg aggatggttg cacgaaacac 4260

actggggaat ggagcaaaac agtctttgaa tatcgaacac gcaaggctgt gagactacct 4320

attgtagata ttgcacccta tgacattggt ggtcctgatc aagaatttgg tgtggacgtt 4380

ggccctgttt gctttttata a 4401

<210> 106
<211> 1578
<212> DNA
<213> Homo sapiens

<400> 106
atgttttctt tcgtagattt gcgactcctc ttgttgcttg ctgctaccgc tctccttact 60

catggacagg aagaaggcca ggtggaagga caggacgaag acattcctcc tataacttgt 120

gtgcagaacg gactgaggta tcatgacaga gatgtgtgga agccagagcc ttgtcgaata 180

tgcgtttgtg acaacggaaa ggtgctatgt gatgatgtta tctgtgatga gacaaagaac 240

tgtcctggcg ctgaagttcc agagggagaa tgttgtccag tttgtcctga tggatcggag 300

tcacccactg atcaagagac tacgggagta gaaggtccaa aaggtgacac tggtcccaga 360

ggcccaagag gacctgccgg cccccctggt agggatggca ttcctggtca acccggccta 420

ccaggccccc caggtccacc tggcccccca ggaccccctg gtttaggagg taacttcgct 480

cctcaactgt cttacggtta cgatgaaaag tctactggtg gaatctctgt tcctggacca 540

atgggtccgt ctggtccaag gggtctacct ggtcctcccg gtgcacctgg tccgcagggt 600

tttcaaggtc caccaggtga accgggagaa ccgggagctt caggtccaat gggtcccagg 660

ggtcctcctg gccctcctgg aaagaacggc gatgatggcg aggctggtaa gccgggacca 720

ccagggccgc ctggtcctcc tggtccacct ggtccgccca gtgcaggatt cgacttttcc 780

ttcttacccc aaccgccaca ggaaaaggca cacgatggtg gtagatatta ccgtgctgat 840

gatgccaatg tcgtgagaga tagagactta gaagttgata cgacattaaa atcactttct 900

caacaaattg agaacattag atctccggag ggtagcagaa agaacccggc caggacttgc 960

cgtgatctta agatgtgcca ctccgattgg aagtcgggag agtactggat cgatcctaat 1020

caaggttgta acttggatgc aataaaagtc ttctgtaaca tggagacagg agaaacttgc 1080

gtttacccaa ctcaaccaag tgttgcacag aaaaattggt atatttccaa gaatccaaag 1140

gataagcgtc atgtttggtt tggagaatct atgaccgatg gctttcaatt cgagtatgga 1200

ggtcagggta gcgacccagc tgatgtcgct attcaattga cctttttgag acttatgtcc 1260

acagaagctt cgcaaaacat tacatatcac tgtaagaata gcgtagccta catggaccaa 1320

cagactggta atctgaaaaa ggccctgttg ttgcaaggca gtaatgaaat cgaaattcgt 1380

gccgaaggaa attccagatt cacatactct gttaccgttg acggctgcac ctctcatacc 1440

ggtgcctggg gtaaaactgt aattgagtat aaaacgacaa agacctcccg tcttcctatc 1500

attgatgttg ctccattaga tgtcggtgcc cctgaccagg agtttggttt tgacgtaggc 1560

cccgtctgct tcctgtaa 1578

<210> 107
<211> 1284
<212> DNA
<213> Homo sapiens

<400> 107
atgctctctt tcgttgatac tagaactcta ttgctgttag cagttacttt gtgtttagca 60

acctgtcaat ccttgcaaga agaaaccgtt agaaaaggtc ctgcaggaga tagaggtcca 120

agaggcgaga gagggccccc gggaccccca gggcgagatg gtgaggatgg tccgacaggt 180

ccaccaggac ctcctggacc tcccggacct ccgggactcg gtggtaactt cgctgctcaa 240

tatgatggta aaggcgttgg tcttggaccg ggaccaatgg gtctgatggg tcctagagga 300

ccaccagggg cagcaggggc acctggtccc caaggattcc aaggacccgc aggggaaccc 360

ggggagccag gacagacagg acctgcaggg gctagaggac ccgccggtcc accaggtaag 420

gctggtgagg acggccaccc aggtaaaccc ggacctcctg gcccaccagg tcctccgggt 480

ccacctggag tttcaggcgg aggttacgac tttggatatg acggagattt ttatagagct 540

gatcaacctc gttcagcccc ctctttgaga ccaaaagact atgaagtgga cgctacattg 600

aagagtctca acaatcagat cgaaaccttg cttactccgg aaggttcaag aaaaaatcct 660

gctaggacct gcagagacct gagactttct catccagagt ggtcttccgg ctattattgg 720

attgatccta accaaggctg tacaatggat gctattaagg tctactgcga cttttccacc 780

ggtgagacat gtataagagc tcaacctgaa aacattcctg ctaaaaattg gtacagatca 840

agtaaagaca aaaagcatgt gtggttgggt gaaactatta atgccggctc tcagtttgaa 900

tacaatgtcg agggagtcac ttctaaagag atggctacgc aacttgcttt catgcgttta 960

ttagccaatt acgccagtca aaatatcacg taccactgca agaatagcat tgcttacatg 1020

gacgaggaga ctggtaacct taaaaaggcc gttattctgc aaggtagcaa tgacgtagag 1080

ctggtcgctg aaggtaactc acgattcact tatactgtcc tggttgacgg ttgttcaaaa 1140

aagaccaacg aatggggaaa aacgattatc gagtacaaga caaataagcc aagcaggctg 1200

ccttttttag acattgcacc actagacata ggcggggctg atcaggaatt tttcgtggac 1260

attggtcccg tctgttttaa gtaa 1284

<210> 108
<211> 3045
<212> DNA
<213> Homo sapiens

<400> 108
atgttttctt tcgtagattt gcgactcctc ttgttgcttg ctgctaccgc tctccttact 60

catggacagg aagaaggcca ggtggaagga caggacgaag acattcctcc tataacttgt 120

gtgcagaacg gactgaggta tcatgacaga gatgtgtgga agccagagcc ttgtcgaata 180

tgcgtttgtg acaacggaaa ggtgctatgt gatgatgtta tctgtgatga gacaaagaac 240

tgtcctggcg ctgaagttcc agagggagaa tgttgtccag tttgtcctga tggatcggag 300

tcacccactg atcaagagac tacgggagta gaaggtccaa aaggtgacac tggtcccaga 360

ggcccaagag gacctgccgg cccccctggt agggatggca ttcctggtca acccggccta 420

ccaggccccc caggtccacc tggcccccca ggaccccctg gtttaggagg taacttcgct 480

cctcaactgt cttacggtta cgatgaaaag tctactggtg gaatctctgt tcctggacca 540

atgggtccgt ctggtccaag gggtctacct ggtcctcccg gtgcacctgg tccgcagggt 600

tttcaaggtc caccaggtcc acagggtcca ggaggtcctc ctggacctaa aggaaatagt 660

ggagaaccag gagctccagg gagtaaagga gacactggtg ctaagggaga gccaggacca 720

gtgggggttc aaggtcctcc cggacccgct ggagaagagg gaaaaagagg tgcacgtggt 780

gagcctggtc ctactggtct gccaggtcca cctggggaaa ggggtggtcc tgggtcaaga 840

ggcttccccg gtgctgacgg agttgctggt ccgaaaggac cagccggaga gcgaggatcc 900

ccaggtcctg caggccctaa ggggtcccct ggtgaggcag gaagaccggg tgaggctggt 960

ttgcccggtg ccaaaggtct cacgggctcc ccaggctctc ctggcccaga cggcaaaact 1020

ggcccacctg gccctgccgg acaagacgga agacctggac ctccaggacc acctggtgcc 1080

agaggtcagg ccggtgtgat ggggtttcca ggtcccaagg gtgctgcagg tgaaccaggg 1140

aaagccggtg aacgaggtgt ccccggtcct ccaggtgctg ttgggccggc tggaaaggat 1200

ggtgaggccg gtgcacaagg gcctccaggc cctgctggtc ctgccggtga aagaggagaa 1260

cagggaccag ccggttctcc tggctttcaa ggtctgcccg gacctgccgg gcctccagga 1320

gaagctggta aaccaggaga acaaggtgtc ccaggagatt tgggagcccc tgggccctca 1380

ggcgctagag gtgagagggg atttcctgga gaacgtggtg tacaaggtcc cccgggtcca 1440

gcaggtccga ggggagctaa tggagctcca ggaaacgacg gtgctaaagg cgacgcaggt 1500

gctccaggag caccaggatc acagggtgcc ccaggtttgc aaggcatgcc aggggaaaga 1560

ggagcagcag gacttccagg ccccaaaggc gacaggggtg acgccggtcc aaaaggggcc 1620

gatggttctc ctggcaaaga cggcgttcga ggcttgacag gtccgatcgg tccacctgga 1680

cccgccggtg caccaggtga taagggagaa tctggtcctt caggccctgc cggtccaacc 1740

ggggctagag gcgctcctgg ggatagaggt gaacccggtc caccagggcc agccggcttc 1800

gctggaccac caggtgctga tggacagccc ggagctaagg gtgagccagg tgatgctggt 1860

gctaaaggtg acgctggacc tccgggtccc gcaggacctg ctggtccccc tgggccaatt 1920

ggtaatgttg gtgccccagg agcaaaagga gccagaggtt ctgcaggccc accaggagcc 1980

acaggattcc ccggtgctgc tggaagagtc ggaccaccgg gtccatccgg gaacgccgga 2040

cctccaggtc ctccgggtcc tgctggtaaa gaaggaggta agggtccaag aggtgaaaca 2100

gggcctgctg gccgtcctgg tgaagtcgga cctcctggcc cacccggtcc agctggggaa 2160

aagggatcac caggagctga tggaccacca gggccgcctg gtcctcctgg tccacctggt 2220

ccgcccagtg caggattcga cttttccttc ttaccccaac cgccacagga aaaggcacac 2280

gatggtggta gatattaccg tgctgatgat gccaatgtcg tgagagatag agacttagaa 2340

gttgatacga cattaaaatc actttctcaa caaattgaga acattagatc tccggagggt 2400

agcagaaaga acccggccag gacttgccgt gatcttaaga tgtgccactc cgattggaag 2460

tcgggagagt actggatcga tcctaatcaa ggttgtaact tggatgcaat aaaagtcttc 2520

tgtaacatgg agacaggaga aacttgcgtt tacccaactc aaccaagtgt tgcacagaaa 2580

aattggtata tttccaagaa tccaaaggat aagcgtcatg tttggtttgg agaatctatg 2640

accgatggct ttcaattcga gtatggaggt cagggtagcg acccagctga tgtcgctatt 2700

caattgacct ttttgagact tatgtccaca gaagcttcgc aaaacattac atatcactgt 2760

aagaatagcg tagcctacat ggaccaacag actggtaatc tgaaaaaggc cctgttgttg 2820

caaggcagta atgaaatcga aattcgtgcc gaaggaaatt ccagattcac atactctgtt 2880

accgttgacg gctgcacctc tcataccggt gcctggggta aaactgtaat tgagtataaa 2940

acgacaaaga cctcccgtct tcctatcatt gatgttgctc cattagatgt cggtgcccct 3000

gaccaggagt ttggttttga cgtaggcccc gtctgcttcc tgtaa 3045

<210> 109
<211> 2751
<212> DNA
<213> Homo sapiens

<400> 109
atgctctctt tcgttgatac tagaactcta ttgctgttag cagttacttt gtgtttagca 60

acctgtcaat ccttgcaaga agaaaccgtt agaaaaggtc ctgcaggaga tagaggtcca 120

agaggcgaga gagggccccc gggaccccca gggcgagatg gtgaggatgg tccgacaggt 180

ccaccaggac ctcctggacc tcccggacct ccgggactcg gtggtaactt cgctgctcaa 240

tatgatggta aaggcgttgg tcttggaccg ggaccaatgg gtctgatggg tcctagagga 300

ccaccagggg cagcaggggc acctggtccc caaggattcc aaggacccgc agggccagtt 360

ggcgcagctg gtgctacggg agctaggggt ctagtgggcg aaccaggtcc agcagggtct 420

aagggtgaat ccggtaacaa gggtgaacct ggaagtgccg gtcctcaggg acctcctggt 480

ccatcagggg aggaaggaaa gagaggccct aatggcgagg ctggtagtgc aggaccaccg 540

ggtcctcctg gattgagagg ttcccccggg tctcgtggac tacctggtgc cgacggtaga 600

gctggcgtta tgggtcctcc cggaagcaga ggagcttctg gacccgcagg tgtcaggggt 660

ccaaacggtg atgctggaag gccaggtgaa ccaggattga tgggtccacg tggattgccc 720

ggctctcctg gaaacattgg acctgccggt aaagaaggcc ctgtgggttt gccaggtatt 780

gatggcagac caggtccaat cggcccagca ggcgctagag gagagcctgg taacatagga 840

ttccctggtc ctaagggacc aactggtgat ccaggcaaga acggtgataa gggtcatgcc 900

gggctagctg gggctagagg tgcacctggc ccagatggga acaacggagc ccaaggccca 960

ccgggtccac agggggtaca aggtggcaag ggcgaacagg gtccagctgg cccgcctggt 1020

tttcagggac ttccgggtcc tagcggtccc gctggagaag ttggaaagcc tggtgaacgt 1080

ggactacatg gagaattcgg actgcctggt cctgctggtc cacgtggaga aagaggtcct 1140

cccggggaga gtggagctgc tggtcctacc ggaccaattg ggtcacgtgg accctccggt 1200

cctccaggac cagatgggaa taagggtgaa ccaggagtcg taggggcagt tggtacagct 1260

ggcccttccg gtccttcggg ccttccagga gaaagaggag ccgccggaat ccctggtggt 1320

aaaggcgaaa aaggagaacc tggtttaagg ggtgagattg gaaatccagg tagagatggt 1380

gcccgaggag ccccaggtgc cgtcggtgct cccgggccag ccggagccac tggcgatagg 1440

ggagaagccg gagctgctgg accagccggt ccagctggac caagaggatc cccgggtgag 1500

agaggagaag ttgggccagc cggtccgaac ggtttcgccg gaccagccgg tgcagccggt 1560

caaccaggtg ctaagggcga gagaggtgct aaaggcccta aaggtgagaa tggtgtcgtt 1620

ggccccactg gccctgttgg agcagctgga cccgctggtc caaacggacc acccggtcct 1680

gctggatcga ggggtgacgg tggtccacca ggtatgacgg gatttccagg tgcagctggt 1740

agaactggtc cccctggtcc gtccgggatt tcaggtccac ctgggcctcc aggacctgct 1800

ggaaaagagg gcttgagggg tccgagagga gatcaaggcc ctgtaggaag aaccggggaa 1860

gttggtgccg ttggaccccc gggttttgct ggggagaaag gaccatctgg agaagcagga 1920

cctcctggcc caccaggtcc tccgggtcca cctggagttt caggcggagg ttacgacttt 1980

ggatatgacg gagattttta tagagctgat caacctcgtt cagccccctc tttgagacca 2040

aaagactatg aagtggacgc tacattgaag agtctcaaca atcagatcga aaccttgctt 2100

actccggaag gttcaagaaa aaatcctgct aggacctgca gagacctgag actttctcat 2160

ccagagtggt cttccggcta ttattggatt gatcctaacc aaggctgtac aatggatgct 2220

attaaggtct actgcgactt ttccaccggt gagacatgta taagagctca acctgaaaac 2280

attcctgcta aaaattggta cagatcaagt aaagacaaaa agcatgtgtg gttgggtgaa 2340

actattaatg ccggctctca gtttgaatac aatgtcgagg gagtcacttc taaagagatg 2400

gctacgcaac ttgctttcat gcgtttatta gccaattacg ccagtcaaaa tatcacgtac 2460

cactgcaaga atagcattgc ttacatggac gaggagactg gtaaccttaa aaaggccgtt 2520

attctgcaag gtagcaatga cgtagagctg gtcgctgaag gtaactcacg attcacttat 2580

actgtcctgg ttgacggttg ttcaaaaaag accaacgaat ggggaaaaac gattatcgag 2640

tacaagacaa ataagccaag caggctgcct tttttagaca ttgcaccact agacataggc 2700

ggggctgatc aggaattttt cgtggacatt ggtcccgtct gttttaagta a 2751

<210> 110
<211> 4611
<212> DNA
<213> Homo sapiens

<400> 110
atgttttctt tcgtagattt gcgactcctc ttgttgcttg ctgctaccgc tctccttact 60

catggacagg aagaaggcca ggtggaagga caggacgaag acattcctcc tataacttgt 120

gtgcagaacg gactgaggta tcatgacaga gatgtgtgga agccagagcc ttgtcgaata 180

tgcgtttgtg acaacggaaa ggtgctatgt gatgatgtta tctgtgatga gacaaagaac 240

tgtcctggcg ctgaagttcc agagggagaa tgttgtccag tttgtcctga tggatcggag 300

tcacccactg atcaagagac tacgggagta gaaggtccaa aaggtgacac tggtcccaga 360

ggcccaagag gacctgccgg cccccctggt agggatggca ttcctggtca acccggccta 420

ccaggccccc caggtccacc tggcccccca ggaccccctg gtttaggagg taacttcgct 480

cctcaactgt cttacggtta cgatgaaaag tctactggtg gaatctctgt tcctggacca 540

atgggtccgt ctggtccaag gggtctacct ggtcctcccg gtgcacctgg tccgcagggt 600

tttcaaggtc caccaggtcc acagggtcca ggaggtcctc ctggacctaa aggaaatagt 660

ggagaaccag gagctccagg gagtaaagga gacactggtg ctaagggaga gccaggacca 720

gtgggggttc aaggtcctcc cggacccgct ggagaagagg gaaaaagagg tgcacgtggt 780

gagcctggtc ctactggtct gccaggtcca cctggggaaa ggggtggtcc tgggtcaaga 840

ggcttccccg gtgctgacgg agttgctggt ccgaaaggac cagccggaga gcgaggatcc 900

ccaggtcctg caggccctaa ggggtcccct ggtgaggcag gaagaccggg tgaggctggt 960

ttgcccggtg ccaaaggtct cacgggctcc ccaggctctc ctggcccaga cggcaaaact 1020

ggcccacctg gccctgccgg acaagacgga agacctggac ctccaggacc acctggtgcc 1080

agaggtcagg ccggtgtgat ggggtttcca ggtcccaagg gtgctgcagg tgaaccaggg 1140

aaagccggtg aacgaggtgt ccccggtcct ccaggtgctg ttgggccggc tggaaaggat 1200

ggtgaggccg gtgcacaagg gcctccaggc cctgctggtc ctgccggtga aagaggagaa 1260
cagggaccag ccggttctcc tggctttcaa ggtctgcccg gacctgccgg gcctccagga 1320
gaagctggta aaccaggaga acaaggtgtc ccaggagatt tgggagcccc tgggccctca 1380
ggcgctagag gtgagagggg atttcctgga gaacgtggtg tacaaggtcc cccgggtcca 1440
gcaggtccga ggggagctaa tggagctcca ggaaacgacg gtgctaaagg cgacgcaggt 1500
gctccaggag caccaggatc acagggtgcc ccaggtttgc aaggcatgcc aggggaaaga 1560
ggagcagcag gacttccagg ccccaaaggc gacaggggtg acgccggtcc aaaaggggcc 1620
gatggttctc ctggcaaaga cggcgttcga ggcttgacag gtccgatcgg tccacctgga 1680
cccgccggtg caccaggtga taagggagaa tctggtcctt caggccctgc cggtccaacc 1740
ggggctagag gcgctcctgg ggatagaggt gaacccggtc caccagggcc agccggcttc 1800
gctggaccac caggtgctga tggacagccc ggagctaagg gtgagccagg tgatgctggt 1860
gctaaaggtg acgctggacc tccgggtccc gcaggacctg ctggtccccc tgggccaatt 1920
ggtaatgttg gtgccccagg agcaaaagga gccagaggtt ctgcaggccc accaggagcc 1980
acaggattcc ccggtgctgc tggaagagtc ggaccaccgg gtccatccgg gaacgccgga 2040
cctccaggtc ctccgggtcc tgctggtaaa gaaggaggta agggtccaag aggtgaaaca 2100
gggcctgctg gccgtcctgg tgaagtcgga cctcctggcc cacccggtcc agctggggaa 2160
aagggatcac caggagctga tggaccccag ggccccggcg gccctcctgg tcccaagggt 2220
aacagcggtg aacctggtgc tcctggcagc aaaggagaca ctggtgctaa gggagagcct 2280
ggccctgttg gtgttcaagg accccctggc cctgctggag aggaaggaaa gcgaggagct 2340
cgaggtgaac ccggacccac tggcctgccc ggaccccctg gcgagcgtgg tggacctggt 2400
agccgtggtt tccctggcgc agatggtgtt gctggtccca agggtcccgc tggtgaacgt 2460
ggttctcctg gccctgctgg ccccaaagga tctcctggtg aagctggtcg tcccggtgaa 2520
gctggtctgc ctggtgccaa gggtctgact ggaagccctg gcagccctgg tcctgatggc 2580
aaaactggcc cccctggtcc cgccggtcaa gatggtcgcc ccggaccccc aggcccacct 2640
ggtgcccgtg gtcaggctgg tgtgatggga ttccctggac ctaaaggtgc tgctggagag 2700
cccggcaagg ctggagagcg aggtgttccc ggaccccctg gcgctgtcgg tcctgctggc 2760
aaagatggag aggctggagc tcaggacccc cctggccctg ctggtcccgc tggcgagaga 2820
ggtgaacaag gccctgctgg ctcccccgga ttccagggtc tccctggtcc tgctggtcct 2880
ccaggtgaag caggcaaacc tggtgaacag ggtgttcctg gagaccttgg cgcccctggc 2940
ccctctggag caagaggcga gagaggtttc cctggcgagc gtggtgtgca aggtccccct 3000
ggtcctgctg gtccccgagg ggccaacggt gctcccggca acgatggtgc taagggtgat 3060
gctggtgccc ctggagctcc cggtagccag ggcgcccctg gccttcaggg aatgcctggt 3120

gaacgtggtg cagctggtct tccagggcct aagggtgaca gaggtgatgc tggtcccaaa 3180

ggtgctgatg gctctcctgg caaagatggc gtccgtggtc tgaccggccc cattggtcct 3240

cctggccctg ctggtgcccc tggtgacaag ggtgaaagtg gtcccagcgg ccctgctggt 3300

cccactggag ctcgtggtgc ccccggagac cgtggtgagc ctggtccccc cggccctgct 3360

ggctttgctg gcccccctgg tgctgacggc caacctggtg ctaaaggcga acctggtgat 3420

gctggtgcta aaggcgatgc tggtccccct ggccctgccg gacccgctgg accccctggc 3480

cccattggta atgttggtgc tcctggagcc aaaggtgctc gcggcagcgc tggtccccct 3540

ggtgctactg gtttccctgg tgctgctggc cgagtcggtc ctcctggccc ctctggaaat 3600

gctggacccc ctggccctcc tggtcctgct ggcaaagaag gcggcaaagg tccccgtggt 3660

gagactggcc ctgctggacg tcctggtgaa gttggtcccc ctggtccccc tggccctgct 3720

ggcgagaaag gatcccctgg tgctgatgga ccaccagggc cgcctggtcc tcctggtcca 3780

cctggtccgc ccagtgcagg attcgacttt tccttcttac cccaaccgcc acaggaaaag 3840

gcacacgatg gtggtagata ttaccgtgct gatgatgcca atgtcgtgag agatagagac 3900

ttagaagttg atacgacatt aaaatcactt tctcaacaaa ttgagaacat tagatctccg 3960

gagggtagca gaaagaaccc ggccaggact tgccgtgatc ttaagatgtg ccactccgat 4020

tggaagtcgg gagagtactg gatcgatcct aatcaaggtt gtaacttgga tgcaataaaa 4080

gtcttctgta acatggagac aggagaaact tgcgtttacc caactcaacc aagtgttgca 4140

cagaaaaatt ggtatatttc caagaatcca aaggataagc gtcatgtttg gtttggagaa 4200

tctatgaccg atggctttca attcgagtat ggaggtcagg gtagcgaccc agctgatgtc 4260

gctattcaat tgaccttttt gagacttatg tccacagaag cttcgcaaaa cattacatat 4320

cactgtaaga atagcgtagc ctacatggac caacagactg gtaatctgaa aaaggccctg 4380

ttgttgcaag gcagtaatga aatcgaaatt cgtgccgaag gaaattccag attcacatac 4440

tctgttaccg ttgacggctg cacctctcat accggtgcct ggggtaaaac tgtaattgag 4500

tataaaacga caaagacctc ccgtcttcct atcattgatg ttgctccatt agatgtcggt 4560

gcccctgacc aggagtttgg ttttgacgta ggccccgtct gcttcctgta a 4611

<210> 111
<211> 4317
<212> DNA
<213> Homo sapiens

<400> 111
   atgctctctt tcgttgatac tagaactcta ttgctgttag cagttacttt gtgtttagca 60
   acctgtcaat ccttgcaaga agaaaccgtt agaaaaggtc ctgcaggaga tagaggtcca 120
   agaggcgaga gagggccccc gggaccccca gggcgagatg gtgaggatgg tccgacaggt 180
   ccaccaggac ctcctggacc tcccggacct ccgggactcg gtggtaactt cgctgctcaa 240
   tatgatggta aaggcgttgg tcttggaccg ggaccaatgg gtctgatggg tcctagagga 300
   ccaccagggg cagcaggggc acctggtccc caaggattcc aaggacccgc agggccagtt 360
   ggcgcagctg gtgctacggg agctaggggt ctagtgggcg aaccaggtcc agcagggtct 420
   aagggtgaat ccggtaacaa gggtgaacct ggaagtgccg gtcctcaggg acctcctggt 480
   ccatcagggg aggaaggaaa gagaggccct aatggcgagg ctggtagtgc aggaccaccg 540
   ggtcctcctg gattgagagg ttcccccggg tctcgtggac tacctggtgc cgacggtaga 600
   gctggcgtta tgggtcctcc cggaagcaga ggagcttctg gacccgcagg tgtcaggggt 660
   ccaaacggtg atgctggaag gccaggtgaa ccaggattga tgggtccacg tggattgccc 720
   ggctctcctg gaaacattgg acctgccggt aaagaaggcc ctgtgggttt gccaggtatt 780
   gatggcagac caggtccaat cggcccagca ggcgctagag gagagcctgg taacatagga 840
   ttccctggtc ctaagggacc aactggtgat ccaggcaaga acggtgataa gggtcatgcc 900
   gggctagctg ggctagaggt tgcacctggc ccagatggga acaacggagc ccaagggcca 960
   ccgggtccac agggggtaca aggtggcaag ggcgaacagg gtccagctgg cccgcctggt 1020
   tttcagggac ttccgggtcc tagcggtccc gctggagaag ttggaaagcc tggtgaacgt 1080
   ggactacatg gagaattcgg actgcctggt cctgctggtc cacgtggaga aagaggtcct 1140
   cccggggaga gtggagctgc tggtcctacc ggaccaattg ggtcacgtgg accctccggt 1200
   cctccaggac cagatgggaa taagggtgaa ccaggagtcg taggggcagt tggtacagct 1260
   ggcccttccg gtccttcggg ccttccagga gaaagaggag ccgccggaat ccctggtggt 1320
   aaaggcgaaa aaggagaacc tggtttaagg ggtgagattg gaaatccagg tagagatggt 1380
   gcccgaggag ccccaggtgc cgtcggtgct cccgggccag ccggagccac tggcgatagg 1440
   ggagaagccg gagctgctgg accagccggt ccagctggac caagaggatc cccgggtgag 1500
   agaggagaag ttgggccagc cggtccgaac ggtttcgccg gaccagccgg tgcagccggt 1560
   caaccaggtg ctaagggcga gagaggtgct aaaggcccta aaggtgagaa tggtgtcgtt 1620
   ggccccactg gccctgttgg agcagctgga cccgctggtc caaacggacc acccggtcct 1680
   gctggatcga ggggtgacgg tggtccacca ggtatgacgg gatttccagg tgcagctggt 1740
   agaactggtc cccctggtcc gtccgggatt tcaggtccac ctgggcctcc aggacctgct 1800
   ggaaaagagg gcttgagggg tccgagagga gatcaaggcc ctgtaggaag aaccggggaa 1860
   gttggtgccg ttggaccccc gggttttgct ggggagaaag gaccatctgg agaagcaggc 1920
   cctgttggtg ctgccggtgc tactggtgcc agaggacttg ttggtgagcc tggtccagct 1980
   ggctccaaag gagagagcgg taacaagggt gagcccggct ctgctgggcc ccaaggtcct 2040
   cctggtccca gtggtgaaga aggaaagaga ggccctaatg gggaagctgg atctgccggc 2100
   cctccaggac ctcctgggct gagaggtagt cctggttctc gtggtcttcc tggagctgat 2160
   ggcagagctg gcgtcatggg ccctcctggt agtcgtggtg caagtggccc tgctggagtc 2220
   cgaggaccta atggagatgc tggtcgccct ggggagcctg gtctcatggg acccagaggt 2280
   cttcctggtt cccctggaaa tatcggcccc gctggaaaag aaggtcctgt cggcctccct 2340
   ggcatcgacg gcaggcctgg cccaattggc ccagctggag caagaggaga gcctggcaac 2400
   attggattcc ctggacccaa aggccccact ggtgatcctg gcaaaaacgg tgataaaggt 2460
   catgctggtc ttgctggtgc tcggggtgct ccaggtcctg atggaaacaa tggtgctcag 2520
   ggacctcctg gaccacaggg tgttcaaggt ggaaaaggtg aacagggtcc cgctggtcct 2580
   ccaggcttcc agggtctgcc tggcccctca ggtcccgctg gtgaagttgg caaaccagga 2640
   gaaaggggtc tccatggtga gtttggtctc cctggtcctg ctggtccaag aggggaacgc 2700
   ggtcccccag gtgagagtgg tgctgccggt cctactggtc ctattggaag ccgaggtcct 2760
   tctggacccc cagggcctga tggaaacaag ggtgaacctg gtgttgttgg tgctgtgggc 2820
   actgctggtc catctggtcc tagtggactc ccaggagaga ggggtgctgc tggcatacct 2880
   ggaggcaagg gagaaaaggg tgaacctggt ctcagaggtg aaattggtaa ccctggcaga 2940
   gatggtgctc gtggtgctcc tggtgctgta ggtgcccctg gtcctgctgg agccacaggt 3000
   gaccggggcg aagctggggc tgctggtcct gctggtcctg ctggtcctcg gggaagccct 3060
   ggtgaacgtg gtgaggtcgg tcctgctggc cccaatggat ttgctggtcc tgctggtgct 3120
   gctggtcaac ctggtgctaa aggagaaaga ggagccaaag ggcctaaggg tgaaaacggt 3180
   gttgttggtc ccacaggccc cgttggagct gctggcccag ctggtccaaa tggtcccccc 3240
   ggtcctgctg gaagtcgtgg tgatggaggc ccccctggta tgactggttt ccctggtgct 3300
   gctggacgga ctggtccccc aggaccctct ggtatttctg gccctcctgg tccccctggt 3360
   cctgctggga aagaagggct tcgtggtcct cgtggtgacc aaggtccagt tggccgaact 3420
   ggagaagtag gtgcagttgg tccccctggc ttcgctggtg agaagggtcc ctctggagag 3480
   gctggacctc ctggcccacc aggtcctccg ggtccacctg gagtttcagg cggaggttac 3540
   gactttggat atgacggaga tttttataga gctgatcaac ctcgttcagc cccctctttg 3600
   agaccaaaag actatgaagt ggacgctaca ttgaagagtc tcaacaatca gatcgaaacc 3660
   ttgcttactc cggaaggttc aagaaaaaat cctgctagga cctgcagaga cctgagactt 3720
   tctcatccag agtggtcttc cggctattat tggattgatc ctaaccaagg ctgtacaatg 3780
   gatgctatta aggtctactg cgacttttcc accggtgaga catgtataag agctcaacct 3840
   gaaaacattc ctgctaaaaa ttggtacaga tcaagtaaag acaaaaagca tgtgtggttg 3900
   ggtgaaacta ttaatgccgg ctctcagttt gaatacaatg tcgagggagt cacttctaaa 3960
   gagatggcta cgcaacttgc tttcatgcgt ttattagcca attacgccag tcaaaatatc 4020
   acgtaccact gcaagaatag cattgcttac atggacgagg agactggtaa ccttaaaaag 4080
   gccgttattc tgcaaggtag caatgacgta gagctggtcg ctgaaggtaa ctcacgattc 4140
   acttatactg tcctggttga cggttgttca aaaaagacca acgaatgggg aaaaacgatt 4200
   atcgagtaca agacaaataa gccaagcagg ctgccttttt tagacattgc accactagac 4260
   ataggcgggg ctgatcagga atttttcgtg gacattggtc ccgtctgttt taagtaa 4317

<210> 112
<211> 429
<212> DNA
<213> Artificial Sequence

<220>
<223> synthesized DNA of human FKBP13A ORF having codon optimized for ye
ast

<400> 112
atgcgtcttt cctggtttcg tgttcttacc gttttatcta tttgcttgtc tgcagtagcc 60

actgctactg gagctgaagg taaacgaaaa ctgcaaattg gtgttaagaa gagagtcgat 120

cattgtccta ttaaatccag aaagggtgat gttcttcata tgcactacac gggaaaattg 180

gaagatggta cagaatttga cagctcattg ccccagaacc aaccatttgt tttctcattg 240

ggaaccggtc aggtgatcaa agggtgggac caaggcttgt taggaatgtg tgaaggtgaa 300

aagagaaagt tagtgattcc aagtgaacta ggttatggag agaggggcgc cccaccgaaa 360

atccctggtg gggctacatt ggtcttcgag gtcgagctgc tcaagataga gagaagaact 420

gagctgtaa 429

<210> 113
<211> 1713
<212> DNA
<213> Artificial Sequence

<220>
<223> synthesized DNA of human FKBP63 ORF having codon optimized for yea
st

<400> 113
atggctttta gaggatggag acctccacct cctcctttac ttttgttgtt gctttgggta 60

accggacagg ccgcacccgt ggctggtttg ggctctgacg ccgagcttca aattgaaaga 120

aggtttgtgc ccgacgagtg cccaagaacc gtaagaagtg gagatttcgt ccgttaccat 180

tacgtcggta cttttccaga cggacaaaag ttcgattcct cctatgatcg agattctacc 240

tttaatgttt tcgttggaaa ggggcagtta attacaggaa tggatcaggc cttagtcggt 300

atgtgtgtta atgaacgaag atttgtcaaa attccaccga agcttgctta tggtaacgaa 360

ggtgtttctg gtgtcatacc acctaatagc gtgttgcact ttgatgtcct cttgatggat 420

atttggaata gcgaagatca ggtgcaaatc catacttatt tcaagcctcc cagttgtcca 480

cgtacaattc aagtgtccga ctttgtccgt taccactaca atggtacttt tttggatggt 540

actctgttcg acagttcgca caatcgtatg aaaacttatg atacttatgt cggtattggt 600

tggttgatcc caggcatgga taagggatta ctaggaatgt gtgtcggtga aaagagaatt 660

atcactattc ccccattcct ggcctacggt gaagatggtg atggaaagga tataccaggt 720

caggcttcac tcgttttcga tgttgctttg ttggatcttc acaacccaaa agattccatt 780

agtatcgaga ataaggttgt accagagaat tgtgagagga tatctcaatc cggagacttc 840

ttaagatacc actacaacgg gactttgtta gatgggaccc tgttcgactc gtcgtattca 900

agaaacagaa cattcgacac atatatcggc caaggatacg ttattccggg aatggacgaa 960

ggattattgg gcgtatgtat aggcgagaag cgaaggatcg tagttcctcc acatttgggt 1020

tatggtgagg aagggcgtgg taatattcca gggtcagctg ttttggtttt tgacattcat 1080

gttattgact ttcataaccc atcagactcc atttctatca catcacacta taagccccct 1140

gactgctctg tgctttctaa aaagggtgac tatctaaagt accattacaa cgcatctctt 1200

ttggatggta cgttgctaga ctctacgtgg aacttgggta aaacatacaa catcgttctt 1260

ggtagcggtc aggtcgttct gggtatggac atgggactac gagagatgtg cgtgggtgag 1320

aagaggaccg ttataatccc tccacatctg ggatacggag aagcaggcgt ggatggggaa 1380

gttcctggtt cagctgttct tgtgtttgat atagaactgc tggagttggt agctggcctg 1440

cctgagggat atatgtttat ttggaacggc gaagtatccc cgaatctatt cgaggaaatc 1500

gataaagacg gtaacggaga agttctctta gaggaattct ctgaatacat ccatgcacaa 1560

gttgccagtg gtaaaggcaa actagctcct ggatttgatg ctgaactcat tgttaagaat 1620

atgtttacca accaagatag aaacggtgat ggaaaagtca ctgccgaaga gtttaaactg 1680

aaagatcaag aagcaaaaca cgatgagttg taa 1713

<210> 114
<211> 1749
<212> DNA
<213> Artificial Sequence

<220>
<223> synthesized DNA of human FKBP65 ORF having codon optimized for yea
 st

<400> 114
atgttccctg ctggtcctcc ttctcattcg ttacttcgtc tgccgttact tcagttactg 60

cttctggttg tccaggccgt cggtagaggt ctaggtagag cctccccagc aggtggacct 120

cttgaggatg tcgttattga gagataccat ataccacgtg cttgtccgag agaagtgcaa 180

atgggcgact tcgtccgata tcattacaac ggcacttttg aggacggaaa gaaattcgat 240

agtagctacg atagaaatac actggttgct atcgttgtag gggttggacg actcatcacc 300

ggaatggata ggggtttgat gggaatgtgt gtgaacgaga ggcgtcgttt aatagtccct 360

ccccacctgg ggtatggatc tatcggtttg gctggattga ttccacctga tgccactctc 420

tacttcgatg tcgttttact ggatgtatgg aacaaggagg atactgttca agtttccacg 480

ttgttgaggc caccgcactg ccctaggatg gttcaggatg gtgatttcgt tcgttaccac 540

tataatggta ctctcttaga cggaaccttt ttcgatacct cttacagcaa aggcggaact 600

tacgatacat atgtgggttc tggttggtta atcaagggaa tggatcaggg gttgttgggt 660

atgtgtccag gcgagagaag gaaaattatt attcctccat ttcttgcata cggtgaaaag 720

ggatacggta ctgttattcc tccccaagca tcactggtgt tccacgttct cctgattgat 780

gtccataacc caaaggatgc cgtacaactt gagaccctag agttgcctcc aggttgcgtt 840

agaagagccg gtgcaggtga ttttatgaga taccattata acggctctct catggacggt 900

actttgttcg actcttcata ttcaagaaac cacacttata atacatacat cggccagggc 960

tatatcattc cagggatgga tcaaggacta cagggtgcat gtatgggtga acgaagaaga 1020

attactatac cgccacattt ggcttatggt gagaatggta cgggtgacaa gattcctgga 1080

tcggctgtgc tgatttttaa cgtgcatgtg atcgacttcc acaatccagc agatgttgta 1140

gaaattagaa cattgagtag accctcagaa acatgcaatg aaaccactaa attgggtgac 1200

tttgtcagat accattacaa ttgtagttta ttggacggga ctcaattgtt cacgtcccac 1260

gactatggtg ctccacaaga ggctacatta ggggctaaca aagttattga aggtcttgac 1320

actggattgc agggaatgtg tgtaggtgag agaagacaat tgatcgtccc ccctcatttg 1380

gcccacggcg aaagtggagc tcgtggtgtt cccggatctg ctgtattgct atttgaagtc 1440

gaacttgttt ccagagaaga cggattgcca accggatatc tgtttgtttg gcataaggat 1500

ccacctgcta acttgtttga agatatggac ctaaacaagg atggcgaggt gccacctgaa 1560

gaattttcca cctttattaa ggctcaagtg tcagagggaa aaggaaggct tatgcccggt 1620

caagatccag aaaagaccat cggggatatg tttcaaaatc aggaccgaaa tcaagatggt 1680

aaaataacag ttgacgaact taaactaaaa tctgacgagg atgaagaaag agtacacgaa 1740

gagctataa 1749

<210> 115
<211> 1185
<212> DNA
<213> Artificial Sequence

<220>
<223> synthesized DNA of Zeocin(TM)-resistant cassette

<400> 115
cggccgccca cacaccatag cttcaaaatg tttctactcc ttttttactc ttccagattt 60

tctcggactc cgcgcatcgc cgtaccactt caaaacaccc aagcacagca tactaaattt 120

tccctctttc ttcctctagg gtgtcgttaa ttacccgtac taaaggtttg gaaaagaaaa 180

aagagaccgc ctcgtttctt tttcttcgtc gaaaaaggca ataaaaattt ttatcacgtt 240

tctttttctt gaaatttttt tttttagttt ttttctcttt cagtgacctc cattgatatt 300

taagttaata aacggtcttc aatttctcaa gtttcagttt catttttctt gttctattac 360

aacttttttt acttcttgtt cattagaaag aaagcatagc aatctaatct aaggggcggt 420

gttgacaatt aatcatcggc atagtatatc ggcatagtat aatacgacaa ggtgaggaac 480

taaaccatgg ccaagttgac cagtgccgtt ccggtgctca ccgcgcgcga cgtcgccgga 540

gcggtcgagt tctggaccga ccggctcggg ttctcccggg acttcgtgga ggacgacttc 600

gccggtgtgg tccgggacga cgtgaccctg ttcatcagcg cggtccagga ccaggtggtg 660

ccggacaaca ccctggcctg ggtgtgggtg cgcggcctgg acgagctgta cgccgagtgg 720

tcggaggtcg tgtccacgaa cttccgggac gcctccgggc cggccatgac cgagatcggc 780

gagcagccgt gggggcggga gttcgccctg cgcgacccgg ccggcaactg cgtgcacttc 840

gtggccgagg agcaggactg acacgtccga cggcggccca cgggtcccag gcctcggaga 900

tccgtccccc ttttcctttg tcgatatcat gtaattagtt atgtcacgct tacattcacg 960

ccctcccccc acatccgctc taaccgaaaa ggaaggagtt agacaacctg aagtctaggt 1020

ccctatttat ttttttatag ttatgttagt attaagaacg ttatttatat ttcaaatttt 1080

tctttttttt ctgtacagac gcgtgtacgc atgtaacatt atactgaaaa ccttgcttga 1140

gaaggttttg ggacgctcga aggctttaat ttgcaagctc ggccg 1185

<210> 116
<211> 727
<212> PRT
<213> Homo sapiens

<400> 116

Met Arg Pro Leu Leu Leu Leu Ala Leu Leu Gly Trp Leu Leu Leu Ala
1               5                   10                  15

Glu Ala Lys Gly Asp Ala Lys Pro Glu Asp Asn Leu Leu Val Leu Thr
            20                  25                  30

Val Ala Thr Lys Glu Thr Glu Gly Phe Arg Arg Phe Lys Arg Ser Ala
        35                  40                  45

Gln Phe Phe Asn Tyr Lys Ile Gln Ala Leu Gly Leu Gly Glu Asp Trp
    50                  55                  60

Asn Val Glu Lys Gly Thr Ser Ala Gly Gly Gly Gln Lys Val Arg Leu
65                  70                  75                  80

Leu Lys Lys Ala Leu Glu Lys His Ala Asp Lys Glu Asp Leu Val Ile
                85                  90                  95

Leu Phe Thr Asp Ser Tyr Asp Val Leu Phe Ala Ser Gly Pro Arg Glu
            100                 105                 110

Leu Leu Lys Lys Phe Arg Gln Ser Arg Ser Gln Val Val Phe Ser Ala

115                 120                 125

Glu Glu Leu Ile Tyr Pro Asp Arg Arg Leu Glu Thr Lys Tyr Pro Val
    130                 135                 140

Val Ser Asp Gly Lys Arg Phe Leu Gly Ser Gly Gly Phe Ile Gly Tyr
145                 150                 155                 160

Ala Pro Asn Leu Ser Lys Leu Val Ala Glu Trp Glu Gly Gln Asp Ser
                165                 170                 175

Asp Ser Asp Gln Leu Phe Tyr Thr Lys Ile Phe Leu Asp Pro Glu Lys
            180                 185                 190

Arg Glu Gln Ile Asn Ile Thr Leu Asp His Arg Cys Arg Ile Phe Gln
        195                 200                 205

Asn Leu Asp Gly Ala Leu Asp Glu Val Val Leu Lys Phe Glu Met Gly
    210                 215                 220

His Val Arg Ala Arg Asn Leu Ala Tyr Asp Thr Leu Pro Val Leu Ile
225                 230                 235                 240

His Gly Asn Gly Pro Thr Lys Leu Gln Leu Asn Tyr Leu Gly Asn Tyr
                245                 250                 255

Ile Pro Arg Phe Trp Thr Phe Glu Thr Gly Cys Thr Val Cys Asp Glu
            260                 265                 270

Gly Leu Arg Ser Leu Lys Gly Ile Gly Asp Glu Ala Leu Pro Thr Val
        275                 280                 285

Leu Val Gly Val Phe Ile Glu Gln Pro Thr Pro Phe Val Ser Leu Phe
    290                 295                 300

Phe Gln Arg Leu Leu Arg Leu His Tyr Pro Gln Lys His Met Arg Leu
305                 310                 315                 320

Phe Ile His Asn His Glu Gln His His Lys Ala Gln Val Glu Glu Phe
                325                 330                 335

Leu Ala Gln His Gly Ser Glu Tyr Gln Ser Val Lys Leu Val Gly Pro
            340                 345                 350

Glu Val Arg Met Ala Asn Ala Asp Ala Arg Asn Met Gly Ala Asp Leu
        355                 360                 365

Cys Arg Gln Asp Arg Ser Cys Thr Tyr Tyr Phe Ser Val Asp Ala Asp
    370                 375                 380

Val Ala Leu Thr Glu Pro Asn Ser Leu Arg Leu Leu Ile Gln Gln Asn
385                 390                 395                 400

Lys Asn Val Ile Ala Pro Leu Met Thr Arg His Gly Arg Leu Trp Ser
                405                 410                 415

Asn Phe Trp Gly Ala Leu Ser Ala Asp Gly Tyr Tyr Ala Arg Ser Glu
            420                 425                 430

Asp Tyr Val Asp Ile Val Gln Gly Arg Arg Val Gly Val Trp Asn Val
        435                 440                 445

Pro Tyr Ile Ser Asn Ile Tyr Leu Ile Lys Gly Ser Ala Leu Arg Gly
    450                 455                 460

Glu Leu Gln Ser Ser Asp Leu Phe His His Ser Lys Leu Asp Pro Asp
465                 470                 475                 480

Met Ala Phe Cys Ala Asn Ile Arg Gln Gln Asp Val Phe Met Phe Leu
                485                 490                 495

Thr Asn Arg His Thr Leu Gly His Leu Leu Ser Leu Asp Ser Tyr Arg
            500                 505                 510

Thr Thr His Leu His Asn Asp Leu Trp Glu Val Phe Ser Asn Pro Glu
        515                 520                 525

Asp Trp Lys Glu Lys Tyr Ile His Gln Asn Tyr Thr Lys Ala Leu Ala
    530                 535                 540

Gly Lys Leu Val Glu Thr Pro Cys Pro Asp Val Tyr Trp Phe Pro Ile
545                 550                 555                 560

Phe Thr Glu Val Ala Cys Asp Glu Leu Val Glu Glu Met Glu His Phe
                565                 570                 575

Gly Gln Trp Ser Leu Gly Asn Asn Lys Asp Asn Arg Ile Gln Gly Gly
            580                 585                 590

Tyr Glu Asn Val Pro Thr Ile Asp Ile His Met Asn Gln Ile Gly Phe
        595                 600                 605

Glu Arg Glu Trp His Lys Phe Leu Leu Glu Tyr Ile Ala Pro Met Thr
    610                 615                 620

Glu Lys Leu Tyr Pro Gly Tyr Tyr Thr Arg Ala Gln Phe Asp Leu Ala
625                 630                 635                 640

Phe Val Val Arg Tyr Lys Pro Asp Glu Gln Pro Ser Leu Met Pro His
                645                 650                 655

His Asp Ala Ser Thr Phe Thr Ile Asn Ile Ala Leu Asn Arg Val Gly
            660                 665                 670

Val Asp Tyr Glu Gly Gly Gly Cys Arg Phe Leu Arg Tyr Asn Cys Ser
        675                 680                 685

Ile Arg Ala Pro Arg Lys Gly Trp Thr Leu Met His Pro Gly Arg Leu
    690                 695                 700

Thr His Tyr His Glu Gly Leu Pro Thr Thr Arg Gly Thr Arg Tyr Ile
705                 710                 715                 720

Ala Val Ser Phe Val Asp Pro
                725

<210>  117
<211>  2184
<212>  DNA
<213>  Homo sapiens

<400>  117
atgcggcccc tgctgctact ggccctgctg ggctggctgc tgctggccga agcgaagggc      60

gacgccaagc cggaggacaa ccttttagtc ctcacggtgg ccactaagga gaccgaggga     120

ttccgtcgct tcaagcgctc agctcagttc ttcaactaca agatccaggc gcttggccta     180

ggggaggact ggaatgtgga gaaggggacg tcggcaggtg gagggcagaa ggtccggctg     240

ctgaagaaag ctctggagaa gcacgcagac aaggaggatc tggtcattct cttcacagac     300

agctatgacg tgctgtttgc atcgggcccc cgggagctcc tgaagaagtt ccggcagtcc     360

aggagccagg tggtcttctc tgctgaggag ctcatctacc cagaccgcag gctggagacc     420

aagtatccgg tggtgtccga tggcaagagg ttcctgggct ctggaggctt catcggttat     480

gcccccaacc tcagcaaact ggtggccgag tgggagggcc aggacagcga cagcgatcag     540

ctgttttaca ccaagatctt cttggacccg gagaagaggg agcagatcaa tatcaccctg     600

gaccaccgct gccgtatctt ccagaacctg gatggagcct tggatgaggt cgtgctcaag     660

tttgaaatgg gccatgtgag agcgaggaac ctggcctatg acaccctccc ggtcctgatc     720

catggcaacg ggccaaccaa gctgcagttg aactacctgg gcaactacat cccgcgcttc     780

tggaccttcg aaacaggctg caccgtgtgt gacgaaggct tgcgcagcct caagggcatt     840

ggggatgaag ctctgcccac ggtcctggtc ggcgtgttca tcgaacagcc cacgccgttt     900

gtgtccctgt tcttccagcg gctcctgcgg ctccactacc cccagaaaca catgcgactt     960

ttcatccaca accacgagca gcaccacaag gctcaggtgg aagagttcct ggcacagcat    1020

ggcagcgagt accagtctgt gaagctggtg ggccctgagg tgcggatggc gaatgcagat 1080

gccaggaaca tgggcgcaga cctgtgccgg caggaccgca gctgcaccta ctacttcagc 1140

gtggatgctg acgtggccct gaccgagccc aacagcctgc ggctgctgat ccaacagaac 1200

aagaacgtca ttgccccgct gatgacccgg catgggaggc tgtggtcgaa cttctggggg 1260

gctctcagtg cagatggcta ctatgcccgt tccgaggact acgtggacat tgtgcagggg 1320

cggcgtgttg gtgtctggaa tgtgccctat atttcaaaca tctacttgat caagggcagt 1380

gccctgcggg gtgagctgca gtcctcagat ctcttccacc acagcaagct ggaccccgac 1440

atggccttct gtgccaacat ccggcagcag gatgtgttca tgttcctgac caaccggcac 1500

acccttggcc atctgctctc cctagacagc taccgcacca cccacctgca caacgacctc 1560

tgggaggtgt tcagcaaccc cgaggactgg aaggagaagt acatccacca gaactacacc 1620

aaagccctgg ccgggaagct ggtggagacg ccctgcccgg atgtctattg gttccccatc 1680

ttcacggagg tggcctgtga tgagctggtg gaggagatgg agcactttgg ccagtggtct 1740

ctgggcaaca acaaggacaa ccgcatccag ggtggctacg agaacgtgcc gactattgac 1800

atccacatga accagatcgg ctttgagcgg gagtggcaca aattcctgct ggagtacatt 1860

gcgcccatga cggagaagct ctaccccggc tactacacca gggcccagtt tgacctggcc 1920

tttgtcgtcc gctacaagcc tgatgagcag ccctcactga tgccacacca tgatgcctcc 1980

accttcacca tcaacatcgc cctgaaccga gtcggggtgg attacgaggg cgggggctgt 2040

cggttcctgc gctacaactg ttccatccga gccccaagga agggctggac cctcatgcac 2100

cctggacgac tcacgcatta ccatgagggg ctccccacca ccaggggcac ccgctacatc 2160

gcagtctcct tcgtcgatcc ctaa 2184

<210> 118
<211> 738
<212> PRT
<213> Homo sapiens

<400> 118

<210>  119
<211>  2217
<212>  DNA
<213>  Homo sapiens

<400>  119
atgacctcct cggggcctgg accccggttc ctgctgctgc tgccgctgct gctgccccct      60

gcggcctcag cctccgaccg gccccggggc cgagacccgg tcaacccaga gaagctgctg     120

gtgatcactg tggccacagc tgaaaccgag gggtacctgc gtttcctgcg ctctgcggag     180

ttcttcaact acactgtgcg gaccctgggc ctgggagagg agtggcgagg gggtgatgtg     240

gctcgaacag ttggtggagg acagaaggtc cggtggttaa agaaggaaat ggagaaatac     300

gctgaccggg aggatatgat catcatgttt gtggatagct acgacgtgat tctggccggc     360

agccccacag agctgctgaa gaagttcgtc cagagtggca gccgcctgct cttctctgca     420

gagagcttct gctggcccga gtgggggctg gcggagcagt accctgaggt gggcacgggg     480

aagcgcttcc tcaattctgg tggattcatc ggttttgcca ccaccatcca ccaaatcgtg     540

cgccagtgga agtacaagga tgatgacgac gaccagctgt tctacacacg gctctacctg 600

gacccaggac tgagggagaa actcagcctt aatctggatc ataagtctcg gatctttcag 660

aacctcaacg gggctttaga tgaagtggtt ttaaagtttg atcggaaccg tgtgcgtatc 720

cggaacgtgg cctacgacac gctccccatt gtggtccatg gaaacggtcc cactaagctg 780

cagctcaact acctgggaaa ctacgtcccc aatggctgga ctcctgaggg aggctgtggc 840

ttctgcaacc aggaccggag gacactcccg ggggggcagc ctcccccccg ggtgtttctg 900

gccgtgtttg tggaacagcc tactccgttt ctgccccgct tcctgcagcg gctgctactc 960

ctggactatc cccccgacag ggtcaccctt ttcctgcaca acaacgaggt cttccatgaa 1020

ccccacatcg ctgactcctg gccgcagctc caggaccact tctcagctgt gaagctcgtg 1080

gggccggagg aggctctgag cccaggcgag gccagggaca tggccatgga cctgtgtcgg 1140

caggaccccg agtgtgagtt ctacttcagc ctggacgccg acgctgtcct caccaacctg 1200

cagaccctgc gtatcctcat tgaggagaac aggaaggtga tcgcccccat gctgtcccgc 1260

cacggcaagc tgtggtccaa cttctggggc gccctgagcc ccgatgagta ctacgcccgc 1320

tccgaggact acgtggagct ggtgcagcgg aagcgagtgg gtgtgtggaa tgtaccatac 1380

atctcccagg cctatgtgat ccggggtgat accctgcgga tggagctgcc ccagagggat 1440

gtgttctcgg gcagtgacac agacccggac atggccttct gtaagagctt tcgagacaag 1500

ggcatcttcc tccatctgag caatcagcat gaatttggcc ggctcctggc cacttccaga 1560

tacgacacgg agcacctgca ccccgacctc tggcagatct tcgacaaccc cgtcgactgg 1620

aaggagcagt acatccacga gaactacagc cgggccctgg aaggggaagg aatcgtggag 1680

cagccatgcc cggacgtgta ctggttccca ctgctgtcag aacaaatgtg tgatgagctg 1740

gtggcagaga tggagcacta cggccagtgg tcaggcggcc ggcatgagga ttcaaggctg 1800

gctggaggct acgagaatgt gcccaccgtg gacatccaca tgaagcaggt ggggtacgag 1860

gaccagtggc tgcagctgct gcggacgtat gtgggcccca tgaccgagag cctgtttccc 1920

ggttaccaca ccaaggcgcg ggcggtgatg aactttgtgg ttcgctaccg gccagacgag 1980

cagccgtctc tgcggccaca ccacgactca tccaccttca ccctcaacgt tgccctcaac 2040

cacaagggcc tggactatga gggaggtggc tgccgcttcc tgcgctacga ctgtgtgatc 2100

tcctccccga ggaagggctg ggcactcctg caccccggcc gcctcaccca ctaccacgag 2160

gggctgccaa cgacctgggg cacacgctac atcatggtgt cctttgtcga cccctga 2217

<210> 120
<211> 418
<212> PRT
<213> Homo sapiens

<400> 120
   Met Arg Ser Leu Leu Leu Leu Ser Ala Phe Cys Leu Leu Glu Ala Ala
   1               5                   10                  15
   Leu Ala Ala Glu Val Lys Lys Pro Ala Ala Ala Ala Ala Pro Gly Thr
               20                  25                  30
   Ala Glu Lys Leu Ser Pro Lys Ala Ala Thr Leu Ala Glu Arg Ser Ala
           35                  40                  45
   Gly Leu Ala Phe Ser Leu Tyr Gln Ala Met Ala Lys Asp Gln Ala Val
       50                  55                  60
   Glu Asn Ile Leu Val Ser Pro Val Val Val Ala Ser Ser Leu Gly Leu
   65                  70                  75                  80
   Val Ser Leu Gly Gly Lys Ala Thr Thr Ala Ser Gln Ala Lys Ala Val
                   85                  90                  95
   Leu Ser Ala Glu Gln Leu Arg Asp Glu Glu Val His Ala Gly Leu Gly
               100                 105                 110
   Glu Leu Leu Arg Ser Leu Ser Asn Ser Thr Ala Arg Asn Val Thr Trp
           115                 120                 125
   Lys Leu Gly Ser Arg Leu Tyr Gly Pro Ser Ser Val Ser Phe Ala Asp
       130                 135                 140
   Asp Phe Val Arg Ser Ser Lys Gln His Tyr Asn Cys Glu His Ser Lys
   145                 150                 155                 160
   Ile Asn Phe Arg Asp Lys Arg Ser Ala Leu Gln Ser Ile Asn Glu Trp
                   165                 170                 175
   Ala Ala Gln Thr Thr Asp Gly Lys Leu Pro Glu Val Thr Lys Asp Val
               180                 185                 190
   Glu Arg Thr Asp Gly Ala Leu Leu Val Asn Ala Met Phe Phe Lys Pro
           195                 200                 205
   His Trp Asp Glu Lys Phe His His Lys Met Val Asp Asn Arg Gly Phe
       210                 215                 220
   Met Val Thr Arg Ser Tyr Thr Val Gly Val Met Met Met His Arg Thr
   225                 230                 235                 240
   Gly Leu Tyr Asn Tyr Tyr Asp Asp Glu Lys Glu Lys Leu Gln Ile Val
                   245                 250                 255
   Glu Met Pro Leu Ala His Lys Leu Ser Ser Leu Ile Ile Leu Met Pro
   260 265 270
   His His Val Glu Pro Leu Glu Arg Leu Glu Lys Leu Leu Thr Lys Glu
   275 280 285
   Gln Leu Lys Ile Trp Met Gly Lys Met Gln Lys Lys Ala Val Ala Ile
   290 295 300
   Ser Leu Pro Lys Gly Val Val Glu Val Thr His Asp Leu Gln Lys His
   305 310 315 320
   Leu Ala Gly Leu Gly Leu Thr Glu Ala Ile Asp Lys Asn Lys Ala Asp
   325 330 335
   Leu Ser Arg Met Ser Gly Lys Lys Asp Leu Tyr Leu Ala Ser Val Phe
   340 345 350
   His Ala Thr Ala Phe Glu Leu Asp Thr Asp Gly Asn Pro Phe Asp Gln
   355 360 365
   Asp Ile Tyr Gly Arg Glu Glu Leu Arg Ser Pro Lys Leu Phe Tyr Ala
   370 375 380
   Asp His Pro Phe Ile Phe Leu Val Arg Asp Thr Gln Ser Gly Ser Leu
   385 390 395 400
   Leu Phe Ile Gly Arg Leu Val Arg Pro Lys Gly Asp Lys Met Arg Asp
   405 410 415
   Glu Leu

<210>  121
<211>  1257
<212>  DNA
<213>  Homo sapiens

<400>  121
atgcgctccc tcctgcttct cagcgccttc tgcctcctgg aggcggccct ggccgccgag      60

gtgaagaaac ctgcagccgc agcagctcct ggcactgcgg agaagttgag ccccaaggcg     120

gccacgcttg ccgagcgcag cgccggcctg gccttcagct tgtaccaggc catggccaag     180

gaccaggcag tggagaacat cctggtgtca cccgtggtgg tggcctcgtc gctggggctc     240

gtgtcgctgg gcggcaaggc gaccacggcg tcgcaggcca aggcagtgct gagcgccgag     300

cagctgcgcg acgaggaggt gcacgccggc ctgggcgagc tgctgcgctc actcagcaac     360

tccacggcgc gcaacgtgac ctggaagctg ggcagccgac tgtacggacc cagctcagtg     420

agcttcgctg atgacttcgt gcgcagcagc aagcagcact acaactgcga gcactccaag     480

atcaacttcc gcgacaagcg cagcgcgctg cagtccatca acgagtgggc cgcgcagacc 540

accgacggca agctgcccga ggtcaccaag gacgtggagc gcacggacgg cgccctgtta 600

gtcaacgcca tgttcttcaa gccacactgg gatgagaaat tccaccacaa gatggtggac 660

aaccgtggct tcatggtgac tcggtcctat accgtgggtg tcatgatgat gcaccggaca 720

ggcctctaca actactacga cgacgagaag gaaaagctgc aaatcgtgga gatgcccctg 780

gcccacaagc tctccagcct catcatcctc atgccccatc acgtggagcc tctcgagcgc 840

cttgaaaagc tgctaaccaa agagcagctg aagatctgga tggggaagat gcagaagaag 900

gctgttgcca tctccttgcc caagggtgtg gtggaggtga cccatgacct gcagaaacac 960

ctggctgggc tgggcctgac tgaggccatt gacaagaaca aggccgactt gtcacgcatg 1020

tcaggcaaga aggacctgta cctggccagc gtgttccacg ccaccgcctt tgagttggac 1080

acagatggca acccctttga ccaggacatc tacgggcgcg aggagctgcg cagccccaag 1140

ctgttctacg ccgaccaccc cttcatcttc ctagtgcggg acacccaaag cggctccctg 1200

ctattcattg ggcgcctggt ccggcctaag ggtgacaaga tgcgagacga gttatag 1257